# EUROPEAN PATENT SPECIFICATION

(11) **EP 1 373 477 B1**
(45) Date of publication and mention of the grant of the patent: **20.07.2011**
(21) Application number: 02753754.7
(22) Date of filing: 07.02.2002
(51) Int. Cl.: C12N 7/00, A61K 39/155, C07K 14/115, C12N 15/45, C12N 15/85, C07K 16/10, A61K 39/42, C12N 7/04, G01N 33/569, C12Q 1/68, C12Q 1/70, G01N 33/50, A01K 67/027

(54) **A NOVEL VIRUS (CRYPTOVIRUS) WITHIN THE RUBULAVIRUS GENUS AND USES THEREFOR**
VIRUS (CRYPTOVIRUS) INNERHALB DER GATTUNG RUBULAVIRUS UND VERWENDUNGEN DAFÜR
NOUVEAU VIRUS (CRYPTOVIRUS) FAISANT PARTIE DU GENRE RUBULAVIRUS ET UTILISATIONS

(30) Priority: 07.02.2001 US 267253 P
(43) Date of publication of application: 02.01.2004
(73) Proprietor: Cryptic Afflictions, LLC, Baldwin City, KS 66006 (US)
(72) Inventor: ROBBINS, Steven, J., Baldwin City, KS 66006 (US)
(74) Representative: HOFFMANN EITLE
(86) International application number: PCT/US2002/004117
(87) International publication number: WO 2002/077211

(56) References cited:
- DATABASE EMBL [Online] 23 March 1998 (1998-03-23) retrieved from EBI Database accession no. AF052755 XP002246954

## Description

Throughout the application various publications are referenced in parentheses. The disclosures of these publications in their entireties are hereby incorporated by reference in the application in order to more fully describe the state of the art to which this invention pertains.

### BACKGROUND OF THE INVENTION

### 1. Field of the Invention

This invention relates to the medical arts and particularly to the field of virology, and, more particularly, to a novel human *Rubulavirus* which has been designated as a "*Cryptovirus*".

### 2. Discussion of the Related Art

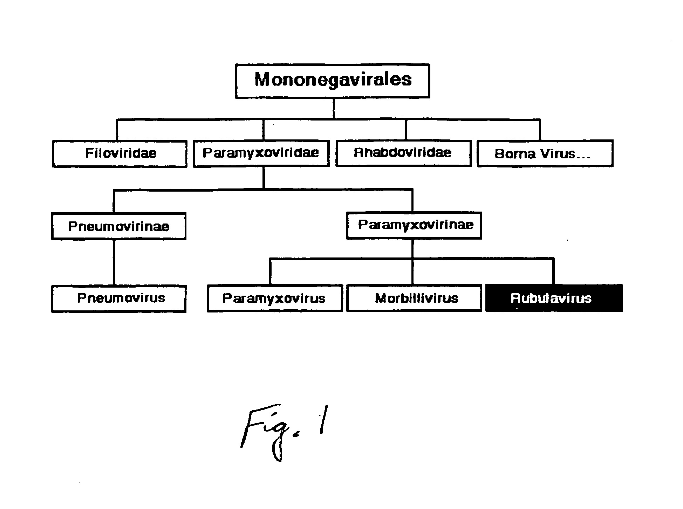

The *Rubulavirus* genus of the Paramyxoviridae (see Fig. 1) are enveloped viruses characterized by a single minus-stranded RNA genome. There is substantial evidence that some rubulaviruses infect domestic animals and can cause neurological diseases in them. These neuropathic rubulaviruses include Canine Parainfluenza Virus Type 2, Porcine Rubulavirus (*aka* La Piedad-Michoacan-Mexico Virus), and Menangle Virus. The strong sequence homologies amongst these viruses imply that each of these viruses represent separate host-adapted species of a common ancestral Rubulavirus species.

Canine Parainfluenza Virus Type 2 (which is also known as CPIV) is associated with infectious tracheobronchitis in dogs. This is a non-lethal disease (usually) of the respiratory tract (Appel and Binn, Canine Infectious Tracheobronchitis. In Virus Infections of Canivores. Elsevier Science Publishing Co., New York, N.Y., pp 201-211, 1987). However, the virus has also been found to be associated with posterior paresis (Evermann et al. J.A.V.M.A. 177:1132-1134, 1980), neurological dysfunction (Baumgartner et al. Infect. Immun. 31:1177-1183,1981), encephalitis (Evermann et al. Arch. Virol. 68:165-172, 1981) and hydrocephalus (Baumgartner et al. Vet. Pathol. 19:79-92, 1982) in dogs.

Porcine Rubulavirus (which is also known as La Piedad-Michoacan-Mexico Virus or LPMV) is associated with Blue-Eye Disease (BED) of pigs. The symptoms of the disease include corneal opacity, extreme "nervousness" in young pigs and infertility in sows and boars (Ramirez-Mendoza et a/., J. Comp. Pathol. 117:237-252, 1997). The virus persists in the central nervous system of pigs after recovery from BED (Wiman *et al.* 1998; Hjertner *et al.* 1998). This virus also shares more than 95% of its nucleotide sequence with *Cryptovirus*.

Menangle virus is an emergent rubulavirus that was first identified in Australia in 1997 (Philbey et al., Emerging Infectious Diseases 4:269-271, 1998). This virus is associated with severe degeneration of the brain and spinal cord of stillborn piglets (Philbey *et al.* 1998). Neurons of these animals contained Menangle virus inclusion bodies (nucleocapsids). Serological studies have also found neutralizing antibodies to the virus in pigs, fruit bats and at least two human piggery workers.

A number of human rubulaviruses are known to cause illness in humans. These include: (1) mumps virus (causes human mumps); (2) human parainfluenza virus type 2 (*aka* HPIV-2; associated with relatively mild upper respiratory, flu-like illness; (3) human parainfluenza virus types 4A and 4B (*aka* HPIV-4A, HPIV-4B; also associated with relatively mild upper respiratory, flu-like illnesses). With mumps there is also evidence of nervous system involvement in a significant number of patients, although this is virtually never life threatening.

In contrast, there are no published studies clearly demonstrating that another *Rubulavirus,* Simian Virus 5 (SV5), causes disease either in humans or in experimentally-infected animals, and there has been at least one published study demonstrating that SV5 does not cause disease in experimentally-infected mice, even mice with severe combined immunodeficiency (SCID mice) (Didcock et al., J. Virol. 73:3125-3133, 1999).

In 1978, a report described the isolation of an "infectious agent" from the bone marrow of patients with multiple sclerosis (MS). (Mitchell, DN et al., Isolation ofan infectious agent from bone marrow of patients with mutiple sclerosis, Lancet ii:387-391 [1978]). Subsequent reports described five separate "human SV5 isolates" derived from different MS patients. (Goswami, KKA et al., Does simian virus 5 infect humans? Journal of General Virology 65:1295-1303 [1984]; Goswami, KKA et al., Evidence for the persistence of paramyxoviruses in human bone marrows, Journal of General Virology 65:1881-1888 [1985]; Randall, RE et a/., Isolation and characterization of monoclonal antibodies to simian virus 5 and their use in revealing antigenic differences between human, canine and simian isolates, Journal of General Virology 68:2769-2780 [1987]). Nevertheless, a causal link between SV5 and MS remained speculative.

In 1987, Goswami *et al.* reported that the cerebrospinal fluid (CSF) of some MS patients contained antibodies to SV5. (Goswami KKA et al., Antibodies against the paramyxovirus SV5 in the cerebrospinal fluids of some multiple sclerosis patients, Nature 327:244-247 [1987]). However, this report was controversial, since the results subsequently failed to be reproducible by other well respected paramyxovirologists. (Vandvik, B. and Norrby, E., Paramyxovirus SV5 and multiple sclerosis, Nature 338:769-771 [1989]; but see, Russell, WC and Randall, RE, Multiple sclerosis and paramyxovirus, Nature 340:104 [1989]). Therefore, a clear causal link between SV5 and MS was not established in the art.

Multiple Sclerosis is a chronic degenerative central nervous system disease that most commonly affects young and early middle-aged adults (between 18 and 40 years of age). It is less commonly diagnosed in adolescents and even less so in children. Affecting 350,000 Americans, MS is one of the most frequent causes of neurologic disability except for traumatic injuries. (S.L. Hauser, Multiple Sclerosis and other demyelinating diseases In: Harrison's principles of Internal Medicine, 13th ed., K.J. Isselbacher et al. (eds.), McGraw-Hill, pp.2287-95 [1994]). The onset, progression and outcome of the disease are highly variable with patients manifesting one of several patterns of illness. For example, for reasons that are unclear, MS affects twice as many females as males. Although the individual components that comprise the diagnostic, clinical tableau of MS have long been delineated, their sequence and severity of presentation from case to case are subject to great variation. (Hallpike J.F., Adams C.W.M., and Tourtellotte W.W., Eds, 1983, Multiple Sclerosis: Pathology, Diagnosis and Management, Chapman & Hall, London; McAlpine E. et al., 1972, Multiple Sclerosis: A Reappraisal, Churchill Livingstone, Edinburgh; Rose A.S., 1972, Multiple Sclerosis: A Clinical Interpretation. In Multiple Sclerosis: Immunology, Virology and Ultrastructure Wolfgram F., Ellison G.W., Stevens J.G., and Andrews J.M., Eds., Academic Press, New York). It is fair to say that no two patients with MS are alike, and, consequently, there is contention as to what constitutes the stereotypic clinical history.

Most commonly, MS first presents as a series of neurological attacks followed by complete or partial remissions where symptoms lessen only to return after some period of stability (relapsing-remitting MS). In other patients, the disease is characterized by a gradual decline with no clear remissions but sometimes with brief plateaus or minor relief of symptoms (primary-progressive MS). In still other patients, there can be a relapsing and remitting course of illness in the early stages followed by progressive decline (secondary-progressive MS).

In general, the primary manifestations of chronic progressive and chronic relapsing MS do not vary greatly. Evidence for an insidious disease (apathy, depression, fatigue, loss of weight, muscle pains) often can be uncovered from the patient's chart before the first neurological manifestations. Among the first signs in about 50% of all definite MS cases are limb weakness, numbness, or tingling (parathesias) in one or more limbs, the extremities, or around the trunk. There is often discordance between signs and symptoms. Adams and Victor (1997, Multiple sclerosis and allied demyelinative diseases. In Principles of Neurology. Adams R.D. and Victor M., Eds., McGraw Hill, New York) mention that "it is a common aphorism that the patient with MS presents with symptoms in one leg and signs (bilateral Babinski) in both." Another common initial sign is a short-lived episode of retrobulbar neuritis affecting one or both eyes. Many MS patients will display papillitis (swelling of the optic nerve head), which depends on the proximity of the demyelinated plaque to the nerve head. There is considerable debate as to whether optic neuritis in a significant percentage of cases constitutes a separate disease or subclass of MS, but in about 50% of cases, the disease progresses to MS (Arnason et al., J. Neurol. Sci. 22:419, 1974).

As diagnosis becomes established, a more regular group of clinical syndromes develops either progressively or in a remitting fashion. The majority of patients display a mixed or generalized type of disease involving optic nerves, brain stem, cerebellum, and spinal cord. About one third will exhibit a spinal form, and about 5% will display a cerebellar or pontobulbar-cerebellar form, and a similar percentage will have an amaurotic form. Adams and Victor (*supra*) estimate that at least 80% of their own clinical material comprised cerebrospinal and spinal forms of the disease.

Psychologic disturbances are frequently observed and can present as an inappropriate euphoric state, attributed by Adams and Victor (*supra*) probably to extensive white matter lesions in the frontal lobes. In a much higher percentage of MS patients depression and irritability are observed.

Until relatively recently, MS and epilepsy were considered discrete entities. The publication of numerous recent studies demonstrating an "overlap" between these disorders has corrected this misperception. Epidemiological and demographic studies conducted over the last decade have provided substantial evidence of concurrent epileptiform symptomology in a significant proportion of MS patients. While the concurrence of epileptiform symptoms is markedly higher in early onset MS (i.e. in children and adolescents), the overall prevalence of epilepsy in MS patients is many times higher than in the general population.

Human epilepsy is an enigmatic medical condition which, in fact, is not a specific disease - or even a single syndrome - but, rather a broad category of symptom complexes arising from any number of disordered brain functions that themselves can be secondary to a variety of pathologic processes. Today, a large number of clinical phenomena are recognized as epileptic seizures, some of which (*e.g.*, myoclonic and atonic seizures) are currently poorly understood and could, in fact, reflect neuronal mechanisms that are somewhat different from the pathophysiologic processes traditionally considered to be "epileptic." Perhaps the best reflection of the enigmatic and complex nature of these illnesses is the simple fact that the etiology of the disease, in the overwhelming majority of the cases (greater than 70%), is either "cryptogenic" (*i.e.*, of obscure, indeterminate origin) or "idiopathic" (*i.e.*, of unknown cause).

Epilepsy is more than seizures. Epileptics typically exhibit a spectrum of responses, from little or no seizure activity, through mild activity (*petit mal* or "absence" seizures), to recurrent and intractable *grand mal* seizures (the occurrence of which is often misunderstood by the lay public to be the defining symptom of all forms of epilepsy; *see* Epilepsy: A Comprehensive Textbook, Engel, Jr. J. and Pedley, T.A., Eds., Lippincott-Raven, 1997). The condition in its entirety is comprised of many facets, different for each individual, that contribute to disability and impaired quality of life. While the physical spectrum of symptoms ranges from extremely subtle *petit mal* or "absence" seizures to profoundly disabling *grand mal* seizures, many patients experience other co-morbid processes (*e.g.*, memory loss, confusion, lethargy, sleep disturbances, and clinical depression) which can be equally disabling. Treatment that focuses solely on seizures often does little to lessen disability. This is perhaps best illustrated by the patient who, having undergone successful surgical resection of epileptogenic brain tissue, becomes seizure-free but remains socially isolated and unemployed, with little evidence of an improved life. Therapeutic intervention can be optimal only when the multiple and interacting medical, psychological, and environmental factors that constitute epilepsy are addressed.

Another epileptiform disease is Subacute Sclerosing Panencephalitis (SSPE), a rare and fatal degenerative central nervous system disease of children and adolescents. (Sever and Zeman, editors, Measles Virus and Subacute Sclerosing Panencephalitis. Neurology (Supplement 1) 18:1-192, 1968; Payne and Baublis, Perspectives in Virology 7:179-195, 1971; Johannes and Sever, Ann. Rev. Med. 26:589-601, 1975; Meulen et al., Comp. Virol. 18:105-159, 1983; Dyken, Neurol. Clin. 3:179-196, 1985). In its early stages it commonly presents as an affective or other behavioral disorder and progresses over a period of months to profound epileptiform neurological disease. Its later stages are characterized by intractable seizures, decerebrate rigidity, coma and death. At some time, virtually all SSPE patients are "misdiagnosed" with epilepsy.

Cases of SSPE have been described in both industrialized and developing countries throughout the world (Canal and Torck, J. Neurol. Sci. 1:380-389, 1964; Pettay et al., J. Infect. Dis. 124:439-444, 1971; Haddad et al., Lancet 2:1025, 1974; Soffer et al., Israeli J. Med. Sci. 11:1-4, 1975; Naruszewicz-Lesiuk et al., Przeg Epidemiologiczna 23:1-8, 1979; Moodie et al., South African Med. J. 58:964-967, 1980). The frequency of the disease varies greatly, ranging from 0.06 and 0.10 cases per million total population per year (cpmpy) in Britain (Dick, Brit. Med. J. 3:359-360, 1973) and the United States (Jabbour et al., J. A. M A. 220:959-962, 1972) to 3.40 and 7.70 cpmpy in Israel (Soffer, et al., Israeli J. Med. Sci. 11:1-4, 1975) and New Zealand (Baguley and Glasgow, Lancet 2:763-765, 1973). The factor(s) that are responsible for the ultimate etiopathogeneis of the disease are unclear.

Numerous studies have shown that the central nervous system tissues of SSPE patients are persistently-infected with measles virus. Substantial evidence indicates that the disease involves the recrudescence of a persistent measles virus infection acquired earlier in life. Specific findings in SSPE patients which support this hypothesis include: (1) a history of childhood measles, (2) markedly elevated titers of measles virus-specific antibodies in serum, (3) the presence of measles virus-specific antibodies in cerebrospinal fluid, (4) the presence of measles virus antigens in CNS tissues demonstrated by specific immunofluorescence, (5) intracellular inclusions of paramyxoviral nucleocapsids in oligodendroglial and neuronal cells, and (6) the isolation of infectious measles virus from brain and lymphatic tissues when co-cultivated with susceptible cells (Bouteille et al., Revue Neurologie 113:454-458, 1965; Connolly et al., Lancet 1:542-544, 1967; Legg, Brit. Med. J. 3:350-354, 1967; Payne et al., New Eng. J. Med. 281585-589, 1969; Horta-Barbosa et al., Nature 221:974, 1969). Finally, and perhaps most convincingly, epidemiological evidence suggests that vaccination against measles substantially reduces the risk of developing the disease (Modlin et al., Pediatrics 59:505-512, 1977; Halsey et al., Am. J. Epidemiol. 111:415-424, 1980; Dyken et al., Morb. Mortal. Weekly Report 31:585-588, 1982).

Despite these findings, there are a number of anomalies that have been observed which are inconsistent with measles virus alone being the sole cause of the illness. These include the following.

First, neurovirulence. Clinical isolates of measles virus from patients with rubeola have not been shown to cause an SSPE-like illness in experimentally infected animals. Cell-associated SSPE-derived strains, however, have been shown to cause such disease in ferrets, marmosets and monkeys (Katz et al., J. Infect. Dis. 121:188-195, 1970; Thormar et al., J. Infect. Dis. 127:678-685, 1973; Ueda et al., Biken Journal 18:179-181, 1975; Yamanouchi et al., Japan. J. Med. Sci. Biol. 29177-186, 1976; Thormar et al., J. Infect. Dis. 136:229-238, 1977; Albrecht et al., Science 195:64-66, 1977; Thormar et al., J. Exp. Med. 148:674-691, 1978; Ohuchi et al., Microbiol. Immunol. 25:887-893 [1981]).

Second, distribution and morphology of virus inclusion bodies. Measles virus antigens in infected cells form large coalescing intracytoplasmic inclusions when examined by fluorescent antibody techniques. When labeled with SSPE patient sera, virus antigens demonstrate distinctly different patterns in cell-associated SSPE-derived virus strains and in experimentally-infected animal CNS tissues. In such materials, intracellular inclusion bodies demonstrate a "peppery," particulate and/or "splattered" distribution (Doi et al., Japan. J. Med. Sci. Biol. 25:321-333, 1972; Kimoto and Baba, Biken Journal 18:123-133, 1975; de Felici et al., Annales Microbiologie 126:523-538, 1975; Ohuchi et al., Microbiol. Immunol. 23:877-888, 1979).

Third, ultrastructural morphology of virus nucleocapsids. In measles virus infected cells, cytoplasmic nucleocapsids are predominantly of a "fuzzy" or "granular" morphology (Tawara, Virus (Osaka) 14:85-88, 1965; Matsumoto, Bull. Yamaguchi Med. School 13: 167-189, 1966; Nakai et al., Virology 38:50-67, 1969; Nakai and Imagawa, J. Virol. 3:187-197, 1969). In SSPE-derived CNS tissues, both fuzzy and smooth nucleocapsids have been consistently observed (Oyanagi et al., J. Virol. 7:176-182, 1971; Dubois-Dalcq et al., Arch. Neurol. 31:355-364, 1974). Smooth cytoplasmic nucleocapsids have also been observed in most cell-associated SSPE-derived cell lines (Doi et al., Japan. J. Med. Sci. Biol. 25:321-333, 1972; Makino et al., Microbiol. Immunol. 21:193-205, 1977; Ueda et al., Biken Journal 18:113-122, 1975; Bumstein et al., Infect. Immun. 10:1378-1382, 1974; Mirchamsy et al., Intervirology 2:106-118, 1978; Schott et al., Revue Neurologie 135:653-664, 1979) with some containing only such structures (Doi et al., Japan. J. Med. Sci. Biol. 25:321-333, 1972; Thormar et al., J. Exp. Med. 148:674-691, 1978).

Fourth, immunoreactivity of virus nucleocapsids. While fuzzy nucleocapsid aggregates are labeled with HRP-conjugated measles virus-specific antibody experimentally raised in animals, smooth virus nucleocapsids are not (Dubois-Dalcq et al., Lab. Invest. 30:241-250, 1974; Brown et al., Acta Neuropathologica 50:181-186, 1980). Most interestingly, both can readily be labeled with SSPE sera (Brown et al., Acta Neuropathologica 50:181-186, 1980).

And fifth, epidemiology. The least understood feature of the measles virus theory of SSPE aetiopathogenesis is the extremely low incidence of the disease. Despite numerous investigations into the role of socioeconomic, demographic and genetic factors (Canal and Torck, J. Neurol. Sci. 1:380-389, 1964; Pettay et al., J. Infect. Dis. 124:439-444, 1971; Haddad et al., Lancet 2:1025, 1974; Soffer et al., Israeli J. Med. Sci. 11:1-4, 1975; Naruszewicz-Lesiuk et al., Przeg Epidemiologiczna 23:1-8, 1979; Moodie et al., South African Med. J. 58:964-967, 1980; Dick, Brit. Med. J. 3:359-360, 1973; Jabbour et al., J. A. M A. 220:959-962, 1972; Baguley and Glasgow, Lancet 2:763-765, 1973; Modlin et al., Pediatrics 59:505-512, 1977; Halsey et al., Am J. Epidemiol. 111:415-424, 1980; Dyken et al., Morb. Mortal. Weekly Report 31:585-588, 1982), it has, until now, been completely unclear why SSPE is so rare when measles virus annually infects millions of children throughout the world.

A much more common idiopathic neurological and/or neuropsychiatric disease, which affects more than a half million Americans, is chronic fatigue syndrome (CFS), which frequently involves concurrent epileptiform symptomology. (P. H. Levine, What we know about chronic fatigue syndrome and its relevance to the practicing physician, Am. J. Med. 105(3A):100S-03S [1998]). Chronic fatigue syndrome is characterized by a sudden onset of persistent, debilitating fatigue and energy loss that lasts at least six months and cannot be attributed to other medical or psychiatric conditions; symptoms include headache, cognitive and behavioral impairment (e.g., short-term memory loss), sore throat, pain in lymph nodes and joints, and low grade fever. (M. Terman et al., Chronic Fatigue Syndrome and Seasonal; Affective Disorder: Comorbidity, Diagnostic Overlap, and Implications for Treatment, Am. J. Med. 105(3A):115S-24S [1998]). Depression and related symptoms are also common, including sleep disorders, anxiety, and worsening of premenstrual symptoms or other gynecological complications. (A.L. Komaroff and D. Buchwald, Symptoms and signs of chronic fatigue syndrome, Rev. Infect. Dis. 13:S8-S11 [1991]; B.L. Harlow et al., Reproductive correlates of chronic fatigue syndrome, Am. J. Med. 105(3A):94S-99S [1998]). Other physiologic abnormalities are also associated with CFS in many patients, including neurally-mediated hypotension, hypocortisolism, and immunologic dysregulation. (P.H. Levine [1998]). A subgroup of CFS patients complain of exacerbated mood state, diminished ability to work and difficulty awakening during winter months, reminiscent of seasonal affective disorder. (M. Terman *et al.* [1998]).

The etiology of CFS has been unknown, and the heterogeneity of CFS symptoms has precluded the use of any particular diagnostic laboratory test. (P.H. Levine [1998]). Symptomatic parallels have been suggested between CFS and a number of other disease conditions, resulting from viral or bacterial infection, toxic exposure, orthostatic hypotension, and stress, but none of these has been shown to have a causal role in CFS. (E.g., I.R. Bell et al., Illness from low levels of environmental chemicals: relevance to chronic fatigue syndrome and fibromyalgia, Am. J. Med. 105(3A):74S-82S [1998]; R.L. Bruno et al., Parallels between post-polio fatigue and chronic fatigue syndrome: a common pathophysiology?, Am. J. Med. 105(3A):66S-73S [1998]; R. Glaser and J.K. Kiecolt-Glaser, Stress-associated immune modulation: relevance to viral infections and chronic fatigue syndrome, Am. J. Med. 105(3A):35S-42S [1998]; P.C. Rowe and H. Calkins, Neurally mediated hypotension and chronic fatigue syndrome, Am. J. Med. 105(3A):15S-21S [1998]; L.A. Jason et al., Estimating the prevalence of chronic fatigue syndrome among nurses, Am. J. Med. 105(3A):91S-93S [1998]). Accordingly, there has been no known cause to which diagnosis and/or treatment of CSF could be directed. Consequently, the diagnosis and treatment of CFS have continued to be directed to symptoms, rather than to an underlying treatable cause. For example, the use of relaxin has been described for relaxing the involuntary muscles and thus relieve pain associated with CFS. (S.K. Yue, *Method of treating myofascial pain syndrome with relaxin,* U.S. Patent No. 5,863,552).

There remains a need for an underlying causal factor for many idiopathic neurological, neurodegenerative, neuropsychological and neuropsychiatric disorders and primary tracheobronchial and/or lymphadenopathy-associated diseases, to which diagnostic testing, research and development, including screening of potential new antiviral drugs, and treatment can be directed. This and other benefits of the present invention are described herein.

### SUMMARY OF THE INVENTION

The present invention is based on the discovery and isolation of a novel human virus that has been designated as a *"Cryptovirus",* which falls within the genus *Rubulavirus* of the family Paramyxoviridae. The genome of the isolated *Cryptovirus* of the present invention is a minus strand RNA having a nucleotide sequence entirely complementary to (SEQ ID NO:1).

The present invention relates to isolated nucleic acids that are *Cryptovirus*-specific. The inventive *Cryptovirus*-specific nucleic acids encompass: (A) nucleotide sequence of contiguous nucleotide positions 1-15246 of (SEQ ID NO:1), such as, but not limited, to plus strand RNAs (e.g., mRNAs) and cDNAs; or (B) a nucleotide sequence complementary to contiguous nucleotide positions 1-15246 of (SEQ ID NO:1), such as, but not limited to minus strand RNAs (e.g., genomic or cloned RNAs) and cDNAs; or (C) *Cryptovirus*-specific fragments of (A) or (B), such fragments being at least about five nucleotides long. The present invention encompasses both RNAs and DNAs, and thus it is understood by the skilled artisan that the present invention encompasses nucleic acids, i.e., RNAs, in which uracil residues ("U") replace the thymine residues ("T") in (SEQ ID NO:1). The inventive nucleic acids include useful *Cryptovirus-*specific probes and primers.

Inventive nucleic acid constructs, including cloning vectors and expression vectors, are provided that contain the inventive nucleic acid. Such inventive recombinant vectors are contained in a host cell of the present invention.

The present invention also relates to an isolated *Cryptovirus* protein encoded by a *Cryptovirus*-specific nucleic acid segment. The inventive *Cryptovirus* proteins include isolated *Cryptovirus* nucleocapsid and envelope proteins and chimeric proteins comprising a *Cryptovirus* protein moiety.

The invention relates to an isolated virion or other viral particle that contains the inventive *Cryptovirus* nucleic acid, such as a viral expression vector, or contains the inventive *Cryptovirus* protein, such as an inventive pseudotyped virion or an inventive isolated *Cryptovirus* virion or other *Cryptovirus* particle.

Inventive compositions of matter that include the inventive *Cryptovirus* nucleic acid, *Cryptovirus* protein, or isolated virions and other viral particles, together with a carrier, are also included in the present invention.

Moreover, the present invention provides a method of isolating a *Cryptovirus* virion. The inventive method involves culturing a plurality of peripheral blood mononuclear cells (PBMNCs) that have been obtained from a human having a *Cryptovirus* infection. The PBMNCs are cultured in an artificial aqueous medium that includes an agent that increases cellular guanylyl cyclase activity, such as but not limited to cyclic GMP. The PBMNCs are then co-cultured in with a plurality of mammalian amnion cells in fresh aqueous medium including the agent, and the co-culture is passaged one or more times. Passaging is followed by co-cultivating a plurality of mammalian epithelial cells together with the PBMNCs and the mammalian amnion cells in fresh aqueous medium comprising the agent. This co-cultivation results in the production of *Cryptovirus* virions that are released into the aqueous medium. A supernatant of the aqueous medium is separated from the cells in the culture, to obtain the *Cryptovirus* virions, which are found in the supernatant. The inventive method facilitates the isolation from cellular material of *Cryptovirus* virions in great numbers. Virions isolated thereby can be further propagated by an inventive method of propagating a *Cryptovirus,* which the present invention provides.

The inventive method of propagating a *Cryptovirus* involves exposing a plurality of mammalian epithelial cells to a plurality of cell-free *Cryptovirus* virions, thus isolated, and further cultivating the *Cryptovirus* virion-exposed mammalian epithelial cells in an artificial aqueous medium comprising an agent that increases the activity of cellular guanylyl cyclase. Thus, a mammalian epithelial cell acutely infected with *Cryptovirus* is provided, which inventive cell is produced by the method.

The present invention also relates to a method of producing a mammalian cell line nonproductively infected with *Cryptovirus.* The method involves co-culturing PBMNCs that have been obtained from a human having a *Cryptovirus* infection, with mammalian amnion cells (e.g., rodent or primate amnion cells), in an artificial aqueous medium comprising an agent that increases cellular guanylyl cyclase activity, such that the mammalian amnion cells become nonproductively infected by *Cryptovirus.* After passaging the nonproductively infected mammalian amnion cells with the peripheral blood mononuclear cells, the co-culture becomes a monoculture of the nonproductively infected mammalian amnion cells. The present invention also relates to a cell nonproductively infected with *Cryptovirus,* which cell is produced in accordance with the method.

*Cryptovirus* is associated with cryptogenic and idiopathic forms of human disease, e.g., epilepsy. *Cryptovirus* is also associated with other human neurological, neurodegenerative, and/or neuropsychiatric diseases where neural dysfunction and neuropathology are evident and where epileptiform symptomology is always concurrent (e.g. subacute sclerosing panencephalitis, SSPE) or is frequently concurrent (e.g., multiple sclerosis [MS] and chronic fatigue syndrome [CFS]). Thus, the inventive cell lines, viral particles and virions are particularly useful for screening potential antiviral agents to discover those that could be effective in treating mammals, including humans, infected with *Cryptovirus.*

In particular, useful in vitro methods of screening a potential antiviral therapeutic agent are provided. In accordance with the in vitro screening methods, the inventive *Cryptovirus*-infected cells are cultured, and then exposed to the potential antiviral therapeutic agent. If acutely infected mammalian epithelial cells are used, then the effect of the potential antiviral therapeutic agent on *Cryptovirus* replication and/or *Cryptovirus* virion assembly is measured (e.g., effect on *Cryptovirus* genomic replication, *Cryptovirus* transcription, and/or translation, i.e., protein synthesis, from *Cryptovirus* mRNAs, effect on numbers of *Cryptovirus* virions produced or completeness of *Cryptovirus* particles). Inhibition of *Cryptovirus* replication and/or *Cryptovirus* virion assembly, relative to a control not receiving the agent, indicates antiviral activity of the potential therapeutic agent. Alternatively, if nonproductively infected cells are used, measurement is made of the effect of the potential antiviral therapeutic agent on *Cryptovirus* replication, *Cryptovirus* genome replication, and/or *Cryptovirus*-specific transcription. Inhibition of *Cryptovirus* replication, *Cryptovirus* genome replication, and/or *Cryptovirus*-specific transcription, relative to a control not receiving the agent, indicates antiviral activity of the potential therapeutic agent. These inventive methods are useful for identifying, screening, or isolating promising new antiviral drugs. Once the potential of a chemical agent is identified by the inventive methods, then, further research can be done to ascertain its clinical usefulness. Thus, the inventive methods of screening a potential chemotherapeutic agent are of benefit in finding and developing pharmaceutical antiviral drugs aimed at treating *Cryptovirus-*related conditions and other conditions associated with other viruses of the Mononegavirales.

The present invention now also provides an animal model for the study of human diseases, for example a neurological, neurodegenerative, and/or neuropsychiatric disease (e.g., idiopathic epileptiform diseases, such as epilepsy, SSPE, MS, and CFS). The animal model involves a non-human mammal, which has been inoculated with an infectious cell-free *Cryptovirus* having a genome comprising a single stranded RNA complementary to (SEQ ID NO:1), or has been inoculated with a cell nonproductively-infected with the *Cryptovirus.* The inoculated non-human mammal of the animal model exhibits at least one symptom characteristic of a human disease after being thus inoculated, which was not previously exhibited by the non-human mammal before inoculation.

The animal model is useful in an in vivo method of screening a potential antiviral therapeutic agent. The method involves administering the potential therapeutic agent to be screened, to the inventive animal model. Before administration of the potential therapeutic agent, the non-human mammal exhibits at least one symptom characteristic of a human disease. After administration of the potential therapeutic agent, the presence or absence of a beneficial antiviral effect is detected; the presence of a beneficial antiviral effect, in comparison to a control animal not receiving the agent, indicates activity of the potential therapeutic agent.

Employing an alternative embodiment of the inventive animal model, an in vivo method of screening a potential antiviral prophylactic agent is provided. The method involves administering a potential prophylactic agent to be screened to a non-human mammal, which does not have a symptom of a human disease, for example a neurological, neurodegenerative, and/or neuropsychiatric disease. Then the animal is inoculated, as previously described, with an infectious cell-free *Cryptovirus* having a genome comprising a single stranded RNA complementary to (SEQ ID NO:1), or with a mammalian cell nonproductively-infected with the *Cryptovirus.* Subsequently, the presence or absence in the non-human mammal of a beneficial antiviral effect is detected, compared to a control not receiving the potential prophylactic agent. The subsequent presence of a beneficial antiviral effect in the inoculated non-human mammal indicates activity of the potential prophylactic agent.

The inventive nucleic acid constructs, *Cryptovirus* proteins, and particles and virions are also particularly useful in producing *Cryptovirus*-specific antibodies, and in the production or manufacture of vaccines, which antibodies and vaccines are directed specifically against *Cryptovirus* proteins, such as the nucleocapsid or envelope proteins of *Cryptovirus.* These vaccines can include live attenuated virus; killed virus; recombinant chimeric viruses; proteins or other parts of virus; or one or more isolated or recombinantly expressed *Cryptovirus* proteins.

The present invention relates also to an isolated antibody that specifically binds a *Cryptovirus* protein and the use of the inventive antibody in manufacturing a medicament for the treatment of *Cryptovirus* infections. Also provided are compositions of matter comprising the antibody and a carrier.

In other aspects, the invention is usefully directed to methods and assays, e.g., for determining whether biological materials are contaminated with *Cryptovirus* or whether a mammal, including a human, is or has been infected with *Cryptovirus.*

In particular, the invention provides methods of detecting the presence or absence of a *Cryptovirus* protein, *Cryptovirus*-specific RNA, or *Cryptovirus*-specific antibody in a sample of a biological material, such as serum.

In the method of detecting *Cryptovirus* protein, the sample of the biological material is contacted with an inventive antibody that specifically binds a *Cryptovirus* protein; and if the presence of specific binding of the antibody to a constituent of the sample is detected, this indicates the presence of the *Cryptovirus* protein in the sample.

Similarly, in the method of detecting *Cryptovirus*-specific RNA in a sample of a biological material containing RNA, the sample is contacted with the inventive Cryptovirus-specific probe under at least moderately stringent hybridization conditions, and the formation of detectable hybridization products indicates the presence of the *Cryptovirus* RNA in the sample.

Alternatively, the sample containing RNA is subjected to an amplification of *Cryptovirus-*specific RNA in the sample, using at least one inventive *Cryptovirus*-specific primer in an amplification reaction mixture. By detecting the presence or absence of *Cryptovirus*-specific nucleic acid amplification products in the amplification reaction mixture, the presence or absence of *Cryptovirus*-specific RNA in the sample can be determined, with the presence of *Cryptovirus*-specific amplification products in the reaction mixture indicating the presence of the *Cryptovirus*-specific RNA in the sample.

The present invention also provides a method of detecting the presence or absence of a *Cryptovirus*-specific antibody in a sample of an antibody-containing biological material, such as serum. The method involves contacting the sample of biological material with the inventive protein, such as a *Cryptovirus* envelope protein, or alternatively, with the inventive virion or viral particle, under conditions allowing the formation of a specific protein-antibody complex, or antibody-bound virus complex, respectively. Detection of the presence of such specific protein-antibody complexes, or antibody-bound virus complexes, indicates the presence of the *Cryptovirus*-specific antibody in the sample. Inventive anti-*Cryptovirus* antibody detecting kits are also provided, which are useful for practicing the method.

Thus, by practicing any of the foregoing inventive methods of detecting the presence or absence of a *Cryptovirus* protein, *Cryptovirus*-specific RNA, or *Cryptovirus*-specific antibody, with a sample of biological materials from a mammal, including a human, an inventive method of detecting or diagnosing a *Cryptovirus* infection in the mammal is provided, as indicated by the presence in the sample of *Cryptovirus* protein, *Cryptovirus*-specific RNA, or *Cryplovirus*-specific antibody. These diagnostic methods are valuable because, regardless of the therapeutic strategy, it is advantageous to begin therapy at the time of "primary" infection (*i.e.*, the first exposure to the virus) or as soon as possible thereafter (*i.e.*, during development of the primary infection).

Other features, objects, and advantages of the invention will be apparent from the accompanying drawings and the detailed description of the preferred embodiments hereinbelow.

### BRIEF DESCRIPTION OF THE DRAWINGS

Fig. 1 is a chart showing the taxonomic assignment of the human *Cryptovirus* of the present invention to the genus *Rubulavirus* of the Paramyxoviridae.

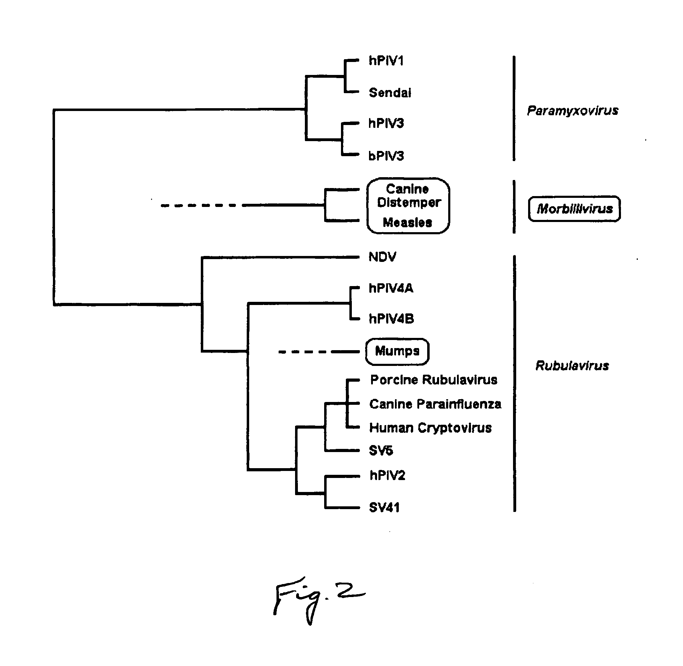

Fig. 2 is a phylogenetic tree, modified from the version appearing in Collins *et al.* (Chapter 41, page 1206, Parainfluenza Viruses, in Virology, 3rd Ed., Fields, Knipe, and Howley, Eds., Lippincott-Raven, Philadelphia, 1996). The modified tree emphasizes the clustering of three Rubulavirus species (Porcine *Rubulavirus*; Canine Parainfluenza Virus Type 2; and the human *Cryptovirus* of the present invention) as distinct from the prototype Rubulavirus Simian Virus 5.

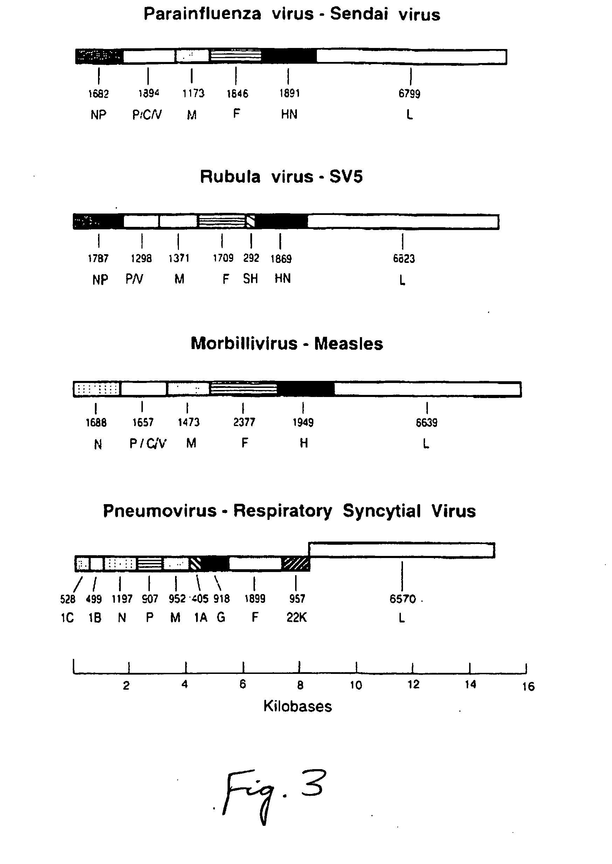

Fig. 3 is a representation of the genetic maps of typical members of each genus of the family Paramyxoviridae. The gene size is drawn to scale. Vertical lines demark gene boundaries. The pneumovirus L gene transcription overlaps that of the 22K (M2) gene and is thus shown in a staggered format. This overlap configuration is seen in human and animal viruses, but not in other pneumoviruses (Lamb and Kolakofsky, Chapter 40, page 1181, The Viruses and Their Replication, in Virology*, supra*).

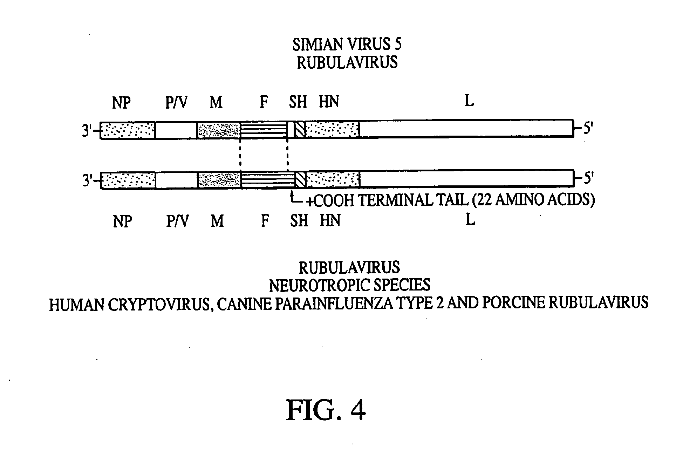

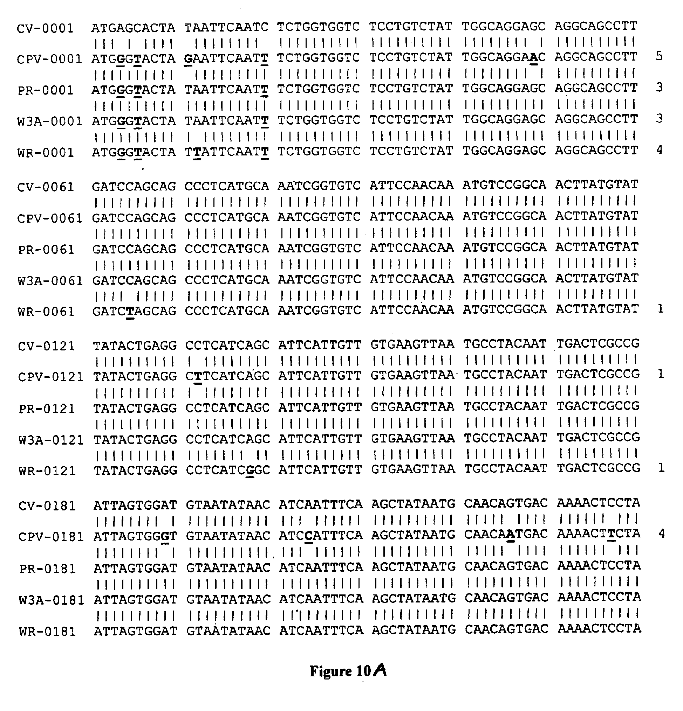

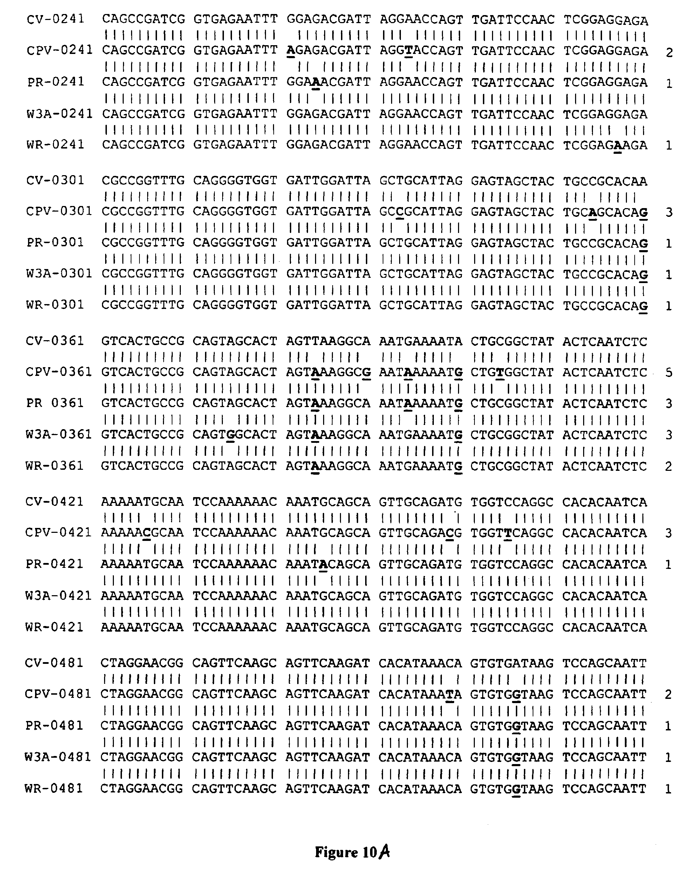

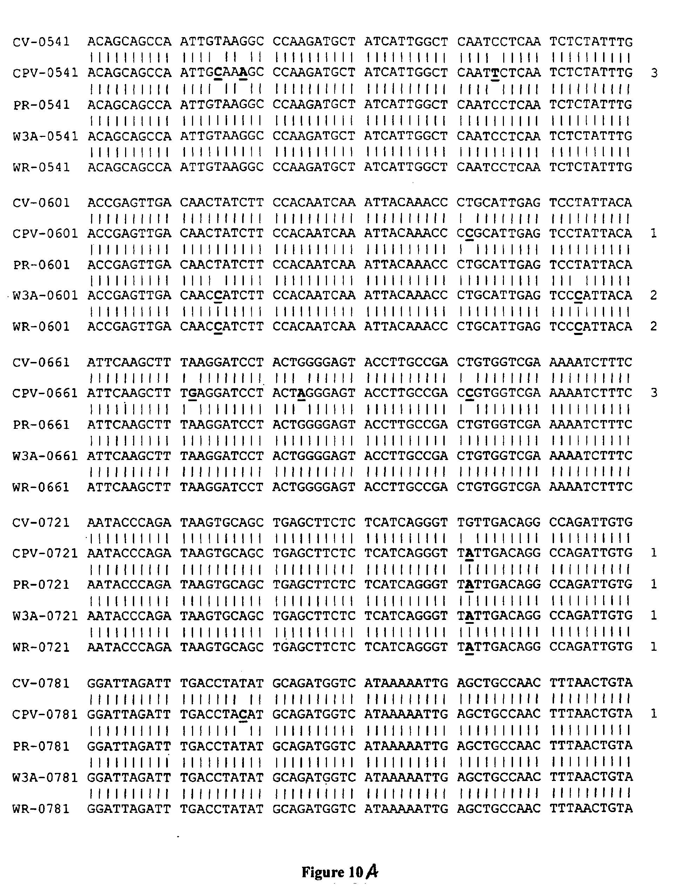

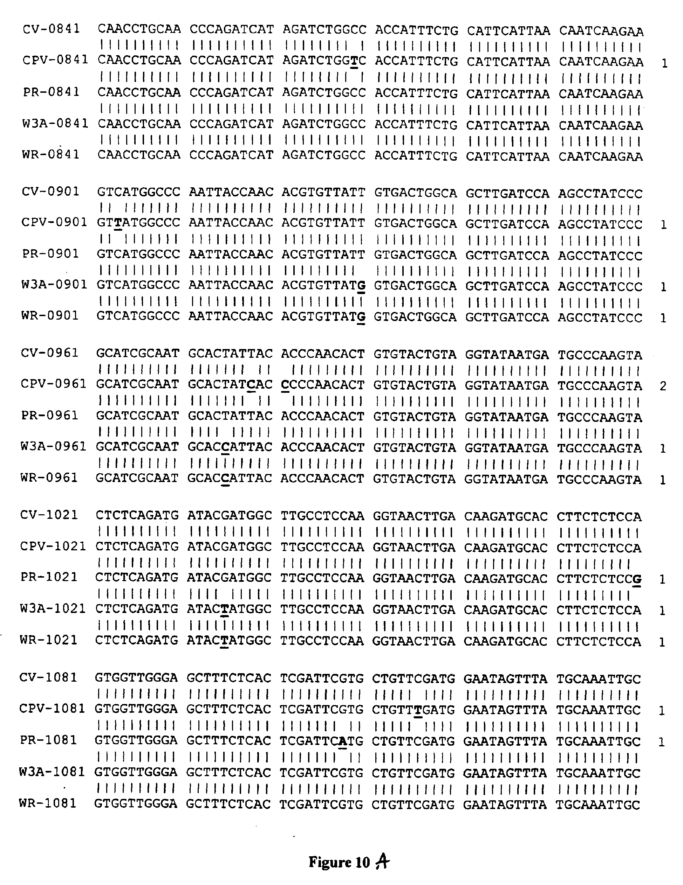

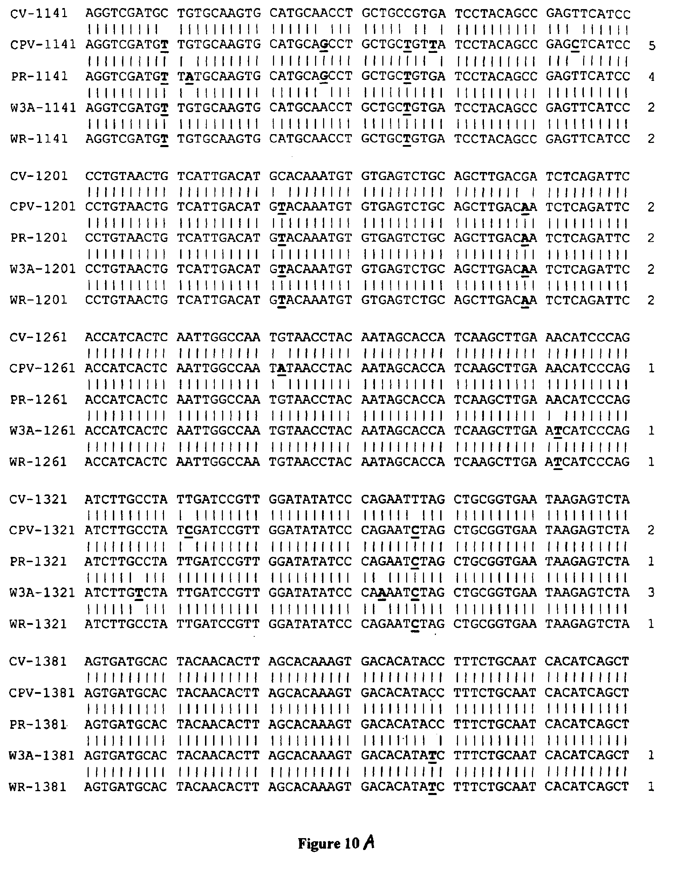

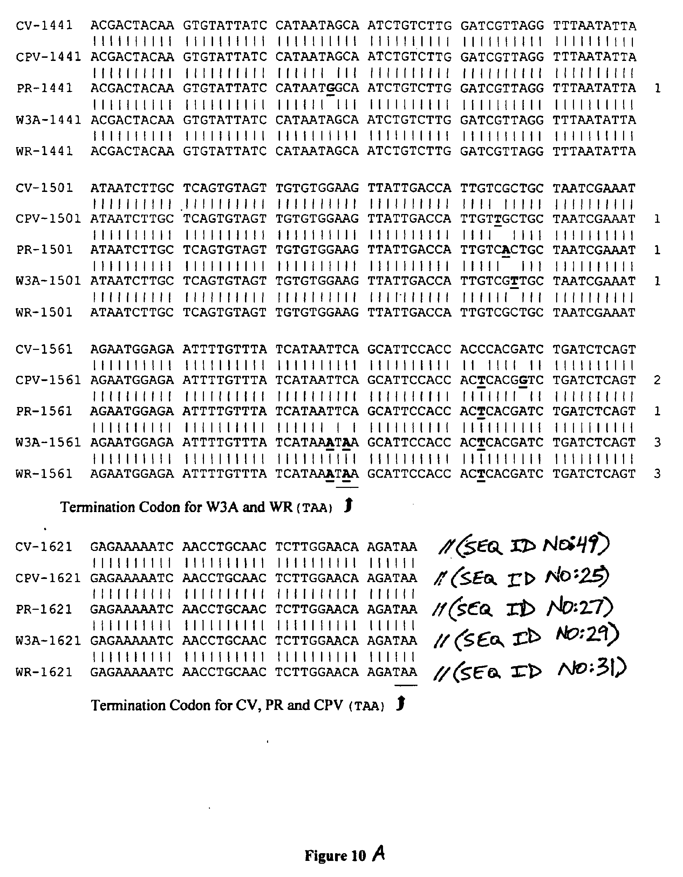

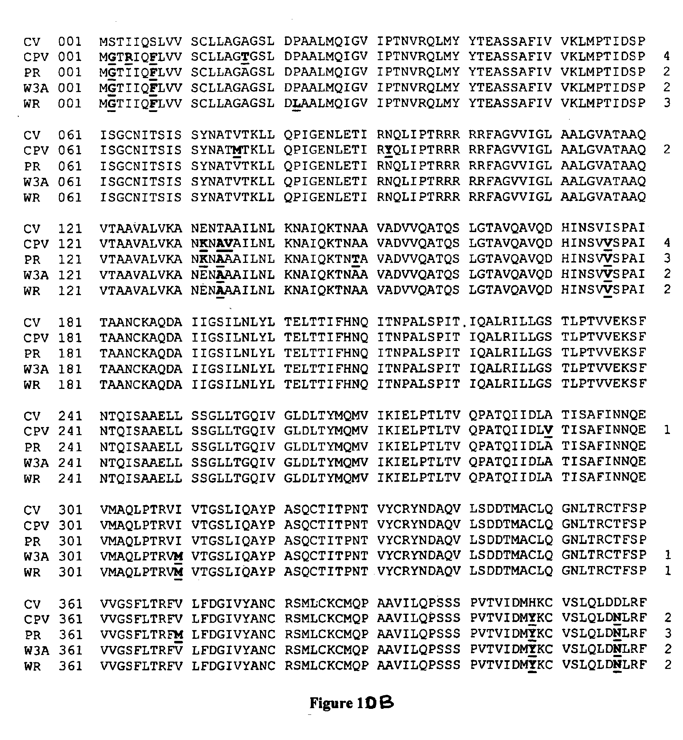

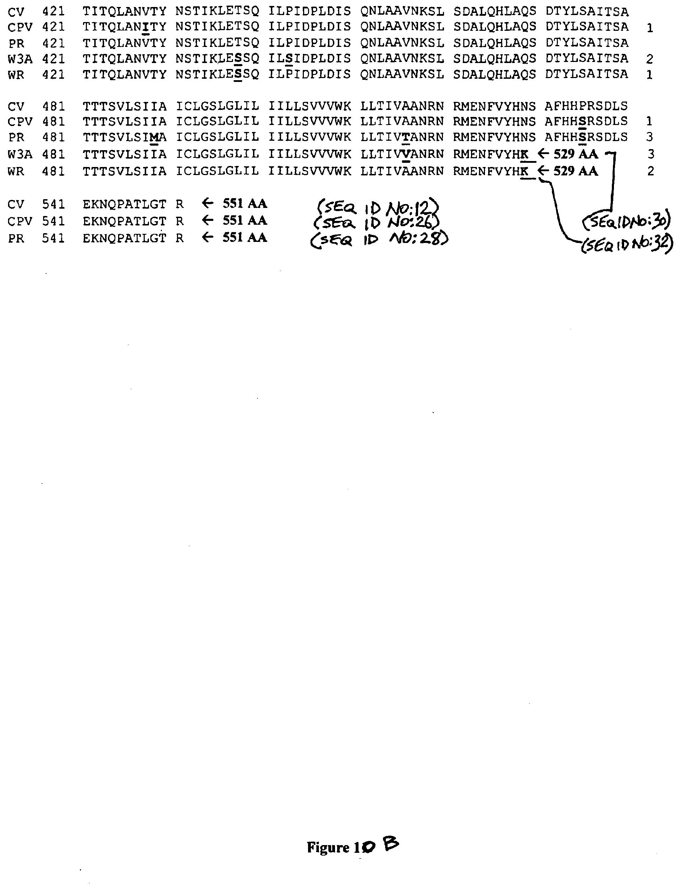

Fig. 4 is a representation of revised Rubulavirus genetic maps, which distinguish Simian Virus 5 from a cluster of related viruses that demonstrate neurotropism and encode an additional 22 amino acid "tail" at the carboxy terminus of their fusion proteins (*i.e.*, the "neurotropic species" of human *Cryptovirus,* Canine Parainfluenza Type 2, and Porcine *Rubulavirus*). The fusion (F) proteins of each neurotropic virus species are more closely related to each other than they are to the fusion protein of Simian Virus 5 (see Fig. 10).

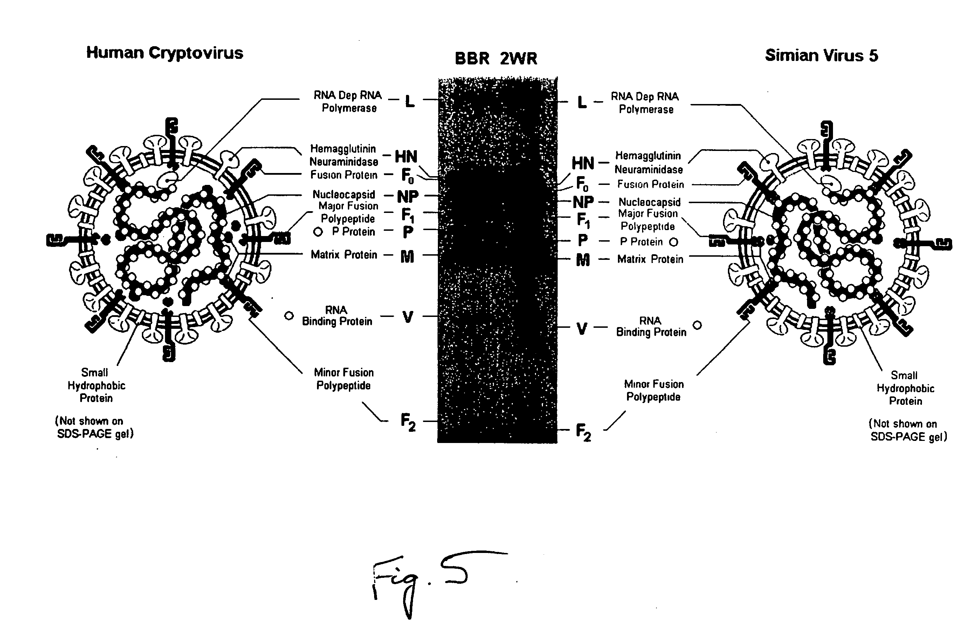

Fig. 5 shows a schematic and comparative autoradiograms of the [³⁵S]-methionine labeled proteins of gradient-purified human *Cryptovirus* (*Strain BBR*) and Simian Virus 5 (*NIH 21005-2WR* strain) following SDS-PAGE on 10% acrylamide Laemmli slab gels under reducing conditions.

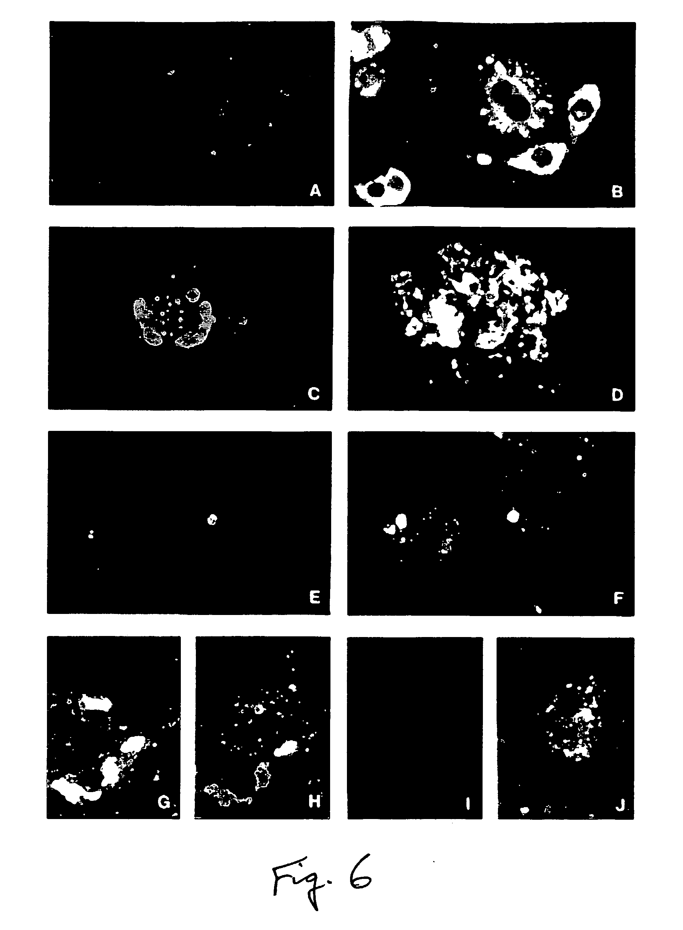

Fig. 6 is a collage of matched sets of fluorescent photomicrographs taken of various SSPE-derived nonproductively infected cell cultures following direct double labeling with rhodamine isothiocyanate-labeled goat anti-measles virus serum (Panels A, C, E, G and 1) and rabbit anti-*Cryptovirus* serum, then followed with fluorescein isothiocyanate-labeled goat anti-rabbit IgG (Panels B, D, F, H and J). Panels A and B represent AV₃/SSPE/MV cells persistently-infected with *Cryptovirus* and also infected with the Edmonston strain of measles virus before being passaged onto coverslips for these immunofluorescent studies; Panels C and D represent the nonproductive SSPE-derived cell line designated "Kitaken" (Ueda et al., Biken Journal 18:179-181, 1975); Panels E and F represent the nonproductive SSPE-derived cell line designated "Niigata" (Doi et al., Japan. J. Med. Sci. Biol. 25:321-333, 1972); Panels G and H and I and J, respectively, represent the nonproductive SSPE-derived cell line designated "Biken" (Yamanouchi et al., Japan. J. Med. Sci. Biol. 29:177-186, 1976; Ohuchi et al., Microbiol. Immunol. 25:887-983, 1981).

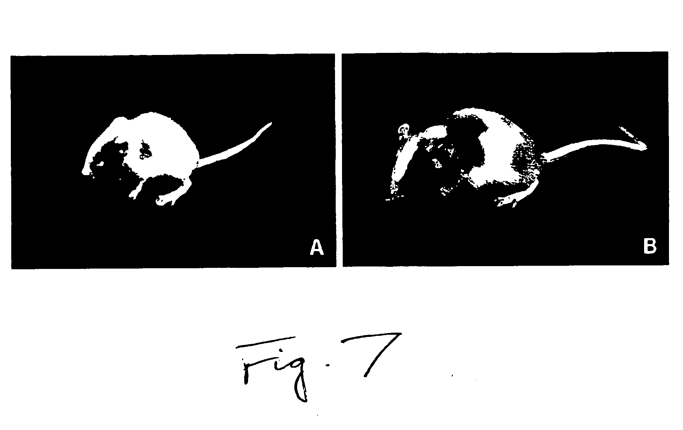

Fig. 7 shows photographs of two male Colored mice, born of the same litter, two months after neonatal (two days after birth) intracerebral inoculation with plaque-purified *Cryptovirus* (strain BBR; Fig. 7A) or with the NIH 21005-2WR strain of Simian Virus 5 (SV5; Fig. 7B).

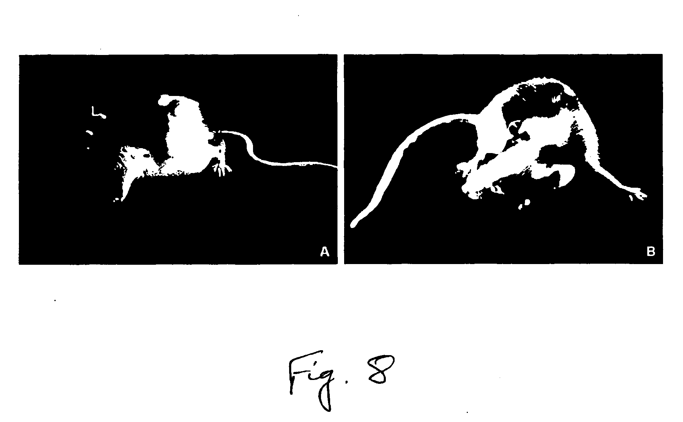

Fig. 8 shows photographs of two female Colored mice, born of the same litter, three months after neonatal (two days after birth) intracerebral inoculation with plaque-purified Cryptovirus (strain BBR; Fig. 8A) and six months after neonatal (two days after birth) intracerebral inoculation with plaque-purified Cryptovirus (Fig. 8B).

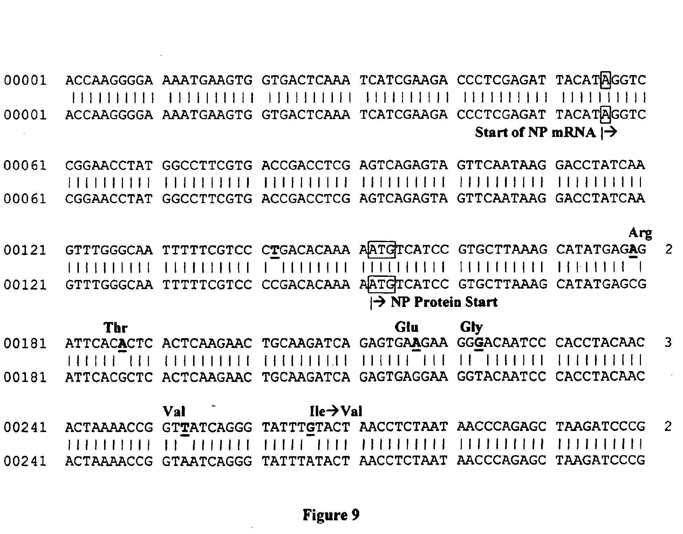

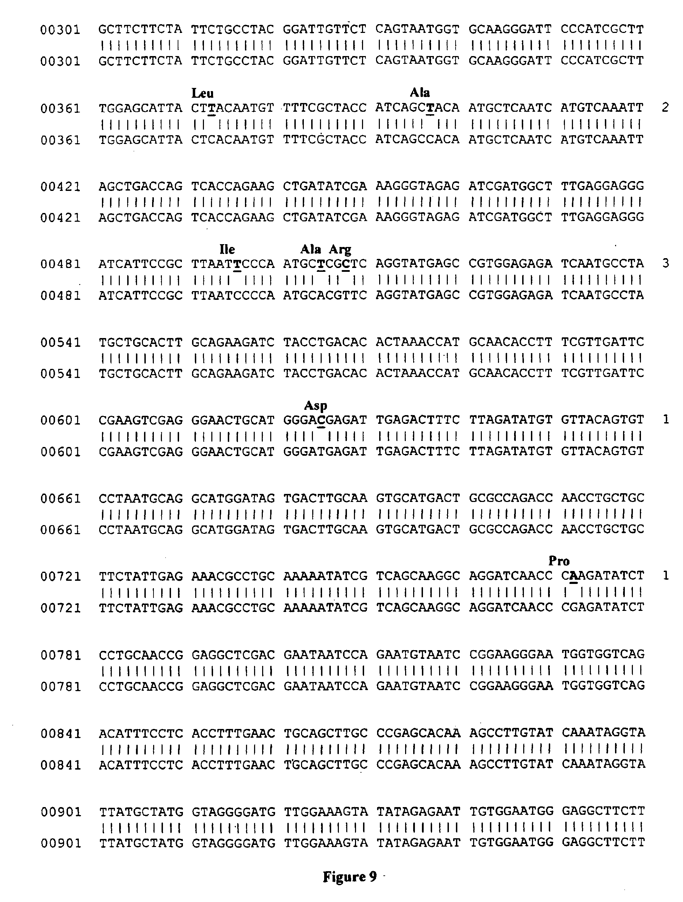

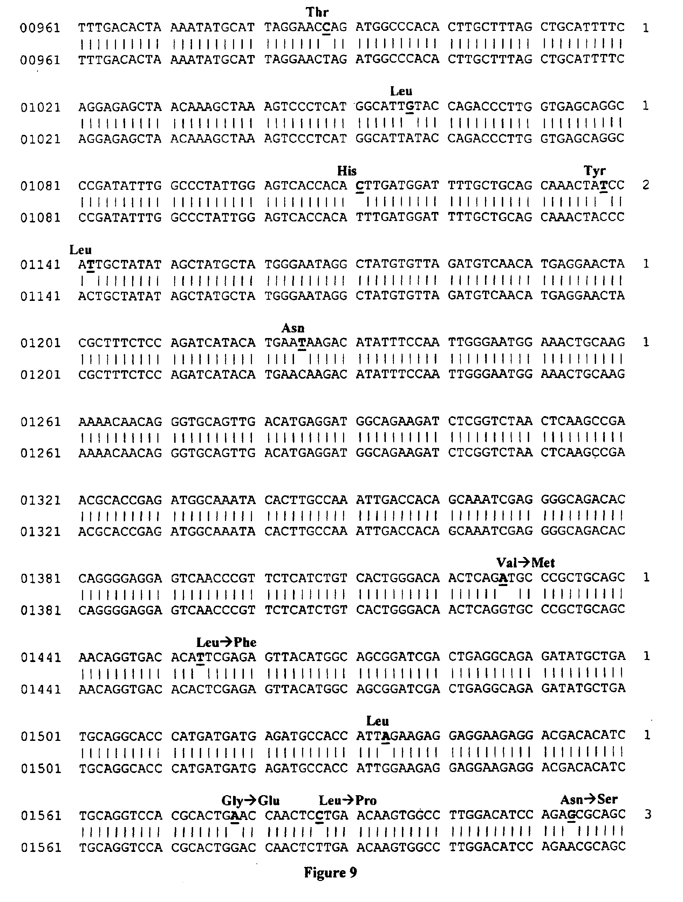

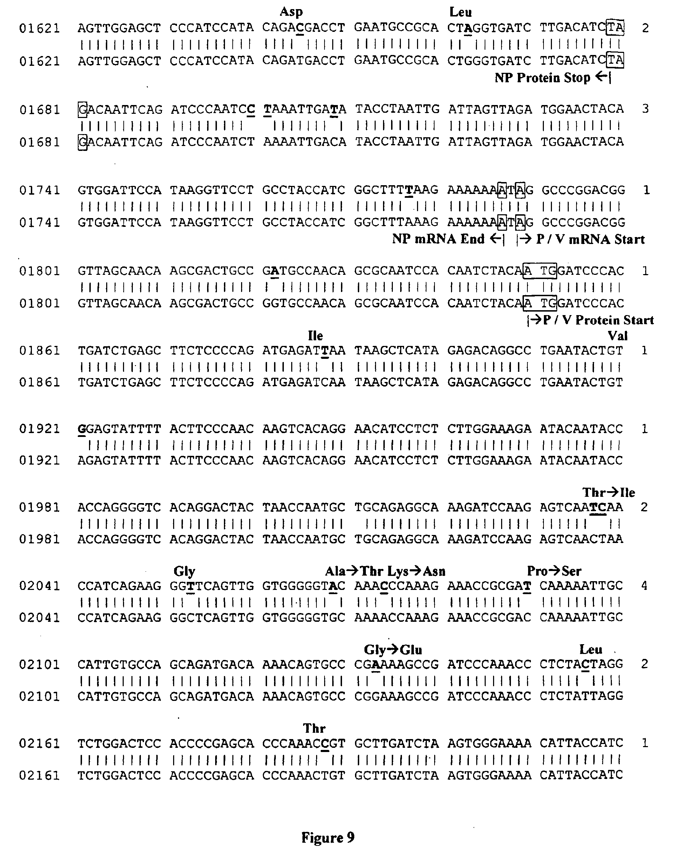

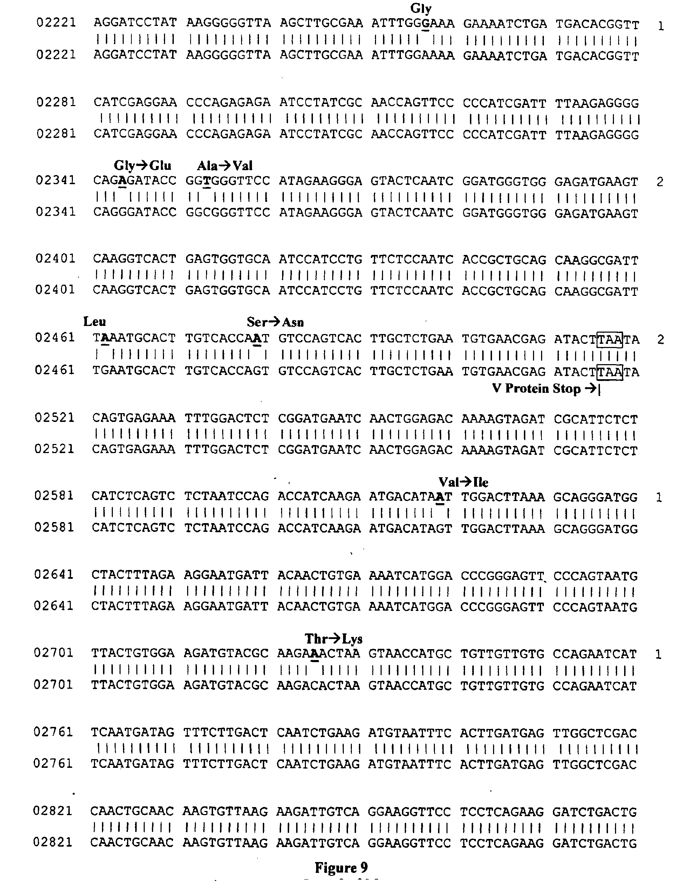

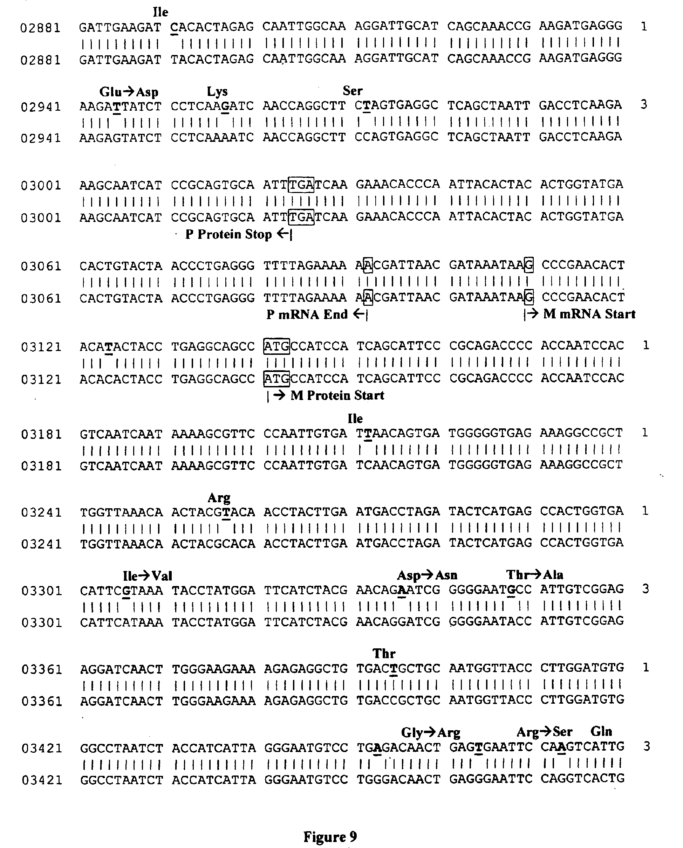

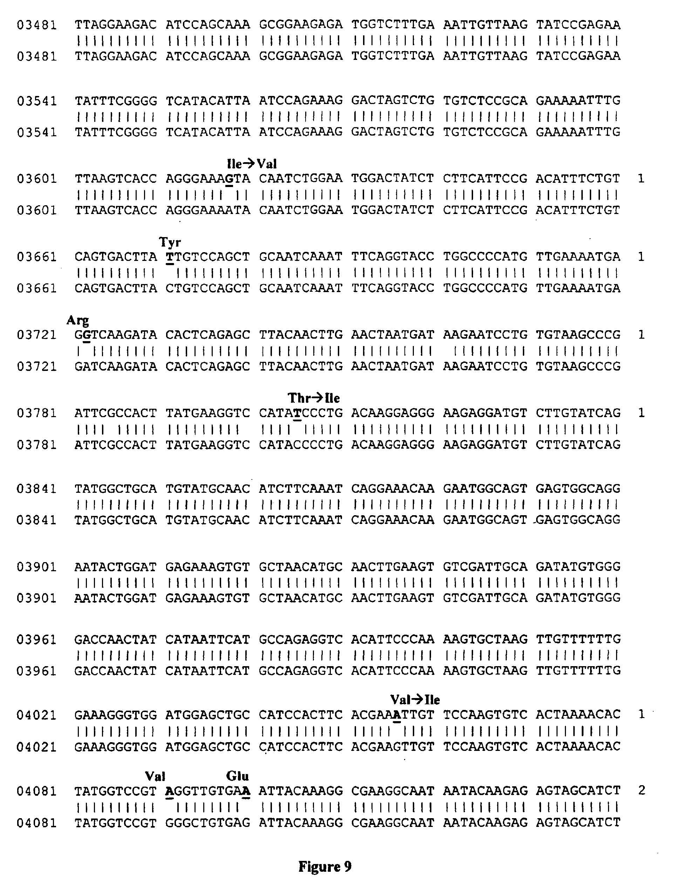

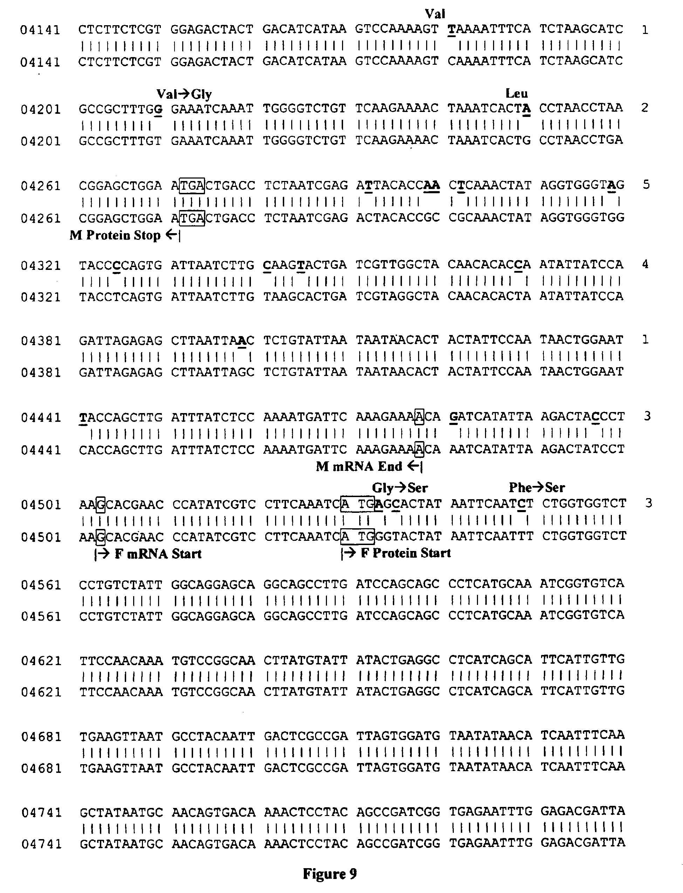

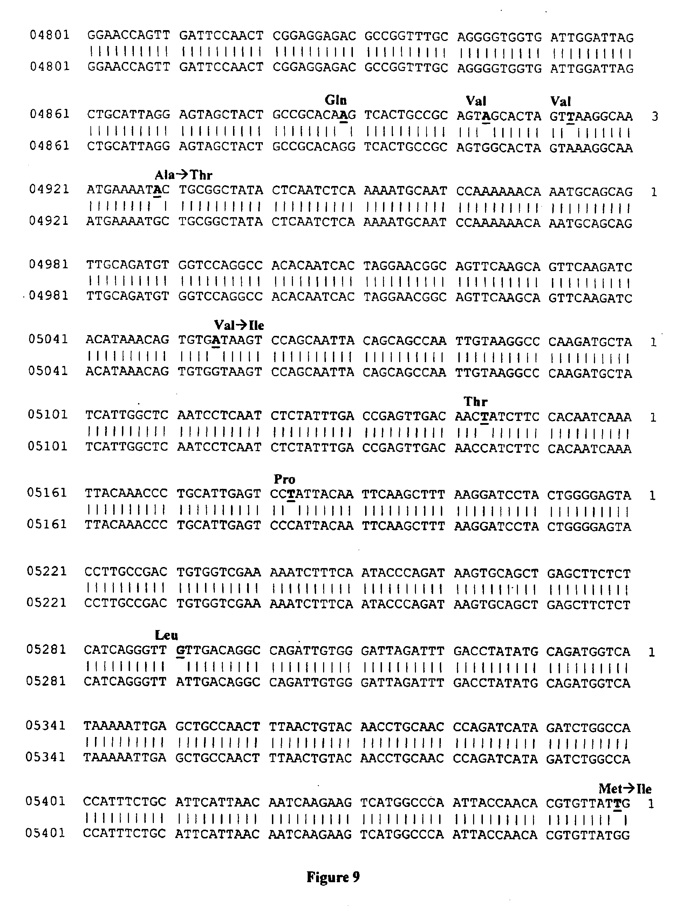

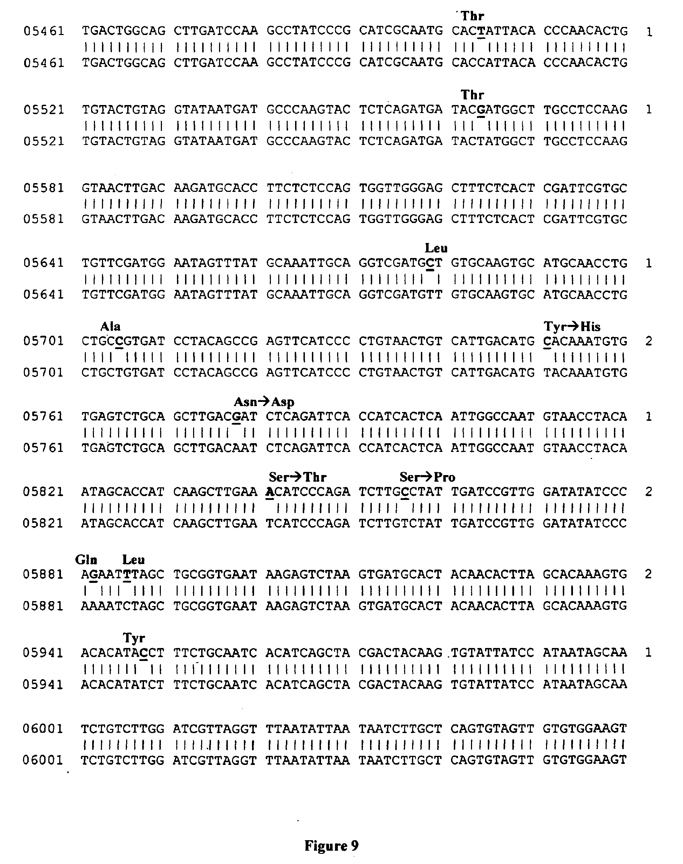

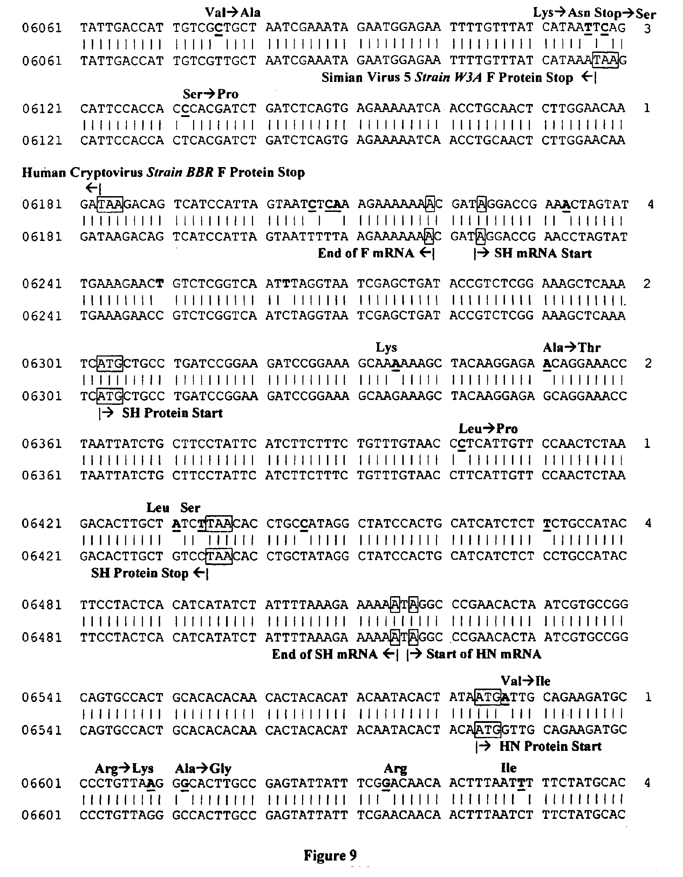

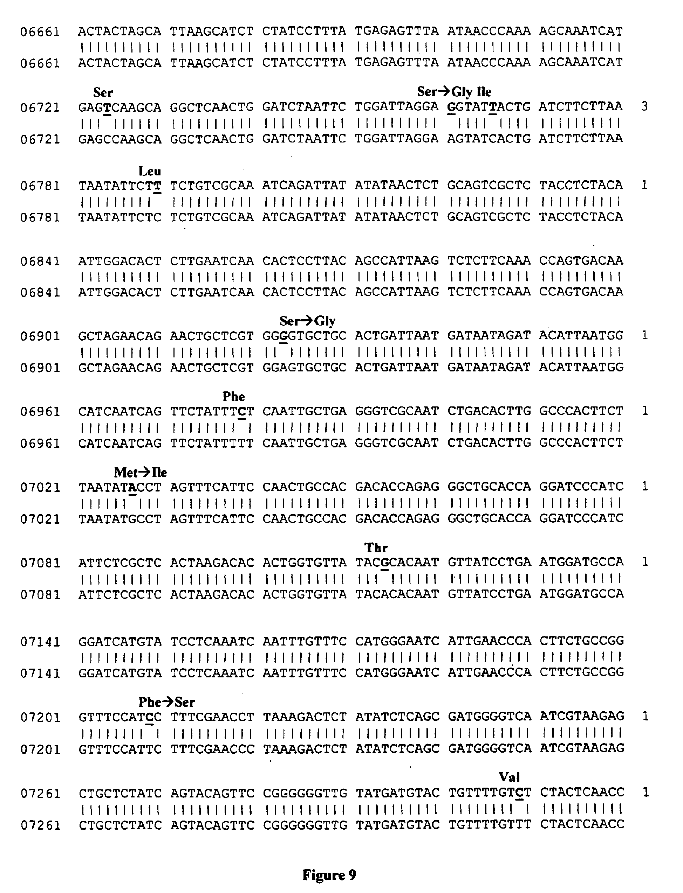

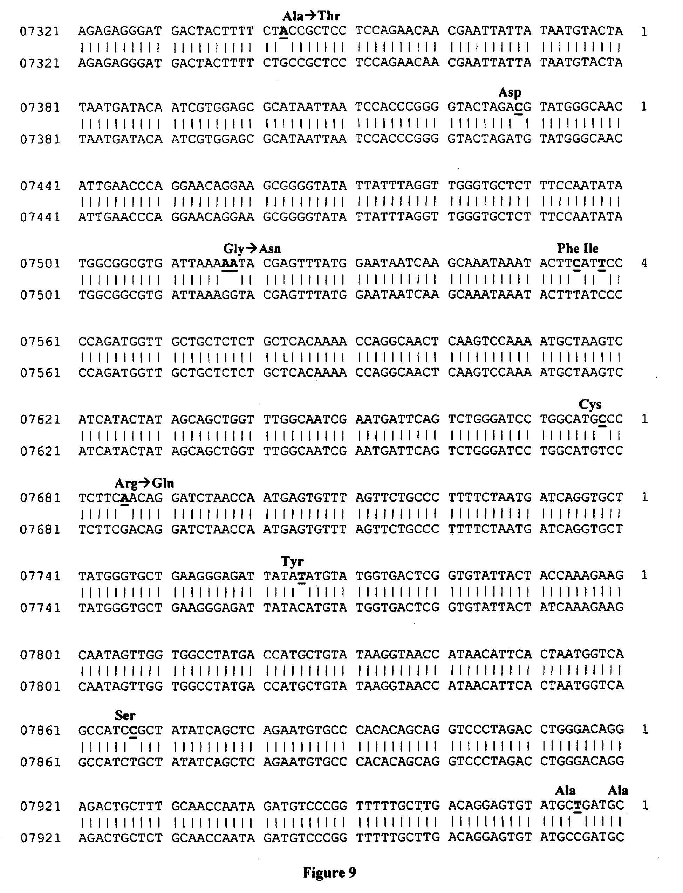

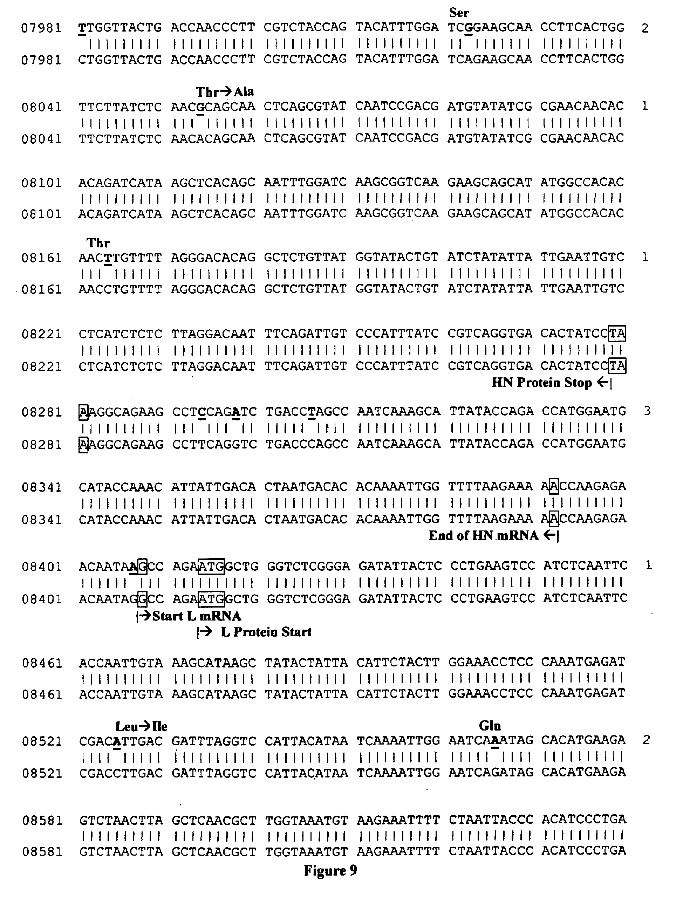

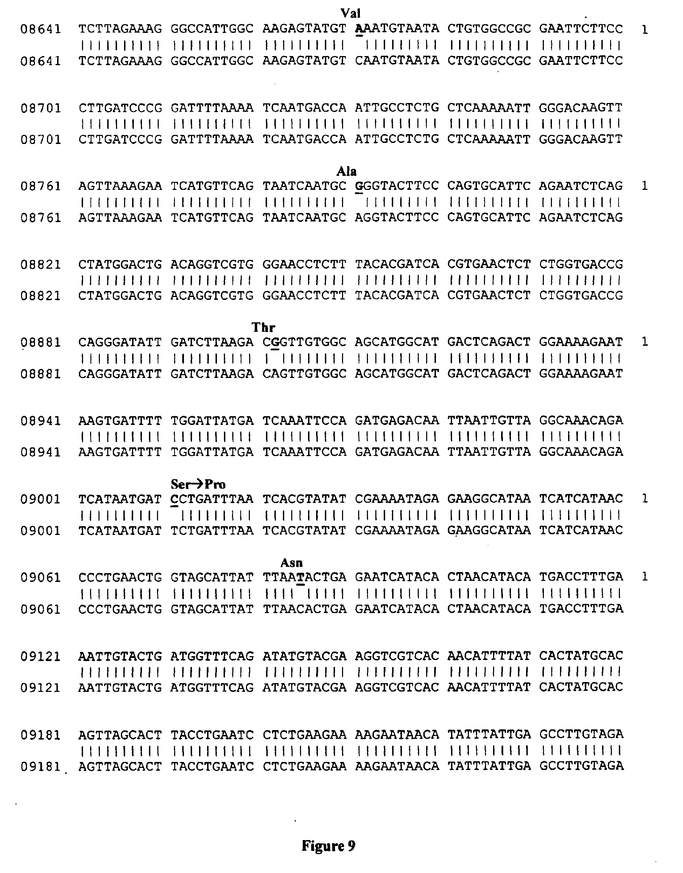

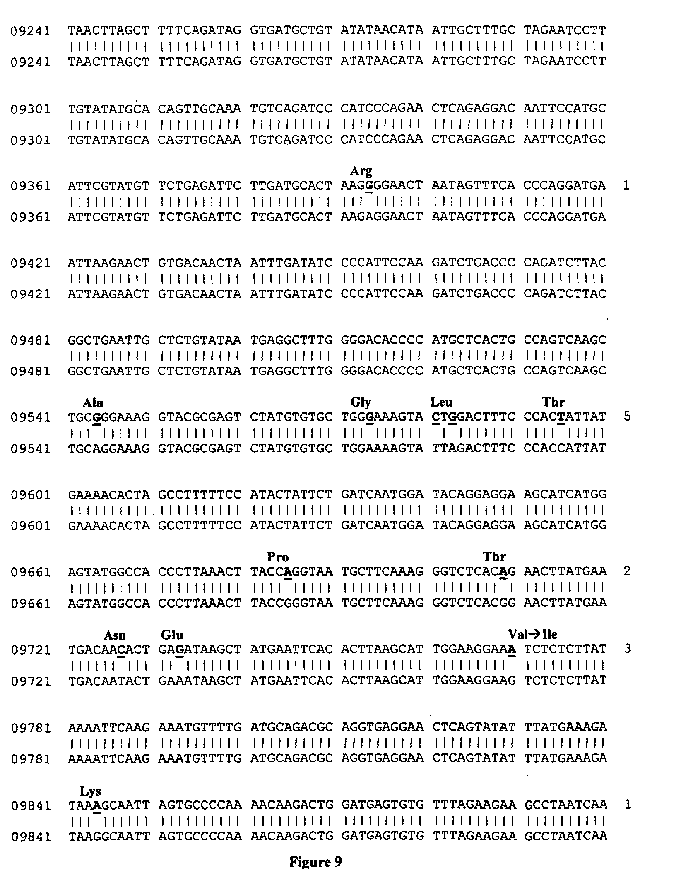

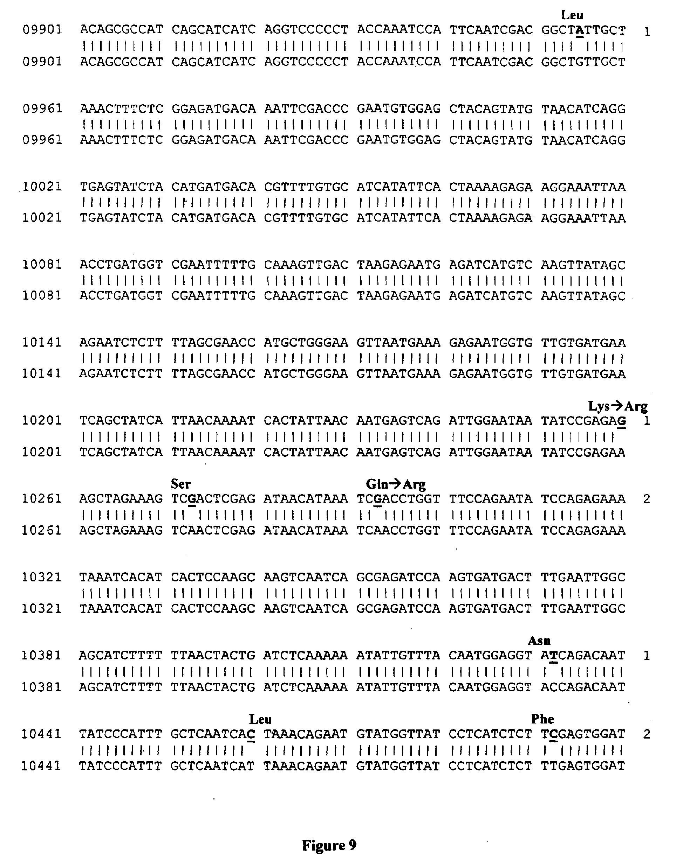

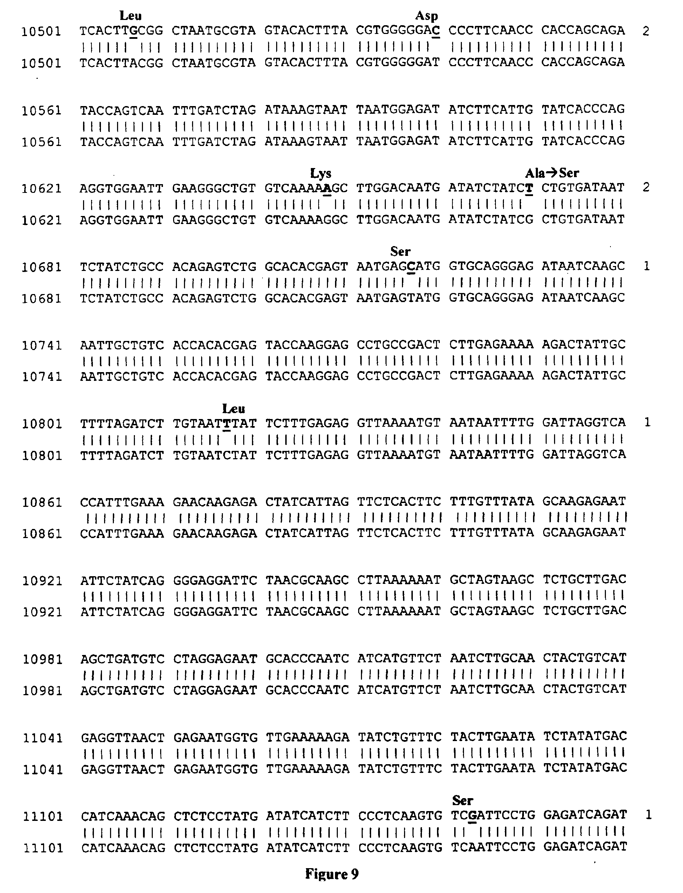

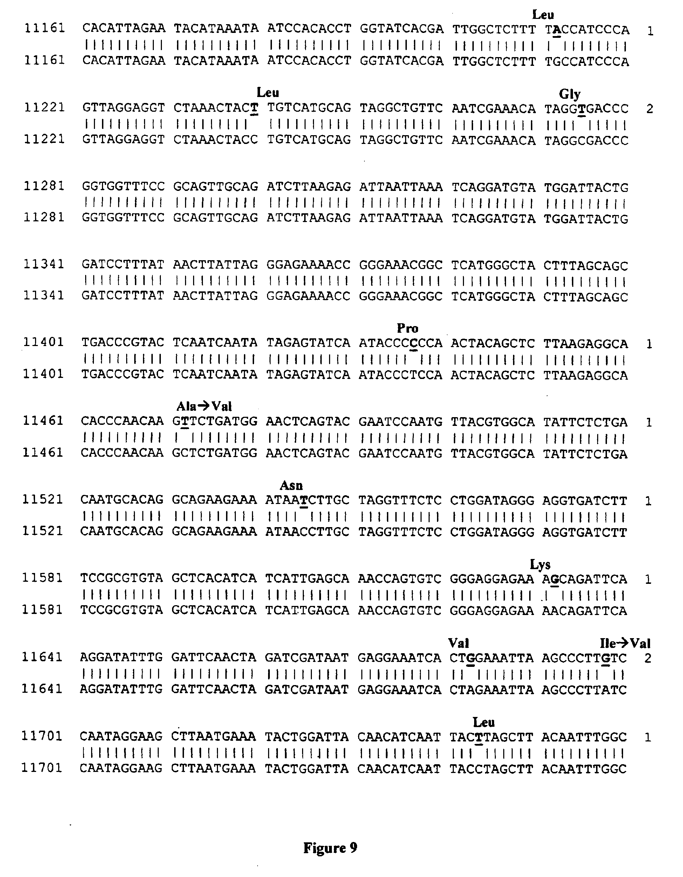

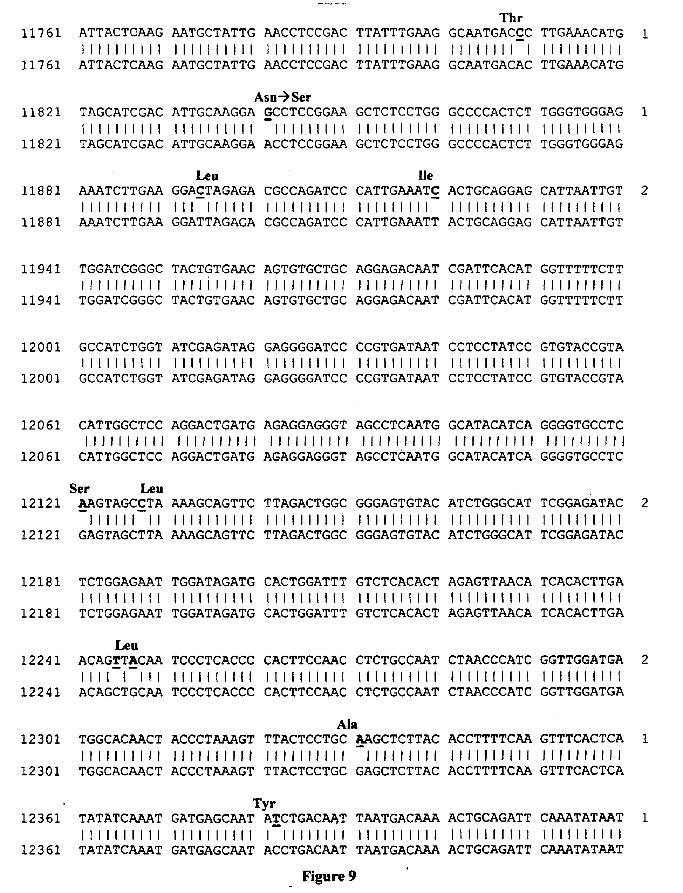

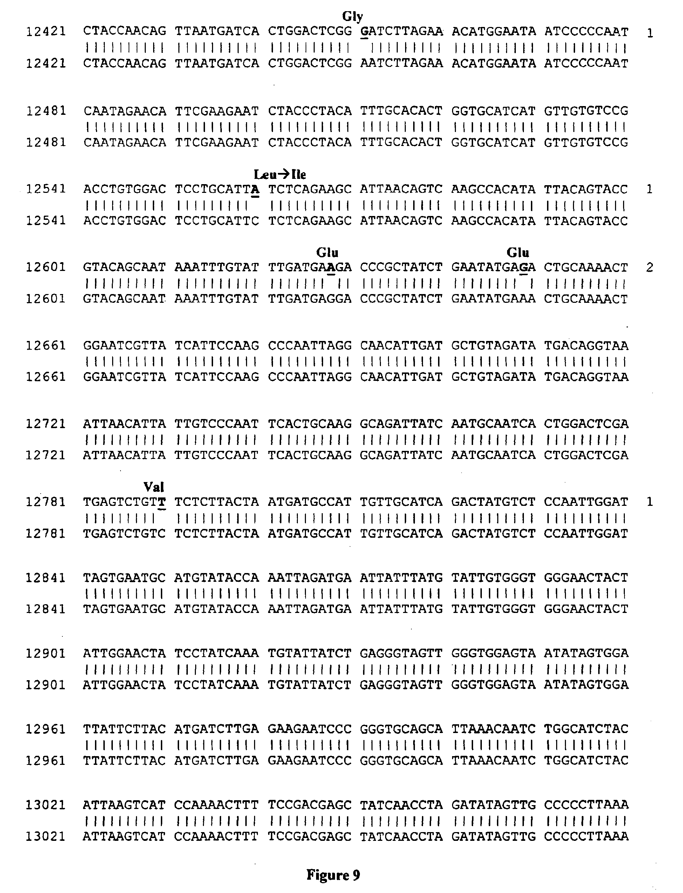

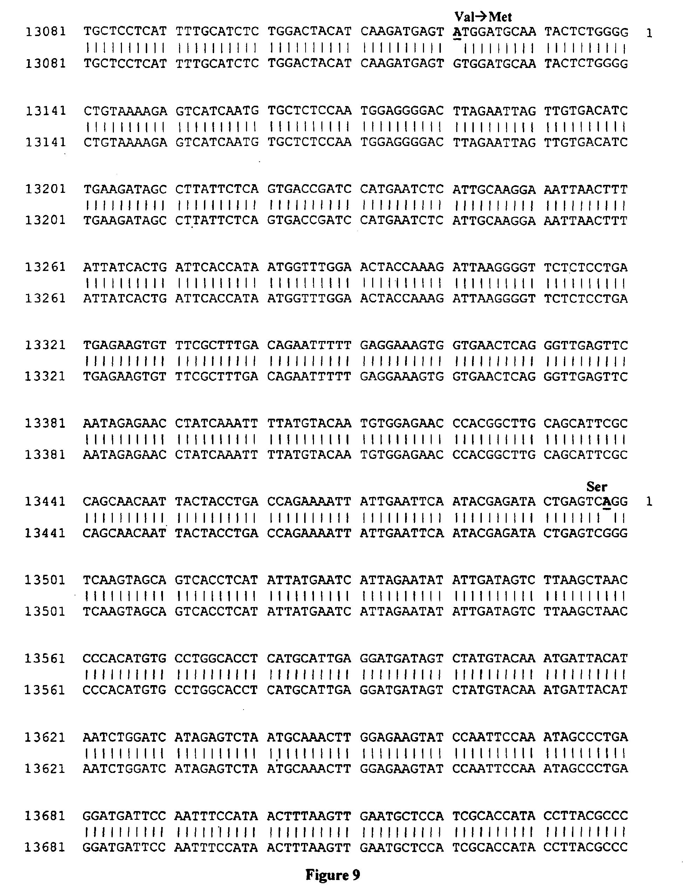

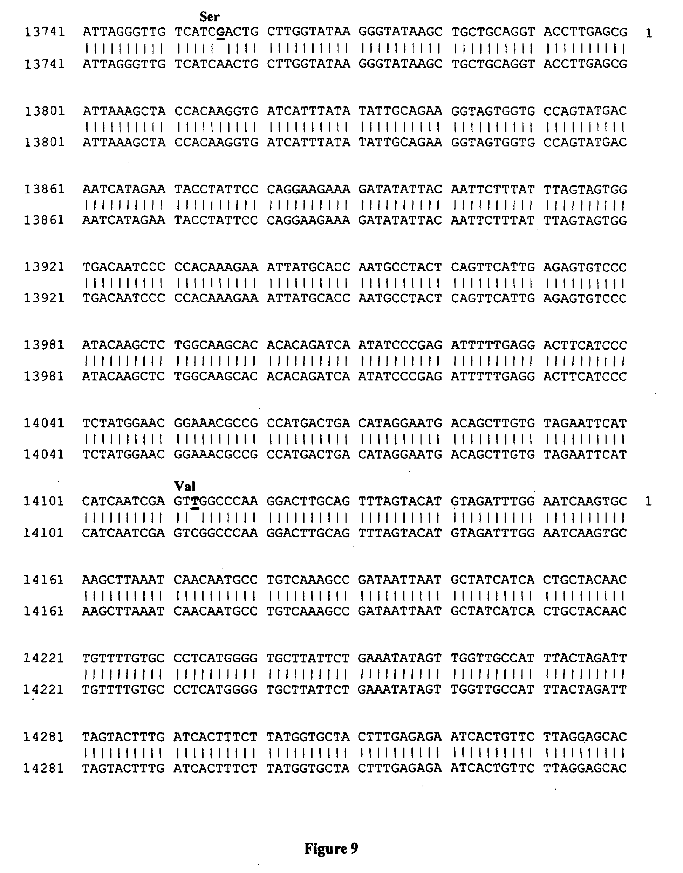

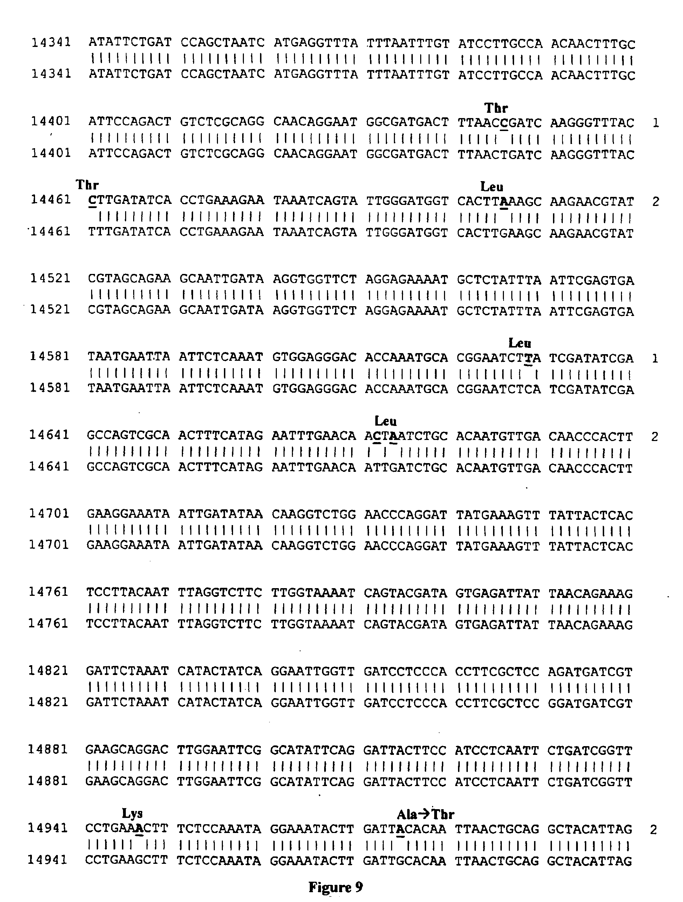

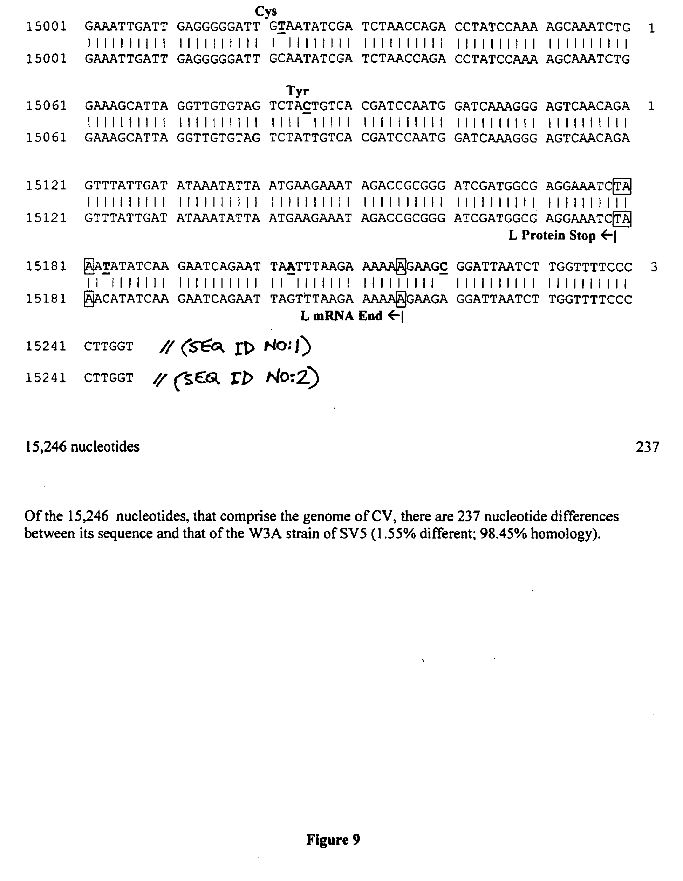

Fig. 9 is a comparison of the FASTA formatted (i.e., mRNA sense: 5' to 3') sequence of human *Cryptovirus Strain BBR* (SEQ ID NO:1) and Simian Virus 5 *Strain W3A* (SEQ ID NO:2). The number of variations from (SEQ ID NO:1) in each line of (SEQ. ID.NO:2) is tallied in the right-hand margin. The *Cryptovirus*-specific nucleotide positions that differ from the sequence of SV5 are in bold underlined type; if the difference is in a coding region, the relevant amino acid encoded is printed above the codon of the *Cryptovirus* nucleotide sequence, and if the different *Cryptovirus* nucleotide results in a codon encoding a different amino acid than the SV5 codon in the analogous position, an arrow leads from the SV5 amino acid to the different amino acid in the analogous *Cryptovirus* protein. Boxed nucleotides indicate known SV5 and analogous *Cryptovirus* start or stop sites, as indicated.
Fig. 10 is a comparison of Rubulavirus F Protein nucleotide (Fig. 10A; comparison of the FASTA formatted, i.e., mRNA sense: 5' to 3' sequence) and encoded amino acid (Fig. 10B) sequences. The first line (uppermost) represents an embodiment of the sequence of an inventive *Cryptovirus* F protein ("CV" [*Strain BBR*]); the second line represents Canine Parainfluenza Virus Type 2 ("CPV" [*Strain Tl*]; see Ito et al., J. Gen. Virol. 81 719-727, 2000); the third line represents Porcine Rubulavirus ("PR"; Klenk and Klenk, *Direct Submission to EMBL* / *GenBank Databases,* September 2000, GenBank Accession AJ278916); the fourth line represents Simian Virus 5 ("W3A" *[Strain W3A];* Paterson et al., Proc. Natl. Acad. Sci. USA 81:6706-6710, 1984); and the fifth (bottom) line represents Simian Virus 5 ("WR" [*Strain WR*]; Ito et al., J. Virol. 71:9855-9858, 1997). Amino acids that are bold and underlined denote amino acids that differ from those in the analogous sequence of the Cryptovirus F protein, and the tallies in the right margin are the number of differences for each sequence block.

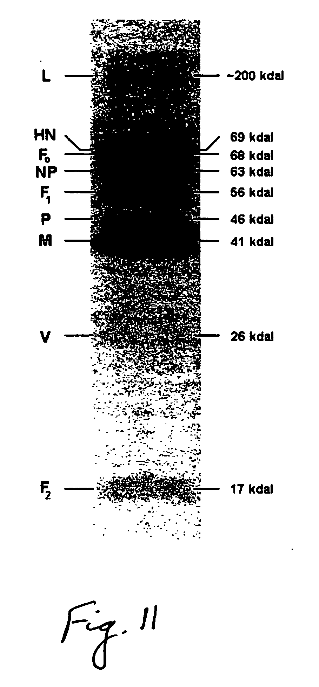

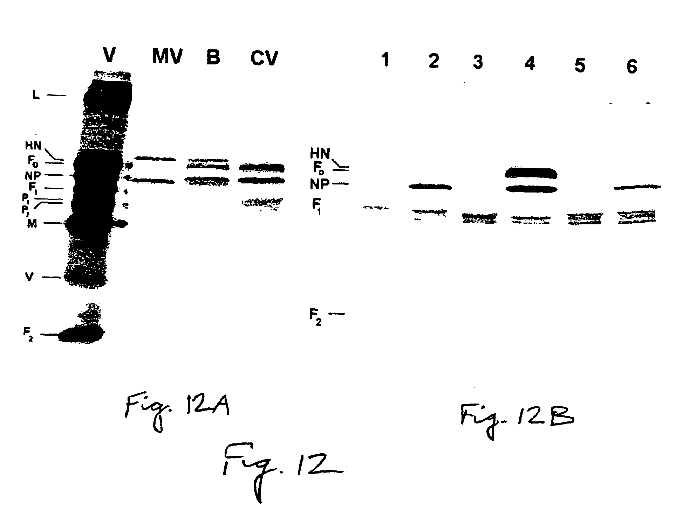

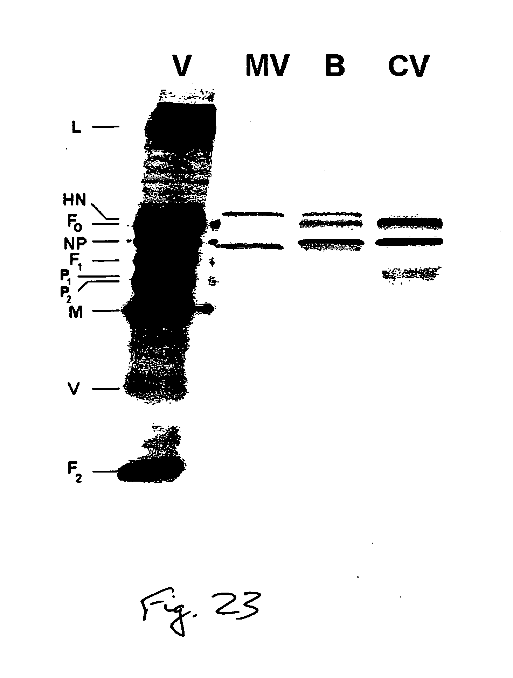

Fig. 11 demonstrates expression of *Cryptovirus* proteins. Fig. 11 is a photograph of an autoradiogram of gradient-purified [³¹S] -methionine-labeled *Cryptovirus* virions produced in acutely-infected Vero cells after SDS-PAGE under reducing conditions. The approximate molecular weights of the proteins indicated on the right side of Fig. 12A were calculated by comparing their migrations to marker proteins of known molecular weight (Sigma Biochemicals). L = the largest nucleocapsid associated protein, the major component of the virion-associated RNA dependent RNA polymerase; HN = the hemagglutinin protein, one of the envelope-associated glycoproteins; Fₒ = the uncleaved fusion protein, a second envelope-associated glycoprotein; NP = the nucleocapsid protein, the major structural protein associated with the nucleocapsid; F₁ = the larger fragment of the cleaved fusion protein; P = the nucleocapsid associated phosphoprotein; M = the virion-associated matrix or membrane protein; V = a minor RNA binding protein thought to be a component of the viral polymerase; F₂ = the smaller fragment of the cleaved fusion protein. Note: the SH protein (about 5 kD), a small envelope-associated protein, ran off the gel and is not shown. Fig. 12 shows photographs of autoradiograms of typical radioimmunoassay profiles (RIPs) obtained by the precipitation and SDS-PAGE separation of [³⁵S]-methionine-labeled virus-specific proteins using the cerebrospinal fluids (CSFs) of a patient diagnosed with subacute sclerosing panenecephalitis (Fig. 12A) and the CSFs of six randomly-selected neurology/ neurosurgery patients who had CSF taken for microbiological screening (Fig. 12B). Fig. 12A shows the RIPs resulting from the precipitation of [³⁵S]-methionine-labeled CV-1_{c} cells acutely-infected with the Edmonston strain of measles virus (Lane MV), identically-labeled CV-1_{c} cells acutely-infected with the BBR strain of *Cryptovirus* (Lane CV) or a mixture of both (Lane B) by the CSF of an 11 year male SSPE patient. Lane V represents a SDS-PAGE profile of [³⁵S]-methionine-labeled gradient purified *Cryptovirus* virions (see also Fig. 11). Fig. 12B shows the RIPs resulting from the precipitation of proteins from [³⁵S]-methionine-labeled CV-1_{c} cells acutely-infected with the BBR strain of *Cryptovirus* by the CSFs of six neurology/neurosurgery patients. The patient whose RIP profile appears in Lane 2 was an adult male who had presented with ataxia, confusion and memory loss and had not been given a specific diagnosis. The patient whose RIP profile appears in Lane 4 was an infant female who presented with hydrocephalus and intractable seizures and subsequently died in *status epilepticus*. None of the CSFs from the patients in Fig. 12B precipitated any of the envelope proteins of measles virus (data not shown). Fig. 12A is the same as Fig. 23 (described below), but is reduced to the same scale as Fig. 12B for the purpose of comparison.

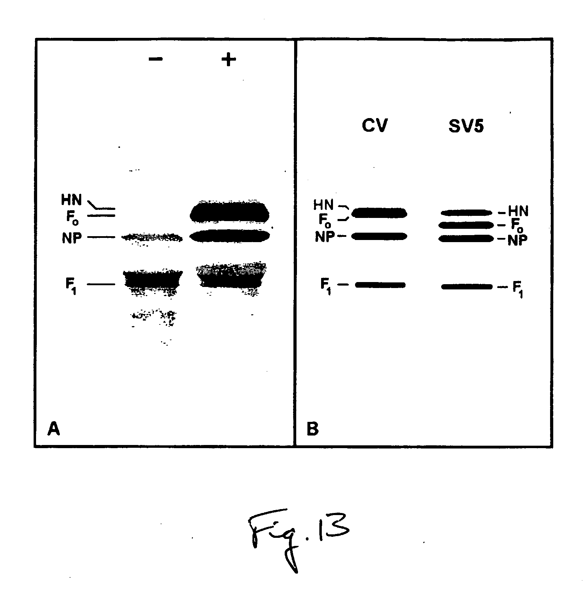

Fig. 13 shows a higher resolution autoradiogram of the radioimmunoassay profiles (RIPs) of the *Cryptovirus*-specific proteins precipitated from [³⁵S]-methionine-labeled CV-1_{c} cells acutely-infected with the BBR strain of *Cryptovirus* by two CSF specimens (Fig. 13A) and a schematic showing the migration of the major corresponding structural proteins of gradient-purified virions of the BBR strain of *Cryptovirus* (Fig. 13B Lane CV) and the NIH 21005-WR strain of SV5 (Fig. 13B Lane SV5). The RIPs in Fig. 13A represent CSF precipitates from patients assessed as *Cryptovirus-*negative (Lane "-"; i.e. not containing *Cryptovirus*-specific antibodies) and *Cryptovirus*-positive (Lane "+" ; i.e. containing *Cryptovirus*-specific antibodies). Fig. 13B is a schematic showing the near co-migration of the Fₒ and HN proteins of *Cryptovirus* and their separate migration in Simian Virus 5 (see also SDS-PAGE profiles in Fig. 5).

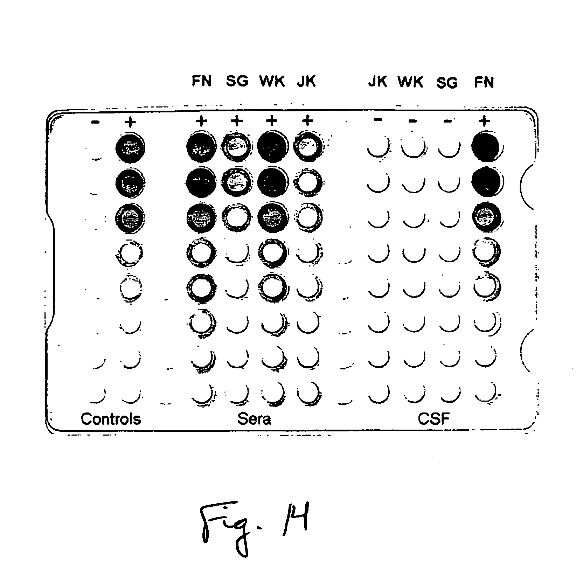

Fig. 14 shows an ELISA of matched serum and CSF specimens from four seropositive neurology/neurosurgery patients using gradient-purified *Cryptovirus* virions as the target. Control sera were rabbit antisera generated against mock-infected CV-1_{c} cells (column 1; "-") and hyperimmune rabbit antisera generated against gradient-purified *Cryptovirus* virions (column 2; "+"). FN = infant female diagnosed with hydrocephalus and intractable seizures; SG = adult female diagnosed with idiopathic intracranial hypertension; WK = male child diagnosed with acute viral meningitis; JK = adult male having an undetermined diagnosis. Serum dilutions began at 1:20 (in the top rows) and proceeded by 2-fold serial dilution to the bottom. CSF dilutions began at 1:2, at the top, before proceeding likewise. Serum specimens were aliquoted from left to right while CSF specimens were aliquoted from right to left. Note that although all of the patients had *Cryptovirus-*specific antibodies in their serum, only the patient with a seizure disorder (FN) had such antibodies in her CSF.

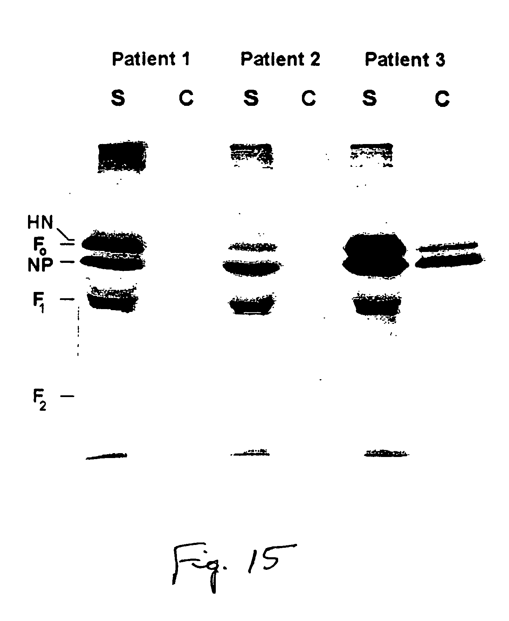

Fig. 15 is a photograph of RIP assays using three sets of matched serum (S) and CSF (C) samples from patients diagnosed with Alzheimer's disease.

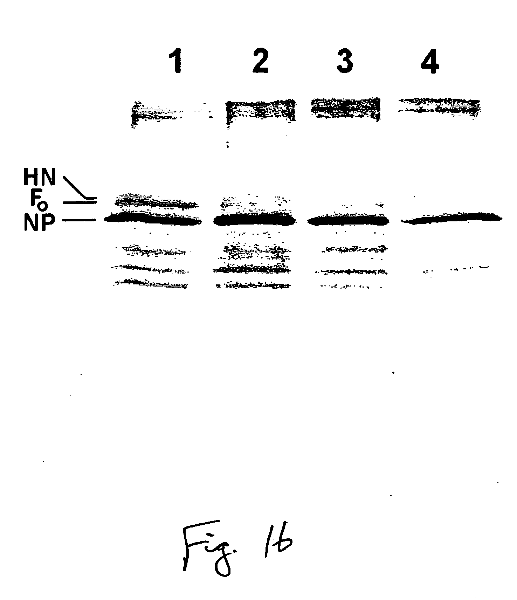

Fig. 16 is a photograph of an autoradiogram following an RIP analysis using four CSF specimens from patients diagnosed with chronic fatigue syndrome (CFS). Lanes 1-3 were assessed as "*Cryptovirus* positive"; Lane 4 assessed as "*Cryptovirus* negative".

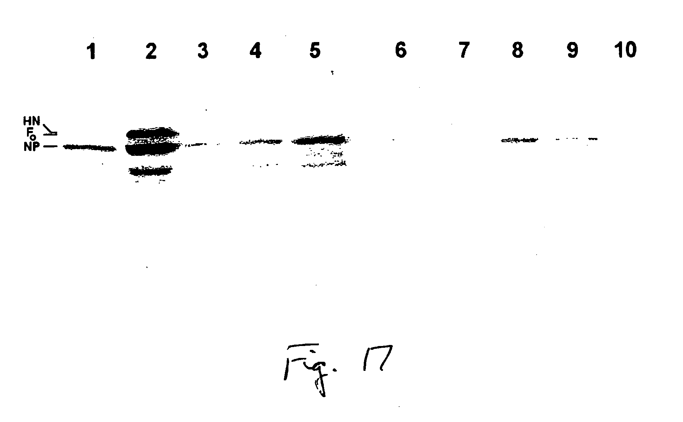

Fig. 17 is a photograph of an autoradiogram following an RIP analysis using CSF samples obtained as "Collection 1" (see hereinbelow). The positive CSF precipitate in Lane 2 was subsequently found to have been obtained from a 55 year-old adult male who presented with ataxia, memory loss, blackouts, seizures, diplopia, and headaches.

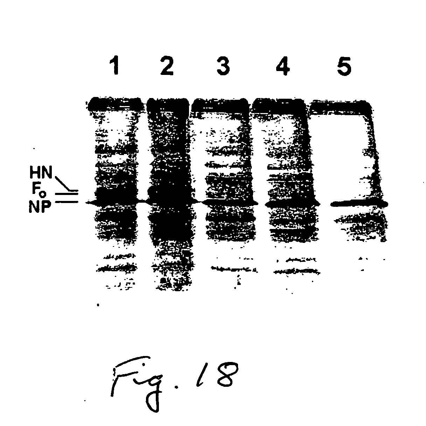

Fig. 18 is a photograph of an autoradiogram following a RIP analysis using CSF samples obtained as "Collection 2" (see hereinbelow).

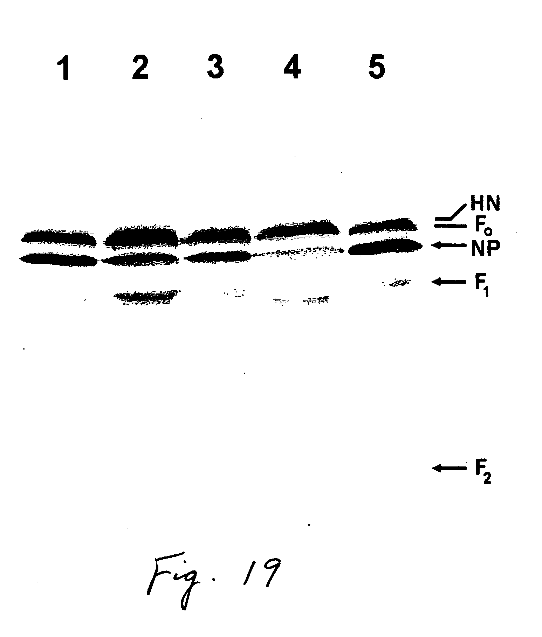

Fig. 19 is a photograph of an autoradiogram following an RIP assay conducted with serum samples obtained from 5 MS patients (out of the 38 samples obtained).

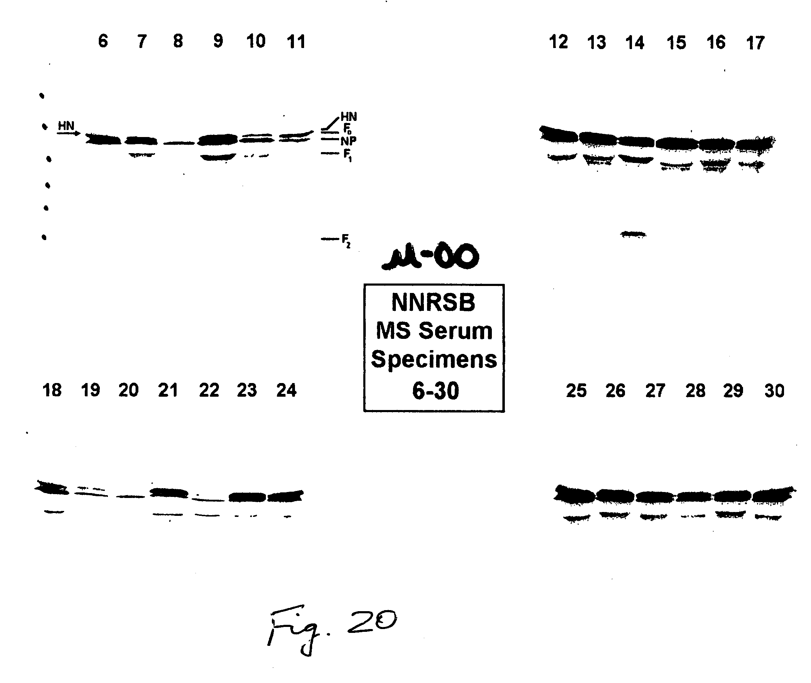

Fig. 20 is a photograph of an autoradiogram following an RIP assay conducted with serum samples from an 25 additional MS patients (out of the 38 samples obtained).

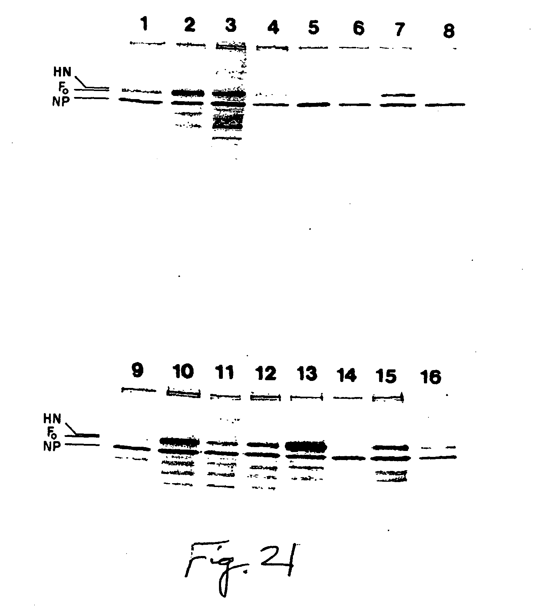

Fig. 21 is a photograph of an autoradiogram following an RIP assay conducted with 16 CSF specimens obtained from 16 MS patients.

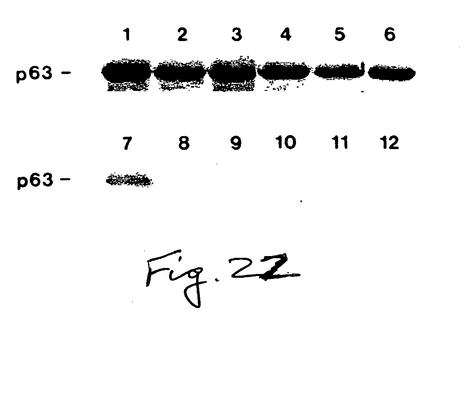

Fig. 22 is a photograph of an autoradiogram obtained following creation of RIP profiles of the *Cryptovirus* NP protein (p63) precipitated from [³⁵S]-methionine-labeled AV₃/SSPE cells by the sera of six Australian SSPE patients (Lanes 1-6) and six control sera (Lanes 7-12; sera from pediatric patients without antibodies to the *Cryptovirus* major envelope proteins (F₀ and HN).
Fig. 23 is a photograph of an autoradiogram of RIP profiles of measles virus-specific proteins or *Cryptovirus*-specific proteins precipitated from [³⁵S]-methionine-labeled measles virus-infected CV-1_{c} cells (Lane MV), *Cryptovirus*-infected CV-1_{c} cells (Lane CV) or a mixture of both (Lane B) by CSF from an 11 year old male diagnosed with SSPE. Lane V = gradient-purified *Cryptovirus* virions from [³⁵S]-methionine-labeled *Cryptovirus*-infected CV-1_{c} cells. L = the largest nucleocapsid associated protein, the major component of the virion-associated RNA dependent RNA polymerase; HN = the hemagglutinin protein, one of the envelope-associated glycoproteins; Fₒ = the uncleaved fusion protein, a second envelope-associated glycoprotein; NP = the nucleocapsid protein, the major structural protein associated with the nucleocapsid; F₁ = the larger fragment of the cleaved fusion protein; P = the nucleocapsid associated phosphoprotein; M = the virion-associated matrix or membrane protein; V = a minor RNA binding protein thought to be a component of the viral polymerase; F₂ = the smaller fragment of the cleaved fusion protein. Note: the SH protein (about 5 kD), a small envelope-associated protein, ran off the gel and is not shown.

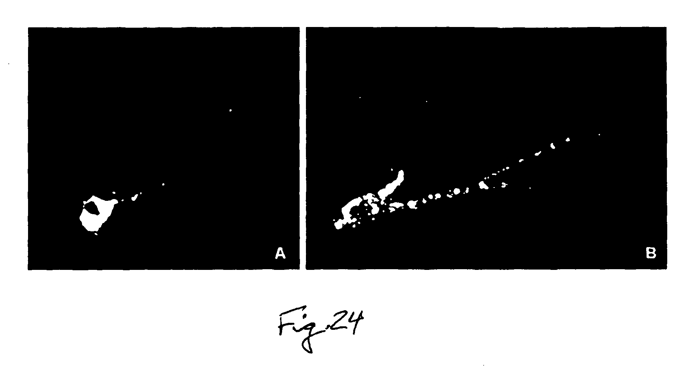

Fig. 24 shows photomicrographs of *Cryptovirus*-infected neurons. Fig. 24A demonstrates *Cryptovirus*-specific immunofluorescence in a single neuron in the brain of a Colored mouse inoculated when two days old with *Cryptovirus Strain BBR* (sacrificed 2 months post inoculation after presenting with seizures). Fig. 24B demonstrates cytoplasmic immunofluorescence in a single neuron from the brain of a guinea pig presenting with a subacute encephalopathy after inoculation with the Niigata-1 strain of SSPE-derived cell-associated virus (detected by an indirect fluorescent antibody technique using SSPE serum)(Doi et al., Japan. J. Med. Sci. Biol. 25:321-333, 1972).

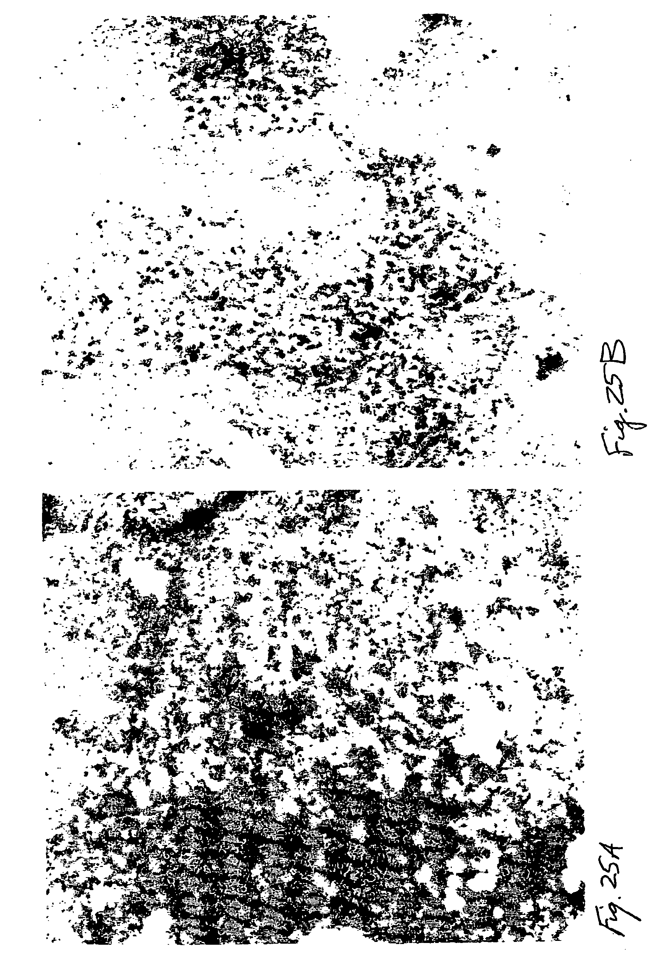

Fig. 25 shows photomicrographs of differential immunogold-labeling of the intracellular nucleocapsids of *Cryptovirus* and measles virus in persistently-infected AV₃/SSPE/MV cells. Fig. 25A shows labeling of the about 15-nm to about 17-nm "smooth" and narrow nucleocapsids of *Cryptovirus* with 10-nm gold beads. The etching technique used results in a loss of resolution of the fine structure of the smooth nucleocapsids making the herringbone pattern somewhat difficult to see. Fig. 25B shows labeling of the 25-nm "fuzzy" and wide nucleocapsids of measles virus with 15-nm gold beads. Magnification is approximately 500,000X.

### DETAILED DESCRIPTION OF THE PREFERRED EMBODIMENTS

The novel virus described herein has been designated a *Cryptovirus* (CV) on the basis of its inapparent, or "cryptic," cytopathology in cultured human cells and its slow and "encrypted" pathogenesis in experimentally infected animals. Given the nucleotide sequence present in the virus, and its structural, biological, and pathogenetic characteristics, *Cryptovirus* fits best within the *Rubulavirus* genus of the family Paramyxoviridae (Fig. 1). More specifically, this virus most closely resembles the viruses known as Canine Parainfluenza Virus Type 2 (which is also known as Canine Parainfluenza Virus, CPI and CPIV) and Porcine Rubulavirus (which is also known as La-Piedad-Michoacan-Mexico Virus and LPMV). (see Fig. 2). The relationships between *Cryptovirus* and these two viruses can be seen in the relationships between their sequences and their structural, biological, and pathogenetic characteristics (see Fig. 3). CPIV shares more than 95% of its nucleotide sequence with *Cryptovirus.* The extent of Menangle virus nucleotide sequence homology with *Cryptovirus* is presently unknown as the sequence of the Menangle virus genome has not yet been published.

There is also an apparent relationship between *Cryptovirus* and: (1) simian parainfluenza virus type 5 (which is also known as simian virus 5 and SV5; see Fig. 4, Fig. 5, Fig. 9, and Fig. 10; here, there is a relationship between the sequences and structural and immunological properties of the viruses but little or no biological or pathogenetic similarity); (2) human mumps virus (here, there are certain structural, biological, and pathogenetic relationships); and (3) human measles virus (here again, there are certain structural, biological and pathogenetic relationships). These relationships help to classify *Cryptovirus* and to establish its novelty.

In addition to having a role in idiopathic and cryptogenic forms of epilepsy or epileptiform disease, i.e., an illness, disorder, or condition having epileptiform symptomology (e.g., CFS, MS, SSPE), *Cryptovirus* is also implicated in a spectrum of idiopathic disorders of the central nervous system (CNS) that present with compulsive or iterative physical, behavioral, or psychological symptoms. The manifestation of symptoms of these disorders as a consequence of *Cryptovirus* infection is exclusively subacute or slow in nature taking weeks, months, or even years to develop. The spectrum of physical symptoms that have been presented by human patients that have been infected with *Cryptovirus* includes febrile response, opthalmological disorders (photosensitivity, blurred vision, nystagmus, loss of vision) parathesias, paralysis, tremor, myoclonus, and *grand mal* and *petit mal* (absence) seizures. The spectrum of behavioral or psychological symptoms that have been presented by patients includes repetitive movements and compulsive behaviors (characteristic of obsessive compulsive disorder), sleep disturbances, memory loss, and dysophoria, anorexia nervosa, autism, mental retardation, affective disorder, dysthymia (clinical depression), schizophrenia, and bipolar disorder.

While not essential features of the present invention, the portal of entry for *Cryptovirus* infection can be the oral mucosa of the throat (*i.e.*, the tracheo-bronchial epithelium), and the virus' incubation period can be of subacute duration (*i.e.*, many days to weeks). Newly infected individuals can develop a febrile pharyngitis and lymphadenopathy of prolonged duration, not unlike infectious mononucleosis. Alternatively, it is thought that the portal of entry for the virus can also be transplacental, so that a mother carrying the virus can transmit it to her child *in utero*, and the child can subsequently develop a neurological, neurodegenerative, and/or neuropsychiatric disease or other developmental disorder (e.g., autism, cerebral palsy, hydrocephalus, birth defect, partial paralysis). Such a child is frequently diagnosed or labelled as "retarded". Indeed, the incidence of epilepsy and seizures are dramatically higher in the severely "mentally-retarded" (as much as 50-fold higher than the general population).

The nucleotide sequence of the human *Cryptovirus* genome (15,246 contiguous nucleotides), in FASTA format (*i.e.*, mRNA sense; 5' to 3'), is shown in Fig. 9 (SEQ ID NO:1). The actual genome of the virus is negative-stranded (antisense to mRNA), having a nucleotide sequence entirely complementary to (SEQ ID NO:1).

Accordingly, the present invention encompasses an isolated human negative-stranded RNA virus that, in FASTA format (i. e. in positive-stranded, mRNA-sense, the reverse and complementary sequence to the actual genome), has the sequence of SEQ ID NO:1. In Fig. 9, nucleotides that vary from those of the W3A strain of Simian Virus 5 are highlighted and the number of variations in each line is tallied in the right margin. The FASTA formatted sequence of human Cryptovirus *Strain BBR* was compared to Simian Virus 5 *Strain W3A* (SEQ ID NO:2; see Fig. 9). Comparisons between various Rubulavirus F Protein amino acid sequences have also been made (Fig 10).

A *Cryptovirus* "particle" is an entire *Cryptovirus* virion, as well as encompassing particles which are intermediates in virion formation (e.g., nucleocapsids), or otherwise partial. *Cryptovirus* particles generally have one or more *Cryptovirus* proteins associated with the *Cryptovirus*-specific nucleic acid they contain. A preferred *Cryptovirus* particle or virion is *Cryptovirus* Strain BBR, which is deposited as ATCC Accession No..

The present invention also relates to a composition of matter comprising the inventive Cryptovirus particle and a carrier.

As used herein a "carrier" can be an organic or an inorganic carrier or excipient, such as water or an aqueous solution, or an emulsion such as an oil/water or water/oil emulsion, and various types of wetting agents. The active ingredient, such as the inventive viral particle, nucleic acid construct, protein, or antibody, can optionally be compounded in a composition formulated, for example, with non-toxic, physiologically acceptable carriers for infusions, tablets, pellets, capsules, solutions, emulsions, suspensions, or in any other formulation suitable for its intended in vitro or in vivo use. Such carriers also include glucose, lactose, gum acacia, gelatin, mannitol, starch paste, magnesium trisilicate, talc, corn starch, keratin, colloidal silica, potato starch, urea, medium chain length triglycerides, dextrans, normal saline, phosphate buffered saline and other carriers suitable for use in manufacturing preparations, in solid, semisolid, or liquid form. In addition auxiliary, stabilizing, thickening and coloring agents and perfumes can be used as appropriate. Other example of suitable carriers are described hereinbelow, but any suitable carrier known in the art is intended.

In the inventive method of isolating the *Cryptovirus* virion, as described hereinabove, the PBMNCs that have been obtained from a human having a *Cryptovirus* infection are cultured in an artificial aqueous medium that includes, importantly, an agent that increases cellular guanylyl cyclase activity.

For purposes of the present invention, the artificial aqueous medium is made by adding the agent that increases cellular guanylyl cyclase activity to a known minimal cell culture medium, such as IMEMZO, MEM, HYQPF Vero (Hyclone), or RPMI, buffered (e.g., with HEPES) to pH 6.8-7.8 and most preferably to pH 6.8-7.2. The agent operates to permit and facilitate the isolation and/or propagation of Cryptovirus in accordance with the invention.

Optionally, fetal calf serum (about 2% v/v to about 10% v/v) is added to the medium. Antibiotics, such as penicillin or streptomycin, in conventional amounts, can also be added to the medium.

It is not essential to the present invention that cellular guanylyl cyclase activity actually be measured. In addition, the present invention is dependent neither upon any particular mechanism by which the agent may actually operate to increase cellular guanylyl cyclase activity (or not), nor upon any mechanism by which the agent operates to permit and/or facilitate the isolation and/or propagation of Cryptovirus in accordance with the invention.

Useful examples of the agent that increases cellular guanylyl cyclase activity include most preferably guanosine 3',5'-cyclic monophosphate ("cyclic GMP") (free acid, or preferably, a pharmaceutically acceptable salt thereof, such as a sodium, potassium, magnesium, calcium, or ammonium salt, or the like), insulin (preferably human insulin), zinc dication (preferably provided in a chloride, sulfate, carbonate, bicarbonate, nitrate, acetate, or other pharmaceutically acceptable salt thereof), or a combination of any or all of these.

Preferably, the cyclic GMP is used in a concentration of about 0.05 to about 5 mM in the artificial aqueous medium. More preferably, the cyclic GMP concentration in the medium is about 0.5 to about 2.5 mM, and most preferably about 0.75 mM to about 1.25 mM. A concentration above about 5 mM cyclic GMP is not optimally conducive to cultivating, propagating, or isolating *Cryptovirus.*

A preferred concentration range for insulin in the artificial aqueous medium is about 1 to about 10 mg/L, more preferably about 2 to about 6 mg/L, and most preferably about 3 to about 5 mg/L.

A preferred concentration range for zinc dication in the artificial aqueous medium is equivalent to about 0.05 to about 0.25 mg/L of ZnSO₄·7H₂O, more preferably equivalent to about 0.10 to about 0.20 mg/L of ZnSO₄·7H₂O, or most preferably equivalent to about 0.13 to about 0.15 mg/L of ZnSO₄·7H₂O.

Alternatively, in some embodiments, the agent that increases cellular guanylyl cyclase activity is nitric oxide or a nitric oxide donor. Nitric oxide gas is fully permeable across biological membranes. Inhalable nitric oxide gas can be administered to a mammalian subject by, for example, a mask in a controlled gas mixture as is known in the art. (E.g., Kieler-Jensen, N. et al., Inhaled nitric oxide in the evaluation of heart transplant candidates with elevated pulmonary vascular resistance, J Heart Lung Transplant. 13(3):366-75 [1994]; Rajek, A. et al., Inhaled nitric oxide reduces pulmonary vascular resistance more than prostaglandin E(1) during heart transplantation, Anesth Analg. 90(3):523-30 [2000]; Solina, A. et al., A comparison of inhaled nitric oxide and milrinone for the treatment of pulmonary hypertension in adult cardiac surgery patients, J Cardiothorac Vasc. Anesth. 14(1):12-17 [2000]; Fullerton, D.A. et al., Effective control of pulmonary vascular resistance with inhaled nitric oxide after cardiac operation, J Thorac Cardiovasc Surg 111(4):753-62, discussion 762-3 [1996]). The concentration in the gas mixture of nitric oxide (NO) is preferably about 1 to 100 ppm NO, more preferably about 4 to 80 ppm NO, and most preferably about 20 to 40 ppm NO. The gas mixture also contains appropriate concentrations of oxygen and nitrogen and/or other inert gases, such as carbon dioxide, helium or argon.

Nitric oxide donors are compounds that produce NO-related physiological activity when applied to biological systems. Thus, NO-donors can mimic an endogenous NO-related response or substitute for an endogenous NO deficiency. The skilled artisan is aware that in biological systems there are at least three redox states of NO that can be released by various NO donors (NO⁺, NO⁰, or NO⁻), all of which are encompassed by the terms "nitric oxide" or "NO" for purposes of the present invention. The redox state of NO makes a substantial difference to the NO donors reactivity towards other biomolecules, the profile of by-products, and the bioresponse (Feelisch, M., *The use of nitric oxide donors in pharmacological studies,* Naunyn-Schmiedebergs Arch. Pharmacol.358:113-22 [1998]). Some classes of NO donors require enzymatic catalysis, while others produce NO non-enzymatically; some NO donors require reduction, for example by thiols, and some oxidation, in order to release NO.

Preferred examples of nitric oxide donors include organic nitrate compounds, which are nitric acid esters of mono- and polyhydric alcohols. Typically, these have low water solubility, and stock solutions are prepared in ethanol or dimethyl sulfoxide (DMSO). Examples are glyceryl trinitrate (GTN) or nitroglycerin (NTG), pentaerythrityl tetranitrate (PETN), isosorbide dinitrate (ISDN), and isosorbide 5-mononitrate (IS-5-N). Administration of organic nitrates can be done intravenously, intraperitoneally, intramuscularly, transdermally, or in the case of PETN, ISDN, NTG, and IS-5-N, orally.

Other preferred examples of nitric oxide donors are S-nitrosothiol compounds, including S-nitroso-N-acetyl-D,L-penicillamine (SNAP), S-nitrosoglutathione (SNOG), S-nitrosoalbumin, S-nitrosocysteine. S-nitrosothiol compounds are particularly light-sensitive, but stock solutions kept on ice and in the dark are stable for several hours, and chelators such as EDTA can be added to stock solutions to enhance stability. Administration is preferably by an intravenous or intra-arterial delivery route.

Other preferred examples of nitric oxide donors include sydnonimine compounds, such as molsidomine (N-ethoxycarbonyl-3-morpholino-sydnonimine), linsidomine (SIN-1; 3-morpholino-sydnonimine or 3-morpholinylsydnoneimine or 5-amino-3morpholinyl-1,2,3-oxadiazolium, e.g., chloride salt), and pirsidomine (CAS 936). Stock solutions are typically prepared in DMSO or DMF, and are stable at 4°C to room temperature, if protected from light. Linsidomine is highly water soluble and stable in acidic solution in deoxygenated distilled water, adjusted to about pH 5, for an entire day. At physiological pH, SIN-1 undergoes rapid non-enzymatic hydrolysis to the open ring form SIN-1A, also a preferred nitric oxide donor, which is stable at pH 7.4 in the dark. Administration is preferably by an intravenous or intra-arterial delivery route.

Also useful as nitric oxide donors are iron nitrosyl compounds, such as sodium nitroprusside (SNP; sodium pentacyanonitrosyl ferrate(II)). Aqueous stock solutions are preferably made freshly in deoxygenated water before use and kept in the dark; stability of stock solutions is enhanced at pH 3-5. Inclusion in the delivery buffer of a physiologically compatible thiol, such as glutathione, can enhance release of NO. SNP is administered by intravenous infusion, and the skilled practitioner is aware that long-term use is precluded by the release of five equivalents of toxic CN-per mole SNP as NO is released.

A most preferred nitric oxide donor is chosen from among the so-called NONOate compounds. The NONOates are adducts of NO with nucleophilic residues (X⁻), such as an amine or sulfite group, in which an NO dimer is bound to the nucleophilic residue via a nitrogen atom to form a functional group of the structure X[-N(O)NO]⁻. The NONOates typically release NO at predictable rates largely unaffected by biological reactants, and NO release is thought to be by acid-catalyzed dissociation with the regeneration of X⁻ and NO.

NONOates include most preferably diethylamine-NONOate (DEA/NO; N-Ethylethanamine: 1, 1-Diethyl-2-hydroxy-2-nitrosohydrazine (1:1) or 1-[N,N-diethylamino]diazen-1-ium-1,2-diolate). Other preferred NONOates include diethylene triamine-NONOate(DETA/NO; 2,2'-Hydroxynitrosohydrazino]bis-ethanamine), spermine-NONOate (SPER/NO; N-(4-[-1-(3-Aminopropyl)-2-hydroxy-2-nitrosohydrazino] butyl)-1,3-propanediamine), propylamino-propylamine-NONOate (PAPA/NO; 3-(2-Hydroxy-2-nitroso-1-propylhydrazino)-1-propanamine or (Z)-1-[N-(3-aminopropyl)-N-(n-propyl)amino]diazen-1-ium-1,2-diolate), MAHMA-NONOate (MAHMA/NO; 6-(2-Hydroxy-1-methyl-2- nitrosohydrazino)-N-methyl-1-hexanamine), dipropylenetriamine-NONOate (DPTA/NO; 3,3'- (Hydroxynitrosohydrazino)bis-1-propanamine), PIPERAZI/NO, proli-NONOate (PROLI/NO; 1-([2-carboxylato]pyrrolidin-1-yl)diazen-1-ium-1,2-diolate-methanol, e.g., disodium salt), SULFO-NONOate (SULFO/NO; hydroxydiazenesulfonic acid 1-oxide, e.g., diammonium salt), the sulfite NONOate (SULFI/NO), and Angelis salt (OXI/NO).

Almost all NONOate compounds are highly soluble in water, and aqueous stock solutions are prepared in cold deoxygenated 1 to 10 mM NaOH (preferably about pH 12) just prior to use. Alkaline stock solutions are stable for several hours if kept on ice in the dark. The characteristic UV absorbance of NONOates can be used for spectrophotometric quantification of NONOate in aqueous solutions. NONOates are preferably administered intravenously or intra-arterially.

Nitric oxide donors have different potencies (Ferraro, R. et al., Comparative effects of several nitric oxide donors on intracellular cyclic GMP levels in bovine chromaffin cells: correlation with nitric oxide production, Br. J. Pharmacol. 127(3):779-87 [1999]). For example, DEA/NO is among the most potent nitric oxide donors, with a half-life of about 2 to 4 minutes; less potent are PAPA/NO (t)_{1/2} about 15 minutes),SPER/NO (t_{1/2} about 34-40 minutes); even less potent are DETA/NO (t_{1/2} about 20 hours) and SNAP (t_{1/2} about 33 to 41 hours, although this can be shortened in the presence of a physiological reductant such as glutathione). SNP is also a potent NO donor. (See, Ferrero *et al.* [1999]; Salom, J.B. et al., Relaxant effects of sodium nitroprusside and NONOates in rabbit basilary artery, Phannacol. 57(2):79-87 [1998]; Salom, J.B. et al., Comparative relaxant effects of the NO donors sodium nitroprusside, DEA/NO and SPER/NO in rabbit carotid arteries, Gen. Pharmacol. 32(1):75-79 [1999]; Salom, J.B. et al., Relaxant effects of sodium nitroprusside and NONOates in goat middle cerebral artery: delayed impairment by global ischemia-reperfusion, Nitric Oxide 3(1):85-93 [1999]; Kimura, M. et al., Responses ofhuman basilar and other isolated arteries to novel nitric oxide donors, J. Cardiovac. Pharmacol. 32(5):695-701 [1998]). Consequently, effective concentrations or doses of NONOates or other NO donors will vary, but can be determined by routine screening.

Stock solutions of NO donors are preferably made up freshly before use (at the appropriate pH for each particular NO donor), chilled on ice, and protected from light (e.g., by the use of darkened glass vials wrapped in aluminum foil), although organic nitrates can be stored for months to years if the vial is properly sealed. Preferably, immediately before administration to the subject, final dilutions are prepared in pharmaceutically acceptable buffer and the final pH of the NO donor-containing buffer is checked for physiological suitability, especially when strongly acidic (e.g., hydrochloride salts) or alkaline (e.g., NONOates) stock solutions are used.

The product of NO exposure time and NO concentration largely determines the quality and magnitude of the biological response to exogenously supplied NO. Short-lived NO donors, such as DEA/NO, are most preferably administered by continuous infusion rather than by bolus to avoid delivering only a short burst of NO.

In accordance with the invention, the artificial aqueous medium preferably, but not necessarily, further includes glutamine at a preferred concentration of about 0.5 to about 5 mM concentration. A more preferred concentration of glutamine in the medium is about 1 to about 3 mM.

In the inventive methods of isolating a *Cryptovirus* virion and of producing a mammalian cell line nonproductively infected with *Cryptovirus,* the PBMNCs are co-cultured with mammalian amnion cells in the artificial aqueous medium, as described above.

Examples of useful mammalian cells include, but are not limited to, rodent, lagomorph, primate, ovine, bovine, canine, feline or porcine cells.

In accordance with the present invention, one preferred embodiment is a primate cell, i.e., a cell originating from a primate source. A primate is a member of the mammalian order Primates, including lemurs, tarsiers, monkeys (e.g., African Green Monkeys, colobus monkeys, and baboons), apes (e.g., chimpanzees, gorillas, orangutans, and gibbons), and humans.

An amnion cell is a cultured cell originally derived from an amniotic membrane or amniotic sac.

A preferred primate amnion cell is a human amnion cell, e.g., AV₃.

An example of the inventive cell nonproductively infected by the method is AV₃/SSPE, which is deposited as ATCC Accession No..

In these inventive methods, passaging of a co-culture of PBMNCs and mammalian amnion cells is done one or more times. Passaging of cultured cells into fresh culture medium (culture medium as described above), is typically done about twice per week. Preferably, at least about two to about 12 passages are done in accordance with the methods. Typically after about eight to twelve passages of the co-culture, virtually all mammalian amnion cells are nonproductively infected with *Cryptovirus.* Generally, within about two to about three passages, the PBMNCs have disappeared from the culture, leaving the mammalian amnion cells.

In accordance with the present invention, a mammalian epithelial cell is a cultured cell originally derived from a mammalian epithelial tissue. In one preferred embodiment, the mammalian epithelil cell is a rodent epithelial cell, such as baby hamster kidney (BHK) cells. In another preferred embodiment, the mammalian epithelial cell is a simian epithelial cell, for example a Vero or a CV-1 cell. Most preferably the CV-1 cell is subline CV-1_{c}, which is deposited as ATCC Accession No.

An example of a primate epithelial cell acutely infected with *Cryptovirus,* in accordance with the method of propagating a *Cryptovirus* is deposited as ATCC Accession No..

In addition to the sequence information provided herein to identify the inventive *Cryptovirus* particle, the inventive *Cryptovirus* and its viral subcomponents can be, and have been, characterized by numerous virological, biochemical, and molecular techniques, including the following, by way of example:

*Plaque Titration Assay:* Formation of macroscopically visible plaques on monolayers of mammalian epithelial cells (e.g., BHK, Vero or CV-1_{c}) can be used to quantitate preparations of infectious *Cryptovirus* (Robbins et al., J. Infect. Disease 143:396-403, 1981).

*Neutralization Titration Assay:* Plaque formation can be inhibited by serial dilutions of clinical serum specimens and *Cryptovirus-specific* antisera generated in rabbits (*see* e.g., Robbins et al., J. Infect. Disease 143:396-403, 1981). Neutralization titration assays are routinely used in medical virological research to demonstrate that a given patient has neutralizing antibodies to a particular virus. A neutralization assay can be used diagnostically for the presence or absence of neutralizing antibody to *Cryptovirus.* In a typical example of a neutralization assay, serial dilutions of a biological material, such as a sample of serum or CSF to be tested, are typically incubated for about one hour at 4°C with sufficient infectious virus to yield a net plating concentration of between about 100-200 plaque forming units of the virus per 0.2 mL of final diluent (including the diluted serum or CSF). After incubation, about 0.2 mL of the diluted virus-serum (or CSF) mixtures are then typically plated onto monolayers of susceptible cells (*e.g.* Vero or CV-1) and the cells are incubated at 37°C in a partial CO₂ atmosphere (e.g., 5% v/v) (typically, with redistribution of the inoculum every 15 minutes). At the end of the incubation period, inoculated monolayers are typically overlayed with sufficient volumes of a 2% (w/v) solution of carboxymethylcellulose in an artificial aqueous cell culture medium (e.g., IMEMZO medium, buffered at pH between about 6.8 and about 7.4, typically containing fetal calf serum, and a suitable quantity of antibiotic, such as about 200 Units penicillin / mL and/or 100 µg streptomycin / mL) to last 10-12 days (*i.e.*, enough volume so that the monolayers won't dry out). Optimally, the plates must not be moved during the incubation period. After 10-12 days, the overlay is aspirated and the cells are fixed with formalin fixative and stained with a protein stain (*e.g.*, Giemsa). The number of plaques formed on each plate is then enumerated and the PRD₅₀ calculated (PRD₅₀ = the Plaque Reduction Dilution; the dilution of serum or CSF at which a 50% reduction in the number of plaques formed on controls [tubes containing virus and saline only] is observed).

*Density Gradient Purification*: Virions and intracellular nucleocapsids from productively-(e.g., Vero and CV-1_{c}) and nonproductively-infected (e.g., AV₃/SSPE) cells can be purified on sucrose-potassium tartrate gradients (virions) and CsCl gradients (nucleocapsids) (see Robbins et al., J. Infect. Disease 143:396-403, 1981; Rapp and Robbins, Intervirology 16:160-167, 1981; Robbins and Rapp, Arch. Virol. 71:85-91, 1982; and Robbins and Abbott-Smith, J. Virol. Meth. 11:253-257, 1985).

*Electron Microscopy:* Electron microscopy, such as transmission or scanning electron microscopy are useful for examining the inventive *Cryptovirus* virion. When examined by electron microscopy, the *Cryptovirus* has been shown to have a morphology and ultrastructure consistent with other members of the Paramyxoviridae (*i.e*., enveloped pleomorphic virions, about 100 nm to about 120 nm in diameter, containing helical nucleocapsids). Intracellular inclusions of the virus in thin sections of productively- (e.g., Vero and CV-1_{c}) and nonproductively-infected cells (e.g., AV₃/SSPE) have also been shown to be comprised of aggregates of filamentous structures with dimensions similar to the nucleocapsids of other members of the Paramyxoviridae (*i.e*., helical herringbone-like structures, about 15 to about 17 nm in diameter) (see Robbins et al., J. Infect. Disease 143:396-403, 1981 and Robbins and Rapp, Arch. Virol. 71:85-91, 1982).

*Radioimmunoprecipitation* (RIP) *Assay:* Extensive data has been generated by the comparative analysis of *Cryptovirus-specific* immunoprecipitates of [³⁵S]-methionine-labeled uninfected, nonproductively- and productively-infected mammalian cells by sodium dodecyl sulfate polyacrylamide gel electrophoresis (SDS-PAGE; see below).

*SDS-PAGE:* Purified virions and cytoplasmic nucleocapsids of the virus have been analyzed by SDS-PAGE under reducing and non-reducing conditions (see Fig. 11 and Fig. 12), an autoradiogram of gradient-purified [³⁵S]-methionine-labeled *Cryptovirus* virions produced in acutely-infected Vero cells after SDS-PAGE under reducing conditions. The approximate molecular weights of the proteins indicated on the right side of Fig. 11 were calculated by comparing their migrations to marker proteins of known molecular weights (Sigma Chemical Co., St. Louis, MO). The SH protein, a small envelope-associated protein having a MW of about 5 kD, is not shown in Fig. 11, because it has run off the gel.

*Immuno-ultrastructural Analysis (Immunogold Analysis).* The intracellular nucleocapsids of nonproductively and productively-infected mammalian cells are typically localized under the electron microscope using *Cryptovirus-specific* or *Cryptovirus* nucleocapsid-specific antibodies, for example hyperimmune rabbit antibodies, and an indirect immunogold labeling technique (discussed hereinbelow).

The preceding are merely illustrative, and not an exhaustive list, of the known techniques typically useful for characterizing the isolated *Cryptovirus* virion of the present invention. Additional conventional techniques, or virological techniques yet to be discovered, can also be employed to further characterize the inventive *Cryptovirus* virion.

Additional characteristics of *Cryptovirus* include the following:
*Latency and Persistence. Cryptovirus* latently and persistently infects human peripheral blood mononuclear cells (PBMNCs). No other member of the Paramyxoviridae has been shown to do this. The evidence for infection includes: (1) detection of *Cryptovirus-specific* proteins by an indirect immunofluorescent antibody technique in PBMNCs following *in vitro* cultivation and induction with mitogens and/or cyclic GMP, (2) recovery of the virus from PBMNCs by serial cocultivation with mammalian cells (see Robbins et al., J. Infect. Dis. 143:396-403, 1981) and (3) the ability to repeatedly recover the virus from PBMNCs drawn from an SSPE patient over a period of 18 months.
*Defective Fusion Activity.* Cell fusion, which is a hallmark of the Paramyxoviridae, is either defective or extremely limited in experimental *Cryptovirus* infections *in vitro* (*i.e.,* in dysgenic nonproductive infections of human amnion cells (AV₃) and productive infections of monkey kidney cells (e.g., Vero and CV-1_{c}; *see* Robbins et al., J. Infect. Dis. 143:396-403, 1981, and Robbins and Rapp, Arch. Virol. 71:85-91, 1982).
*Restricted Expression in Latently-Infected Cells. Cryptovirus-specific* protein expression is dysgenic in experimental nonproductive latent infections of mammalian amnion cells (e.g., human AV₃ cells). This restriction involves severely decreased expression, or non-expression, of the virus-encoded envelope proteins (F, HN and SH) (see Robbins and Rapp, Arch. Virol. 71:85-91, 1982).
*B Cell Lymphotropism. Cryptovirus* demonstrates a tropism for B cells, and can be harbored by such cells *in situ.* This has been demonstrated by successfully infecting EBV-transformed B cell lines from human donors with the virus (*i.e*., by detecting the progressive formation of *Cryptovirus-*specific inclusion bodies in the cytoplasm of experimentally-infected EBV-transformed B cell lines by *Cryptnvirus-*specific immunofluorescence). In contrast, *Cryptovirus-specific* proteins could not be detected in an experimentally-infected human T cell line, CCRF-CEM. Accordingly, *Cryptovirus* can reside in B cells in infected individuals.
*Neurotropism. Cryptovirus* also demonstrates a clear tropism for neurons in mice following intracerebral inoculation of neonatal animals (as detected by *Cryptovirus-specific* immunofluorescence). It is less clear whether other nervous system tissues are infected. While neurotropism, itself, may not be unique to *Cryptovirus* when compared and contrasted to other human members of the Paramyxoviridae (*e.g.*, Measles Virus, Mumps Virus), some of the neuropathological consequences of *Cryptovirus* infection of CNS tissues (including neurons) appear to be (see below).
*Hind Limb Atrophy and Paralysis.* Hind limb paralysis and atrophy were seen in approximately 33% of Quackenbush mice intracerebrally-inoculated with *Cryptovirus* as newborns. In addition, hind limb atrophy and paralysis was observed in some of the offspring of adult female Quackenbush mice that had been inoculated with *Cryptovirus* as newborns but did not develop any overt symptomology. The frequency of the symptoms appearing in the latter situation was difficult to assess because the mothers tended to cannibalize the newborn animals that were born with, or subsequently developed, such characteristics.
*Subacute*/*Slow Encephalopathic and Epileptigenic Potential.* Approximately 30% of neonatal Colored mice that were inoculated with infectious *Cryptovirus* preparations went on to develop subacute/slow encephalopathic and/or epileptiform illness (the specific symptoms displayed by such animals are described below). The number of animals that actually developed encephalopathic and/or epileptiform illness was likely higher than 30%, because a number of previously asymptomatic animals were found dead in their cages in clonic postures (a symptom associated with death following or during intractable seizure). On at least two occasions, this occurred in animals after they had suffered from recurrent seizures the day before. The animals that developed such illnesses were predominantly male (approximately 2:1, male: female).
*Slow Psychopathogenic Potential.* Of the Colored mice that survived intracerebral inoculation with infectious *Cryptovirus* preparations as newborns and did not develop epileptiform illness as adolescent or young adult animals, approximately 30% went on to develop profound physical and behavioral abnormalities as adults. The abnormalities displayed by these animals are described hereinbelow. Sudden death was not seen in this group of animals. The animals which developed such symptoms were predominantly female (approximately 3:1, female: male).

The preceding are merely illustrative, and are not an exhaustive list, of some of the observable properties of the inventive *Cryptovirus* particle.

The present invention also relates to isolated nucleic acids and isolated proteins that are *"Cryptovirus-specific,"* i.e., unique to *Cryptovirus.*

A *Cryptovirus-specific* nucleic acid segment or protein is determined by sequence similarity or homology to known sequences of bases or amino acids, respectively, for example other rubulavirus nucleic acid or protein sequences. Base and amino acid sequence homology is determined by conducting a sequence similarity search of a genomics/proteomics data base, such as the GenBank database of the National Center for Biotechnology Information (NCBI; www.ncbi.nlm.nih.gov/BLAST/), using a computerized algorithm, such as PowerBLAST, QBLAST, PSI-BLAST, PHI-BLAST, gapped or ungapped BLAST, or the "Align" program through the Baylor College of Medicine server (www.hgsc.bcm.tmc.edu/seq_data). (E.g., Altchul, S.F., et al., Gapped BLAST and PSI-BLAST: a new generation of protein database search programs, Nucleic Acids Res. 25(17):3389-402 [1997]; Zhang, J., & Madden, T.L., PowerBLAST: a new network BLAST application for interactive or automated sequence analysis and annotation, Genome Res. 7(6):649-56 [1997]; Madden, T.L., et al., Applications of network BLAST server, Methods Enzymol. 266:131-41 [1996]; Altschul, S.F., et al., Basic local alignment search tool, J. Mol. Biol. 215(3):403-10 [1990]).

For purposes of the present invention the term "isolated" encompasses "purified". Thus, an isolated nucleic acid, protein, viral particle, or antibody that is further purified to a greater level of homogeneity, is also "isolated."

For purposes of the present invention the term "nucleic acid" includes a polynucleotide, of any length, either polymeric ribonucleotides (RNA) or polymeric deoxyribonucleotides (DNA), such as cDNA.

The term "isolated nucleic acid" refers to a *Cryptovirus* genomic RNA which is essentially free, i.e., contains less than about 50%, preferably less than about 70%, and even more preferably less than about 90% of the polypeptides with which the *Cryptovirus* genome is naturally associated. Alternatively, an "isolated" nucleic acid of the present invention is a *Cryptovirus-specific* "recombinant polynucleotide", which as used herein intends a polynucleotide of genomic RNA, sense RNA (i.e., mRNA sense), cDNA, semisynthetic, or synthetic origin, which, by virtue of its origin or manipulation: (1) is not associated with all or a portion of a polynucleotide with which it is associated in nature, (2) is linked to a polynucleotide other than that to which it is linked in nature, or (3) does not occur in nature. The inventive nucleic acid can be in a sense or antisense orientation.

As used herein, the "sense strand" of a nucleic acid contains the sequence that has sequence homology to that of mRNA. The "anti-sense strand" contains a sequence which is complementary to that of the "sense strand." Inventive nucleic acids also include double- and single-stranded DNA and RNA.

Techniques for purifying viral polynucleotides from viral particles are known in the art, and include for example, disruption of the particle with a chaotropic agent, differential extraction and separation of the polynucleotide(s) and polypeptides by ion-exchange chromatography, affinity chromatography, and sedimentation according to density.

Inventive nucleic acids also encompass polynucleotides with known types of modifications, for example, labels, methylation, "caps", substitution of one or more of the naturally occurring nucleotides with a nucleotide analog, internucleotide modifications such as, for example, polynucleotides with uncharged linkages (e.g., methyl phosphonates, phosphotriesters, phosphoamidates, carbamates, etc.) and with charged linkages (e.g., phosphorothioates, phosphorodithioates, etc.), those containing pendant moieties, such as, for example proteins (including for e.g., nucleases, toxins, antibodies, signal peptides, poly-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide.

A "nucleic acid segment" is a polynucleotide subportion of a larger nucleic acid.

A nucleotide sequence complementary to an inventive *Cryptovirus-specific* nucleotide sequence, as used herein, is one binding specifically or hybridizing with a *Cryptovirus-specific* nucleotide base sequence. The phrase "binding specifically" or "hybridizing" encompasses the ability of a polynucleotide sequence to recognize a complementary base sequence and to form double-helical segments therewith via the formation of hydrogen bonds between the complementary base pairs. Thus, a complementary sequence includes, for example, an antisense sequence with respect to a sense sequence or coding sequence. As known to those of skill in the art, the stability of hybrids is reflected in the melting temperature (Tₘ) of the hybrids. In general, the stability of a hybrid is a function of sodium ion concentration and temperature. Typically, the hybridization reaction is performed under conditions of relatively low stringency, followed by washes of varying, but higher, stringency. Reference to hybridization stringency relates to such washing conditions.

As used herein, the phrase "moderately stringent hybridization" refers to conditions that permit target-RNA or DNA to bind a complementary nucleic acid that has at least about 95%, about 96%, about 97%, about 98%, about 99%, or about 100% identity to the target RNA segment or DNA segment. Preferably, moderately stringent conditions are conditions approximately equivalent in stringency to hybridization in 50% formamide, 5 x Denhart's solution, 5 x SSPE, 0.2% SDS at 42°C, followed by washing in 0.2 x SSPE, 0.2% SDS, at 65°C.

The phrase "high stringency hybridization" refers to conditions that permit hybridization of only those nucleic acid sequences that form stable hybrids in 0.018 M NaCl at 65°C (i.e., if a hybrid is not stable in 0.018 M NaCl at 65°C, it will not be stable under high stringency conditions, as contemplated herein). High stringency conditions can be provided, for example, by hybridization in 50% formamide, 5X Denhart's solution, 5X SSPE, 0.2% SDS at 42°C, followed by washing in 0.1 x SSPE, and 0.1% SDS at 65°C.

The phrase "low stringency hybridization" typically refers to conditions equivalent to hybridization in 10% formamide, 5X Denhart's solution, 6 x SSPE, 0.2% SDS at 42°C, followed by washing in 1 x SSPE, 0.2% SDS, at 50°C. Denhart's solution and SSPE (see, e.g., Sambrook et al., Molecular Cloning, A Laboratory Manual, Cold Spring Harbor Laboratory Press [1989]) are well known to those of skill in the art, as are other suitable hybridization buffers.

The inventive nucleic acids include a *Cryptovirus*-specific nucleic acid fragment at least about five contiguous nucleotides long, and up to 15245 contiguous nucleotides long, of SEQ ID NO:1, or a complementary sequence.

Thus, useful fragments include nucleic acid segments consisting of an open reading frame of the Cryptovirus genome or a complementary sequence. An "open reading frame" (ORF) is a region of a polynucleotide sequence which encodes a polypeptide; this region may represent a portion of a coding sequence or an entire coding sequence.

A "coding sequence" is a polynucleotide sequence which is transcribed into mRNA and/or translated into a polypeptide when placed under the control of appropriate regulatory sequences. The boundaries of the coding sequence are determined by a translation start codon at the 5'-terminus and a translation stop codon at the 3'-terminus. A coding sequence can include, but is not limited to, mRNA, cDNA, and recombinant polynucleotide sequences.

Useful examples of the fragment include nucleic acid segments that encode *Cryptovirus* proteins, such as (i) contiguous nucleotide positions 152-1678 of (SEQ ID NO:1)(also designated [SEQ ID NO:3]); (ii) contiguous nucleotide positions 1850-2515 of (SEQ ID NO:1)(also designated [SEQ ID NO:5]); (iii) contiguous nucleotide positions 1850-3023 of (SEQ ID NO:1)(also designated [SEQ ID NO:33]); (iv) contiguous nucleotide positions 1850-3023 of (SEQ ID NO:1) combined with a further insertion of two guanine (G) residues between nucleotide position 2339 of (SEQ ID NO:1) and nucleotide position 2340 of (SEQ ID NO:1)(the combined sequence including the "GG" insertion being designated [SEQ ID NO:7]); (v) contiguous nucleotide positions 3141-4271 of (SEQ ID NO:1)(also designated [SEQ ID NO:9]); (vi) contiguous nucleotide positions 4530-6182 of (SEQ ID NO:1)(also designated [SEQ ID NO:11]); (vii) contiguous nucleotide positions 4587-6182 of (SEQ ID NO:1)(also designated [SEQ ID NO:13]); (viii) contiguous nucleotide positions 4587-4835 of (SEQ ID NO:1)(also designated [SEQ ID NO:15]); (ix) contiguous nucleotide positions 4836-6182 of (SEQ ID NO:1)(also designated [SEQ ID NO: 17]); (x) contiguous nucleotide positions 4272-6515 of (SEQ ID NO:1)(also designated [SEQ ID NO:34]); (xi) contiguous nucleotide positions 6303-6434 of (SEQ ID NO:1)(also designated [SEQ ID NO:19]); (xii) contiguous nucleotide positions 6584-8278 of (SEQ ID NO:1)(also designated [SEQ ID NO:21]); or (xiii) contiguous nucleotide positions 8414-15178 of (SEQ ID NO:1)(also designated [SEQ ID NO:23]). A nucleotide complementary to any of (SEQ ID NOS:3, 5, 7, 9, 11, 3, 15, 17, 19, 21, 23, 33, or 34), or a degenerate coding sequence of any of (SEQ ID NOS:3, 5, 7, 9, 11, 13, 15, 17, 19, 21, 23, 33, or 34) is also encompassed by the nucleic acid fragment.

As used herein, the term "degenerate coding sequence", or interchangeably, "degenerate sequence", refers to a protein-encoding nucleic acid sequence that has at least one codon that differs in at least one nucleotide position from any reference nucleic acid, e.g., any of SEQ ID NO:3, 5, 7, 9, 11, 13, 15, 17, 19, 21, or 23, but which encodes the same amino acids as the reference nucleic acid. For example, codons specified by the triplets "UCU", "UCC", "UCA", and "UCG" are degenerate with respect to each other since all four of these codons encode the amino acid serine.

In some embodiments, the *Cryptovirus-specific* fragment is up to about 500 nucleotides long. In other embodiments, the fragment is up to about 50 nucleotides long. Other embodiments of the inventive nucleic acid fragment are about fifteen nucleotides to about 35 nucleotides long; for example, this is a preferred length for a *Cryptovirus-specific* primer of the present invention, which is a *Cryptovirus-specific* oligonucleotide for use in nucleic acid amplification reactions. Most preferably, the inventive *Cryptovirus-specific* primer is about 17 to about 22 nucleotides long, but primers as short as 7 contiguous nucleotides may be useful for some gene-specific sequences. (E.g., Vincent, J., et al., Oligonucleonucleotides as short as 7-mers can be used for PCR amplification, DNA Cell Biol. 13(1):75-82 [1994]).

The inventive probe is preferably about 7 to about 500 nucleotides long, most preferably about 15 to about 150 nucleotides long, and comprises, for at least part of its length, a *Cryptovirus-*specific nucleotide sequence at least 7 to 15 nucleotides long, such that the probe hybridizes to a *Cryptovirus-specific* single stranded nucleic acid under suitably stringent hybridization conditions. For example, probes comprising the inventive oligonucleotide primer sequences described herein can be labeled for use as probes for detecting or analyzing *Cryptovirus-specific* nucleic acid, including nucleic acid amplification products.

Non-limiting examples of the *Cryptovirus-specific* fragments useful as primers or probes include nucleic acids comprising: contiguous nucleotide positions 1684-1701 of SEQ ID NO:1 (designated SEQ ID NO:35); contiguous nucleotide positions 1700-1717 of SEQ ID NO:1 (designated SEQ ID NO:36); contiguous nucleotide positions 4283-4300 of SEQ ID NO:1 (designated SEQ ID NO:37); contiguous nucleotide positions 4299-4316 of SEQ ID NO:1 (designated SEQ ID NO:38); contiguous nucleotide positions 4285-4302 of SEQ ID NO:1 (designated SEQ ID NO:39); contiguous nucleotide positions 4300-4317 of SEQ ID NO:1 (designated SEQ ID NO:40); contiguous nucleotide positions 4518-4535 of SEQ ID NO:1 (designated SEQ ID NO:41); contiguous nucleotide positions 4533-4550 of SEQ ID NO:1 (designated SEQ ID NO:42); contiguous nucleotide positions 6116-6133 of SEQ ID NO:1 (designated SEQ ID NO:44); contiguous nucleotide positions 6192-6209 of SEQ ID NO:1 (designated SEQ ID NO:45); contiguous nucleotide positions 6191-6208 of SEQ ID NO:1 (designated SEQ ID NO:43); contiguous nucleotide positions 7501-7518 of SEQ ID NO:1 (designated SEQ ID NO:46); contiguous nucleotide positions 7517-7534 of SEQ ID NO:1 (designated SEQ ID NO:47); or a nucleotide sequence complementary to any of the preceding SEQ ID NOS:35-47. A polynucleotide particularly useful as a probe, especially for probing nucleic acids in samples of biological materials originating from a human or amplification products derived therefrom, is a nucleic acid comprising contiguous nucleotide positions 4292-4549 of SEQ ID NO:1 (designated SEQ ID NO:48) or a complementary sequence. For probing nucleic acids derived from a sample of biological material from a human, even a large nucleic acid segment of SEQ ID NO:1 can be used, for example a nucleic acid comprising contiguous nucleotide positions 4272-6515 of SEQ ID NO: 1 designated SEQ ID NO:34) or a complementary sequence.

The primer is capable of acting as a point of initiation of synthesis of a polynucleotide strand when placed under appropriate conditions. The primer will be completely or substantially complementary to a region of the polynucleotide strand to be copied. Thus, under conditions conducive to hybridization, the primer will anneal to the complementary region of the analyte strand. Upon addition of suitable reactants, (e.g., a polymerase, nucleotide triphosphates, and the like), the primer is extended by the polymerizing agent to form a copy of the analyte strand. The primer may be single-stranded, or alternatively maybe partially or fully double-stranded.

The terms "analyte polynucleotide" and "analyte strand" refer to a single- or double-stranded nucleic acid molecule which is suspected of containing a *Cryptovirus-specific* target sequence, and which may be present in a sample of biological material.

The inventive probe is a structure comprised of a polynucleotide which forms a hybrid structure with a *Cryptovirus-specific* target sequence, due to complementarity of at least one nucleotide sequence in the probe with a sequence in the target region. The polynucleotide regions of probes may be composed of DNA, and/or RNA, and/or synthetic nucleotide analogs. "Target region" refers to a region of the nucleic acid which is to be amplified and/or detected. The term "target sequence" refers to a *Cryptovirus-specific* sequence with which a probe or primer will form a stable hybrid under desired conditions.

Included within probes are "capture probes" and "label probes". Preferably the probe does not contain a sequence complementary to sequence(s) used to prime a nucleic acid amplification reaction.

The term "capture probe" as used herein refers to a polynucleotide comprised of a single-stranded polynucleotide coupled to a binding partner. The single-stranded polynucleotide is comprised of a targeting polynucleotide sequence, which is complementary to a target sequence in a target region to be detected in the analyte polynucleotide. This complementary region is of sufficient length and complementarity to the target sequence to afford a duplex of stability which is sufficient to immobilize the analyte polynucleotide to a solid surface (via the binding partners). The binding partner is specific for a second binding partner; the second binding partner can be bound to the surface of a solid support, or may be linked indirectly via other structures or binding partners to a solid support.

The term "binding partner" as used herein refers to a molecule capable of binding a ligand molecule with high specificity, as for example an antigen and an antibody specific therefor. In general, the specific binding partners must bind with sufficient affinity to immobilize the analyte copy/complementary strand duplex (in the case of capture probes) under the isolation conditions. Specific binding partners are known in the art, and include, for example, biotin and avidin or streptavidin, IgG and protein A, the numerous known receptor-ligand couples, and complementary polynucleotide strands. In the case of complementary polynucleotide binding partners, the partners are generally at least about 15 bases in length, and may be at least 40 bases in length; in addition, they have a content of Gs and Cs of at least about 25% and as much as about 75%. The polynucleotides may be composed of DNA, RNA, or synthetic nucleotide analogs.

"Coupled" as used herein refers to attachment by covalent bonds or by strong non-covalent interactions (e.g., hydrophobic interactions, hydrogen bonds, etc.). Covalent bonds may be, for example, ester, ether, phosphoester, amide, peptide, imide, carbon-sulfur bonds, carbon-phosphorus bonds, and the like.

A "support" refers to any solid or semi-solid surface to which a desired binding partner may be anchored. Suitable supports include glass, plastic, metal, polymer gels, and the like, and may take the form of beads, wells, dipsticks, membranes, and the like.

As used herein, the term "label probe" refers to an oligonucleotide which is comprised of a targeting polynucleotide sequence, which is complementary to a target sequence to be detected in the analyte polynucleotide. This complementary region is of sufficient length and complementarity to the target sequence to afford a duplex comprised of the "label probe" and the "target sequence" to be detected by the label. The oligonucleotide is coupled to a label either directly, or indirectly via a set of ligand molecules with high specificity for each other.

A "label" includes any atom or moiety which can be used to provide a detectable (preferably quantifiable) signal, and which can be attached by conventional means to a polynucleotide or to polypeptide, such as an antibody. The label can be used alone or in conjunction with additional reagents. Such labels are themselves well-known in the art. The label can be a radioisotope, such as ¹⁴C, ³²P, ³⁵S, ³H, or ¹⁵O, which is detected with suitable radiation detection means. Alternatively, the label can be a fluorescent labeling agent that chemically binds to antibodies or antigens without denaturation to form a fluorochrome (dye) that is a useful immunofluorescent tracer. A description of immunofluorescent analytic techniques is found in DeLuca, "Immunofluorescence Analysis", in Antibody As a Tool, Marchalonis et al., eds., John Wiley & Sons, Ltd., pp. 189-231 (1982). As well, any of diverse fluorescent dyes can optionally be used to label probes or primers or amplification products for ease of detection and/or analysis. Useful fluorescent dyes include, but are not limited to, rhodamine, fluorescein, SYBR Green I, Y1O-PRO-1, thiazole orange, Hex (i.e., 6-carboxy-2',4',7',4,7-hexachlorofluoroscein), pico green, edans, fluorescein, FAM (i.e., 6-carboxyfluorescein), or TET (i.e., 4,7,2',7'-tetrachloro-6-carboxyfluoroscein). (E.g., J. Skeidsvoll and P.M. Ueland, Analysis of double-stranded DNA by capillary electrophoresis with laser-induced fluorescence detection using the monomeric dye SYBR green I, Anal. Biochem. 231(20):359-65 [1995]; H. Iwahana et al., Multiple fluorescence-based PCR-SSCP analysis using internal fluorescent labeling of PCR products, Biotechniques 21(30:510-14, 516-19 [1996]).

The inventive nucleic acid constructs include recombinant cloning and expression vectors (including plasmids and viral expression vectors, such as retroviral or adenoviral vectors), that contain the inventive nucleic acid. A "vector" is a replicon in which another polynucleotide segment is attached, so as to bring about the replication and/or expression of the attached segment. A "replicon" is any genetic element, e.g., a plasmid, a chromosome, a virus, a cosmid, etc., that behaves as an autonomous unit of polynucleotide replication within a cell, i.e., being capable of replication under the replicon's own control. Inventive recombinant expression vectors contain one or more inventive nucleic acid segments and include at least a promoter region operatively linked to the inventive nucleic acid segment in a transcriptional unit. Preferred examples of inventive nucleic acid constructs are those that include one or more nucleic acid segments encoding a *Cryptovirus* protein, the *Cryptovirus* coding sequence(s) being thus suitably placed and operatively linked to suitable regulatory sequences within one or more transcriptional units within the construct.

"Control" or "regulatory" sequences, elements or regions refers to polynucleotide sequences which are necessary to effect the expression of coding sequences to which they are ligated. The nature of such control sequences differs depending upon the host organism; in prokaryotes, such control sequences generally include promoter, ribosomal binding site, and terminators; in eukaryotes, generally, such control sequences include promoters, terminators and, in some instances, enhancers. The term "control sequences" is intended to include, at a minimum, all components whose presence is necessary for expression, and may also include additional components whose presence is advantageous, for example, leader sequences.

As used herein, "expression" refers to the process by which genes are transcribed into mRNA, which is in turn translated into peptides, polypeptides, or proteins. With respect to a recombinant expression vector, a promoter region refers to a segment of nucleic acid that controls transcription of a coding sequence to which it is operatively linked. The promoter region includes specific sequences that are sufficient for RNA polymerase recognition, binding and transcription initiation. In addition, the promoter region includes sequences that modulate this recognition, binding and transcription initiation activity of RNA polymerase. These sequences may be *cis* acting or may be responsive to *trans* acting factors. Promoters, depending upon the nature of the regulation, may be constitutive or regulated developmentally or inducibly. Exemplary promoters contemplated for use in the practice of the present invention include the SV40 early promoter, the cytomegalovirus (CMV) promoter, the mouse mammary tumor virus (MMTV) steroid-inducible promoter, Moloney murine leukemia virus (MMLV) promoter, and the like.

For optimal expression of foreign genes in mammalian cells (e.g., the *Cryptovirus* genes of the present invention), the expression vector may also require terminator sequences and poly A addition sequences; enhancer sequences which increase expression may also be included, and sequences which cause amplification of the gene may also be desirable. Such sequences are known in the art.

As used herein, the term "operatively linked" refers to the functional relationship of nucleic acid with regulatory (control or effector) nucleotide sequences, such as promoters, enhancers, transcriptional and translational stop sites, and other signal sequences. For example, operative linkage of DNA or RNA to a promoter refers to the physical and functional relationship between the DNA or RNA and the promoter such that the transcription of such DNA or RNA is initiated from the promoter by an RNA polymerase that specifically recognizes, binds to and transcribes the DNA or RNA, respectively. A regulatory sequence "operatively linked" to a coding sequence is ligated in such a way that expression of the coding sequence is achieved under conditions compatible with the regulatory sequences. Thus, for example, within a transcriptional unit, the promoter sequence, is located upstream (i.e., 5' in relation thereto) from the coding sequence and the coding sequence, is 3' to the promoter, or alternatively is in a sequence of genes or open reading frames 3' to the promoter and expression is coordinately regulated thereby. Both the promoter and coding sequences are oriented in a 5' to 3' manner, such that transcription can take place in vitro in the presence of all essential enzymes, transcription factors, co-factors, activators, and reactants, under favorable physical conditions, e.g., suitable pH and temperature. This does not mean that, in any particular cell, conditions will favor transcription. For example, transcription from a tissue-specific promoter is generally not favored in heterologous cell types from different tissues.

The inventive expression vector, comprising a *Cryptovirus-specific* nucleic acid, is used to transform a cell. "Transformation", as used herein, refers to the insertion of an exogenous polynucleotide into a host cell, irrespective of the method used for the insertion, for example, direct uptake, transfection, transduction, f-mating, microparticle bombardment, or electroporation. The exogenous polynucleotide may be maintained as a non-integrated vector, for example, a plasmid, or alternatively, may be integrated into the host genome. A "transformed" host cell refers to both the immediate cell that has undergone transformation and its progeny that maintain the originally exogenous polynucleotide.

Appropriate expression vectors are well known to those of skill in the art and include those that are replicable in eukaryotic cells and/or prokaryotic cells and those that remain episomal or those which integrate into the host cell genome.

Exemplary, eukaryotic expression vectors, include the cloned bovine papilloma virus genome, the cloned genomes of the murine retroviruses, and eukaryotic cassettes, such as the pSV-2 gpt system (described by Mulligan and Berg, 1979, Nature Vol. 277:108-114) the Okayama-Berg cloning system (Mol. Cell Biol. Vol. 2:161-170, 1982), pGAL4, pCI (e.g., pCI-neo), and the expression cloning vector described by Genetics Institute (Science Vol. 228:810-815, 1985), are available which provide substantial assurance of at least some expression of the protein of interest in the transformed mammalian cell.

Preferred are vectors which contain regulatory elements that can be linked to the inventive nucleic acids, for transfection or transduction of mammalian cells. Examples are cytomegalovirus (CMV) promoter-based vectors such as pcDNAI (Invitrogen, San Diego, CA), MMTV promoter-based vectors such as pMAMNeo (Clontech, Palo Alto, CA) and pMSG (Pharmacia, Piscataway, NJ), and SV40 promoter-based vectors such as pSVβ (Clontech, Palo Alto, CA). In one embodiment of the present invention, adenovirus-transferrin/polylysine-DNA (TfAdp1-DNA) vector complexes (Wagner et al., 1992, PNAS, USA, 89:6099-6103; Curiel et al., 1992, Hum. Gene Therapy, 3:147-154; Gao et al., 1993, Hum. Gene Ther., 4:14-24) are employed to transduce mammalian cells with heterologous *Cryptovirus-specific* nucleic acid. Any of the plasmid expression vectors described herein may be employed in a TfAdpl-DNA complex.

In addition, expression vectors may contain appropriate packaging signals that enable the vector to be packaged by a number of viral virions, e.g., retroviruses, such as human immune-deficiency virus, lentiviruses, mumps virus, herpes viruses, adenoviruses, resulting in the formation of a "viral vector." (See, e.g., Anderson, W.F., Gene therapy scores against cancer, Nat. Med. 6(8):862-63 [2000]). These viral vectors include, for example, Herpes simplex virus vectors (e.g., Geller et al., 1988, Science, 241:1667-1669), Vaccinia virus vectors (e.g., Piccini et al., 1987, Meth. in Enzymology, 153:545-563); Cytomegalovirus vectors (Mocarski et al., in Viral Vectors, Y. Gluzman and S.H. Hughes, Eds., Cold Spring Harbor Laboratory, Cold Spring Harbor, N.Y., 1988, pp. 78-84), Moloney murine leukemia virus vectors (Danos et al., 1980, PNAS, USA, 85:6469), adenovirus vectors (e.g., Logan et al., 1984, PNAS, USA, 81:3655-3659; Jones et al., 1979, Cell, 17:683-689; Berkner, 1988, Biotechniques, 6:616-626; Cotten et al., 1992, PNAS, USA, 89:6094-6098; Graham et al., 1991, Meth. Mol. Biol., 7:109-127), adeno-associated virus vectors, retrovirus vectors (see, e.g., U.S. Patent 5,252,479, WIPO publications WO 92/07573, WO 90/06997, WO 89/05345, WO 92/05266 and WO 92/14829, U.S. Patent Nos. 4,405,712 and 4,650,764; Shackleford et al., 1988, PNAS, USA, 85:9655-9659), and the like.

A preferred viral vector is Moloney murine leukemia virus and the pseudotyped retroviral vector derived from Moloney virus called vesicular-stomatitis-virus-glycoprotein (VSV-G)-Moloney murine leukemia virus. A most preferred viral vector is a pseudotyped (VSV-G) lentiviral vector derived from the HIV virus, which is used to transduce mammalian cells. (Naldini, L., et al., In vivo gene delivery and stable transduction of nondividing cells by a lentiviral vector, Science 272: 263-267 [1996]). This gene delivery system employs retroviral particles generated by a three-plasmid expression system. In this system a packaging construct contains the human cytomegalovirus (hCMV) immediate early promoter, driving the expression of all viral proteins. The construct's design eliminates the cis-acting sequences crucial for viral packaging, reverse transcription and integration of these transcripts. The second plasmid encodes a heterologous envelope protein (*env*), namely the G glycoprotein of the vesicular stomatitis virus (VSV-G). The third plasmid, the transducing vector (pHR'), contains cis-acting sequences of human immunodeficiency virus (HIV) required for packaging, reverse transcription and integration, as well as unique restriction sites for cloning heterologous complementary DNAs (cDNAs). For example, a genetic selection marker, such as green fluorescent protein (GFP), enhanced green fluorescent protein (EGFP), blue fluorescent protein, yellow fluorescent protein, a fluorescent phycobiliprotein, β-galactosidase, and/or a gene encoding another preselected product is cloned downstream of the hCMV promoter in the HR'vector, and is operatively linked so as to form a transcriptional unit. A VSV-G pseudotyped retroviral vector system is capable of infecting a wide variety of cells including cells from different species and of integrating into the genome. Some retroviruses, i.e., lentiviruses, such as HIV, have the ability to infect non-dividing cells. Lentiviruses have a limited capacity for heterologous DNA sequences, the size limit for this vector being 7-7.5 kilobases (Verma, I.M. and Somia, N., Gene Therapy - promises, problems and prospects, Nature 389:239-242 [1997]). *In vivo* experiments with lentiviruses show that expression does not shut off like other retroviral vectors and that *in vivo* expression in brain, muscle, liver or pancreatic-islet cells, is sustained at least for over six months - the longest time tested so far (Verma and Somia [1997]; Anderson, WF., Human Gene Therapy, Nature (Suppl). 392:25-30 [1998]).

All of the above viruses may require modification to render them non-pathogenic or less antigenic. Other known viral vector systems, however, are also useful within the confines of the invention.

A particularly useful expression vector which is useful to express foreign cDNA and which may be used in vaccine preparation is *Vaccinia* virus. In this case, the heterologous cDNA is inserted into the *Vaccinia* genome. Techniques for the insertion of foreign cDNA into the vaccinia virus genome are known in the art, and utilize, for example, homologous recombination. The insertion of the heterologous cDNA is generally into a gene which is non-essential in nature, for example, the thymidine kinase gene (tk), which also provides a selectable marker. Plasmid vectors that greatly facilitate the construction of recombinant viruses have been described (see, for example, Mackett et al. (1984) in "DNA Cloning" Vol II IRL Press, p. 191, Chakrabarti et al. (1985), Mol. Cell Biol. 5:3403; Moss (1987) in "Gene Transfer Vectors for Mammalian Cells" (Miller and Calos, eds., p. 10). Expression of the desired polypeptides containing immunoreactive regions then occurs in cells and mammals that are infected and/or immunized with the live recombinant *Vaccinia* virus.

The inventive "nucleic acid construct" also encompasses a construct that is not contained in a vector, for example, a synthetic antisense oligonucleotide, such as a phosphorothioate oligodeoxynucleotide. Synthetic antisense oligonucleotides, or other antisense chemical structures designed to recognize and selectively bind to mRNA, are constructed to be complementary to portions of the *Cryptovirus* coding strand, for example, to coding sequences shown in SEQ ID NO:3, 5, 7, 9, 11, 13, 15, 17, 19, 21, or 23. When taken up by a mammalian cell, the antisense oligonucleotide prevents translational expression of at least part of the *Cryptovirus* coding region, the inventive antisense oligonucleotide is useful to prevent expression of a *Cryptovirus* protein. Antisense oligonucleotides inactivate target mRNA sequences by either binding thereto and inducing degradation of the mRNA by, for example, RNase I digestion, or inhibiting translation of mRNA target sequence by interfering with the binding of translation-regulating factors or ribosomes, or by inclusion of other chemical structures, such as ribozyme sequences or reactive chemical groups which either degrade or chemically modify the target mRNA. Gene-based therapy strategies employing antisense oligonucleotides are well known in the art. (E.g., Rait, A. et al., 3'-End conjugates of minimally phosphorothioate-protected oligonucleotides with I-O-hexadecylglycerol: synthesis and anti-ras activity in radiation-resistant cells, Bioconjug Chem., 11 (2):153-60 [2000]; Stenton, G. R. et al., Aerosolized syk Antisense suppresses syk expression, mediator release from macrophages, and pulmonary inflammation, J. Immunol., 164(7):3790-7 [2000]; Suzuki, J. et al., Antisense Bcl-x oligonucleotide induces apoptosis and prevents arterial neointimal formation in murine cardiac allografts, Cardiovas. Res., 45(3):783-7 [2000]; Kim, J. W. et al., Antisense oligodeoxynucleotide of glyceraldehyde-3-phosphate dehydrogenase gene inhibits cell proliferation and induces apoptosis in human cervical carcinoma cell line, Antisense Nucleic Acid Drug Dev., 9(6):507-13 [1999]; Han, D. C. et al., therapy with antisense TGF-betal oligodeoxynucleotides reduces kidney weight and matrix mRNAs in diabetic mice, Am. J. Physiol. Renal Physiol., 278(4):F628-F634 [2000]; Scala, S. et al., Adenovirus-mediated suppression of HMGI (Y) protein synthesis as potential therapy of human malignant neoplasias, Proc. Natl. Acad. Sci. USA., 97(8):4256-4261 [2000]; Arteaga, C. L., et al., Tissue-targeted antisense c-fos retroviral vector inhibits established breast cancer xenografts in nude mice, Cancer Res., 56(5):1098-1103 [1996]; Muller, M. et al., Antisense phosphorothioate oligodeoxynucleotide down-regulation of the insulin-like growth factor I receptor in ovarian cancer cells, Int. J. Cancer, 77(4):567-71 [1998]; Engelhard, H. H., Antisense Oligodeoxynucleotide Technology: Potential Use for the Treatment of Malignant Brain Tumors, Cancer Control, 5(2):163-170 [1998]; Alvarez-Salas, L. M. et al., Growth inhibition of cervical tumor cells by antisense oligodeoxynucleotides directed to the human papillomavirus type 16 E6 gene, Antisense Nucleic Acid Drug Dev., 9(5):441-50 [1999]; Im, S. A., et al., Antiangiogenesis treatment for gliomas: transfer of antisense-vascular endothelial growth factor inhibits tumor growth in vivo, Cancer Res., 59(4):895-900 [1999]; Maeshima, Y. et al., Antisense oligonucleotides to proliferating cell nuclear antigen and Ki-67 inhibit human mesangial cell proliferation, J. Am. Soc. Nephrol., 7(10):2219-29 [1996]; Chen, D. S. et al., Retroviral Vector-mediated transfer of an Antisense cyclin GI construct inhibit osteosarcoma tumor growth in nude mice, Hum. Gene Ther, 8(14):1667-74 [1997]; Hirao, T. et al., Antisense epidermal growth factor receptor delivered by adenoviral vector blocks tumor growth in human gastric cancer, Cancer Gene Ther. 6(5):423-7 [1999]; Wang, X. Y. et al., Antisense inhibition of protein kinase Calpha reverses the transformed phenotype in human lung carcinoma cells, Exp. Cell Res., 250(1):253-63 [1999]; Sacco, M.G. et al., In vitro and in vivo antisense-mediated growth inhibition of a mammary adenocarcinoma from MMTV-neu transgenic mice, Gene Ther., 5(3);388-93 [1998]; Leonetti, C. et al., Antitumor effect of c-myc antisense phosphorothioate oligodeoxynucleotides on human melanoma cells in vitro and in mice, J. Natl. Cancer Inst., 88(7):419-29 [1996]; Laird, A. D. et al., Inhibition of tumor growth in liver epithelial cells transfected with a transforming growth factor alpha antisense gene, Cancer Res. 54(15):4224-32 (Aug 1, 1994); Yazaki, T. et al., Treatment of glioblastoma U-87 by systemic administration of an antisense protein kinase C-alpha phosphorothioate oligodeoxynucleotide, Mol. Pharmacol., 50(2):236-42 [1996]; Ho, P. T. et al., Antisense oligonucleotides as therapeutics for malignant diseases, Semin. Oncol., 24(2):187-202 [1997]; Muller, M. et al., Antisense phosphorothioate oligodeoxynucleotide down-regulation of the insulin-like growth factor I receptor in ovarian cancer cells, Int. J. Cancer, 77(4):567-71 [1998]; Elez, R. et al., Polo-like kinasel, a new target for antisense tumor therapy, Biochem. Biophys. Res. Commun., 269(2):352-6 [2000]; Monia, B. P. et al., Antitumor activity of a phosphorothioate antisense oligodeoxynucleotide targeted against C-raf kinase, Nat. Med., 2(6):668-75 [1996]).

The present invention relates to an isolated *Cryptovirus* protein. The term "protein" refers to a polymer of amino acids of any length, i.e., a polypeptide, and does not refer to a specific length of the product; thus, "polypeptides", "peptides", and "oligopeptides", are included within the definition of "protein", and such terms are used interchangeably herein with "protein". The term "protein" also includes post-expression modifications of the polypeptide, for example, glycosylations, acetylations, phosphorylations and the like. Included within the definition of "protein" are, for example, polypeptides containing one or more analogs of an amino acid (including, for example, unnatural amino acids, etc.), polypeptides with substituted linkages, as well as other modifications known in the art, both naturally occurring and non-naturally occurring. Methods of inserting analogs of amino acids into a peptide sequence are known in the art.

The phrase "isolated *Cryptovirus* protein" refers to a cryptoviral protein which is substantially free, i.e., contains less than about 50%, preferably less than about 70%, and even more preferably less than about 90%, of the cellular components and/or contaminants normally associated with a native in vivo environment. An isolated *Cryptovirus* protein of the present invention can also be further isolated from one or more other components of a *Cryptovirus* particle, for example, other *Cryptovirus* protein species, phospholipid components of the viral envelope, or the viral genome. In some useful embodiments the inventive isolated *Cryptovirus* protein is purified to homogeneity by known virological and biochemical methods.

A protein is provided which is encoded by a *Cryptovirus*-specific nucleic acid segment, which nucleic acid segment comprises:
(i) contiguous nucleotide positions 152-1678 of (SEQ ID NO:1)(also designated SEQ ID NO:3), or a degenerate coding sequence, which encodes the *Cryptovirus* nucleocapsid (NP) protein;
(ii) contiguous nucleotide positions 1850-2515 of (SEQ ID NO:1)(also designated SEQ ID NO:5), or a degenerate coding sequence, which encodes a *Cryptovirus* RNA binding (V) protein thought to be a component of the viral RNA-dependent RNA polymerase;
(iii) contiguous nucleotide positions 1850-3023 of (SEQ ID NO:1)(also designated SEQ ID NO:33) combined with a further insertion of two guanine (G) residues between nucleotide position 2339 of (SEQ ID NO:1) and nucleotide position 2340 of (SEQ ID NO:1)(the combined coding sequence including the "GG" insertion being designated [SEQ ID NO:7]), or a degenerate coding sequence thereof; this frameshift-causing insertion into the mRNA encoding the *Cryptovirus* nucleocapsid-associated phosphoprotein (P protein) occurs during processing of the mRNA and is not templated by the Cryptovirus minus stranded RNA genomic sequence;
(iv) contiguous nucleotide positions 3141-4271 of (SEQ ID NO:1)(also designated SEQ ID NO:9), or a degenerate coding sequence, which encodes the *Cryptovirus* virion-associated matrix or membrane (M) protein;
(v) contiguous nucleotide positions 4530-6182 of (SEQ ID NO:1)(also designated SEQ ID NO:11) or a degenerate coding sequence, which encodes the *Cryptovirus* (uncleaved) fusion (F) protein, which is a propeptide form of a major envelope-associated glycoprotein, and includes a 19-amino acid signal region at its amino terminus;
(vi) contiguous nucleotide positions 4587-6182 of (SEQ ID NO:1)(also designated SEQ ID NO:13), or a degenerate coding sequence, which encodes the *Cryptovirus* (uncleaved) fusion (F₀) protein, which is a propeptide form of a major envelope-associated glycoprotein, minus the 19-amino acid signal region at its amino terminus;
(vii) contiguous nucleotide positions 4587-4835 of (SEQ ID NO:1)(also designated SEQ ID NO:15), or a degenerate coding sequence, which encodes the cleaved F₂ protein;
(viii) contiguous nucleotide positions 4836-6182 of (SEQ ID NO:1)(also designated SEQ ID NO:17), or a degenerate coding sequence, which encodes the cleaved F₁ protein, including a 22-amino acid carboxy terminal peptide segment that is thought to be important to *Cryptovirus* infectivity;
(ix) contiguous nucleotide positions 6303-6434 of (SEQ ID NO:1)(also designated SEQ ID NO:19), or a degenerate coding sequence, which encodes the *Cryptovirus* SH protein, a small envelope-associated protein;
(x) contiguous nucleotide positions 6584-8278 of (SEQ LD NO:1)(also designated SEQ ID NO:21), or a degenerate coding sequence, which encodes the *Cryptovirus* hemagglutinin (HN) protein, another major envelope protein; or
(xi) contiguous nucleotide positions 8414-15178 of (SEQ ID NO:1)(also designated SEQ ID NO:23), or a degenerate coding sequence, which encodes the *Cryptovirus* largest nucleocapsid associated protein (L protein).

These are the amino acid sequences corresponding to the preceding proteins in the same order:
(i) NP has the following amino acid sequence (SEQ ID NO:4):
(ii) V has the following amino acid sequence (SEQ ID NO:6):
(iii) P has the following amino acid sequence (SEQ ID NO:8):
(iv) M has the following amino acid sequence (SEQ ID NO:10):
(v) F has the following amino acid sequence (SEQ ID NO:12):
(vi) F₀ has the following amino acid sequence (SEQ ID NO:14):
(vii) F₂ has the folowing amino acid sequence (SEQ ID NO:16):
(viii) F₁ has the amino acid sequence (SEQ ID NO:18);
(ix) SH has the following amino acid sequence (SEQ ID NO:20):
(x) HN has the following amino acid sequence (SEQ ID NO:22): and
(xi) L has the following amino acid sequence (SEQ ID NO:24):

The protein also encompasses a polypeptide having substantially the same amino acid sequence as (SEQ ID NO:4), (SEQ ID NO:6) (SEQ ID NO:8). (SEQ ID NO:10), (SEQ ID NO:12), (SEQ ID NO:14), (SEQ ID NO:16), (SEQ ID NO:18), (SEQ ID NO:20), (SEQ ID NO:22), or (SEQ ID NO:24). As employed herein, the term "substantially the same amino acid sequence" refers to amino acid sequences having at least about 80%, still more preferably about 90% amino acid identity with respect to a reference amino acid sequence; with greater than about 95% amino acid sequence identity being especially preferred. It is recognized, however, that polypeptide containing less than the described levels of sequence identity arising as splice variants or that are modified by conservative amino acid substitutions are also encompassed within the scope of the present invention. The degree of sequence homology is determined by conducting an amino acid sequence similarity search of a protein data base, such as the database of the National Center for Biotechnology Information (NCBI; www.ncbi.nlm.nih.gov/BLAST/), using a computerized algorithm, such as PowerBLAST, QBLAST, PSI-BLAST, PHI-BLAST, gapped or ungapped BLAST, or the "Align" program through the Baylor College of Medicine server (www.hgsc.bcm.tmc.edu/seq_data). (E.g., Altchul, S.F., et al., Copped BLAST and PSI-BLAST: a new generation of protein database search programs, Nucleic Acids Res. 25(17):3389-402 [1997]; Zhang, J., & Madden, T.L., PowerBLAST: a new network BLAST application for interactive or automated sequence analysis and annotation, Genome Res. 7(6):649-56 [1997]; Madden, T.L., et al., Applications of network BLAST server, Methods Enzymol. 266:131-41 [1996]; Altschul, S.F., et al., Basic local alignment search tool, J. Mol. Biol. 215(3):403-10 [1990]).

Also encompassed by the term *Cryptovirus* protein, are biologically functional or active peptide analogs thereof. The term peptide "analog" includes any polypeptide having an amino acid residue sequence substantially identical to a sequence specifically shown herein in which one or more residues have been conservatively substituted with a functionally similar residue and which displays the ability to mimic the biological activity of a native *Cryptovirus* protein.

Examples of conservative substitutions include the substitution of one non-polar (hydrophobic) residue such as isoleucine, valine, leucine or methionine for another, the substitution of one polar (hydrophilic) residue for another such as between arginine and lysine, between glutamine and asparagine, between glycine and serine, the substitution of one basic residue such as lysine, arginine or histidine for another, or the substitution of one acidic residue, such as aspartic acid or glutamic acid for another.

The phrase "conservative substitution" also includes the use of a chemically derivatized residue in place of a non-derivatized residue, provided that such polypeptide displays the requisite biological activity.

"Chemical derivative" refers to a subject polypeptide having one or more residues chemically derivatized by reaction of a functional side group. Such derivatized molecules include, for example, those molecules in which free amino groups have been derivatized to form amine hydrochlorides, p-toluene sulfanyl groups, carbobenzoxy groups, t-butyloxycarbonyl groups, chloroacetyl groups or formyl groups. Free carboxyl groups may be derivatized to form salts, methyl and ethyl esters or other types of esters or hydrazides. Free hydroxyl groups may be derivatized to form O-acyl or O-alkyl derivatives. The imidazole nitrogen of histidine may be derivatized to form N-im-benzylhistidine. Also included as chemical derivatives are those peptides which contain one or more naturally occurring amino acid derivatives of the twenty standard amino acids. For example, 4-hydroxyproline may be substituted for proline; 5-hydroxylysine may be substituted for lysine; 3-methylhistidine may be substituted for histidine; homoserine may be substituted for serine; and ornithine may be substituted for lysine. The inventive polypeptide also includes any polypeptide having one or more additions and/or deletions of residues, relative to the sequence of an inventive polypeptide whose sequence is shown herein, so long as the requisite biological activity is maintained.

A variant of a *Cryptovirus* protein designated by (SEQ ID NO:4), (SEQ ID NO:6) (SEQ ID NO:8), (SEQ ID NO:10), (SEQ ID NO:12), (SEQ ID NO:14), (SEQ ID NO:16). (SEQ ID NO:18), (SEQ ID NO:20), (SEQ ID NO:22), or (SEQ ID NO:24) is also provided, a "variant" refers to a polypeptide in which the amino acid sequence of the designated *Cryptovirus* protein has been altered by the deletion, substitution, addition or rearrangement of one or more amino acids in the sequence. Methods by which variants occur (for example, by recombination) or are made (for example, by site directed mutagenesis) are known in the art.

The *Cryptovirus* protein can also include one or more labels, which are known to those of skill in the art.

The inventive proteins are isolated or purified by a variety of known biochemical means, including, for example, by the recombinant expression systems described herein, precipitation, gel filtration, ion-exchange, reverse-phase and affinity chromatography, electrophoresis, and the like. Other well-known methods are described in Deutscher et al., Guide to Protein Purification: Methods in Enzymology Vol. 182, (Academic Press, [1990]).

The isolated *Cryptovirus* protein of the present invention can also be chemically synthesized. For example, synthetic polypeptide can be produced using Applied Biosystems, Inc. Model 430A or 431A automatic peptide synthesizer (Foster City, CA) employing the chemistry provided by the manufacturer and the amino acid sequences provided herein. Alternatively, the *Cryptovirus* protein can be isolated or purified from native cellular sources. Alternatively, the *Cryptovirus* protein can be isolated from the inventive chimeric proteins by the use of suitable proteases.

Alternatively, the *Cryptovirus* proteins can be recombinantly derived, for example, produced by eukaryotic or prokaryotic cells genetically modified to express *Cryptovirus* protein-encoding polynucleotides in accordance with the inventive technology as described herein. Recombinant methods are well known, as described, for example, in Sambrook *et al.,* supra., 1989). An example of the means for preparing the inventive *Cryptovirus* protein is to express nucleic acids encoding the *Cryptovirus* protein of interest in a suitable host cell that contains the inventive expression vector, such as a bacterial cell, a yeast cell, an insect cell, an amphibian cell (i.e., oocyte), or a mammalian cell, using methods well known in the art, and recovering the expressed polypeptide, again using well-known methods.

"Recombinant host cells" "host cells", "cells", "cell lines", "cell cultures" and other such terms denoting prokaryotic or eukaryotic cell lines cultured as unicellular or monolayer entities, and refer to cells which can be, or have been, used as recipients for a recombinant expression vector or other foreign nucleic acids, such as DNA or RNA, and include the progeny of the original cell which has been transfected. It is understood that the progeny of a single parental cell may not necessarily be completely identical in morphology or in genomic or total DNA complement as the original parent, due to natural, accidental, or deliberate mutation.

The present invention includes chimeric proteins. The term "chimeric protein" generally refers to a polypeptide comprising an amino acid sequence drawn from two or more individual proteins that are not naturally so linked. In the present invention, "chimeric protein" is used to denote a polypeptide comprising a *Cryptovirus* protein, or a truncate or polypeptide variant thereof having an altered amino acid sequence, fused to a non*-Cryptovirus* protein or polypeptide moiety. Chimeric proteins are most conveniently produced by expression of a fused gene, which encodes a portion of one polypeptide at the 5' end and a portion of a different polypeptide at the 3' end, where the different portions are joined in one reading frame which may be expressed in a suitable host cell. In some embodiments, the *Cryptovirus* protein is positioned at the carboxy terminus of the chimeric protein. In other embodiments the *Cryptovirus* protein is positioned at the amino terminus of the chimeric protein.

The *non-Cryptovirus* protein moiety, or "chimeric partner", of the inventive chimeric protein can be a functional enzyme fragment. Suitable functional enzyme fragments are those polypeptides which exhibit a quantifiable activity when expressed fused to the *Cryptovirus* protein. Exemplary enzymes include, without limitation, ß-galactosidase (ß-gal), ß-lactamase, horseradish peroxidase (HRP), glucose oxidase (GO), human superoxide dismutase (hSOD), urease, and the like. These enzymes are convenient because the amount of chimeric protein produced can be quantified by means of simple colorimetric assays. Alternatively, one may employ antigenic proteins or fragments, e.g., human superoxide dismutase (hSOD), to permit simple detection and quantification of chimeric proteins using antibodies specific for the non*-Cryptovirus* protein chimeric partner. In chimeric proteins, useful chimeric partners include amino acid sequences that provide for secretion from a recombinant host, enhance the immunological reactivity of a *Cryptovirus* protein epitope(s), or facilitate the coupling of the *Cryptovirus* polypeptide to a support or a vaccine carrier. (See, e.g., EPO Pub. No. 116,201; U.S. Pat. No. 4,722,840; EPO Pub. No. 259,149; U.S. Pat. No. 4,629,783).

Embodiments of the inventive *Cryptovirus* protein are useful as immunoreactive polypeptides, including use for the production of a *Cryptovirus-specific* antibody. "Immunoreactive" refers to the ability of a polypeptide to bind immunologically to an antibody and/or to a lymphocyte antigen receptor due to antibody or receptor recognition of a specific epitope contained within the polypeptide; or the ability of a polypeptide to be immunogenic. An "immunogenic" protein is a polypeptide that elicits a cellular and/or humoral immune response, whether alone or linked to a carrier in the presence or absence of an adjuvant. The immunogenicity of various isolated *Cryptovirus* proteins, or *Cryptovirus* protein fragments, of interest is determined by routine screening. Immunological reactivity may be determined by antibody binding, more particularly by the kinetics of antibody binding, and/or by competition in binding using as competitor(s) a known polypeptide(s) containing an epitope against which the antibody is directed. The techniques for determining whether a polypeptide is immunologically reactive with an antibody are known in the art. Particularly useful examples of immunogenic *Cryptovirus* proteins are the envelope proteins, i.e., F, F₀, F₂, F₁, , HN, and SH proteins.

As used herein, "epitope" refers to an antigenic determinant of a polypeptide. An epitope can comprise 3 amino acids in a spatial conformation which is unique to the epitope. Epitopes typically are mapped to comprise at least about five amino acids, more usually at least about 8 amino acids to about 10 amino acids, or more. Methods of determining the spatial conformation of amino acids are known in the art, and include, for example, x-ray crystallography and two-dimensional nuclear magnetic resonance.

Immunogenic *Cryptovirus* proteins are useful for producing or manufacturing vaccines. As described above, *Cryptovirus* belongs to the Paramyxoviridae family of viruses. Numerous vaccines have been developed for humans and domestic animals against viruses in this virus family. For example, there are effective vaccines against measles virus, mumps virus, canine distemper virus, canine parainfluenza virus type 2, and Newcastle's disease virus (of fowl). These viruses were amenable to the development of effective vaccines because they have a narrow species tropism (i.e., they infect only one, or only a few, species), they exist as only one, or only one predominant, serotype (making a vaccine universally protective against the virus), they cause significant morbidity in their host (i.e., they are significant causes of human illness), and they are pandemic (i.e., there is a worldwide concern).

*Cryptovirus* is amenable to vaccine development for the same reasons other family members have proven so. There is evidence for cross- neutralizability of hyper immune rabbit antiserum made against different sources of the virus, and very similar nucleotide sequences of the virus genome have been obtained from two sources of the virus (BBR strain and Niigata cell-associated strain).

In accordance with the invention, multivalent or monovalent vaccines can be prepared against one or more *Cryptovirus* proteins. In particular, vaccines are contemplated comprising one or more *Cryptovirus* proteins, such as, but not limited to, envelope proteins F, F₀, F₂, F₁, HN, and/or SH. Methods for manufacturing vaccines which contain an immunogenic polypeptide as an active ingredient, are known to one skilled in the art. Typically, such vaccines are prepared as injectable compositions comprising the *Cryptovirus* protein(s), either as liquid solutions or suspensions; solid forms suitable for solution in, or suspension in, liquid prior to injection may also be prepared. The preparation may also be emulsified, or the protein encapsulated in liposomes. The active immunogenic ingredients are typically mixed with excipients which are pharmaceutically acceptable and compatible with the active ingredient. Suitable excipients or carriers are, for example, water, saline, dextrose, glycerol, ethanol, or the like and combinations thereof. In addition, if desired, the vaccine may contain minor amounts of auxiliary substances such as wetting or emulsifying agents, pH buffering agents, and/or adjuvants which enhance the effectiveness of the vaccine.

Examples of adjuvants which may be effective include but are not limited to: aluminum hydroxide, N-acetyl-muramyl-L-theronyl-D-isoglutamine (thr-MDP), N-acetyl-nor-muramyl-L-alanyl-D-isoglutamine (CGP 11637, referred to as nor-MDP), N-acetylmuramyl-L-alanyl-D-isoglutaminyl-L-alanine-2-(1-2-dipalmitoyl-sn-glycero-3-hydroxyphosphoryloxy)-ethylamine (CGP 19835A, referred to as MTP-PE), and RIBI, which contains three components extracted from bacteria, monophosphoryl lipid A, trehalose dimycolate and cell wall skeleton (MPL+TDM+CWS) in a 2% squalene/Tween 80 emulsion. The effectiveness of a particular adjuvant can be determined by measuring the amount of antibodies directed against an immunogenic *Cryptovirus* protein resulting from administration of this protein in vaccines which are also comprised of the adjuvant.

The proteins can be formulated into the vaccine as neutral or salt forms. Pharmaceutically acceptable salts include the acid addition salts (formed with free amino groups of the peptide) and which are formed with inorganic acids such as, for example, hydrochloric or phosphoric acids, or organic acids such as acetic, oxalic, tartaric, maleic, and the like. Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

The vaccines are conventionally administered parenterally by injection, for example, either subcutaneously or intramuscularly, but they can also be delivered intranasally, enterically, or by any other delivery route. Administration can be with or without adjuvants. Additional formulations of the vaccine composition that are suitable for other non-injection modes of administration include suppositories and, in some cases, oral formulations. For suppositories, traditional binders and carriers can include, for example, polyalkylene glycols or triglycerides; such suppositories may be formed from mixtures containing the active ingredient in the range of 0.5% to 10%, preferably 1%-2%. Oral formulations include such normally employed excipients as, for example, pharmaceutical grades of mannitol, lactose, starch, magnesium stearate, sodium saccharine, cellulose, magnesium carbonate, and the like. These compositions can take the form of solutions, suspensions, emulsions, tablets, pills, capsules, caplets, sustained release formulations or powders and contain typically 10%-95% of active ingredient, preferably 25%-70%.

In addition to the above, it is also possible to manufacture live vaccines of attenuated microorganisms, e.g., weakened or avirulent virus particles, including mutated *Cryptovirus* particles, and other attenuated virus particles containing an inventive recombinant nucleic acid encoding one or more *Cryptovirus* proteins, or host cells that express recombinant *Cryptovirus* proteins encoded by inventive expression vectors, as described herein. Suitable attenuated microorganisms are known in the art and include, for example, viruses (e.g., vaccinia virus) as well as bacteria.

Alternatively, killed *Cryptovirus* particles or virions, or killed pseudotyped viral particles or virions bearing *Cryptovirus* envelope proteins, are useful in the manufacture of a vaccine. Virions are killed for vaccine purposes by known methods.

The vaccines are administered in a manner compatible with the dosage formulation, and in such amount as will be prophylactically and/or therapeutically effective. The quantity to be administered, which is generally in the range of about 5 µg to about 250 µg of antigen per dose, depends on the subject to be vaccinated, capacity of the subject's immune system to synthesize antibodies, and the degree of protection desired. Precise amounts of active ingredient required to be administered depend on the judgment of the practitioner and can be particular to each vaccinated animal or human. Any suitable vertebrate animal can be vaccinated, particularly a member of a mammalian species, including rodents, lagomorphs, goats, pigs, cattle, sheep, and primates.

The vaccine may be given in a single dose schedule, or preferably in a multiple dose schedule. A multiple dose schedule is one in which a primary course of vaccination may be with 1-10 separate doses, followed by other doses given at subsequent time intervals required to maintain and/or reinforce the immune response, for example, at 1-4 months for a second dose, and if needed, a subsequent dose(s) after several months. The dosage regimen will also, at least in part, be determined by the immunological characteristics and needs of the individual animal or human to be vaccinated and must be dependent upon the judgment of the practitioner.

In addition, the vaccine containing the *Cryptovirus* proteins described above, can be administered in conjunction with other immunoregulatory agents, for example, immunoglobulins.

Compositions of the present invention can be administered to individual animals or humans to generate polyclonal antibodies (purified or isolated from serum using conventional techniques) which can then be used in a number of applications. For example, the polyclonal antibodies can be used to passively immunize an animal or human, or as immunochemical reagents, as described hereinbelow.

The present invention is also directed to an isolated antibody that specifically binds a *Cryptovirus* protein, such as the *Cryptovirus* NP, V, P, M, F, F₀, F₂, F₁, SH, HN, or L proteins. The term "antibody" refers to a polypeptide or group of polypeptides which are comprised of at least one antibody binding domain. A "binding domain" is formed from the folding of variable domains of an antibody molecule to form three-dimensional binding spaces with an internal surface shape and charge distribution complementary to the features of an epitope of an antigen, which allows an immunological reaction with the antigen. An antibody binding domain can be formed from a heavy and/or a light chain domain (V_{H} and V_{L}, respectively), which form hypervariable loops which contribute to antigen binding. Typical vertebrate antibodies are tetramers or aggregates thereof, comprising light and heavy chains which are typically aggregated in a "Y" configuration and which may or may not have covalent linkages between the chains. In vertebrate antibodies, the amino acid sequences of all the chains of a particular antibody are homologous with the chains found in one antibody produced by the lymphocyte which produces that antibody in vivo, or in vitro (for example, in hybridomas).

An ``isolated" antibody is an antibody, for instance polyclonal antibody, removed from the body of an animal or human that produced it. The inventive polyclonal antibody can be further purified from cellular material, e.g., in blood, lymph, or milk. A preferred embodiment is in the form of an antiserum directed against one or more *Cryptovirus* proteins. Alternatively, an "isolated" antibody of the present invention includes antibodies the production of which involves a manipulation or human intervention, for example, monoclonal antibody or chimeric antibody.

The inventive "antibody" includes any immunoglobulin, including IgG, IgM, IgA, IgD or IgE, or antibody fragment that binds a specific *Cryptovirus* epitope. Such antibodies can also be produced by hybridoma, chemical synthesis or recombinant methods known in the art. (E.g., Sambrook et al., Molecular Cloning: A Laboratory Manual (2 ed.), Cold Spring Harbor Laboratory Press, Cold Spring Harbor, New York, USA [1989]); Harlow and Lane, Antibodies: A Laboratory Manual (Cold Spring Harbor Laboratory [1988]). Both *anti-Cryptovirus* protein and anti-chimeric protein antibodies can be useful within the scope of the present invention. (See, e.g., Bahouth et al., Trends Pharmacol. Sci. 12:338 [1991]; Ausubel et al., Current Protocols in Molecular Biology (John Wiley and Sons, NY [1989]). Examples of chimeric antibodies are discussed in U.S. Pat. Nos. 4,816,397 and 4,816,567. Flurorescent-labeled antibodies, enzyme-conjugated antibodies, or antibodies otherwise labeled for facility of detection, as known in the art, are also included within "antibody."

"Antibody" also includes "chimeric antibody." Chimeric antibodies are antibodies in which the heavy and/or light chains are chimeric proteins. Typically the constant domain of the chains is from one particular species and/or class, and the variable domains are from a different species and/or class. Also included is any antibody in which either or both of the heavy or light chains are composed of combinations of sequences mimicking the sequences in antibodies of different sources, whether these sources be differing classes, or different species of origin, and whether or not the fusion point is at the variable/constant boundary. Thus, it is possible to produce antibodies in which neither the constant nor the variable region mimic known antibody sequences. It then becomes possible, for example, to construct antibodies whose variable region has a higher specific affinity for a particular antigen, or whose constant region can elicit enhanced complement fixation, or to make other improvements in properties possessed by a particular constant region.

Included also within the definition of "antibody" are Fab and F(ab')₂ fragments of antibodies. The "Fab" region refers to those portions of the heavy and light chains which are roughly equivalent, or analogous, to the sequences which comprise the branch portion of the heavy and light chains, and which have been shown to exhibit immunological binding to a specified antigen, but which lack the effector F_{c} portion. A "Fab" fragment is an aggregate of one heavy and one light chain. A F(ab')₂ fragment, which also lacks the effector F_{c} portion, is a tetramer containing the 2H and 2L chains, which are capable of selectively reacting with a designated antigen or antigen family. Methods of producing "Fab" and F(ab')₂ fragments of antibodies are known within the art and include, for example, proteolysis, and synthesis by recombinant techniques. Thus, the inventive anti-*Cryptovirus* antibodies can also be Fab or F(ab')₂ antibody fragments.

Also useful is a "single domain antibody" (dAb), which is an antibody which is comprised of a V_{H} domain, which reacts immunologically with a *Cryptovirus* antigen. A dAB does not contain a V_{L} domain, but may contain other antigen binding domains known to exist in antibodies, for example, the kappa and lambda domains. Methods for preparing dABs are known in the art. (See, e.g., Ward et al., Nature 341: 544 [1989]).

Other preferred embodiments include altered antibodies such as humanized, CDR-grafted or bifunctional, i.e., divalent antibodies, all of which can also be produced by methods well known in the art. "Altered antibodies", which refers to antibodies in which the naturally occurring amino acid sequence in a vertebrate antibody has been varied. Utilizing recombinant DNA techniques, antibodies can be redesigned to obtain desired characteristics. The possible variations are many, and range from the changing of one or more amino acids to the complete redesign of a region, for example, the constant region. Changes in the constant region, in general, to attain desired cellular process characteristics, e.g., changes in complement fixation, interaction with membranes, and other effector functions. Changes in the variable region may be made to alter antigen binding characteristics. The antibody may also be engineered to aid the specific delivery of a molecule or substance to a specific cell or tissue site. The desired alterations may be made by known techniques in molecular biology, e.g., recombinant techniques, site directed mutagenesis, etc.

A preferred embodiment of the inventive antibody specifically binds a *Cryptovirus* envelope protein described hereinabove. Another preferred embodiment of the inventive antibody specifically binds a *Cryptovirus* nucleocapsid protein, but antibodies that specificaally bind any other *Cryptovirus* protein are also useful and preferred.

The inventive antibody can be used, inter alia, in diagnostic or assay methods and systems to detect *Cryptovirus* proteins present in a sample of biological material. With respect to the detection of such polypeptide, the antibodies can be used for in vitro diagnostic or assay methods, or in vivo imaging methods. Such antibodies can also be used for the immunoaffinity or affinity chromatography purification of the inventive *Cryptovirus* proteins.

The present invention includes an in vitro method of detecting the presence or absence of a *Cryptovirus* protein in a sample of a biological material. The method is particularly, but not exclusively, useful for testing clinical or experimental biological materials for diagnostic or pathology purposes.

The sample is contacted with the inventive antibody described herein; and known immunological procedures are employed for detecting specific binding of the antibody to a constituent of the sample. The presence of specific binding indicates the presence of the *Cryptovirus* protein in the sample.

Immunological procedures, useful for in vitro detection of target *Cryptovirus* proteins in a sample, include immunoassay systems that employ the inventive *Cryptovirus* protein-specific antibody in a detectable form.

Known protocols for such immunoassay techniques and systems are based, for example, upon competition, or direct reaction, or sandwich type assays. Such immunoassay techniques and systems include, for example, ELISA, immunoblotting, immunofluorescence assay (IFA), Pandex microfluorimetric assay, agglutination assays, flow cytometry, serum diagnostic assays and immunohistochemical staining procedures which are well known in the art.

An antibody can be made detectable by various means well known in the art. Assay systems that amplify the signals from a primary antibody-antigen complex are also known; examples of which are assays which utilize biotin and avidin, and enzyme-labeled and mediated immunoassays, such as ELISA assays. A detectable marker can be directly or indirectly attached to a primary or secondary antibody in the assay protocol. Useful markers include, for example, radionuclides, enzymes, fluorogens, chromogens and chemiluminescent labels. Embodiments can employ solid support matrices, or can involve immunoprecipitation.

These same immunoassay techniques and systems can be employed in the inventive method of detecting the presence or absence of a *Cryptovirus-specific* antibody in an antibody-containing biological material. Antibody-containing biological materials include, but are not limited to, whole blood and blood components, plasma, serum, spinal fluid, lymph fluid, the external sections of the respiratory, intestinal, and genitourinary tracts, tears, saliva, milk, white blood cells, and myelomas. Processed antibody-containing fractions or dilutions of any of these are also considered antibody-containing biological materials.

One preferred embodiment of the method of detecting the presence or absence of a *Cryptuvirus-specific* antibody involves contacting the sample originating from an individual suspected of having a *Cryptovirus* infection with a viral virion or other viral particle containing one or more Cryptovirus envelope proteins, as described herein, such that, if antibody selectively binding *Cryptovirus* antigen is present, an antibody-bound complex forms. Then any antibody-bound *Cryptovirus* antigen complexes thus formed are contacted with anti-human antibody-binding immunoglobulin or anti-human antibody-binding fragments thereof, for example, Fab and/or F(ab')₂, fragments, and complexes of the immunoglobulin or the fragments thereof, are allowed to form with the antibody-bound *Cryptovirus* complexes. The presence or absence of any complexes formed is detected, by any known immunodetection means as described herein. The presence of such complexes indicates the presence in the sample of antibody that selectively binds *Cryptovirus* antigen.

Another more preferred embodiment involves contacting the sample originating from an individual suspected of having a *Cryptovirus* infection with the inventive *Cryptovirus* envelope protein, such that, if antibody selectively binding *Cryptovirus* is present, an antibody-bound envelope protein complex forms. Any antibody-bound envelope protein complexes thus formed are then contacted with anti-human antibody-binding immunoglobulin or anti-human antibody-binding fragments, such as Fab and F(ab')₂ fragments. The formation of complexes of the immunoglobulin or the Fab and F(ab')₂ fragments thereof is allowed with the antibody-bound envelope protein complexes; and the presence or absence of any antibody-bound envelope protein complexes thus formed is detected. The presence of such complexes indicating the presence in the sample of antibody selectively binding *Cryptovirus.*

The terms "selective" or "specific" binding of antibody to *Cryptovirus* proteins or *Cryptovirus* antigens therein, includes asymmetric cross-reactive binding with closely related rubulaviruses, such as SV5, but does not include non-specific binding to unrelated antigens or surfaces. The skilled artisan is aware of important controls that are preferably included in any immunoassay system for the determination of non-specific antibody binding. Typically, for example in ELISA, a background level of non-specific binding is determined and used as a baseline.

"Complexed" means that a protein, such as an antibody, is a constituent or member of a complex, i.e., a mixture or adduct resulting from chemical binding or bonding between and/or among the other constituents. Chemical binding or bonding can have the nature of a covalent bond, ionic bond, hydrogen bond, hydrophobic bond, or any combination of these bonding types linking the constituents of the complex at any of their parts or moieties, of which a constituent can have one or a multiplicity of moieties of various sorts. Antibody-antigen binding is typically non-covalent. Not every constituent of a complex need be bound to every other constituent, but each constituent has at least one chemical bond with at least one other constituent of the complex. For example, a secondary antibody in the assay system may not be directly bound with the *Cryptovirus* antigen, or the viral particle or virion, yet it is "complexed" with it.

By way of further non-limiting illustration of a clinical diagnostic embodiment for detecting *Cryptovirus* infection in a human patient, a serum specimen from the patient is screened for *Cryptovirus-specific* antibodies to the major envelope proteins of the virus (F₀ and HN) by ELISA, radio-immunoprecipitation, immunoblotting techniques or any other immunological technique (e.g., direct or indirect fluorescent antibody techniques, immunobeads, *etc.).* Optionally, another blood sample is obtained from any seropositive individual. The virus can be detected in the PBMNCs of such samples by isolating the cells on a suitable gradient medium, culturing the cells in the presence of cyclic GMP and then either (1) screening the cells for intracellular *Cryptovirus-specific* inclusions with *Cryptovirus-specific* fluorescent antibodies or (2) PCR amplification of induced PBMNC with *Cryplovirus-specific* nucleotide primers.

While *Cryptovirus*-specific antibodies have been found in the serum of seropositive individuals, indicating current or former *Cryptovirus* infection, these antibodies are not necessarily indicative of epileptiform or encephalopathic disease. *Cryptovirus* appears to infect and be carried in the PBMNCs of a significant proportion of individuals without necessarily causing encephalopathic disease. These individuals can overtly appear to be asymptomatic. The neuropathological (e.g., epileptiform, encephalopathic, and other neurological, neurodegenerative, and/or neuropsychiatric disease) potential of the virus only appears to become manifest in individuals in whom the virus has infected nervous system tissues. Consequently, only *Cryptovirus-specific* antibodies (i.e. those directed against the major envelope proteins of the virus, F and HN) found in the cerebrospinal fluid (CSF) are fully indicative of neurological, neurodegenerative, and/or neuropsychiatric disease and then, they are virtually always indicative. By way of further example, ten human patients who were previously diagnosed with chronic fatigue syndrome involving significant short-term memory loss, and for whom CSF samples could be obtained, were all found to have *Cryptovirus-specific* antibody in their CSF and electroencephalographic profiles consistent with a diagnosis of absence epilepsy (i.e., *petit mal.* epilepsy).

The present invention also relates to an anti*-Cryptovirus* antibody detecting kit, useful for testing or assaying a biological sample, in particular an antibody-containing biological material. Thus, the inventive kits are beneficial for screening clinical supplies of human blood, serum, platelets, plasma, tissues and organs, to determine their safety for transfusion or transplantation purposes. Diagnostic applications of the inventive kits are also useful.

The inventive kit is particularly useful for practicing the inventive assay methods for detecting antibody that selectively binds *Cryptovirus* and its antigens and the inventive methods of detecting the presence or absence of a *Cryptovirus*-specific antibody. In some preferred embodiments, the kit contains an isolated *Cryptovirus* particle, comprising a genome having a nucleotide sequence entirely complementary to (SEQ ID NO:1). The kit also contains labeled anti-human antibody-binding antibody, preferably anti-human immunoglobulin or labeled anti-human antibody-binding antibody fragments, such as Fab and/or F(ab')₂. A preferred embodiment of the kit further contains a solid matrix for supporting the *Cryptovirus* particle(s). In a preferred embodiment, the Cryptovirus particles in the anti-*Cryptovirus* antibody detecting kit are *Cryptovirus* virions, and in a preferred embodiment the *Cryptovirus* virions are produced from an acutely *Cryptovirus*-infected cell line, such as an acutely infected baby hamster kidney (BHK) cell-derived cell line, a Vero-derived cell line, or a CV-1-derived cell line (e.g., a CV-1_{c}-derived cell line, described hereinbelow, and deposited with ATCC as Accession No.).

Alternatively, in a most preferred embodiment of the inventive *anti-Cryptovirus* antibody detecting kit, which does not include *Cryptovirus* particles, the kit includes a plurality of one or more kinds of isolated *Cryptovirus* proteins and/or chimeric proteins comprising a *Cryptovirus* protein moiety, as described hereinabove. In some preferred embodiments, the Cryptovirus protein or protein moiety is an envelope protein, as described herein. Such embodiments also contain labeled anti-human antibody-binding antibody, such as anti-human immunoglobulin or labeled anti-human antibody-binding antibody fragments, such as Fab and/or F(ab')₂ fragments. A preferred embodiment of such a kit further contains a solid matrix for supporting the *Cryptovirus* proteins.

Solid matrices, or supports, and methods for attaching or adsorbing viral particles and proteins to a solid matrix, are well known in the art. In accordance with the inventive kits, the term "solid matrix" includes any solid or semi-solid support or surface to which the viral particle or protein can be anchored or adhered. Suitable matrices are made of glass, plastic, metal, polymer gels, and the like, and may take the form of beads, wells (e.g., single- or multi-well serum plates) slides, dipsticks, membranes, and the like.

As is known to the skilled artisan in using such kits, e.g., for ELISA, RIA, or sandwich-type assays, the biological sample, optimally solubilized or suspended and in an optimized dilution, is contacted with the viral particles or proteins, typically supported on the surface of the solid matrix (e.g., well of a serum plate), and after appropriate washes and incubations, is further contacted with the labeled anti-human antibody-binding immunoglobulin or labeled anti-human antibody-binding fragments. After further washes, commercially available plate readers and/or other accessory detection equipment are typically employed in conjunction with the inventive kit, for detecting the formation of bound anti-human antibody complexes with human antibody that has bound to *Cryptovirus* particles or proteins.

Instructions for use are included in the kit. "Instructions for use" typically include a tangible expression describing the reagent concentration or at least one assay method parameter, such as the relative amounts of reagent and sample to be admixed, maintenance time periods for reagent/sample admixtures, temperature, buffer conditions, incubations, washes, and the like, typically for an intended purpose, in particular the inventive assay methods as described herein.

Optionally, the kit also contains other useful components, such as, diluents, buffers, or other acceptable carriers, specimen containers, syringes, pipetting or measuring tools, paraphernalia for concentrating, sedimenting, or fractionating samples, or the inventive antibodies for use in controls.

The materials or components assembled in the kit can be provided to the practitioner stored in any convenient and suitable ways that preserve their operability and utility. For example the components can be in dissolved, dehydrated, or lyophilized form; they can be provided at room, refrigerated or frozen temperatures.

The components are typically contained in suitable packaging material(s). As employed herein, the phrase "packaging material" refers to one or more physical structures used to house the contents of the kit. The packaging material is constructed by well known methods, preferably to provide a sterile, contaminant-free environment.

The packaging materials employed in the kit are those customarily utilized in virus- and peptide-based systems. As used herein, the term "package" refers to a suitable solid matrix or material such as glass, plastic, paper, foil, and the like, capable of holding the individual kit components. Thus, for example, a package can be a glass vial used to contain suitable quantities of an inventive composition containing viral or peptide components. The packaging material generally has an external label which indicates the contents, quantities, and/or purpose of the kit and/or its components.

Thus the present invention provides immunoassay methods and kits, useful for research, clinical diagnostics, and screening of blood, blood components or products, and tissue and organs intended for transfusion or transplantation. These applications are of great value and utility, because strong evidence shows that peripheral blood mononuclear cells (PBMNCs) can harbor *Cryptovirus* and can subsequently infect and cause disease in a patient who receives such contaminated blood, blood products, tissues, or cells.

The inventive methods of detecting the presence or absence of a *Cryptovirus-specific* RNA in a sample of a biological material, described hereinabove, are also particularly useful for these and other purposes. In accordance with the most preferred embodiments of the method, *Cryptovirus* RNAs are amplified by any of numerous known methods of amplifying nucleic acid segments, in the form of RNA or cDNA. Before amplification, it is preferable, but not necessary, to extract or separate RNA from DNA in the sample and to amplify nucleic acids remaining in that fraction of the sample separated from the DNA. The amplifications products, if any, are then analyzed to detect the presence of *Cryptovirus-specific* amplification products. If *Cryptovirus*-specific amplification products are present, the findings are indicative of the presence of *Cryptovirus* RNA in the sample. For greater confidence in the interpretation of negatives (i.e., no detectable *Cryptovirus*-specific amplification products), analysis is optionally carried out following a control amplification of mRNAs specific for a cellular housekeeping gene, for example, a gene encoding β-actin, phosphofructokinase (PFK), glyceraldehyde 3-phosphate dehydrogenase, or phosphoglycerate kinase.

The RNAs in the sample, are amplified by a suitable amplification method. For example, in a preferred embodiment, a reverse transcriptase-mediated polymerase chain reaction (RT-PCR) is employed to amplify *Cryplovirus-specific* nucleic acids. Briefly, two enzymes are used in the amplification process, a reverse transcriptase to transcribe *Cryptovirus-specific* cDNA from a *Cryptovirus-specific* RNA template in the sample, a thermal resistant DNA polymerase (e.g., *Taq* polymerase), and *Cryptovirus-specific* primers to amplify the cDNA to produce *Cryptovirus-specific* amplification products. The use of limited cycle PCR yields semi-quantitative results. (E.g., Gelfand *et al., Reverse transcription with thermostable DNA polymerase-high temperature reverse transcription,* U.S. Patent Nos. 5,310,652; 5,322,770; Gelfand *et al., Unconventional nucleotide substitution in temperature selective RT-PCR,* U.S. Patent No. 5,618,703).

In another preferred embodiment of the inventive method, single enzyme RT-PCR is employed to amplify *Cryptovirus-specific* nucleic acids. Single enzymes now exist to perform both reverse transcription and polymerase functions, in a single reaction. For example, the Perkin Elmer recombinant *Thermus thermophilus* (rTth) enzyme (Roche Molecular), or other similar enzymes, are commercially available. Cycling instruments such as the Perkin Elmer ABI Prism 7700, the so-called Light Cycler (Roche Molecular), and/or other similar instruments are useful for carrying out RT-PCR. Optionally, single enzyme RT-PCR technology, for example, employing rTth enzyme, can be used in a PCR system.

By way of illustration only, RT-PCR-based testing is quite sensitive for detection of the virus. For example, a RT-PCR-priming technique has been used to confirm a detectable *Cryptovirus* carriage rate in PBMNCs nonproductively harboring the virus (without culturing, cyclic GMP induction, and passaging) on the order of 1:10⁵ PBMNC. In addition, numerous fragments of the *Cryptovirus* genome have been cloned from AV₃/SSPE cells using *Cryptovirus-specific* primers and a RT-PCR-based amplification technique.

Preferably, amplification and analysis are carried out in an automated fashion, with automated extraction of RNA from a sample, followed by PCR, and fluorescence detection of amplification products using probes, such as TaqMan or Molecular Beacon probes. Typically, the instrumentation includes software that provides quantitative analytical results during or directly following PCR without further amplification or analytical steps.

In another preferred embodiment, transcription-mediated amplification (TMA) is employed to amplify *Cryptovirus-specific* nucleic acids. (E.g., K. Kamisango et al., Quantitative detection of hepatitis B virus by transcription-mediated amplification and hybridization protection assay, J. Clin. Microbiol. 37(2):310-14 [1999]; M. Hirose et al., New methods to measure telomerase activity by transcription-mediated amplification and hybridization protection assay, Clin. Chem. 44(12)2446-52 [1998]). Rather than employing RT-PCR for the amplification of a cDNA, TMA uses a probe that recognizes a *Cryptovirus-specific* (target sequence) RNA; in subsequent steps, from a promoter sequence built into the probe, an RNA polymerase repetitively transcribes a cDNA intermediate, in effect amplifying the original RNA transcripts and any new copies created, for a level of sensitivity approaching that of RT-PCR. The reaction takes place isothermally (one temperature), rather than cycling through different temperatures as in PCR.

Other useful amplification methods include a reverse transcriptase-mediated ligase chain reaction (RT-LCR), which has utility similar to RT-PCR. RT-LCR relies on reverse transcriptase to generate cDNA from mRNA, then DNA ligase to join adjacent synthetic oligonucleotides after they have bound the target cDNA.

Most preferably, amplification of a *Cryptovirus-specific* nucleic acid segment in the sample can be achieved using *Cryptovirus-specific* oligonucleotide primers of the present invention, as described herein.

Optionally, high throughput analysis may be achieved by multiplexing techniques well known in the art, employing multiple primer sets, for example primers directed not only to *Cryplovirus-specific* nucleic acids, but to amplifying expression products of housekeeping genes (controls) or of other potential diagnostic markers, to yield additional diagnostic information. (E.g., Z. Lin et al., Multiplex genotype determination at a large number of gene loci, Proc. Natl. Acad. Sci. USA 93(6):2582-87 [1996]; Demetriou *et al., Method and probe for detection of gene associated with liver neoplastic disease,* U.S. Patent No. 5,866,329).

Hybridization analysis is a preferred method of analyzing the amplification products to detect the presence or absence of *Cryptovirus-specific* nucleic acid amplification products, employing one or more *Cryptovirus-specific* probe(s) that, under suitable conditions of stringency, hybridize(s) with single stranded *Cryptovirus-specific* nucleic acid amplification products comprising complementary nucleotide sequences. The amplification products are typically deposited on a substrate, such as a cellulose or nitrocellulose membrane, and then hybridized with labeled *Cryptovirus-specific* probe(s), optionally after an electrophoresis. Conventional dot blot, Southern, Northern, or fluorescence in situ (FISH) hybridization protocols, *in liquid* hybridization, hybridization protection assays, or other semi-quantitative or quantitative hybridization analysis methods are usefully employed along with the *Cryptovirus-specific* probes of the present invention. As is readily apparent to the skilled artisan, such analytical hybridization techniques and others (e.g., Northern blotting), are useful in accordance with other embodiments of the inventive method of detecting the presence or absence of a *Cryptovirus*-specific RNA in a sample of a biological material that do not involve any amplification step(s). In these embodiments, the inventive *Cryptovirus-specific* probes are contacted directly with RNA in the sample to perform hybridization analysis.

Alternatively, electrophoresis for analyzing or detecting amplification products is done rapidly and with high sensitivity by using any of various methods of conventional slab or capillary electrophoresis, with which the practitioner can optionally choose to employ any facilitating means of nucleic acid fragment detection, including, but not limited to, radionuclides, UV-absorbance or laser-induced fluorescence. (K. Keparnik et al.., Fast detection of a (CA)18 microsatellite repeat in the IgE receptor gene by capillary electrophoresis with laser-induced fluorescence detection, Electrophoresis 19(2);249-55 [1998]; H. Inoue et al., Enhanced separation of DNA sequencing products by capillary electrophoresis using a stepwise gradient of electric field strength, J. Chromatogr. A. 802(1):179-84 [1998]; N.J. Dovichi, DNA sequencing by capillary electrophoresis, Electrophoresis 18(12-13):2393-99 [1997]; H. Arakawa et al., Analysis of single-strand conformation polymorphisms by capillary electrophoresis with laser induced fluorescence detection, J. Phann. Biomed. Anal. 15(9-10):1537-44 [1997]; Y. Baba, Analysis of disease-causing genes and DNA-based drugs by capillary electrophoresis. Towards DNA diagnosis and gene therapy for human diseases, J. Chromatgr B. Biomed. Appl. 687(2):271-302 [1996]; K.C. Chan et al., High-speed electrophoretic separation of DNA fragments using a short capillary, J. Chromatogr B. Biomed. Sci. Appl. 695(1):13-15 [1997]).

Any biological material can be sampled for the purpose of practicing the inventive methods of detecting the presence or absence of a *Cryptovirus-specific* protein or *Cryptovirus-specific* RNA in a sample of a biological material. Preferred biological materials for sampling include blood or serum, lymphoid tissue, nervous tissue, including brain tissue. However, the biological material can be cerebrospinal fluid (CSF), lymph, plasma, feces, semen, prostatic fluid, tears, saliva, milk, gastric juice, mucus, synovial fluid, pleural effusion, peritoneal effusion, pericardial effusion, skin, vascular epithelium, oral epithelium, vaginal epithelium, cervical epithelium, uterine epithelium, intestinal epithelium, bronchial epithelium, esophageal epithelium, or mesothelium, or other biopsy sample of cellular material from any tissue. Cellular material includes any sample containing mammalian cells, including samples of tissues, expressed tissue fluids, tissue wash or tissue rinsate fluids, blood cells (e.g., peripheral blood mononuclear cells), tumors, organs, and also samples of in vitro cell culture constituents (including but not limited to conditioned medium resulting from the growth of cells in cell culture medium, putatively virally infected cells, recombinant cells, and cell components), or the like.

Tissue samples that can be collected include, but are not limited to, cell-containing material from the brain, blood, spleen, lymph node, vasculature, kidney, pituitary, ureter, bladder, urethra, thyroid, parotid gland, submaxillary gland, sublingual gland, bone, cartilage, lung, mediastinum, breast, uterus, ovary, testis, prostate, cervix uteri, endometrium, pancreas, liver, adrenal, esophagus, stomach, and/or intestine.

The sample is alternatively derived from cultured mammalian cells, cell-free extracts, or other specimens indirectly derived from a mammalian subject's body, as well as from substances taken directly from a subject's body. The samples can be stored before detection methods are applied (for example nucleic acid amplification and/or analysis, or immunochemical detection) by well known storage means that will preserve nucleic acids or proteins in a detectable and/or analyzable condition, such as quick freezing, or a controlled freezing regime, in the presence of a cryoprotectant, for example, dimethyl sulfoxide (DMSO), glycerol, or propanediol-sucrose. Samples may also be pooled before or after storage for purposes of amplifying their *Cryplovirus-specific* nucleic acids for analysis and detection, or for purposes of detecting *Cryptovirus* protein.

The sample is optionally pre-treated by refrigerated or frozen storage overnight, by dilution, by phenol-chloroform extraction, or by other like means, to remove factors that may inhibit various amplification reactions that may be employed; such as heme-containing pigments or urinary factors. (E.g., J. Mahony et al., Urine specimens from pregnant and non-pregnant women inhibitory to amplification of Chlamydia trachomatis nucleic acid by PCR, ligase chain reaction, and transcription-mediated amplification: identification of urinary substances associated with inhibition and removal of inhibitory activity, J. Clin. Microbiol. 36(11):3122-26 [1998]).

The present invention is also directed to an animal model for the study of human diseases, preferably, but not limited to, neurological, neurodegenerative, and/or neuropsychiatric diseases. The term "neurological diseases" refers to diseases of the nervous system, including neuropathies manifested in the central nervous system and/or the peripheral nervous system. These include epileptiform diseases and non-epileptiform CNS diseases (e.g. Parkinsonism) and peripheral nervous system disease(s) (e.g. amyotrophic lateral sclerosis or "Lou Gehrig's Disease." "Neurodegenerative" diseases involve wasting or paralytic neurological diseases, which typically present with tremor, weakness and atrophy, for example Lou Gehrig's Disease or Alzheimer's disease. The terms "neuropsychiatric" and "neuropsychological" are used interchangeably herein. "Neuropsychiatric diseases" are neurological diseases that also include behavioral symptoms that derive from the underlying neurophysiological processes. The animal model is a non-human mammal, such as, but not limited to, a rodent, a lagomorph, or a non-human primate.

A rodent is any of the relatively small gnawing mammals of the order Rodentia , such as mice, rats, hamsters, guinea pigs, squirrels, marmots, beavers, gophers, voles, porcupines, and agoutis. A lagomorph is any of various herbivorous mammals belonging to the order Lagomorpha, which includes rabbits, hares, and pikas.

The animal is, or has been, inoculated with an infectious cell-free *Cryptovirus* virion of the present invention. Alternatively, the animal is, or has been, artificially inoculated with a cell nonproductively-infected with *Cryptovirus,* such as AV₃/SSPE.

Inoculation can be by a peripheral or an intracerebral delivery route. For the study of neurological, neurodegenerative, and/or neuropsychiatric diseases, intracerebral inoculation is preferred, although for diseases presenting with involvement of the peripheral nervous system, peripheral inoculation can also be useful.

Intracerebral inoculation is by any suitable means, but preferably by direct injection into the brain, preferably into neural tissue, and most preferably by stereotactic injection means known in the art. Alternatively, intracerebral inoculation with *Cryptovirus* or nonproductitively infected cells can be by intraarterial (e.g., intracarotid) or intravenous injection or infusion, in conjunction with at least transient disruption of the blood brain barrier by physical or chemical means, delivered simultaneously with the *Cryptovirus* or nonproductively infected cells.

"Simultaneously" means that the physical or chemical means for disrupting the blood brain barrier are administered contemporaneously or concurrently with the *Cryptovirus* virions or nonproductively infected cells. "Simultaneously" also encompasses disrupting means being administered within about one hour after the *Cryptovirus* or nonproductively infected cells are last administered, preferably within about 30 minutes after, and most preferably, being administered simultaneously with the *Cryptovirus* or nonproductively infected cells. Alternatively, "simultaneously" means that the medicant is administered within about 30 minutes before, and preferably within about 15 minutes before the *Cryptovirus* or nonproductively infected cells are first administered.

Physical disruption of the blood brain barrier includes by means of "mechanical" injury or other physical trauma that breaches the blood brain barrier in at least one location of the brain's vasculature. Chemical disruption includes by an agent that transiently permeabilizes the blood-brain barrier and allows the *Cryptovirus* to enter the brain from the blood stream via the brain microvasculature. Such permeabilizing agents are known, for example, bradykinin and bradykinin analogs, and activators of calcium-dependent or ATP-dependent potassium channels. (e.g., B. Malfroy-Camine, *Method for increasing blood-brain barrier permeability by administering a bradykinin agonist of blood-brain barrier permeability,* U.S. Patent No. 5,112,596; J.W. Kozarich *et al., Increasing blood brain barrier permeability with permeabilizer peptides,* U.S. Patent No. 5,268,164; Inamura, T. et al., Bradykinin selectively opens blood-tumor barrier in experimental brain tumors, J. Cereb. Blood Flow Metab. 14(5):862-70 [1994]; K.L. Black, *Method for selective opening of abnormal brain tissue capillaries,* U.S. Patent Nos. 5,527,778 and 5,434,137; N.G. Rainov, Selective uptake of viral and monocrystalline particles delivered intra-arterially to experimental brain neoplasms, Hum. Gene. Ther. 6(12):1543-52 [1995]; N.G. Rainov et al., Long-term survival in a rodent brain tumor model by bradykinin-enhanced intra-arterial delivery of a therapeutic herpes simplex virus vector, Cancer Gene Ther. 5(3):158-62 [1998]; F.H. Barnett et al., Selective delivery of herpes virus vectors to experimental brain tumors using RMP-7, Cancer Gene Ther. 6(1):14-20 [1999]; WO 01/54771 A2; and WO 01/54680 A2).

The inoculated non-human mammal exhibits at least one symptom characteristic of a human neurological, neurodegenerative, and/or neuropsychiatric disease after being thus inoculated, which was not previously exhibited by the non-human mammal before inoculation. Such symptoms include subacute symptoms and more slowly developing symptoms.

Generally, the subacute symptoms (developing from about 3 weeks to about 2 months post inoculation) associated with such experimental infections include: (1) cachexia/anorexia (i.e., wasting or diminution of body mass and size); (2) degenerative neurologic wasting or paralysis; (3) atrophy of limb(s); (4) hindlimb paralysis; (5) photosensitivity or repetitive blinking; (6) hyperactivity or hyperesthesia (e.g., nervousness, agitation, racing, jumpiness, extreme sensitivity to touch and sound); (7) ataxia (i.e., loss of balance, wobbly gait); (8) hypesthesia; (9) withdrawal and isolation from other animals, closing of eyes, "hunched" posture; (10) stupor *(i.e.,* rigidity, semi-comatose, somnambulant motionlessness); (11) convulsions or seizures (i.e., flaying of limbs, loss of consciousness, whirling, rolling and/or circling); (12) muscle spasms or myoclonus (e.g., tremor, twitching of muscles, repetitive jerking of muscles); (13) corneal opacity (a clouding of the cornea) and (14) sudden death. Individual animals can present with one or more of the preceding subacute symptoms, but are generally observed displaying a complex of two or more symptoms. Subacute symptoms are more frequently observed in male animals compared to female animals.

More slowly developing symptoms (i.e., those developing after about two months and sometimes not for about six months or more after inoculation) include: (1) obesity; (2) hypesthesia (i.e., decreased sensitivity to sensory stimuli); (3) extreme lethargy and prolonged sleeping; (4) hyperactivity or hyperesthesia (i.e., increased sensitivity to sensory stimuli); (5) aggressiveness (e.g., jumping or biting); (6) obsessive compulsive behavior (e.g., excessive and prolonged washing of the face or continual scratching); (7) self-mutilation (the extreme end of obsessive compulsive washing or scratching where the skin is damaged); (8) still-born fetuses and deformities in newborn animals (usually paralysis or limb atrophy) born to experimentally-infected females; and (9) infanticide (cannibalism of numerous newborns or entire litters). Individual animals can present with one or more of the preceding more slowly developing symptoms, but are generally observed displaying a complex of two or more symptoms. More slowly developing symptoms are more frequently observed in female animals compared to male animals.

The inventive animal model is an excellent model system for the study of neurodegenerative, wasting or paralytic neurological diseases which typically present with tremor, weakness and atrophy. The inventive animal model is also, in particular, an excellent model system for the study of idiopathic epileptiform diseases because the infected animals present with virtually the entire spectrum of symptoms associated with epileptiform illnesses in humans. At present, most existing animal models of epilepsy (e.g., induction of seizure by inoculation with the glutamate receptor agonist, kainite, or by partial suffocation) are contrived to produce seizures and the gross anatomical pathology associated with seizures without reference to the etiology of the actual symptomatic spectrum of the illnesses in humans. While these models are useful in developing therapeutics for seizure activity, there is little or no evidence that they are relevant to the ultimate aetiopathogenesis of epileptiform illnesses in humans or the actual spectrum of symptoms which occur.

In contrast, the animal model of the present invention is a truly homologous animal model; that is, one in which the actual factors/symptoms associated with the disease in humans are extant and can be specifically targeted by both therapeutic and prophylactic strategies. Thus, the inventive animal models disclosed herein can be used to screen antiviral medications or medicaments, including anti-epileptic and anti-psychotropic medicaments, as well as to test vaccines and other prophylactic remedies and to determine how to best coordinate and optimize any and all treatment strategies.

*Cryptovirus* is mildly cytopathic in cell culture but causes profound neuropathological disease in experimentally-infected animals. Any of the cytopathogenic and neuropathogenic traits of the virus can be used as markers in screens designed to identify and test potential antiviral therapeutic and/or prophylactic agents.

Accordingly, the present invention features *in vitro* and *in vivo* methods of screening potential antiviral therapeutic agents and/or antiviral prophylactic agents, including immunoprophylactic agents. A "potential" antiviral therapeutic or prophylactic agent is an agent that has not yet been clinically confirmed (i.e., in phase III clinical trials) to have antiviral properties effective against *Cryptovirus.* Agents that have not been tested clinically against *Cryptovirus* infections or have been tested clinically against *Cryptovirus* infections only with respect to phase I and phase II clinical trials are also encompassed by "potential" antiviral therapeutic and/or prophylactic agents for purposes of the present invention.

In accordance with the inventive *in vitro* methods of screening a potential therapeutic or prophylactic agent, either acutely- or productively-infected mammalian cell cultures (e.g., BHK, Vero, or CV-1_{c} cells) or nonproductively infected carrier cultures (e.g., AV₃/SSPE cells) can be used to evaluate the potential antiviral agent. While the acutely infected (productive) cellular system is preferentially useful for screening agents targeted at the processing and assembly of *Cryptovirus* envelope glycoproteins (e.g., protease inhibition of F₀ cleavage activation), the nonproductively infected cellular system (e.g., AV₃/SSPE cells) is preferred for screening for the efficacy of long-term treatment with transcriptional or other polymerase inhibitors (inhibiting the buildup of intracellular nucleocapsids and the eventual triggering of apoptotic cell death).

The inventive animal model is usefully employed in the *in vivo* method of screening a potential antiviral therapeutic agent. The method involves administering the potential therapeutic agent to be screened, to the inventive animal model after its inoculation with *Cryptovirus,* in accordance with the inventive animal model.

An alternative embodiment of the inventive animal model is employed in the *in vivo* method of screening a potential antiviral prophylactic agent. The method involves administering a potential prophylactic agent to be screened to a non-human mammal, which does not have a symptom of a human disease, such as but not limited to a neurological, neurodegenerative, and/or neuropsychiatric disease (e.g., an epileptiform disease). Then the animal is inoculated, as described herein, with the infectious cell-free *Cryptovirus* or with the mammalian cell nonproductively-infected with the *Cryptovirus.* The method is particularly, but not exclusively, useful for identifying potential anti-epileptic or anti-psychotropic antiviral prophylactic agents.

Administration of the potential prophylactic agent or therapeutic agent is by any suitable delivery route, enteral (e.g., orally or by suppository) or parenteral (e.g., by injection, infusion, transmembrane, transdermal, or inhalation delivery route).

Examples of agents that can be evaluated, in accordance with the invention, include compounds or substances with known antiviral properties against viruses other than *Cryptovirus;* novel compounds or mixtures of compounds, such as cell, plant or animal extracts, with potential antiviral activity; and vaccines, as described hereinabove; or any combination of these.

Using the inventive in vivo method of screening, potential immunoprophylactic agents *(i.e.,* vaccines which stimulate the immune system to respond to, attack or inhibit virus replication, assembly or any other process associated with virus reproduction and spread) are also amenable to testing because non-human mammals can be inoculated with a putative prophylactic agent or vaccine (as mentioned above) and then challenged with infectious *Cryptovirus* to assess its utility in preventing the development of Cryptovirus-associated diseases. The use of such agents discovered in accordance with the invention may ultimately be necessary to control and eradicate *Cryptovirus-*associated diseases in the human population much as measles and mumps vaccines have been used to bring these diseases under control in many countries.

In addition to those named above, one of ordinary skill in the art will recognize numerous potential antiviral chemo- and molecular-therapeutic agents that could be analyzed or evaluated using the *in vitro* (cell culture) or *in vivo* methods of screening provided herein. These potential antiviral therapeutic and/or prophylactic agents can include existing antiviral agents known to affect viruses other than *Cryptovirus (e.g.,* Ribavirin^{™}, which is also known as Virazole^{™}) and new potential antiviral agents. For example, molecular therapeutic agents (e.g., anti-sense nucleotides and ribozymes) or protease inhibitors can also be tested using the inventive in vitro and/or in vivo methods of screening. Agents that might inhibit the cleavage of the viral fusion protein (F₀) can be sought, and these could be particularly valuable, as there is evidence that cleavage of the fusion protein and its association with the viral hemagglutinin/neuraminidase protein (HN) are critical events in determining the pathogenicity of infections by other Paramyxovirdae (Yao et al., J. Virol. 71: 650-656, 1997). Further, these potential antiviral agents may be directed, for example, at *Cryptovirus* replication or assembly, or the expression or activity of *Cryptovirus* genes and proteins, such as, but not limited to, the *Cryptovirus-encoded* RNA-dependent RNA polymerase comprising the L protein and its companion P and V proteins. The inventive screening methods also can be employed to develop broad-spectrum antiviral agents, effective against viruses other than *Cryptovirus.*

Immunotherapeutic agents, such as those that attack, or stimulate the immune system to attack, infected cells or virus particles (by, e.g., passive antibody administration or introduction of *Cryptovirus-specific* monoclonal antibodies) are also amenable to testing because they can block or inhibit the assembly, release or cell-to-cell transfer of virions. However, administration of these agents may be of only limited value in "curing" persistent and chronic *Cryptovirus* infections because the virus appears to survive *in situ* by shutting off production of its envelope proteins and going into a "latent" or inapparent state, in which it appears to be undetectable by the immune system.

Appropriate amounts of potential prophylactic or therapeutic agents vary and are determined by routine screening.

In accordance with the inventive *in vivo* methods of screening a potential therapeutic agent or prophylactic agent, the agent is evaluated for an ability to induce, create, bring about, or result in a beneficial antiviral effect in the inventive animal model. A "beneficial antiviral effect" includes the prevention of infection with *Cryptovirus* or a reduction in the duration or severity of at least one symptom associated with *Cryptovirus* infection, in the animal subjected to the assay, compared to tissues in control animals. A "beneficial antiviral effect" also includes a prevention or reduction of cytopathic effect (CPE) in tissues sampled from the animal subjected to assay by the screening method. Also encompassed by a "beneficial antiviral effect" is an inhibitory effect on *Cryptovirus* replication and/or *Cryptovirus* virion assembly (e.g., inhibitory effect on *Cryptovirus* genomic replication, *Cryptovirus* transcription, and/or translation, i.e., protein synthesis, from *Cryptovirus* mRNAs, or a diminution in the numbers of *Cryptovirus* virions produced or a relative lack of completeness of *Cryptovirus* particles, compared to a suitable control), which effect is measured by known means in cells or tissues sampled from the animal subjected to the assay.

Appropriate controls for use in the screening methods will be self-evident to the skilled artisan. Such controls can include: (1) animals administered with the same potential prophylactic or therapeutic agent and challenged with sterile artificial aqueous culture medium alone or the culture medium containing a strain of SV5; (2) animals mock-treated with saline (or the same carriers used in delivering the potential prophylactic or therapeutic agent) and challenged with *Cryptovirus;* and (3) animals mock-treated with saline (or the same carriers used in delivering the potential prophylactic or therapeutic agent) and challenged with sterile artificial aqueous culture medium alone or the culture medium containing a strain of SV5.

The practice of the present invention will employ, unless otherwise indicated, conventional or other known techniques of biochemistry, molecular biology, microbiology, virology, recombinant nucleic acid technology, and immunology, which are within the skill of the art. Such techniques are explained fully in the literature. (e.g., Maniatis et al., Molecular Cloning: A Laboratory Manual, Cold Spring Harbor Laboratory Press, Cold Spring Harbor, New York, USA (1982); DNA Cloning, Vols. I and II (D. N Glover ed. 1985); Sambrook et al., Molecular Cloning: A Laboratory Manual (2 ed.), Cold Spring Harbor Laboratory Press, Cold Spring Harbor, New York, USA (1989); Davis et al., Basic Methods in Molecular Biology, Elsevier Science Publishing, Inc., New York, USA (1986); or Methods in Enzymology: Guide to Molecular Cloning Techniques Vol. 152, S. L. Berger and A. R. Kimmerl Eds., Academic Press Inc., San Diego, USA (1987); Oligonucleotide Synthesis (M. J. Gait ed, 1984); Nucleic Acid Hybridization (B. D. Hames & S. J. Higgins eds. 1984); Transcription And Translation (B. D. Hames & S. J. Higgins eds. 1984); Animal Cell Culture (R. 1. Freshney ed. 1986); Immobilized Cells And Enzymes (IRL Press, 1986); B. Perbal, A Practical Guide To Molecular Cloning (1984); the series, Methods In Enzymology (Academic Press, Inc.); Gene Transfer Vectors For Mammalian Cells (J. H. Miller and M. P. Calos eds. 1987, Cold Spring Harbor Laboratory), Methods in Enzymology Vol. 154 and Vol. 155 (Wu and Grossman, and Wu, eds., respectively), Mayer and Walker, eds. (1987), Immunochemical Methods In Cell And Molecular Biology (Academic Press, London), Scopes, (1987), Protein Purification: Principles And Practice, Second Edition (Springer-Verlag, N.Y.), and Handbook Of Experimental Immunology, Volumes I-IV (D. M. Weir and C. C. Blackwell eds 1986).

The invention will now be described in greater detail by reference to the following non-limiting examples.

### EXAMPLES

### Example 1. Detection of Cryptovirus in Infected Cells and Isolation and Purification of Cryptovirus Viral Particles.

In accordance with the present invention, cell-free *Cryptovirus* particles were recovered from cells of the buffy coat (peripheral blood mononuclear cells; PBMNC) obtained from the peripheral blood of patients with SSPE. The technique used was modified from that of Robbins et al. (J. Infect. Dis. 143:396-403, 1981). Modifications included the addition of cyclic GMP (to a final concentration of 1 mM) to the aqueous culture medium, in accordance with the present invention, which medium was added to the initial PBMNC cultures and the primary cocultivate with human amnion cells (AV₃). Results were further optimized by using as the mammalian epithelial cell line in a co-cultivation step with the PBMNCs and amnion cells, a clonal subline of CV-1 cells (CV-1_{c}).

Successful isolation of the virus requires viable PBMNCs. Such PBMNCs were separated from other blood components by standard procedures on Ficoll-Hypaque™ gradient media. After centrifugation, the buffy coat cells banded at the interface of the media and were removed with a sterile pipette. They were then gently washed by dilution in 50 volumes of RPMI cell culture media containing 1-2% fetal calf serum and centrifuged in a table-top refrigerated centrifuge (1000 rpm for 5 minutes). The pelleted PBMNCs were then diluted to 2 x 10⁵ cells per ml in RPMI media containing 10% fetal calf serum and 1 mM cyclic GMP (sodium salt) and incubated without disturbance at 37°C for 12-18 hours. Following this incubation, the cultures were seeded with sufficient AV₃ cells in Richter's Modified Minimal Essential Medium (IMEMZO) (supplemented with insulin, zinc and HEPES buffer, 2 mM L-glutamine, 200 Units penicillin/mL, 100 pg streptomycin/mL, 5-10% (v/v) fetal calf serum, pH between 6.8 and 7.0) to yield a net cell concentration of 2 x 10⁵ cells per mL (for all cells in the culture) and were reincubated at 37°C. Once the cultures reached confluence (2-3 days), the monolayer was chelated with a solution of 0.02% w/v EDTA in CMF-PBS (calcium and magnesium free phosphate buffered saline), the cells were dispersed, and passaged at 2 x 10⁵ cells /mL in IMEMZO as before.

The cultures were then blindly passaged in the same way when confluent every 3-4 days (roughly twice a week) for 2-3 weeks. After two weeks, a slide culture was prepared to examine the cells for the presence of *Cryptovirus*-specific inclusions in the cytoplasm using a *Cryptovirus*-specific indirect fluorescent antibody technique (exposure to hyperimmune rabbit anti-*Cryptovirus* antisera followed by labeling with fluorescein-conjugated goat anti-rabbit IgG).

When 5-10% of the cells were positive for *Cryptovirus*-specific inclusions, the cultures were ready for co-cultivation with the permissive CV-1_{c} cells mentioned above. This involved the 1:1 cocultivation of the passaged primary PBMNC/AV₃ cultures with CV-1_{c} cells in Richter's Modified Minimal Essential Medium diluted to yield a net concentration of 2 X 10⁵ cells/mL. These cultures were then monitored for the development of subtle cytopathic effects (CPE; stellation and rounding of cells or the formation of multinucleated cells containing three or more nuclei) over the ensuing 4-5 days. If no CPE developed before the cultures become confluent, they were passaged and monitored again. If no CPE developed after three such passages, the cultures were discarded.

Once CPE was observed, the whole culture was frozen at -70°C, thawed, and the cells were dispersed and dispensed into 1.0 mL aliquots. These aliquots represented the putative primary isolation of the virus. The virus was then plaque-purified by titration on monolayers of CV-1_{c} cells overlaid with a semi-solid solution of 1% w/v sodium carboxymethylcellulose (NaCMC) containing 2% fetal calf serum, which was made up in Richter's Modified Minimal Essential Medium. The cultures were incubated at 37°C in a partial CO₂ atmosphere (5% v/v). Plaques formed in 8-12 days and were then picked and replaqued, as above. Once triply plaque-purified, the virus was aliquoted onto CV-1_{c} monolayers (in IMEMZO media containing 5% fetal calf serum and supplements listed above) in 25-75 cm² tissue culture flasks. Once sufficient CPE developed, involving half or more of the cultured cells, the whole cultures were frozen, thawed, and the lysate was dispersed, re-aliquoted and refrozen at -70°C. Samples of the virus stock were then titrated for further use by the method of Robbins *et al.* (Robbins et al., J. Infect. Disease 143:396-403, 1981).

*Density Gradient Purification:* Virions and intracellular nucleocapsids isolated from productively- (Vero and CV-1_{c}) and nonproductively-infected (AV₃/SSPE) cells were further purified on sucrose-potassium tartrate gradients (virions) and CsCl gradients (nucleocapsids) by the method of Robbins *el al.* (Robbins et al., J. Infect. Disease 143:396-403, 1981; Rapp and Robbins, Intervirology 16:160-167, 1981; Robbins and Rapp, Arch. Virol. 71:85-91, 1982; and Robbins and Abbott-Smith, J. Virol. Meth. 11:253-257, 1985).

### Example 2. Cryptovirus Propagation and Virion Isolation and Purification.

Once isolated, cell-free virus stocks were grown in simian epithelial cell lines (*e.g*. Vero or CV-1 cells). The *Cryptovirus* isolates used in the studies described herein were triply-plaque purified and grown in a clonal subline of CV-1 cells designated CV-1_{c}. Optimal production of infectious virus occurred when using IMEMZO supplemented with insulin, zinc and HEPES buffer, 2 mM L-glutamine, 200 Units penicillin/mL, 100 µg streptomycin/ml, 5-10% (v/v) fetal calf serum, at a pH between 6.8 and 7.0.

The presence of insulin, and optionally zinc dication, in the tissue culture medium was useful in obtaining viable titers of infectious virus. Independent attempts to grow the virus in CV-1 cells using standard media (*e.g*. MEM) produced very poor results. Conversely, the expression of *Cryptovirus* proteins and the productivity of *Cryptovirus* infections in primate cell cultures was dramatically enhanced (50- to 100-fold) by addition of cyclic GMP (1 mM; sodium salt) to standard media (specifically MEM). The enhancement obtained was very similar to the enhancement of measles virus replication published earlier (Robbins, Intervirology 32:204-208, 1991).

*Virion Isolation and Purification.* Virions were isolated and further purified from the supernatant tissue culture medium of acutely infected CV-1 cells 72 hours after infection. The procedure employed involved the separation of the virus particles by differential centrifugation.

The supernatant medium of infected cultures was decanted into a sterile plastic 50-mL Falcon centrifuge tube and clarified at 2000 rpm for 10 minutes. The supernatant was then transferred to an impact resistant glass centrifuge tube (Sorvall) and further clarified at 10,000 rpm for 10 minutes. All clarifications took place at 4° C in an RC2B Sorvall centrifuge. The supernatant fluid from the second clarification step was layered over a 60% w/v sucrose cushion (in 10 mM Tris, 5 mM EDTA, pH 7.2) and centrifuged at approximately 130,000 x G in a Beckman SW-28 rotor at 4° C for 90 minutes in a Beckman L70 ultracentrifuge. Materials were collected from the tissue culture medium-sucrose interface, pooled, diluted with tissue culture medium and recentrifuged onto another 60% sucrose cushion as described above. The materials at the interface were again removed, diluted with tissue culture medium, and centrifuged at 35,000 rpm (280,000 x G) for 60 minutes through a 30% w/v over 60% w/v discontinuous (i.e. layered) sucrose gradient prepared in the Tris EDTA buffer described above). The virions were collection from the 30%:60% sucrose interface, diluted with cold Tris EDTA buffer and pelleted in a Beckman SW41 rotor at 41,000 rpm and 4° C for 60 minutes. Pelleted virions were resuspended in a variable amount of the cold Tris EDTA buffer and frozen at - 70° C until further use. Total protein in each virion preparation was determined by the method of Lowry et al. (1951).

### Example 3. Preparation of Antisera.

Antisera were raised in adult New Zealand White rabbits against sucrose-potassium tartrate gradient-purified virions of *Cryptovirus,* CsCl gradient-purified nucleocapsids (from infected CV-1_{c} cell cytoplasm), and against the major viral nucleocapsid protein, NP, eluted from polyacrylamide gels after SDS-PAGE. Rabbit antisera were also raised against the NIH 21005-2WR strain of SV5, and the Edmonston strain of measles virus.

Animals were inoculated by a pincushion technique which involved three series of three separate inoculations in each animal using a sterile 27 gauge needle and 1.0-mL syringe (one inoculation intradermally on the back; one inoculation intraperitoneally and one inoculation in a hind foot pad). The first series of inoculations were made using gradient purified and dialyzed virions (100 µg of virions in 0.3 mL of a 10 mM Tris 5 mM EDTA solution) mixed 1:1 with Freund's Complete Adjuvant and each inoculation contained approximately 200 µL of the virion:adjuvant mixture. The second series of inoculations were made two weeks later in the same locations but on the opposite side of each animal and consisted of the same amount of virions mixed with Freund's Incomplete Adjuvant. The third series of inoculations were made two weeks later in the same locations as the first inoculations but using only virions (diluted in 0.6 mL of the Tris-EDTA solution described above). Blood was harvested by intracardiac exsanguination of the animals two weeks after the final series of inoculations. The harvested blood was centrifuged (2000 rpm for 10 minutes) and allowed to clot on ice. The upper serum component was harvested and adsorbed against the pelleted component (2000 rpm for 10 minutes) of saline-washed freeze-dried acetone:methanol-extracted monkey kidney tissue (4° C for 1 hour with agitation every 15 minutes). The adsorbed serum was harvested by centrifugation (2,000 for 10 minutes) and stored in 1.0-mL aliquots at -20° C).

All of the *anti-Cryptovirus* antisera were strongly reactive with the corresponding *Cryptovirus-specific* materials from which they were generated when analyzed by (1) immunoprecipitation, (2) immunofluorescence, (3) immunoblotting, (4) ultrastructural immunolabelling techniques (immunogold), and (5) in the case of antisera generated against gradient-purified virus particles, neutralization titration assays. All the hyperimmune virus-specific antisera that were generated in the rabbits had homologous neutralization titers in excess of 1280 and, usually, in excess of 2560 (reciprocal dilution of PRD₅₀).

All experimentally-generated antisera were adsorbed against saline washed, freeze-dried, acetone:methanol extracts of monkey kidney tissue or similar extracts of AV₃ cells and/or CV-1_{c} cells.

While clinical sera were similarly adsorbed, CSF specimens were NOT preadsorbed due to the small volumes that were usually available and the requirement to retain aliquots for duplicate and parallel studies.

The precipitating "titers" of the experimental sera raised against purified nucleocapsids and purified viruses were not specifically determined although, routinely, 5-10 µL were used in positive control immunoprecipitation reactions and 25 µL of positive control antisera (diluted 1:10 or 1:20) for positive controls in ELISA assays.

*Cryptovirus-specific* antisera were also produced in mice experimentally inoculated with gradient-purified infectious *Cryptovirus* virions. These antisera were analyzed by immunoprecipitation, and were found to strongly precipitate all *Cryptovirus* envelope proteins.

There was clear *asymmetric* cross-reactivity between the antisera raised against *Cryptovirus* virions and antiserum raised against virions of the NIH 2WR-21005 strain of SV5. The asymmetry observed in this regard was always such that the heterologous reactions (i.e., *Cryptovirus-specific* antisera vs. SV5 materials and SVS-specific antiserum vs. *Cryptovirus* materials) were two- to fourfold weaker that the homologous reactions (i.e., *Cryptovirus-specific* antisera vs. *Cryptovirus* materials and SVS-specific antisera vs. SV5 materials). Another antiserum, which was independently prepared against NIH 21005-2WR strain of SV5 and kindly provided by Dr. Purnell Choppin, behaved in a similar asymmetric manner to the antiserum against SV5 described hereinabove.

Such cross reactivity is not surprising. Precisely the same sort of asymmetric cross-reactivity occurs when examining other paramyxovirus systems (*e.g*., there is also a two to four fold *asymmetric* cross-reactivity between measles virus antibodies when reacted with the closely related viruses of canine distemper and rinderpest and *vice versa*). There was also limited (*i.e*., much weaker) cross-reactivity between *Cryptovirus*-specific antibodies and other paramyxoviruses (*e.g*., measles virus).

### Example 4. Characterization of isolated Cryptovirus.

*Cryptovirus Neurovirulence and Neurotropism.* As shown in Fig. 24A, *Cryptovirus* demonstrated a tropism for neurons. Intracranial inoculation of mice with infectious *Cryptovirus* or nonproductive virus-carrying cells (AV₃/SSPE) resulted in the subacute/slow development of a spectrum of neuropathological conditions that had epileptiform, neurological and/or neuropsychological components. These responses were similar to the "experimental SSPE" reported earlier in animals following inoculation with "cell-associated measles-like" virus such as Niigata, Kitaken and Biken (see Fig. 24B; Doi et al., Japan. J. Med. Sci. Biol. 25:321-333, 1972; Ueda et al., Biken Journal 18:179-181, 1975; Yamanouchi et al., Japan. J. Med. Sci. Biol. 29:177-186, 1976; Ohuchi et al., Microbiol. Immunol. 25:887-983, 1981).

*Cryptovirus Presence in Neurovirulent SSPE-derived Virus-carrying Cell Lines.* Four virus-carrying cell lines derived from patients with SSPE were tested by immunofluorescence for the presence of measles virus- and/or *Cryptovirus* specific antigens. These were AV₃/SSPE/MV (an SSPE-derived cell line derived from PBMNC from an SSPE patient cocultivated with AV₃ cells which was experimentally-infected with Edmonston strain measles virus; Robbins, unpublished data); the nonproductive SSPE-derived cell line designated "Kitaken" (Ueda et al., Biken Journal 18:179-181, 1975); the nonproductive SSPE-derived cell line designated "Niigata" (Doi et al., Japan. J. Med. Sci. Biol. 25:321-333, 1972); and the nonproductive SSPE-derived cell line designated ``Biken" (Yamanouchi et al., Japan. J. Med Sci. Biol. 29:177-186, 1976; Ohuchi et al., Microbiol. Immunol. 25:887-983, 1981). With the possible exception of the Niigata cell line, all of these virus-carrying cell lines expressed both measles virus-specific and *Cryptovirus-specific* antigens when examined by vims-specific immunofluorescent techniques (shown in Fig. 6). Given that no cell-free clinical isolates of measles virus have ever been shown to cause SSPE-like illnesses in experimentally-infected animals, the presence of *Crytovirus* in these cultures strongly suggests that the subacute/slow neuropathies seen in these animals are due to the presence of *Cryptovirus* in the cultures-not measles virus.

*Cryptovirus* inclusion bodies (i.e. cytoplasmic nucleocapsids) displayed the same sort of "peppery" and or "splattered" distribution in both acutely-infected cells (CV-1_{c}) and nonproductively- and persistently-infected cells (AV₃/SSPE) as that previously described in CNS biopsy and autopsy materials from SSPE patients and in SSPE-derived nonproductive virus-carrying cell lines (e.g., de Felici et al., Annales Microbiologie 126:523-538 [1975]; Makino et al., Microbiology and Immunology 21:193-205 [1977]; Brown et al., Acta Neuropathologica 50:181-186 [1980]). This is most clearly evident in Panels B, D, F, H and J of Fig. 6. These characteristics were in sharp contrast to the discrete and "coalescing" distribution and morphology of intracellular measles virus inclusions bodies (see Panels A, C, E and G of Fig. 6).

*Neutralization Titration Assay.* Formation of macroscopically visible plaques on monolayers of mammalian cells (e.g., BHK, Vero and CV-1_{c}) can be used to quantitate preparations of infectious *Cryptovirus.* Plaque formation can be inhibited by serial dilutions of clinical serum specimens and *Cryptovirus-specific* antisera generated in rabbits (see Robbins et al., J. Infect. Disease 143:396-403, 1981). Plaque titration assays were conducted to determine the PRD₅₀ of isolated *Cryptovirus.*

Briefly, ten-fold serial dilutions of serum or CSF specimens to be tested were incubated for one hour at 4°C with sufficient infectious virus to yield a net plating concentration of between 100-200 plaque forming units of the virus / 0.2 mL of final diluent (including the diluted serum or CSF). After incubation, 0.2 mL of the diluted virus-serum (or virus-CSF) mixtures was then plated onto monolayers of susceptible cells (*e.g*. Vero or CV-1_{c}) and the cells were incubated at 37°C in a partial CO₂ atmosphere (5%v/v) (with redistribution of the inoculum every 15 minutes). At the end of the incubation period, inoculated monolayers were overlayed with sufficient volumes of a 2% (w/v) solution of carboxymethylcellulose (made up in IMEMZO medium containing 2% fetal calf serum, insulin, zinc, and HEPES buffer, 2 mM L-glutamine, 200 Units penicillin / mL, 100 µg streptomycin / mL, pH between 6.8 and 7.0) to last 10-12 days (*i.e*., enough volume so that the monolayers won't dry out). The plates were not moved during the incubation period. After 10-12 days, the overlay was aspirated and the cells were fixed with formalin fixative and stained with a protein stain (*e.g*., Giemsa). The number of plaques formed on each plate was then enumerated and the PRD₅₀ calculated.

In particular, cross neutralization assays involved the determination of the titer of antisera made against each species of virus which would neutralize 50% of the plaque forming units (PFUs) of each virus. Virus stocks of the BBR strain of *Cryptovirus* and the NIH 21005-2WR strain of SV5 were diluted in serum-free minimal essential medium (Eagle's MEM containing 2mM L-glutamine, 200 units of penicillin and 100 µg of streptomycin/ml with the pH adjusted to between 6.8 and 7.0 with NaHCO₃) to a titer of 1,000 PFUs per mL (resulting in 100 PFUs per well after dilution and plating). Antiserum raised in New Zealand White rabbits was serially-diluted in 10-fold increments in the same serum-free MEM. Aliquots (0.5 mL) of the diluted virus stocks were then mixed with 0.5 ml aliquots of each dilution of the antisera, gently mixed with a vortex and incubated on ice for one hour with gentle mixing every 15 minutes. Following this incubation period, the medium was aspirated from monolayers of CV-1 cells in 6-well cluster plates (NUNC), the monolayers were washed with warm saline, the saline was aspirated and 0.2 mL of each of the diluted antisera-virus incubates were plated onto two monolayers. The inoculated cluster plates were subsequently incubated at 37° C in a CO₂ incubator (containing 5% CO₂ v/v) for I hour with manual redistribution of the inocula every 15 minutes. Following this adsorption period, each well was overlayed with 10.0 mL of a semisolid overlay medium (1% w/v sodium carboxymethylcellulose in Eagle's MEM containing 2 mM L-glutamine, 200 units of penicillin and 100 µg of streptomycin/ml, 2% v/v fetal calf serum with the pH adjusted to between 6.8 and 7.0 with NaHCO₃) and incubated for 10-12 days at 37° C in a CO₂ incubator (containing 5% CO₂ v/v) without being disturbed. Following this incubation period, the overlay was aspirated, the monolayers were gently washed with warm saline, and then fixed in formalin fixative (3.7% by weight formaldehyde gas in saline) for 1 hour or longer. Following fixation, the fixative was aspirated and the fixed monolayers were gently washed with distilled water and stained with 1-2 mL per well of Giemsa stain (0.5 gm Giemsa powder dissolved in 42 mL of warmed [55° C] glycerin, 42 mL of absolute methanol, filtered and diluted 1:5 with formalin fixative immediately before use) for 1 hour at room temperature. The stain was subsequently decanted and the monolayers were washed under tap water and allowed to dry at room temperature. Plaques on monolayers were illuminated on a light box, enumerated under a magnifying lens and recorded for each dilution, virus and antisera series. The neutralization titer of each antiserum virus series was calculated to be the reciprocal of the dilution of antisera resulting in a 50% decrease in the number of plaques formed.

The calculated neutralization titer for each crossed neutralization set (i.e. anti-*Cryptovirus* antiserum versus *Cryptovirus* and anti-*Cryptovirus* antiserum versus SV5; anti-SV5 antiserum versus Cryptovirus and anti-SV5 antisera versus SV5) was consistently 2-4 fold less for the heterologous mixtures (i.e. anti-*Crytovirus* antisera versus SV5 and anti-SV5 antisera versus *Cryptovirus*) than for the homologous mixtures (anti-Cryptovirus antisera versus Cryptovirus and anti-SV5 antisera versus SV5). On no occasion did any heterologous mixture have less than a 2-fold difference when compared to the homologous pair (in three separate trials).

*Cryptovirus Ultrastructural and Immunoultrastructural Characterization.* AV₃/SSPE/MV cells (AV₃/SSPE cells persistently and nonproductively infected with the Edmonston strain of measles virus), AV₃/SSPE cells, and CV-1_{c} cells acutely infected with the BBR strain of *Cryptovirus* were fixed in situ (on glass cover slips) in 2% formaldehyde and picric acid and 3% glutaraldehyde in 0.1 M cacodylate buffer, pH 7.2, for 15 minutes at room temperature. Osmium tetroxide post-fixation was omitted for specimens that were to be treated with antibody (i.e. which were prepared for immunoultrastructural studies). Cover slips with fixed cells were washed in three changes of cacodylate buffer, dehydrated to 70% ethanol and embedded in LR White resin. Resin was polymerized at 50°C for 24 hours. Ultrathin sections were cut and mounted on uncoated nickel grids.

Stained thin sections of CV-1_{c} cells acutely-infected with the BBR strain of *Cryptovirus* and AV₃/SSPE cells were examined by electron microscopy. In infected CV-1_{c} cells, pleomorphic virion particles, 100-120 nm in diameter, were seen budding from the surface of acutely-infected cells and numerous accumulations of filamentous structures (helical nucleocapsids, 15-17 nm in diameter) were observed in the cell cytoplasm (data not shown). Both the virions and nucleocapsids were similar to those described for other members of the Paramyxoviridae. While virions were not observed budding from the surfaces of AV₃/SSPE cells, inclusions of intracellular nucleocapsids were seen in abundance and these were identical to those seen in the acutely-infected cells.

The intracellular nucleocapsids of nonproductively and productively-infected mammalian cells can readily be localized under the electron microscope using *Cryptovirus-*specific or *Cryptovirus* nucleocapsid-specific hyperimmune rabbit antibodies and an indirect immunogold labeling technique.

Immunolabelling was performed on sections of AV₃/SSPE/MV cells by floating sections mounted on nickel grids (processed as described without osmium tetroxide post-fixation) on drops of solution (see below) in a closed, humid chamber. Sections were etched according to the method of Ingram et al. (Parasitology Research 74:208-215, 1988). Non-specific labeling was reduced by incubation of the sections with 5% bovine serum albumin in modified Tris buffer (20 mM Tris, 0.5 M NaCl, 20 mM sodium azide and 0.05% Tween 20, pH 8.2) for 30 minutes at 37°C prior to immunolabeling. The modified Tris buffer was used for all dilutions and washes.

In single labeling experiments, sections were incubated with rabbit antisera (anti-Edmonston measles virus or anti-BBR strain of Cryptovirus) diluted 1:20 in modified Tris buffer for 2 hours at 37°C, washed in three changes of buffer, and incubated with a goat anti-rabbit IgG colloidal gold (10 or 15 nm particle size, 1:20 dilution, 1 hour at 37°C). After washing with two changes of buffer, followed by two changes of distilled water, sections were lightly contrasted with 2% uranyl acetate and led citrate, and examined in a JEOL 1200EX transmission electron microscope.

In double labeling experiments, sections were immunolabeled on one face, as described for single labeling, using rabbit anti-Edmonston measles virus and 15 nm colloidal gold particles, and ensuring that the reverse face of the section was not contaminated by labeling solutions. The labeled face was then coated with a thin film of Celloidin to reduce possible cross reaction of antibodies while the reverse face of the section was labeled. Immunolabeling of the reverse face of the sections was performed as described above, using the second antiserum (rabbit anti-BBR strain of *Cryptovirus*) and 10 nm colloidal gold particles. Examination of these double labeled sections allowed simultaneous comparison of labeling patterns of the two antisera.

The results of these studies were unequivocal and are shown in Fig. 25. The first labeling sequence (15 nm gold beads) labeled only the wider "fuzzy" measles virus nucleocapsids (as shown in Fig. 25B), while the second labeling sequence (10 nm gold beads) labeled only the narrower smooth *Cryptovirus* nucleocapsids (as shown in Fig. 25A).

### Example 5. Characterization of Isolated Cryptovirus Proteins

*Radioimmunoprecipitation* (RIP) *Assay:* Extensive data were generated by the comparative analysis of *Cryptovirus-specific* immunoprecipitates of [³⁵S]-methionine-labeled uninfected, nonproductively- and productively-infected human and primate cells by sodium dodecyl sulfate polyacrylamide gel electrophoresis (SDS-PAGE; see below).

CV-1_{c} or Vero cell monolayers were infected with the BBR strain of Cryptovirus, the NIH 21005-2WR strain of SV5 or the Edmonston strain of measles virus at a multiplicity of infection of 1-2 PFU/cell using procedures as described elsewhere (Robbins and Rapp, Virology 106:317-326, 1981).

Labeling was accomplished twenty-four hours after infection by the following procedure. Tissue culture medium was removed from infected cell monolayers and the cells were washed with a serum and methionine-free Eagle's based MEM (starvation medium). The infected cultures were then supplemented with the starvation medium for 60-90 minutes and incubated at 37°C. Following the starvation period, cultures were labeled with the starvation medium containing 100 µCi/mL of [³⁵S]-methionine (Amersham). Labeling was carried out at 37°C in a 5% CO₂ atmosphere for 5-6 hours. Immunoprecipitations were carried out according to the procedure of Lamb et al., Virology 91:60-78, 1978.

*SDS-PAGE:* Virions and immunoprecipitates were analyzed under denaturing and reducing conditions on 10% (or to detect the presence of very small peptide species [e.g., SH protein], 20%) polyacrylamide slab gels (Laemmli, 1970). After electrophoresis, gels were treated with a fluor solution (Amersham), were dried, and were then exposed to X-ray film.

Purified virions of the virus were analyzed by SDS-PAGE under reducing and non-reducing conditions (see Fig. 11, an autoradiogram of gradient-purified [³⁵S] -methionine-labeled *Cryptovirus* virions produced in acutely-infected Vero cells after SDS-PAGE under reducing conditions). The approximate molecular weights of the proteins indicated on the right side of the figure were calculated by comparing their migrations to marker proteins of known molecular weights (Sigma Chemical Co., St. Louis, MO). The annotations are defined in the brief description of the drawing. The SH protein, a small envelope-associated protein having a MW of about 5 kD, is not shown because it has run off the gel (see below).

*SH Protein.* Due apparently to the small size of the SH protein and its relatively low methionine content, the SH protein was difficult to detect in radio-labeled virion preparations of both the BBR strain of *Cryptovirus* and the NIH 21005-2WR strain of SV5. When unlabeled purified virion preparations of both viruses were run on 20% polyacrylamide slab gels under denaturing and reducing conditions alongside of low molecular weight marker proteins (BioRad) and stained with a silver-staining technique (BioRad), a small protein with an Mᵣ of approximately 5 kD, identified as the SH protein, was found in both *Cryptovirus* and SV5 virion preparations. There was no detectable difference between the migration of the SH protein from the BBR strain of *Cryptovirus* or the NIH 21005-2WR strain of SV5.

*F₀ and HN Co-migration Anomaly.* Although the major envelope proteins (F₀ and HN) of many Rubulaviruses (*e.g*., SV5) were readily discernible as separate bands on SDS-PAGE gels, the larger size of the *Cryptovirus* F₀ protein (*i.e*., +22 amino acids) resulted in a significantly slower rate of migration for this protein (Mᵣ = 69 kD). As shown in Figs. 13A-B, close examination of such gels enabled one to discern both proteins, albeit with some difficulty. Figs. 13A and 13B show photographs of migration patterns of the major *Cryptovirus* envelope proteins, F₀ and HN. Fig. 13A illustrates the observed near co-migration of the major *Cryptovirus* envelope proteins, F₀ and HN. Enlargement of the RIP from the CSF-positive patient (right lane) in Fig. 13A shows the "bowed" or "crested" structure that resulted from the near co-migration of the F₀ and HN proteins of *Cryptovirus.* A diagrammatic interpretation of the near co-migration of the F₀ and HN proteins of *Cryptovirus* and the discrete migration of the analogous proteins of Simian virus 5 are shown in Fig. 13B.

### Example 6. Experimental Infection of Mice: Creation of an Animal Model.

Infectious *Cryptovirus* stocks (prepared in CV-1_{c} cells) and live nonproductively-infected AV₃/SSPE cells were used to intracerebrally inoculate two strains of laboratory mice (Quackenbush and Colored, an outbred strain of C57 Black).

Briefly, neonatal mice (1-2 days old) were inoculated by injection with 0.025 mL (phosphate buffered saline, pH 7.4) containing either 5 x 10⁴ PFU of cell-free *Cryptovirus* or 5 x 10³ nonproductively infected human amnion cells (AV₃/SSPE). Following inoculation, the neonatal mice were returned to their mothers who were provided food and water *ad libitum.* Observations of inoculated mice were made daily. Symptom of disease first appeared in affected animals after 21 days, and in others not until after more than 60 days. Observed symptoms included cachexia, muscle spasms, tremors, compulsive behaviors (e.g., extended periods of scratching, rubbing, or running in circles), hyperactivity/hyperesthesia, seizures and convulsions, and stupor. These results demonstrated that intracerebral inoculation with *Cryptovirus* results in subacute central nervous system (CNS) disease. Neurological, neurodegenerative, and/or neuropsychiatric disease presentation in mice is virtually indistinguishable from presentation in humans.

While all of the inoculated animals developed antibodies to the nucleocapsid protein of the virus (NP), not all of them developed antibodies to the envelope proteins (F, HN, and SH). More than 90% of the mice inoculated with *Cryptovirus* virions developed antibodies to the envelope proteins but only 33% of those inoculated with AV₃/SSPE cells did so.

Concurrently, while many of the animals inoculated with infectious *Cryptovirus* stocks developed profound neuropathological disease, fewer of the animals inoculated with nonproductively-infected AV₃/SSPE cells developed such illnesses, and there was a strong correlation between development of antibodies to the envelope proteins of the virus and the development of CNS symptoms. This suggests that the development of CNS disease depends on the establishment of an acute or subacute CNS infection by the virus and the expression of all of the virus' structural proteins in some cells or tissues.

More detailed examples follow:
*Quackenbush mice.* Two litters of 1-2 day old Quackenbush mice were intracerebrally-inoculated (in the right cerebral hemisphere) with 5 x 10⁴ PFUs of either *Cryptovirus* (strain BBR)(10 individuals) or measles virus (Edmonston strain)(8 individuals), were returned to their mothers and were periodically observed over a period of three months. Two animals (one inoculated with *Cryptovirus* and one inoculated with measles virus) were found dead and partially consumed the next morning. Their deaths were attributed to "needle trauma" and/or maternal cannibalism. While none of the mice inoculated with measles virus developed any neurological, neurodegenerative, physiological or neuropsychiatric symptoms over the course of the study, two of the male mice inoculated with *Cryptovirus* developed atrophy and contralateral hindlimb paralysis (in their left hind legs) three to four weeks after inoculation. A third (female) mouse was observed dragging its left hind leg (unatrophied) approximately four weeks after inoculation but was found killed and partially eaten a day later. While 3 of 9 animals inoculated with *Cryptovirus* developed hind limb paralysis (33%), none of the animals showed overt signs of seizure, wasting or neurophychiatric symptoms over the course of the study. Hind limb paralysis was also seen in a number of the offspring of adult female Quackenbush mice that had been inoculated with *Cryptovirus* as newborns but that did not develop any overt symptomology. The frequency of this phenomenon was difficult to assess because the mothers tended to cannibalize the newborn animals that were born with, or subsequently developed, such characteristics.
*Colored mice.* Three litters of 1-2 day old Colored mice (comprising 26 individuals) were observed daily following intracerebral inoculation with 5 x 10⁴ PFUs of *Cryptovirus* (strain BBR), Simian Virus 5 (NIH 21005-2WR strain) or measles virus (Edmonston strain) or mock-infected cells. Half of each litter was inoculated with *Cryptovirus* (13 animals) while the other half was apportioned into three groups and inoculated with either Simian Virus 5 (6 animals), measles virus (4 animals) or mock-infected CV-1_{c} lysate (3 animals). Each group was marked with phenol red stain on the upper skin of one foot to distinguish them (i.e. right front, left front, right rear, left rear). One animal inoculated with *Cryptovirus* died between 24 and 48 hours post-inoculation and this was attributed to "needle trauma/starvation" as it had stopped feeding (or was rejected) when it was returned to its mother. Between three and four weeks later, one male and one female animal were found dead in their cages in tonic-clonic posture-both having been inoculated with *Cryptovirus.* It was noted that both also appeared to be underweight when compared to their littermates. Two months after inoculation with *Cryptovirus,* a third mouse (male) was observed to have cachexia, anorectic wasting, tremors and seizures (Fig. 7A). Over the next month (approximately 11 weeks after inoculation), a fourth animal (also male) developed tremors and seizures although no wasting was observed (data not shown). A male littermate of the *Cryptovirus*-infected mouse shown in Fig 7A, which was inoculated with the NIH 21005-2WR strain of SV5, is shown in Fig. 7B. The same results were obtained when mice were inoculated with either the *Edmonston* strain of measles virus, mock-infected CV-1_{c} cells, or homogenized AV₃/SSPE cells (i.e. all remained healthy and none developed neurological, neurodegenerative or neuropsychiatric symptoms; data not shown).

Over the ensuing six months (observed up to nine months post inoculation), a significant number of the remaining animals (4 of the remaining 8) inoculated with *Cryptovirus* developed physiological, neurological and/or neuropsychiatric symptoms. Such late onset animals presented with symptoms that were in marked contrast to the overt seizure disorders observed in the subacute onset animals (i.e. those that developed symptoms in first three months post inoculation). These symptoms were dominated by physiological, neuropsychiatric and behavioral disturbances rather than more overt neurological symptoms and included: marked weight gain, extreme aggressive/passive responses to stimuli, obsessive/compulsive behaviors, ataxia and tremor. Aggression was most frequently characterized in afflicted animals by physical agitation and a predisposition to biting when handled. Passive animals tended to eat excessively, gain weight, and sleep. Repetitive behavior was also sporadically observed and consisted primarily of endless pacing and/or facial washing so extreme as to result in the loss of fur on the head and neck and the development of abrasive wounds. One of the animals (a female shown in Fig. 8A) had abnormal cranial structure (microcephaly) and manifested a spectrum of physiological and behavioral symptoms at six months including obesity, tics and muscle twitching (along the back and left side) and obsessive/compulsive facial washing and scratching. Episodes of such obsessive/compulsive behavior were observed to last for an hour or more. A second female animal (shown in Fig. 8B) appeared overtly normal during the first five months post inoculation but between five and six months began displaying marked ataxia, tremors and aggression. While this animal maintained a normal body weight and appearance, it was prone to splaying its feet to maintain its balance when resting and stumbling when walking and biting and hissing/snarling when handled or disturbed. Neither animal shown in Fig. 8 developed overt (*grand mal*) seizures, in contrast to the animal shown in Fig. 7A. Two other animals (one male and one female) also developed mixtures of the slow onset symptomology (data not shown). Overt seizure activity was never observed in any of the late onset animals and none of the animals inoculated with SV5, measles virus or mock-infected cells developed any similar symptoms.

Of the 13 neonate animals inoculated with *Cryptovirus,* one died from needle trauma (sex uncertain), two died in tonic-clonic posture with signs of wasting (indicative of sudden death due to *status epilepticus* (one male; one female); two became wasted and developed grand mal seizures (both males); four developed a spectrum of slow onset neurological/neuropsychological symptoms (three females, one male); one committed infanticide after being bred (a female); and three never developed any symptoms (two females and one male). Removing the needle trauma death from the equation, 9 of 12 animals developed neurological, degenerative and/or neuropsychiatric symptoms (75%). Removing the two clonic-posture deaths as well, 7 of 10 animals developed symptoms (70%). This was highly significant compared to the combined results for the control inoculated mice (7 of 10 mice inoculated with *Cryptovirus* presenting with neurological symptoms versus 0 of 13 mice in the control groups; P = 0.0005, 2-sided Fisher exact test), and was also significant compared to just the SV5 inoculated mice (7 of 10 versus 0 of 6; P = 0.01, 2-sided Fisher exact test) and even when compared to just the measles inoculated mice (7 of 10 versus 0 of 4; P = 0.035, one-sided Fisher exact test). Statistical significance was not quite reached compared to the mice inoculated with uninfected cell lysate, because of the small number of mice in this group (7 of 10 versus 0 of 3, P = 0.069, single-sided Fisher exact). However, when the two clonic-posture deaths were included even this small control group was significantly different (9 of 12 versus 0 of 3, P = 0.044, 2-sided Fisher exact). Thus, the disease(s) and symptoms resulting from *Cryptovirus* infection was profoundly significant.

*Infanticide.* Of the four Colored mice which were inoculated as neonates with *Cryptovirus* but did not develop subacute or slow onset symptoms over the first nine months of the study, three were female and were subsequently bred with uninfected males at nine months of age. While all of the offspring of two of the three resulting litters developed normally, all of the offspring in one of the litters (comprising 10 animals) were killed and wholly or partially cannibalized by their mother. Such infanticide did not occur in litters to females that had been inoculated with SV5, measles or mock-infected cells. In a separate study (see below), one of the females which was inoculated with live AV3/SSPE cells-but did not develop any overt neurological symptoms-developed late physiological and behavioral symptoms and also committed infanticide of its whole litter after being bred with an uninfected male.

*Animal Model Employing Inoculation of Nonproductively Infected AV₃*/*SSPE cells.* In a separate study, two litters of neonatal mice (18 animals) were inoculated (1-2 days after birth) with either live or homogenized AV₃/SSPE cells (six animals each) or live or homogenized AV₃ cells (three animals each). There were no needle trauma deaths. While none of the animals inoculated with the homogenized AV₃/SSPE cells, live AV₃ cells or homogenized AV₃ cells (12 animals) developed any subacute or slow onset symptoms whatsoever, one of the six animals inoculated with live AV₃/SSPE cells developed subacute degenerative and neurological symptoms (a male) and one developed slow onset symptoms (1 female). The animal that developed subacute symptomology began presenting with symptoms 24 days after intracerebral inoculation. Over a five day period, the symptoms presented included cachexia, wasting, hunched posture, repetitive chirping and clicking, hyperesthesia, incontinence of urine, tremors, muscle spasms and coma. Overt seizures were not observed. It was sacrificed when coma developed. The animal that developed slow onset symptomology (a female) presented at five to six months (post-inoculation) with repetitive pacing, aggression and progressive obesity. The animal was bred at six months with an uninfected male. Seven days after delivering a litter of eight offspring, it killed the whole litter and partially consumed each individual. No seizures or other signs of overt neurological symptoms were observed. None of the other females which were inoculated with homogenized AV₃/SSPE cells, AV₃ cells or homogenized AV₃ cells and bred (six animals in total) committed infanticide.

### Example 7. Cryptovirus-Specific Antibodies Are Present In The Serum And Cerebrospinal Fluid (CSF) Of Human Patients Diagnosed With Neurological, Neurodegenerative And/Or Neuropsychiatric Diseases.

Evidence for the presence of *Cryptovirus*-specific antibodies to the major envelope proteins of the virus (F₀ and HN) in the serum and CSF of patients was determined by immunoprecipitation of [³⁵S]-methionine-labeled *Cryptovirus*-specific proteins produced in acutely-infected CV-1_{c} cells. Figs. 12A and 12B are photographs of autoradiograms, which serve as examples of RIP profiles of measles virus- or *Cryptovirus*-specific proteins precipitated from [³⁵S]-methionine-labeled acutely infected CV-1_{c} cells by clinical CSF specimens followed by SDS-PAGE (reduced). In Fig. 12A, lane "V" contains gradient-purified *Cryptovirus* virions from acutely-infected, ³⁵[S]methionine-labeled CV-1_{c} cells (BBR Strain). Lane "MV" contains proteins precipitated by the CSF of an 11-year old male SSPE patient from radiolabled CV-1_{c} cells acutely-infected with measles virus *(Edmonston Strain).* Lane "B" contains proteins precipitated by the same CSF specimen from a 1:1 mixture of radiolabled CV-1_{c} cells acutely-infected with either measles virus or *Cryptovirus.* Lane "CV" contains proteins precipitated by the same CSF specimen from radio-labeled CV-1_{c} cells acutely-infected with *Cryptovirus.* In Fig. 12B are shown RIP profiles of the *Cryptovirus*-specific proteins precipitated by the CSFs of six randomly-selected neurology/neurosurgery patients who had CSF taken for diagnostic screening. The patient whose sample appears in Lane 2 was an adult male who had presented with ataxia, confusion and memory loss (tentatively diagnosed with *ataxic cerebellar syndrome*). The patient whose sample appears in Lane 4 was an infant female who presented with hydrocephalus and intractable seizures and who subsequently died in *status epilepticus.*

### Example 8. Cryptovirus Is Implicated In The Aetiopathogenesis Of Disease in Patients Diagnosed with Idiopathic Human Neurological, Neurodegenerative, And/Or Neuropsychiatric Diseases.

*Cryptovirus* is implicated in the aetiopathogenesis of disease in patients diagnosed with idiopathic neurological, neurodegenerative, and/or neuropsychiatric diseases, including anorexia nervosa, multiple sclerosis (MS), epilepsy, subacute sclerosing panencephalitis (SSPE), autism, mental retardation, affective disorder, dysthymia (clinical depression), schizophrenia, obsessive compulsive disorder, manic depression (bipolar disorder), chronic fatigue syndrome (CFS), hydrocephalus, ataxic cerebellar syndrome and atypical viral meningitis. Most patients who had *Cryptovirus*-specific antibodies in their CSF had been given multiple diagnoses. Thus, there is a correlation between the presence of *Cryptovirus*-specific antibody to the major envelope proteins of the virus (F₀ and HN) in the CSF of neurology or neurosurgery patients and prior diagnosis of a condition with a significant "iterative" or compulsive component.

Although *Cryptovirus* seropositivity did not necessarily correlate to CSF positivity (*i.e*., the presence of antibody to the *Cryptovirus* F₀ and HN proteins in the CSF) or a diagnosis of any neuropathological condition, CSF positivity strongly correlated with a prior diagnosis of a significant disorder of the central nervous system. These correlations were consistently found for patients with certain diagnoses (*e.g*., SSPE, MS, CFS, and certain forms of idiopathic epilepsy) and incidentally found for specimens from patients with other diagnoses (*e.g*., Alzheimer's Disease).

Similar results were obtained for two CSF specimens analyzed by an immunblotting technique (data not shown), or for serum and CSF specimens analyzed by an enzyme-linked-immunosorbent-assay (ELISA; see Fig. 14), although these assays were performed on only a proportion of CSF specimens due to the limited volumes available in some samples.

Although some patients presented with some of the above-mentioned symptoms and did not have antibody to the virus in their CSF specimens, in no instances were *Cryptovirus*-specific antibodies found in the CSF of patients that did not present with many of the symptoms and who had not been diagnosed with a significant neuropathological or neuropsychological disorder.

In addition, seropositive individuals (*i.e*., those who have *Cryptovirus*-specific antibodies in their serum) harbor the virus in a nonproductive, inapparent but inducible state in their PBMNCs.

While the presence of the virus in an individual patient's PBMNCs did not symmetrically correlate with the development of neuropathological disorder, these findings imply that the virus can gain entry into the CNS via a microvascular incident (*i.e*., leakage of *Cryptovirus* carrying PBMNCs into the CNS) or by immune system responses to other CNS stimuli (*i.e*., diapedisis of *Cryptovirus-*carrying lymphocytes into the CNS as part of an inflammatory response to another infection; a Trojan Horse phenomenon). Reference to a lack of symmetrical correlation means that, while all individuals whose PBMNCs were examined and had *Cryptovirus*-specific antibodies in their CSF carried the virus in those cells, not all individuals who were found to be carrying the virus in their PBMNCs were, at that time, suffering from any neurological, neurodegenerative, and/or neuropsychiatric disorder.

The following examples reveal more detail.

(a) *Alzheimer's Disease.* As shown in Fig. 15, three matched sets of serum and CSF (provided by the National Neurological Research Specimen Bank (NNRSB) in Los Angeles, CA) were examine by RIP analysis for the ability to precipitate the *Cryptovirus* F₀ and HN proteins from radiolabeled acutely-infected CV-1_{c} cells. While all three had *Cryptovirus*-specific antibodies in their serum, only Patient 3 had these antibodies in his or her CSF. This implies that the illness Patient 3 was suffering, diagnosed as Alzheimer's disease, was complicated by concurrent *Cryptovirus* infection of the CNS tissues. Alternatively, Patient 3 could have been misdiagnosed, in which case he or she could actually be suffering from a *Cryptovirus*-related neuropathy.

Even though the sample size is small, it is interesting that all three of the Alzheimer's disease patients had been exposed to *Cryptovirus* and were probably carrying it in their lymphocytes. It appeared, unlikely, however, that *Cryptovirus* plays a role in the development of Alzheimer's disease, because Patients 1 and 2 did not appear to have the virus in their CNS tissues.

(b) *Ataxic Cerebellar Syndrome, Atypical Viral Meningitis, Hydrocephalus, Idiopathic Parasthesia and Status Epilepticus.* A blind screen (i.e., none of the diagnoses or medical histories pertaining to any of the specimens was provided prior to specimen screening) was conducted of 66 CSF specimens from neurology or neurosurgery patients who had CSF specimens taken for diagnostic screening by the Department of Clinical Microbiology at the Royal Brisbane and Royal Children's Hospitals, in Brisbane, Queensland, Australia. Of these CSF specimens, ten were *Cryptovirus*-positive (see Fig. 17). One of the ten *Cryptovirus*-positive CSF specimens was identified as being from an adult male patient who had been diagnosed with ataxic cerebellar syndrome. (see Fig. 12B). Another of the ten *Cryptovirus*-positive CSF specimens was identified as being from an adult female patient who had been diagnosed with atypical viral meningitis (data not shown). A third positive CSF specimen came from a 55 year old male that had presented with ataxia, memory loss, blackouts, seizures, diploplia and headache and had been diagnosed with hydrocephalus, chronic fatigue syndrome and possible epilepsy; a fourth positive CSF specimen was from an adult male who had been diagnosed with idiopathic parasthesia; and a fifth positive CSF specimen was from a female infant who presented with clonic hand movements and intractable seizures and was diagnosed with hydrocephalus and *status epilepticus* (see also c and d, below). Diagnoses and symptoms of the remaining five *Cryptovirus*-positive CSF specimens were unavailable.

(c) *Chronic Fatigue Syndrome (CFS).* A number of adolescent and adult patients who presented with symptoms of CFS were subsequently found to have high titers of anti-*Cryptovirus* antibodies in their sera, demonstrating that primary infection with the virus can manifest as a chronic febrile tracheo-bronchial illness with associated chronic malaise and lymphadenopathy. This is not unlike infectious mononucleosis in presentation (i.e., a sore throat and persistent "glandular fever"). There was no evidence of acute encephalitic (or encephalopathic) disease in such patients or in any other patient found to have *Cryptovirus*-specific antibodies in his or her serum or CSF. "Acute" is taken here to mean presenting with rapid onset and symptoms within seven days.

Fifty-six serum specimens from patients who had been diagnosed with CFS were provided for *Cryptovirus* screening by regional physicians (Brisbane and Southeast Queensland). Eleven matching CSF specimens were subsequently obtained. RIP analysis revealed that 54/56 (96.4%) of the serum samples and 10/11 (90.9%) of the CSF specimens contained *Cryptovirus*-specific antibodies (Fig. 16).

Including the patient who had been codiagnosed with hydrocephalus, epilepsy and CFS (see Fig. 17 and data for Epilepsy, below), a total of 12 CSF specimens from CFS patients were analyzed by RIP analysis and 11/12 (91.7%) had *Cryptovirus*-specific antibodies in them.

Patients who had been diagnosed with CFS almost always had two, or more, concurrent diagnoses. These included: anorexia nervosa, MS, epilepsy, dysthymia (clinical depression), schizophrenia, and manic depression (bipolar disorder). For example, one adolescent girl who was co-diagnosed with both anorexia nervosa and chronic fatigue syndrome (CFS) had *Cryptovirus-*specific antibodies in her CSF. It is of note that the etiology of virtually all of these disorders is idiopathic.

While the symptoms presented by CFS patients cover a broad spectrum, the spectrum is, in fact, fairly discrete and representative of the illness. This is perhaps best illustrated by examination of the medical records of five patients, presented below:

Patient PR was an adult male, 55 years of age, who was suffering primarily from mental confusion, lethargy, memory loss, blurred vision, dysthymia, and *petit mal* seizures. EEG results were abnormal, which indicates epileptiform disease. Patient PR was ambulatory with progressively deteriorating CNS symptoms.

Patient DF was an adult male, 52 years of age, who was suffering primarily from mental confusion, lethargy, memory loss, dysthymia, and *petit mal* seizures. EEG results were abnormal, showing epileptiform, responses in cortical and subcortical functions of the anterior hemispheres. Patient DF was ambulatory with progressively deteriorating CNS symptoms.

Patient NB was an adult female, 36 years of age, who was suffering primarily from mental confusion, lethargy and extreme fatigue, memory loss, dysthymia, ataxia, blurred vision, and parathesias, and had a history of glandular fever, recurrent sore throats of prolonged duration, tremors, and *petit mal* seizures. NB's sister was diagnosed with anorexia and myoclonus. Patient NB was bedridden or partially ambulatory with progressively deteriorating CNS symptoms.

Patient KT was an adult female, 27 years of age, who was suffering primarily from mental confusion, loss of concentration, memory loss, anorexia, lethargy and extreme fatigue, and tremors, and had a history of recurrent febrile lymphadenopathy. Patient KT was stable but bedridden and only partially ambulatory.

Patient SS was an adult female, 23 years of age, who was suffering primarily from loss of concentration, memory loss, and lethargy, and had a history of dysthymia beginning at age 14 and EBV-negative glandular fever. Immediate family members (mother, father, two sisters, and one brother) were all seropositive. In addition, SS's mother had a 9 year history of dysthymia, and *Cryptovirus* antigens were detected in her cultured PBMNC. Two years after sampling, Patient SS was stable and ambulatory.

(d) *Epilepsy and Hydrocephalus.* RIP analysis was used to determine the presence of *Cryptovirus*-specific antibodies in two clinical collections of CSF specimens. The first collection included 66 specimens that were selected at random from those submitted to the Department of Clinical Microbiology at Royal Brisbane Hospital in Brisbane, Queensland by physicians in the Department of Neurology and Neurosurgery (see b above). None of the diagnoses or medical histories pertaining to any of the specimens was provided prior to specimen screening. Fig. 17 illustrates the results of RIP assays conducted with CSF from this collection. The positive CSF precipitate in Lane 2 was subsequently found to have come from a 55-year old adult male (RW) who presented with ataxia, memory loss, blackouts, seizures, diploplia, and headaches. He was determined to have a hydrocephalic condition and underwent surgery to insert a ventricular shunt to alleviate the condition. He had been diagnosed with hydrocephalus, epilepsy and Chronic Fatigue Syndrome (CFS).

Ten of the 66 CSF specimens in Collection 1 were found to contain *Cryptovirus*-specific antibody (15%). Diagnoses were obtained for five of these patients and included: (1) hydrocephalus and intractable seizures in an infant female who subsequently died (see Fig. 12B), (2) ataxic cerebellar syndrome in an adult male (see Fig. 12B), (3) atypical viral meningitis in a female child, (4) parathesia in an adult male, and (5) hydrocephalus, epilepsy and CFS in the patient described in connection with Fig. 17.

Diagnoses were obtained for only two of the 56 *Cryptovirus*-negative CSF specimens: one patient (WK, a male) was diagnosed with acute viral meningitis and one (SG, a female) was diagnosed with idiopathic intracranial hypertension.

The second collection (Collection 2) included 20 CSF specimens from children (<12 years old) that were collected by neurologists at Camperdown Children's Hospital in Sydney, New South Wales, Australia. Again, none of the diagnoses or medical histories pertaining to any of the specimens was provided prior to specimen screening. However, in this collection a request had been made to include an undisclosed number of CSF specimens from children who had either presented with epileptiform illness or had been diagnosed with some form of idiopathic epilepsy.

Fig. 18 illustrates the results of RIP assays conducted with CSF from Collection 2. The CSF precipitate analyzed in Lane 1 was from a newborn infant who developed intractable seizures and died in *status epilepticus* (Patient CT, below) and the precipitate analyzed in Lane 2 was from a child who had been given a diagnosis of Lennox-Gasteau/generalized epilepsy (Patient LB, below), respectively. The background noise in this autoradiogram was high as a result of the long-term exposure (30 days) required to see the bands.

Six of the 20 CSF specimens provided in the (biased) Collection 2 were found to have *Cryptovirus*-specific antibodies, and it was subsequently learned that this screening had identified 6 of the 7 specimens from patients who had been diagnosed with epilepsy or other forms of epileptiform illness and had been included in the collection. The six *Cryptovirus-positive* CSF specimens came from the following patients: (1) CT, a neonate with intractable fits and seizures, who died in *status epilepticus,* (2) LB, who was diagnosed with Lennox-Gasteau epilepsy and generalized epilepsy, (3) BM, who was diagnosed with severe retardation and epilepsy, (4) FZ, a two-month old child with intractable seizures who died in *status epilepticus,* (5) CN, who had hydrocephalus, cerebral palsy, and epileptiform seizures, and (6) LD, who had primary infantile spasms. Hydrocephalus was codiagnosed in 3 of 8 patients diagnosed with epilepsy or other epileptiform illness.

Although one of the 14 *Cryptovirus*-negative CSF specimens was obtained from a patient who had been diagnosed with epilepsy, diagnoses were not provided for the remaining specimens. They were simply characterized as pediatric neurology or neurosurgery specimens from asymptomatic patients (*i.e*., patients who had not presented with epileptiform symptoms or been diagnosed with epileptiform illness).

(e) *Multiple Sclerosis (MS).* Clinical specimens from patients with MS comprise one of the largest groups of materials screened (38 serum samples and 30 CSF samples including 30 matched sets of each). Eight of the serum samples came from MS patients in Brisbane, Queensland who had debilitating disease and were living in a nursing home run by the National Multiple Sclerosis Society of Australia. No CSF specimens were acquired from these patients. The 30 matched sets of serum and CSF were provided by the National Neurological Research Specimen Bank (NNRSB) in Los Angeles.

Fig. 19 illustrates the results of RIP assays conducted with serum samples of 5/30 MS patients provided by the NNRSB. The results obtained from an additional 25 serum specimens provided by the NNRSB are shown in Fig. 20, and the RIP results from 16/30 of the CSF specimens from MS patients provided by the NNRSB are shown in Fig. 21. RIPs performed using the remaining 8 specimens resulted in similar profiles (data not shown). As shown in Figs. 19-21, the results of these analyses demonstrated that all patients had high levels of *Cryptovirus*-specific antibodies in their serum (100%) and 29/30 had *Cryptovirus-specific* antibodies in their CSF (96.7%).

(f) *Subacute Sclerosing Panencephalitis (SSPE).* The anomalies that have been observed which are inconsistent with measles virus alone being the sole cause of SSPE (see Discussion of Related Art) can be explained by the evidence that the aetiopathogenesis of SSPE involves dual infection of the CNS by measles and *Cryptovirus* (which was isolated from SSPE patients).

Sera from SSPE patients were found to precipitate the major nucleocapsid protein of the virus (NP, 63 kD) from nonproductively-infected AV₃/SSPE cells (see Fig. 22). Fig. 22 is a photograph of an autoradiogram obtained following creation of RIP profiles of the *Cryptovirus* NP protein (p63) precipitated from [³⁵S]-methionine-labeled AV₃/SSPE cells by the sera of six Australian SSPE patients (Lanes 1-6) and six control sera (Lanes 7-12; sera from pediatric patients without antibodies to the *Cryptovirus* major envelope proteins (F₀, HN).

Fig. 23 is a photograph of an autoradiogram of RIP profiles of measles virus-specific and *Cryptovirus-specific* proteins precipitated from [³⁵S]-methionine-labeled measles virus-infected CV-1_{c} cells (Lane MV), *Cryptovirus*-infected CV-1_{c} cells (Lane CV) or a mixture of both (Lane B) by CSF sampled from an 11-year old male diagnosed with SSPE. Lane V = gradient-purified *Cryptovirus* virions from [³⁵S]-methionine-labeled *Cryptovirus*-infected CV-1_{c} cells. Fig. 23 shows that CSF from this SSPE patient precipitated both *Cryptovirus* and measles virus proteins. The results of this assay demonstrate that SSPE CSF contains both measles virus-specific and *Cryptovirus*-specific antibodies (i.e. antibody to the HN protein of measles virus (Lane MV) and the HN and F₀ proteins of *Cryptovirus* virus (Lane CV) and that both are present in nearly equal amounts. This was unique, since none of the other CSFs samples that precipitated *Cryptovirus* proteins (e.g., from MS, CFS, or epilepsy patients) also preciptated the measles virus HN protein.

The RIP profile of *Cryptovirus*-specific proteins precipitated by this CSF specimen is typical of those produced by the *"Cryptovirus*-positive" CSFs of MS patients, CFS patients and idiopathic epilepsy patients tested to date (compare Figs. 12A and 17). While there was considerable variation in the strength of the antibody response to the F₀ and HN proteins, there appeared to be little variation in the presence of antibody to one protein or the other. There was, however, a variable response to the F₁ and F₂ proteins (*i.e*., in many patients, such responses appeared to be absent). This paradox may relate to the proteolytic cleavage of the protein *in situ* and corresponding immune responses (*i.e*., the F₀ protein is efficiently cleaved by some patients, generating the F₁ and F₂ fragments and ultimately exposing their immune system to them); in other patients the protein may be cleaved less efficiently (or not at all) and, therefore, these patients do not generate as much of an antibody response to the fragments).

### Example 9. Correlations Between Affected Human Patients and Experimentally-Infected Animals

Some of the examples herein highlight the epileptiform symptomology presented by many of the patients who have *Cryptovirus-*specific antibodies in their cerebrospinal fluid (CSF) or by mice experimentally-infected with the virus. This association is strong, but not all patients with *Cryptovirus*-specific antibodies in their CSF present with overt seizures or convulsions. There is instead a spectrum of responses, from little or no seizure activity, through mild activity (*petit mal* or "absence" seizures), to recurrent and intractable *grand mal* seizures (the occurrence of which is often misunderstood by the lay public to be the defining symptom of all forms of epilepsy; *see* Epilepsy: A Comprehensive Textbook, Engel, Jr. J. and Pedley, T.A., Eds., Lippincott-Raven, 1997).

With regard to the development of symptoms and manifestations of human *Cryptovirus-*infections, it is essential to be cognizant of the spatial ("where"), temporal ("when"), and quantum ("what else") factors involved: (1) which cells, tissues, neural tracts and CNS structures become infected by the virus, (2) the developmental state of those systems at the time of infection, and (3) the role of environmental and host factors in development and progress of the infection, respectively.

For example, the data presented here establish a strong correlation between the development of epileptiform symptomology and *early* CNS infection with the virus (*i.e*., in infancy, early childhood or adolescence and in experimentally-infected neonatal mice), this correlation is less strong in adults (and adult mice who do not develop epileptiform symptoms) and the spectrum of CNS manifestations observed is much wider.

Generally, the characteristic most frequently and consistently presented by humans or animals that have been experimentally infected with the virus is the development of "iterative" or "compulsive" neuropathies and behaviors. This is most likely due to the selective loss of (or immunopathological damage done to) neurons (*e.g*. interneurons) or neuron tracts in different parts of the central nervous system (CNS) at different times or at different stages of CNS development.

When the medical records of patients who had *Cryptovirus*-specific antibodies in their CSF were examined, all of the patients had been diagnosed with one or more serious neurological disorders. These included, but were not limited to:
(1) subacute sclerosing panencephalitis (SSPE): 4 of 4 CSFs tested (100%), all adolescent patients);
(2) idiopathic / cryptogenic epilepsy: 6 of 7 CSFs tested (85.7%), from infants and children presenting with seizures and diagnosed with idiopathic or cryptogenic forms of epilepsy;
(3) multiple sclerosis (MS): 29 of 30 CSFs tested (96.7%) all adult specimens; and
(4) chronic fatigue syndrome (CFS) / clinical depression: 11 of 12 CSFs tested (91.7%), all adult specimens.

These results demonstrate a clear correlation between *Cryptovirus*-specific antibodies in the CSF and a narrow spectrum of CNS diseases. Although the diseases listed above have been defined as representatives of discrete pathognomonic entities, there is in reality substantial overlap between the symptoms presented by these patients and their diagnoses. For example, virtually every patient eventually diagnosed with SSPE is initially diagnosed with epilepsy. Similarly, early stage MS is extremely similar in presentation to CFS, and clinical depression is a common characteristic of both. Not surprisingly, another name for CFS is "atypical multiple sclerosis" (in Bell, The Disease of a Thousand Names, Pollard Publications, Lyndonville, NY [1991]).

There is a strong correlation between (1) the age of those patients who had severe epileptiform illness (SSPE and epilepsy, the majority of whom are infants, children, or adolescents) and (2) the age of those patients who had more diffuse or subtle neurological dysfunction (e.g., MS and CFS patients who were all adults). Furthermore, SSPE, certain forms of idiopathic and cryptogenic epilepsy, and MS have many neuropathological characteristics in common. These include areas of discrete, focal or disseminated sclerosis (scar formation in CNS tissue), dysplastic lesions (either as the result of immunopathological processes or neuron tract loss), and perivascular curing of immune cells (evidence of inflammatory processes in the vicinity of lesions). Thus, each of these diseases could represent a different pathological "complex" of spatial, temporal, and quantum factors that have *Cryptovirus* infection of CNS tissues as a shared characteristic. With respect to SSPE, previous data have established (and the data here confirm) that measles virus is also involved in this illness. SSPE is caused by widespread CNS infection by both viruses (resulting in inflammatory and disseminated sclerosis across the white matter of the brain) while certain forms of idiopathic epilepsy represent early infection of the CNS by *Cryptovirus* alone (resulting in the loss of susceptible interneurons and neuron tracts and the development of discrete dysplastic lesions). MS, occurring almost exclusively in adults, represents the pathological outcome of late and focal *Cryptovirus* infection of the CNS - due to the restriction of *Cryptovirus* replication in fully differentiated CNS tissues and the effective partitioning of brain by the mature glial architecture.

In summary, intracranial inoculation of mice with the virus, or with cells nonproductively-infected with the virus, results in the subacute development of neuropathological diseases in a significant proportion of the animals. These diseases are closely akin to the spectrum of human neuropathies seen in patients with *Cryptovirus*-specific antibodies in their cerebrospinal fluids.

Further, although patients with *Cryptovirus*-positive CSF had been diagnosed with a spectrum of illnesses, there was a clear partitioning of patients into two groups (1) infants, children and adolescents with illnesses dominated by subacute epileptiform physical symptoms which were often life-threatening and (2) young adults and adults with slowly-developing chronic illnesses which presented with less pronounced physical symptoms but significant neuropsychological components which were usually not life-threatening.

These findings support the conclusion that *Cryptovirus* is responsible for a neurological spectrum disorder whose ultimate manifestation depends on (1) the age of the individual when they contract the primary infection, (2) the mechanism by which the virus gains entry into the CNS tissue, (3) the extent of the infection at that time, (4) the stage of development of the CNS when it becomes infected, (5) the part of the CNS which becomes infected, (6) genetic factors (*e.g*., immune system defects, neurological malformations, the presence for absence of virus receptors on CNS tissues, etc.) and (7) other environmental factors (*e.g*., the occurrence of head trauma, neurosurgery of any kind, prior or concurrent infection of CNS tissue by other agents, exposure to drugs or toxic chemicals, *etc.*).

A number of embodiments of the invention have been described. Nevertheless, it will be understood that various modifications can be made without departing from the spirit and scope of the invention. Accordingly, other embodiments are within the scope of the following claims.

## Claims

1. An isolated nucleic acid, comprising:
(A)contiguous nucleotide positions 1-15246 of (SEQ ID NO: 1);
(B)a nucleotide sequence complementary to (A)or
(C)a *Cryptovirus*-specific fragment of (A) or (B), comprising a nucleic acid segment selected from the group consisting of:
(i) contiguous nucleotide positions 152-1678 of (SEQ ID NO:1) ;
(ii) contiguous nucleotide positions 1850-2515 of (SEQ ID NO:1);
(iii)contiguous nucleotide positions 1850-3023 of (SEQ ID NO:1) ;
(iv) contiguous nucleotide positions 1850-3023 of (SEQ ID NO:1) combined with a further insertion of two guanine residues between nucleotide position 2339 of (SEQ ID NO:1) and nucleotide position 2340 of (SEQ ID NO:1);
(v) contiguous nucleotide positions 3141-4271 of (SEQ ID NO:1);
(vi) contiguous nucleotide positions 4530-6182 of (SEQ ID NO:1;
(vii) contiguous nucleotide positions 4587-6182 of (SEQ ID NO:1) ;
(viii) contiguous nucleotide positions 4836-6182 of (SEQ ID NO:1);
(ix) contiguous nucleotide positions 4272-6515 of (SEQ ID NO:1);
(x) contiguous nucleotide positions 6303-6434 of (SEQ ID NO:1);
(xi) contiguous nucleotide positions 6584-8278 of (SEQ ID. NO:1); and
(xii) contiguous nucleotide positions 8414-15178 of (SEQ ID NO:1).

2. The nucleic acid of Claim 1, wherein the nucleic acid is RNA or DNA.

3. A composition of matter, comprising the nucleic acid of Claim 1; and a carrier.

4. A nucleic acid construct, comprising the nucleic acid of Claim 1.

5. Use of the nucleic acid of Claim 1 in manufacturing a vaccine.

6. Use of the nucleic acid construct of Claim 4 in manufacturing a vaccine.

7. An expression vector, comprising the nucleic acid construct of Claim 4.

8. A cloning vector, comprising the nucleic acid construct of Claim 4.

9. A host cell, comprising the expression vector of Claim 7 or the cloning vector of Claim 8.

10. The host cell of Claim 9, wherein the cell is a mammalian cell.

11. An isolated *Cryptovirus* protein comprising an amino acid sequence as defined in any of SEQ ID numbers 4, 6, 8, 10, 12, 14, 18, 20, 22, or 24.

12. The protein of Claim 11, wherein the protein is a *Cryptovirus* envelope protein encoded by a nucleic acid segment comprising an amino acid sequence as defined in any of SEQ ID NOs: 12, 14, 18, 20, or 22.

13. A composition of matter, comprising the protein of Claim 11; and a carrier.

14. A chimeric protein, comprising a *Cryptovirus* protein comprising an amino acid sequence as defined in claim 12.

15. The chimeric protein of Claim 14, wherein the *Cryptovirus* protein is a *Cryptovirus* envelope protein comprising an amino acid sequence as defined in claim 12.

16. Use of the protein of Claim 11 or Claim 14 in producing a *Cryptovirus*-specific antibody.

17. Use of the protein of Claim 11 or Claim 14 in producing a vaccine.

18. An isolated viral particle, comprising the nucleic acid of Claim 1.

19. A composition of matter, comprising the virion of Claim 18; and a carrier.

20. An isolated viral particle, comprising the protein of Claim 12.

21. An isolated *Cryptovirus* particle, comprising a genome having a nucleotide sequence entirely complementary to (SEQ ID NO:1).

22. Use of the viral particle of Claim 20 in manufacturing a vaccine.

23. Use of the *Cryptovirus* particle of Claim 21 in manufacturing a vaccine.

24. The use of Claim 22 or 23, wherein the viral particle is an attenuated virion.

25. A method of detecting the presence or absence of a *Cryptovirus*-specific RNA in a sample of a biological material, comprising:
contacting the sample with the probe comprising a nucleic acid as defined in Claim 1 under at least moderately stringent hybridization conditions, wherein the formation of detectable hybridization products indicates the presence of the *Cryptovirus*-specific RNA in the sample.

26. A method of detecting the presence or absence of a *Cryptovirus*-specific RNA in a sample of a biological material, comprising:
amplifying *Cryptovirus-*specific RNA in the sample using at least one primer comprising a nucleic acid as defined in Claim 1 in an amplification reaction mixture;
then detecting the presence or absence of *Cryptovirus*-specific nucleic acid amplification products in the amplification reaction mixture, wherein the presence of the amplification products in the reaction mixture indicates the presence of the *Cryptovirus* RNA in the sample.

27. The method of any of Claims 25 or 26, wherein the biological material is a cellular material, or
is blood or serum, or
is cerebrospinal fluid, or
is lymphoid tissue, or
is nervous tissue.

28. The method of Claim 27, wherein the nervous tissue is brain tissue.

29. A method of detecting the presence or absence of a *Cryptovirus*-specific antibody in a sample of a biological material, comprising:
contacting the sample with the protein of Claim 11:
allowing the formation of a specific protein-antibody complex; detecting the presence of the specific protein-antibody complex, wherein the presence of a specific protein-antibody complex indicates the presence of the *Cryptovirus-*specific antibody in the sample.

30. A method of detecting the presence or absence of a *Cryptovirus*-specific antibody in a sample of a biological material, comprising:
contacting the sample with the protein of Claim 14; allowing the formation of a specific protein-antibody complex; detecting the presence of the specific protein-antibody complex, wherein the presence of a specific protein-antibody complex indicates the presence of the *Cryptovirus*-specific antibody in the sample.

31. An assay method for detecting the presence or absence of an antibody that selectively binds *Cryptovirus* in a sample of an antibody-containing biological material originating from a human, comprising:
contacting the sample, the sample originating from an individual suspected of having a *Cryptovirus* infection, with the envelope protein of Claim 12, such that, if antibody selectively binding *Cryptovirus* is present, an antibody-bound envelope protein complex forms;
contacting any antibody-bound envelope protein complexes thus formed with anti-human antibody-binding antibody, and allowing the formation of complexes of the antibody, with the antibody-bound envelope protein complexes; and
detecting the presence or absence of any antibody-bound envelope protein complexes thus formed, the presence of such complexes indicating the presence in the sample of antibody selectively binding *Cryptovirus.*

32. An assay method for detecting the presence or absence of antibody that selectively binds *Cryptovirus* antigen in a sample of an antibody-containing biological material originating from a human, the method comprising:
contacting the sample, the sample originating from an individual suspected of having a *Cryptovirus* infection, with the viral particle of Claim 20, such that, if antibody selectively binding *Cryptovirus* antigen is present, an antibody-bound virus complex forms;
contacting any antibody-bound virus complexes thus formed with anti-human antibody-binding antibody, and allowing the formation of complexes of the anti-human antibody-binding antibody with the antibody-bound virus complexes; and detecting the presence or absence of any complexes formed, the presence of such complexes indicating the presence in the sample of antibody selectively binding *Cryptovirus* antigen.

33. A method of detecting a *Cryptovirus* infection in a mammal, comprising:
performing the method of.any of Claims 25, 26, 30, 31, or 32, using the sample, whereby detecting the presence of the *Cryptovirus* protein, *Cryptovirus*-specific RNA, and/or *Cryptovirus-*specific antibody in the sample indicates a *Cryptovirus* infection in the mammal.

34. The method of Claim 33, wherein the mammal is a human, or
wherein the human has a neurological, neurodegenerative, and/or neuropsychiatric disease, or
wherein the human has a primary tracheobronchial and/or
lymphadenopathy-associated illness.

35. A method of isolating a *Cryptovirus* virion, comprising the nucleic acid sequence of Claim 1 the method comprising:
culturing a plurality of peripheral blood mononuclear cells that have been obtained from a human having a *Cryptovirus* infection , the human having been infected with the virus particle of claim 21, in an artificial aqueous medium comprising an agent that increases cellular guanylyl cyclase activity;
co-culturing the plurality of peripheral blood mononuclear cells with a plurality of mammalian amnion cells in fresh artificial aqueous medium comprising an agent that increases cellular guanylyl cyclase activity;
passaging the peripheral blood mononuclear cells with the mammalian amnion cells in co-culture;
co-cultivating a plurality of mammalian epithelial cells together with the peripheral blood mononuclear cells and the mammalian amnion cells in fresh artificial aqueous medium comprising an agent that increases cellular guanylyl cyclase activity; and
separating a supernatant of the aqueous medium from the cells, to obtain a *Cryptovirus* virion in the supernatant.

36. A method of propagating a *Cryptovirus,* comprising:
(a) exposing a plurality of mammalian epithelial cells to a plurality of cell-free *Cryptovirus* virions, said *Cryptovirus* virions having been isolated by the method of Claim 35; and
(b)further cultivating the mammalian epithelial cells, thus virion-exposed, in an artificial aqueous medium comprising an agent that increases the activity of cellular guanylyl cyclase.

37. A method of producing a mammalian cell line nonproductively infected with a *Cryptovirus,* encoded by a nucleic acid of claim 1 comprising:
(a) co-culturing peripheral blood mononuclear cells that have been obtained from a human having a *Cryptovirus* infection, with mammalian amnion cells, in an artificial aqueous medium comprising an agent that increases cellular guanylyl cyclase activity, such that the mammalian amnion cells become nonproductively infected by *Cryptovirus*; and
(b) passaging the nonproductively infected mammalian amnion cells with the peripheral blood mononuclear cells, whereby the co-culture becomes a monoculture of the nonproductively infected mammalian amnion cells.

38. The method of Claim 35 or Claim 37, wherein the mammalian amnion cells are human amnion cells.

39. The method of Claim 38, wherein the human amnion cells are AV₃ cells.

40. The method of Claim 35 or Claim 36, wherein the mammalian epithelial cells are simian epithelial cells selected from the group consisting of Vero or CV-1 cells.

41. The method of Claim 40, wherein the CV-1 cells are subline CV-1 cells.

42. The method of Claims 35, 36, or 37, wherein the agent that increases cellular guanylyl cyclase activity is cyclic GMP, insulin, zinc dication, or a combination of any of these.

43. The method of Claim 42, wherein the cyclic GMP is in a concentration of about 0.05 to about 5 mM in the artificial aqueous medium.

44. The method of Claim 35, 36, or 37, wherein the agent that increases cellular guanylyl cyclase activity is nitric oxide or a nitric oxide donor selected from the group consisting of organic nitrate compounds, iron nitrosyl compounds, S-nitrosothiol compounds, sydnonimine compounds, and NONOate compounds.

45. The method of Claims 35, 36, or 37, wherein the aqueous medium further comprises glutamine.

46. A method of producing a mammalian epithelial cell line acutely infected with *Cryptovirus,* comprising the method of Claim 36.

47. A mammalian epithelial cell acutely infected with *Cryptovirus,* said cell being produced by the method of Claim 36.

48. A cell nonproductively infected with *Cryptovirus,* wherein said cell is produced in accordance with the method of Claim 37.

49. An in vitro method of screening a potential antiviral therapeutic agent, comprising:
(a) culturing the cell of Claim 47;
(b) exposing the cells to the potential antiviral therapeutic agent; and
(c) measuring the effect of the agent on *Cryptovirus* replication and/or *Cryptovirus* virion assembly, wherein inhibition of *Cryptovirus* replication and/or *Cryptovirus* virion assembly relative to a control indicates antiviral activity of the potential therapeutic agent.

50. An in vitro method of screening a potential antiviral therapeutic agent, comprising:
(a) culturing the cell of Claim 47;
(b) exposing the cells to the potential antiviral therapeutic agent; and
(c) measuring the effect of the agent on *Cryptovirus* replication, *Cryptovirus* genome replication, and/or *Cryptovirus-*specific transcription, wherein inhibition of *Cryptovirus* replication, *Cryptovirus* genome replication, and/or *Cryptovirus-*specific transcription, relative to a control, indicates antiviral activity of the potential therapeutic agent.

51. An animal model for the study of human diseases, comprising a non-human mammal, said non-human mammal having been artificially inoculated with an infectious cell-free *Cryptovirus* having a genome comprising a single stranded RNA complementary to (SEQ ID NO:1), or having been inoculated with a cell nonproductively-infected with the *Cryptovirus,* whereby the non-human mammal exhibits at least one symptom characteristic of a human disease after being thus inoculated, said symptom not being previously exhibited by the non-human mammal.

52. The animal model of Claim 51, wherein the non-human mammal is a rodent or lagomorph, or
wherein the non-human mammal is a non-human primate, or wherein the human disease is a neurological, neurodegenerative, and/or neuropsychiatric disease.

53. An anti-*Cryptovirus* antibody detecting kit, comprising:
the *Cryptovirus* particle of Claim 18; and
a labeled anti-human antibody-binding antibody.

54. The anti-*Cryptovirus* antibody detecting kit in accordance with Claim 53, further comprising a solid matrix for supporting said *Cryptovirus* particle.

55. An anti-*Cryptovirus* antibody detecting kit, comprising:
the protein of Claim 11 or Claim 14: and
a labeled anti-human antibody-binding antibody.

56. The anti-*Cryptovirus* antibody detecting kit in accordance with Claim 54, further comprising a solid matrix for supporting said protein.

## Patentansprüche

1. Isolierte Nukleinsäure, umfassend:
(A) zusammenhängende Nukleotidpositionen 1-15246 der (SEQ ID Nr.: 1);
(B) eine Nukleotid-Sequenz, die komplementär zu (A) ist oder
(C) ein *Cryptovirus*-spezifisches Fragment von (A) oder (B), umfassend einen Nukleinsäureabschnitt ausgewählt aus der Gruppe bestehend aus:
(i) zusammenhängende Nukleotidpositionen 152-1678 der (SEQ ID Nr.: 1);
(ii) zusammenhängende Nukleotidpositionen 1850-2515 der (SEQ ID Nr.: 1);
(iii) zusammenhängende Nukleotidpositionen 1850-3023 der (SEQ ID Nr.: 1);
(iv) zusammenhängende Nukleotidpositionen 1850-3023 der (SEQ ID Nr.: 1) kombiniert mit einer zusätzlichen Insertion von zwei Guaninresten zwischen der Nukleotidposition 2339 der (SEQ ID Nr.: 1) und der Nukleotidposition 2340 der (SEQ ID Nr.: 1);
(v) zusammenhängende Nukleotidpositionen 3141-4271 der (SEQ ID Nr.: 1);
(vi) zusammenhängende Nukleotidpositionen 4530-6182 der (SEQ ID Nr.: 1);
(vii) zusammenhängende Nukleotidpositionen 4587-6182 der (SEQ ID Nr.: 1);
(viii) zusammenhängende Nukleotidpositionen 4836-6182 der (SEQ ID Nr.: 1);
(ix) zusammenhängende Nukleotidpositionen 4272-6515 der (SEQ ID Nr.: 1);
(x) zusammenhängende Nukleotidpositionen 6303-6434 der (SEQ ID Nr.: 1);
(xi) zusammenhängende Nukleotidpositionen 6584-8278 der (SEQ ID Nr.: 1); und
(xii) zusammenhängende Nukleotidpositionen 8414-15178 der (SEQ ID Nr.: 1).

2. Nukleinsäure gemäß Anspruch 1, wobei die Nukleinsäure RNA oder DNA ist.

3. Zusammensetzung, umfassend die Nukleinsäure gemäß Anspruch 1 und einen Träger.

4. Nukleinsäurekonstrukt, umfassend die Nukleinsäure gemäß Anspruch 1.

5. Verwendung der Nukleinsäure gemäß Anspruch 1 zur Herstellung eines Impfstoffes.

6. Verwendung des Nukleinsäurekonstrukts gemäß Anspruch 4 zur Herstellung eines Impfstoffs.

7. Expressionsvektor, umfassend das Nukleinsäurekonstrukt gemäß Anspruch 4.

8. Klonierungsvektor, umfassend das Nukleinsäurekonstrukt gemäß Anspruch 4.

9. Wirtszelle, umfassend den Expressionsvektor gemäß Anspruch 7 oder den Klonierungsvektor gemäß Anspruch 8.

10. Wirtszelle gemäß Anspruch 9, wobei die Zelle eine Säugerzelle ist.

11. Isoliertes *Cryptovirus*-Protein, umfassend eine Aminosäuresequenz die durch eine der SEQ ID Nrn. 4, 6, 8, 10, 12, 14, 18, 20, 22 oder 24 definiert ist.

12. Protein gemäß Anspruch 11, wobei das Protein ein *Cryptovirus*-Hüllprotein ist, das durch einen Nukleinsäureabschnitt kodiert wird, der eine Aminosäuresequenz, wie in einer der SEQ ID Nrn. 12, 14, 18, 20 oder 22 definiert, umfasst.

13. Zusammensetzung, umfassend das Protein gemäß Anspruch 11 und einen Träger.

14. Chimäres Protein, umfassend ein *Cryptovirus*-Protein, das eine Aminosäuresequenz, wie in Anspruch 12 definiert, umfasst.

15. Chimäres Protein gemäß Anspruch 14, wobei das *Cryptovirus*-Protein ein *Cryptovirus*-Hüllprotein ist, das eine Aminosäuresequenz, wie in Anspruch 12 definiert, umfasst.

16. Verwendung des Proteins gemäß Anspruch 11 oder Anspruch 14 zur Herstellung eines *Cryptovirus*-spezifischen Antikörpers.

17. Verwendung des Proteins gemäß Anspruch 11 oder Anspruch 14 zur Herstellung eines Impfstoffs.

18. Isoliertes Viruspartikel, umfassend die Nukleinsäure gemäß Anspruch 1.

19. Zusammensetzung, umfassend das Virion gemäß Anspruch 18 und einen Träger.

20. Isoliertes Viruspartikel, umfassend das Protein gemäß Anspruch 12.

21. Isoliertes *Cryptovirus*-Partikel, umfassend ein Genom, das eine Nukleotidsequenz aufweist, die gänzlich komplementär zu (SEQ ID Nr.: 1) ist.

22. Verwendung des Viruspartikels gemäß Anspruch 20 zur Herstellung eines Impfstoffs.

23. Verwendung des *Cryptovirus*-Partikels gemäß Anspruch 21 zur Herstellung eines Impfstoffs.

24. Verwendung gemäß Anspruch 22 oder 23, wobei das Viruspartikel ein attenuiertes Virion ist.

25. Verfahren zum Nachweisen der Gegenwart oder Abwesenheit einer *Cryptovirus*-spezifischen RNA in einer Probe eines biologischen Materials, umfassend:
das In-Kontakt-bringen der Probe mit dem Reagens, das eine Nukleinsäure, wie im Anspruch 1 definiert, umfasst, unter mindestens mäßig stringenten
Hybridisierungsbedingungen, wobei die Bildung von nachweisbaren Hybridisierungsprodukten die Gegenwart der *Cryptovirus*-spezifischen RNA in der Probe anzeigt.

26. Verfahren zum Nachweisen der Gegenwart oder Abwesenheit einer *Cryptovirus*-spezifischen RNA in einer Probe eines biologischen Materials, umfassend:
das Amplifizieren *Cryptovirus*-spezifischer RNA in der Probe unter Verwendung von mindestens einem Primer, der eine Nukleinsäure, wie in Anspruch 1 definiert, umfasst, in einer Amplifikationsreaktionsmischung;
anschließend das Nachweisen der Gegenwart oder Abwesenheit von *Cryptovirus*-spezifischen Nukleinsäure-Amplifikationsprodukte in der
Amplifikationsreaktionsmischung, wobei die Gegenwart der Amplifikationsprodukte in der Reaktionsmischung die Gegenwart der *Cryptovirus*-RNA in der Probe anzeigt.

27. Verfahren gemäß einem der Ansprüche 25 oder 26, wobei das biologische Material
ein zelluläres Material oder
Blut oder Serum oder
Cerebrospinalflüssigkeit oder
Lymphgewebe oder
Nervengewebe
ist.

28. Verfahren gemäß Anspruch 27, wobei das Nervengewebe Gehirngewebe ist.

29. Verfahren zum Nachweisen der Gegenwart oder Abwesenheit eines *Cryptovirus*-spezifischen Antikörpers in einer Probe eines biologischen Materials, umfassend:
das In-Kontakt-bringen der Probe mit dem Protein von Anspruch 11:
das Ermöglichen der Bildung eines spezifischen Protein-Antikörper-Komplexes;
das Nachweisen der Gegenwart des spezifischen Protein-Antikörper-Komplexes, wobei die Gegenwart eines spezifischen Protein-Antikörper-Komplexes die Gegenwart des *Cryptovirus*-spezifischen Antikörpers in der Probe anzeigt.

30. Verfahren zum Nachweisen der Gegenwart oder Abwesenheit eines *Cryptovirus*-spezifischen Antikörpers in einer Probe eines biologischen Materials, umfassend:
das In-Kontakt-bringen der Probe mit dem Protein von Anspruch 14;
das Ermöglichen der Bildung eines spezifischen Protein-Antikörper-Komplexes;
das Nachweisen der Gegenwart des spezifischen Protein-Antikörper-Komplexes, wobei die Gegenwart eines spezifischen Protein-Antikörper-Komplexes die Gegenwart des *Cryptovirus*-spezifischen Antikörpers in der Probe anzeigt.

31. Assayverfahren zum Nachweisen der Gegenwart oder Abwesenheit eines Antikörpers, der selektiv *Cryptovirus* in einer Probe eines Antikörper-haltigen biologischen Materials bindet, das von einem Menschen stammt, umfassend:
Das In-Kontakt-bringen der Probe, die von einem Individuum stammt, bei dem vermutet wird, dass es eine *Cryptovirus*-Infektion hat, mit dem Hüllprotein von Anspruch 12, so dass, falls ein Antikörper, der *Cryptovirus* selektiv bindet, vorhanden ist, sich ein Antikörper-gebundener Hüllproteinkomplex bildet;
das In-Kontakt-bringen der so gebildeten Antikörper-gebundenen Hüllproteinkomplexe mit anti-humanem Antikörper-bindenden Antikörper und das Ermöglichen der Bildung von Komplexen des Antikörpers mit dem Antikörper-gebundenen Hüllproteinkomplexen; sowie
das Nachweisen der Gegenwart oder Abwesenheit von so gebildeten Antikörper-gebundenen Hüllproeinkomplexen, wobei die Gegenwart von solchen Komplexen die Gegenwart von Antikörpern, die *Cryptovirus* selektiv binden, in der Probe anzeigt.

32. Assayverfahren zum Nachweisen der Gegenwart oder Abwesenheit eines Antikörpers, der selektiv *Cryptovirus-*Antigen bindet, in einer Probe eines Antikörper-haltigen biologischen Materials, das von einem Menschen stammt, wobei das Verfahren umfasst:
das In-Kontakt-bringen der Probe, wobei die Probe von einem Individuum stammt, bei dem eine *Cryptovirus-*Infektion vermutet wird, mit dem Virus von Anspruch 20, so dass, falls ein Antikörper, der *Cryptovirus*-Antigen selektiv bindet, vorhanden ist, sich ein Antikörper-gebundener Viruskomplex bildet;
das In-Kontakt-bringen so gebildeter Antikörper-gebundener Viruskomplexe mit anti-humanem Antikörper-bindenden Antikörper und das Ermöglichen der Bildung von Komplexen des anti-humanen Antikörper-bindenden Antikörpers mit den Antikörpern-gebundenen Viruskomplexen;
sowie das Nachweisen der Gegenwart oder Abwesenheit von gebildeten Komplexen, wobei die Gegenwart von solchen Komplexen die Gegenwart eines Antikörpers, der *Cryptovirus*-Antigen selektiv bindet, in der Probe anzeigt.

33. Verfahren zum Nachweisen einer *Cryptovirus*-Infektion in einem Säuger, umfassend:
Durchführen des Verfahrens gemäss einem der Ansprüche 25, 26, 30, 31 oder 32 unter Verwendung der Probe, wobei das Nachweisen der Gegenwart von *Cryptovirus*-Protein, *Cryptovirus*-spezifischer RNA und/oder *Cryptovirus*spezifischem Antikörper in der Probe eine *Cryptovirus-*Infektion in dem Säuger anzeigt.

34. Verfahren gemäß Anspruch 33, wobei der Säuger ein Mensch ist, oder wobei der Mensch eine neurologische, neurodegenerative und/oder neuropsychiatrische Krankheit hat oder
wobei der Mensch eine primäre tracheobronchiale und/oder Lymphadenopathie-assoziierte Krankheit hat.

35. Verfahren zum Isolieren eines *Cryptovirus,* umfassend die Nukleinsäuresequenz von Anspruch 1, wobei das Verfahren umfasst:
das Kultivieren einer Vielzahl von peripheren einkernigen Blutzellen, die von einem Menschen mit einer *Cryptovirus*-Infektion erhalten wurden, wobei der Mensch mit dem Viruspartikel von Anspruch 21 infiziert wurde, in einem künstlichen wässrigen Medium, das ein Mittel umfasst, das die zelluläre Guanylatcyclase-Aktivität erhöht;
das Co-Kultivieren der Vielzahl peripherer einkerniger Blutzellen mit einer Vielzahl von Säugeramnionzellen in frischem künstlichem Medium, das ein Mittel umfasst, das die zelluläre Guanylatcyclase-Aktivität erhöht;
das Passieren der peripheren einkernigen Blutzellen mit den Säugeramnionzellen in Co-Kultur,
das Co-Kultivieren einer Vielzahl von Säugerepithelzellen zusammen mit den peripheren einkernigen Blutzellen und den Säugeramnionzellen in frischem künstlichen wässrigen Medium, das ein Mittel umfasst, das die zelluläre Guanylatcyclase-Aktivität erhöht; sowie
das Trennen des Überstands des wässrigen Mediums von den Zellen, um ein *Cryptovirus*-Virion in dem Überstand zu erhalten.

36. Verfahren zum Propagieren eines *Cryptovirus,* umfassend:
(a) das Exponieren einer Vielzahl von Säugerepithelzellen gegenüber einer Vielzahl von zellfreiem *Cryptovirus*-Virionen, wobei die *Cryptovirus*-Virionen mit dem Verfahren von Anspruch 35 isoliert wurden; sowie
(b) weiterhin das Kultivieren der so Virion-exponierten Säugerepithelzellen in einem künstlichen wässrigen Medium, das ein Mittel umfasst, das die Aktivität der zellulären Guanylatcyclase erhöht.

37. Verfahren zum Herstellen einer Säugerzelllinie, die nicht-produktiv mit einem *Cryptovirus* infiziert ist, das von einer Nukleinsäure von Anspruch 1 kodiert wird, umfassend:
(a) das Co-Kultivieren von peripheren einkernigen Blutzellen, die von einem Menschen mit einer *Cryptovirus*-Infektion erhalten wurden, mit Säugeramnionzellen in einem künstlichen wässrigen Medium, das ein Mittel umfasst, das die zelluläre Guanylatcyclase-Aktivität erhöht, so dass die Säugerzellen nicht-produktiv durch *Cryptovirus* infiziert werden; sowie
(b) das Passieren der nicht-produktiv infizierten Säugeramnionzellen mit den peripheren einkernigen Blutzellen, wobei die Co-Kultur eine Monokultur der nicht-produktiv infizierten Säugeramnionzellen wird.

38. Verfahren gemäß Anspruch 35 oder Anspruch 37, wobei die Säugeramnionzellen humane Amnionzellen sind.

39. Verfahren gemäß Anspruch 38, wobei die humanen Amnionzellen AV₃-Zellen sind.

40. Verfahren gemäß Anspruch 35 oder Anspruch 36, wobei die Säugerepithelzellen Simianepithelzellen sind, die aus der Gruppe von Vero- oder CV-1-Zellen ausgewählt werden.

41. Verfahren gemäß Anspruch 40, wobei die CV-1-Zellen Sublinien-CV-1-Zellen sind.

42. Verfahren gemäß den Ansprüchen 36, 36 oder 37, wobei das Mittel, das die zelluläre Guanylatcyclase-Aktivität erhöht, cyclisches GMP, Insulin, Zink-Dikation oder eine Kombination davon ist.

43. Verfahren gemäß Anspruch 42, wobei das cyclische GMP in einer Konzentration von ungefähr 0,05 bis ungefähr 5 mM in dem künstlichen wässrigen Medium vorliegt.

44. Verfahren gemäß den Ansprüchen 35, 36 oder 37, wobei das Mittel, das die zelluläre Guanylatcyclase-Aktivität erhöht, Stickoxid oder ein Stickoxiddonor, ausgewählt aus der Gruppe bestehend aus organischen Nitratverbindungen, Eisennitrosylverbindungen, S-Nitrosothiolverbindungen, Sydnoniminverbindungen und NONOat-Verbindungen ist.

45. Verfahren gemäß den Ansprüchen 35, 36, oder 37, wobei das wässrige Medium weiterhin Glutamin umfasst.

46. Verfahren zum Herstellen einer Säugerepithelzelllinie, die akut mit *Cryptovirus* infiziert ist, umfassend das Verfahren von Anspruch 36.

47. Säugerepithelzelle, die akut mit *Cryptovirus* infiziert ist, wobei die Zelle durch das Verfahren von Anspruch 36 hergestellt wird.

48. Nicht-produktiv mit *Cryptovirus* infizierte Zelle, wobei die Zelle gemäß dem Verfahren von Anspruch 37 hergestellt wird.

49. In vitro-Verfahren zum Screenen eines möglichen antiviralen Therapiemittels, umfassend:
(a) das Kultivierend der Zelle gemäß Anspruch 47;
(b) das Exponieren der Zellen gegenüber dem möglichen antiviralen Therapiemittel; sowie
(c) das Messen der Wirkung des Mittels auf die *Cryptovirus*-Replikation und/oder den *Cryptovirus-*Viruszusammenbau, wobei die Inhibition der *Cryptovirus*-Replikation und/oder des *Cryptovirus-*Viruszusammenbaus im Vergleich mit einer Kontrolle die antivirale Aktivität des möglichen Therapiemittels anzeigt.

50. In vitro-Verfahren zum Nachweisen eines möglichen antiviralen Therapiemittels, umfassend:
(a) das Kultivieren der Zelle gemäß Anspruch 47;
(b) das Exponieren der Zellen gegenüber dem möglichen antiviralen Therapiemittel; sowie
(c) das Messen der Wirkung des Mittels auf die *Cryptovirus*-Replikation, *Cryptovirus-*Genomreplikation und/oder *Cryptovirus*-spezifische Transkription, wobei die Inhibition der *Cryptovirus*-Replikation, *Cryptovirus-*Genomreplikation und/oder *Cryptovirus*-spezifischer Transkription im Vergleich mit einer Kontrolle die antivirale Aktivität des möglichen Therapiemittels anzeigt.

51. Tiermodell zum Studium von humanen Krankheiten, das einen nicht-menschlichen Säuger umfasst, wobei der nicht-menschlicher Säuger künstlich mit einem infektiösen zellfreien *Cryptovirus* inokuliert wurde, das ein Genom aufweist, das eine einzelsträngige RNA umfasst, die zur (SEQ ID Nr.: 1) komplementär ist oder mit einer Zelle inokuliert wurde, die nicht-produktiv mit dem *Cryptovirus* infiziert wurde, wobei der nicht-menschliche Säuger mindestens ein Symptom zeigt, das für eine humane Krankheit im Anschluss an eine solche Inokulation charakteristisch ist, wobei das Symptom vorher nicht durch den nicht-menschlichen Säuger gezeigt worden war.

52. Tiermodell gemäß Anspruch 51, wobei der nicht-menschliche Säuger ein Nager oder Hasenartiger ist, oder wobei der nicht-menschliche Säuger ein nicht-menschlicher Primat ist, oder wobei die menschliche Krankheit eine neurologische, neurodegenerative und/oder neuropsychiatrische Krankheit ist.

53. Anti-*Cryptovirus*-Antikörper-Nachweiskit, umfassend:
das *Cryptovirus* von Anspruch 18; und
einen markierten anti-humanen Antikörper-bindenden Antikörper.

54. Anti-*Cryptovirus*-Antikörper-Nachweiskit gemäß Anspruch 53, weiterhin umfassend eine feste Matrix zum Anbringen des *Cryptovirus.*

55. Anti-*Cryptovirus*-Antikörper-Nachweiskit, umfassend:
das Protein gemäß Anspruch 11 oder 14; und
einen markierten anti-humanen Antikörper-bindenden Antikörper.

56. Anti-*Cryptovirus*-Antikörper-Nachweiskit gmäß Anspruch 54, weiterhin umfassend eine feste Matrix zum Trägern des Proteins.

## Revendications

1. Acide nucléique isolé, comprenant :
(A) les positions nucléotidiques contigües 1 à 15246 de (SEQ ID NO: 1) ;
(B) une séquence nucléotidique complémentaire de (A) ou
(C) un fragment de (A) ou (B) spécifique de *Cryptovirus,* comprenant un segment d'acide nucléique choisi dans le groupe constitué par :
(i) les positions nucléotidiques contigües 152 à 1678 de (SEQ ID NO: 1) ;
(ii) les positions nucléotidiques contigües 1850 à 2515 de (SEQ ID NO: 1) ;
(iii) les positions nucléotidiques contigües 1850 à 3023 de (SEQ ID NO: 1) ;
(iv) les positions nucléotidiques contigües 1850 à 3023 de (SEQ ID NO: 1) combinées à une insertion supplémentaire de deux résidus guanine entre la position nucléotidique 2339 de (SEQ ID NO: 1) et la position 2340 de (SEQ ID NO: 1) ;
(v) les positions nucléotidiques contigües 3141 à 4271 de (SEQ ID NO: 1) ;
(vi) les positions nucléotidiques contigües 4530 à 6182 de (SEQ ID NO: 1) ;
(vii) les positions nucléotidiques contigus 4587 à 6182 de (SEQ ID NO: 1) ;
(viii) les positions nucléotidiques contiguës 4836 à 6182 de (SEQ ID NO: 1) ;
(ix) les positions nucléotidiques contiguës 4272 à 6515 de (SEQ ID NO: 1) ;
(x) les positions nucléotidiques contiguës 6303 à 6434 de (SEQ ID NO: 1) ;
(xi) les positions nucléotidiques contiguës 6584 à 8278 de (SEQ ID NO: 1) ; et
(xii) les positions nucléotidiques contiguës 8414 à 15178 de (SEQ ID NO: 1).

2. Acide nucléique selon la revendication 1, dans lequel l'acide nucléique est un ARN ou un ADN.

3. Composition de matière, comprenant l'acide nucléique selon la revendication 1, et un support.

4. Construction d'acide nucléique, comprenant l'acide nucléique selon la revendication 1.

5. Utilisation de l'acide nucléique selon la revendication 1 dans la fabrication d'un vaccin.

6. Utilisation de la construction d'acide nucléique selon la revendication 4 dans la fabrication d'un vaccin.

7. Vecteur d'expression, comprenant la construction d'acide nucléique selon la revendication 4.

8. Vecteur de clonage, comprenant la construction d'acide nucléique selon la revendication 4.

9. Cellule hôte, comprenant le vecteur d'expression selon la revendication 7 ou le vecteur de clonage selon la revendication 8.

10. Cellule hôte selon la revendication 9, dans laquelle la cellule est une cellule de mammifère.

11. Protéine de *Cryptovirus* isolée comprenant une séquence acides aminés telle que définie dans l'un quelconque des numéros de SEQ ID 4, 6, 8, 10, 12, 14, 18, 20, 22, ou 24.

12. Protéine selon la revendication 11, dans laquelle la protéine est une protéine d'enveloppe de *Cryptovirus* codée par un segment d'acide nucléique comprenant une séquence d'acides aminés telle que définie dans l'une quelconque de SEQ ID NOs: 12, 14, 18, 20 ou 22.

13. Composition de matière, comprenant la protéine selon la revendication 11, et un support.

14. Protéine chimère, comprenant une protéine de *Cryptovirus* comprenant une séquence d'acides aminés telle que définie dans la revendication 12.

15. Protéine chimère selon la revendication 14, dans laquelle la protéine de *Cryptovirus* est une protéine d'enveloppe de *Cryptovirus* comprenant une séquence d'acides aminés telle que définie dans la revendication 12.

16. Utilisation de la protéine selon la revendication 11 ou la revendication 14 dans la production d'un anticorps spécifique de *Cryptovirus.*

17. Utilisation de la protéine selon la revendication 11 ou la revendication 14 dans la production d'un vaccin.

18. Particule virale isolée, comprenant l'acide nucléique selon la revendication 1.

19. Composition de matière, comprenant le virion selon la revendication 18 ; et un support.

20. Particule virale isolée, comprenant la protéine selon la revendication 12.

21. Particule de *Cryptovirus* isolée, comprenant un génome ayant une séquence nucléotidique entièrement complémentaire de (SEQ ID NO : 1).

22. Utilisation de la particule virale selon la revendication 20 dans la fabrication d'un vaccin.

23. Utilisation de la particule de *Cryptovirus* selon la revendication 21 dans fabrication d'un vaccin.

24. Utilisation selon la revendication 22 ou 23, dans laquelle la particule virale est un virion atténué.

25. Procédé de détection de la présence ou l'absence d'un ARN spécifique de *Cryptovirus* dans un échantillon d'un matériel biologique, comprenant :
la mise en contact de l'échantillon avec la sonde comprenant un acide nucléique tel que défini selon la revendication 1 dans des conditions d'hybridation au moins modérément stringentes, dans lequel la formation de produits d'hybridation détectables indique la présence de l'ARN spécifique de *Cryptovirus* dans l'échantillon.

26. Procédé de détection de la présence ou l'absence d'un ARN spécifique de *Cryptovirus* dans un échantillon d'un matériel biologique, comprenant :
l'amplification d'un ARN spécifique de *Cryptovirus* dans l'échantillon en utilisant au moins une amorce comprenant un acide nucléique tel que défini selon la revendication 1 dans un mélange réactionnel d'amplification ;
puis la détection de la présence ou l'absence de produits d'amplification d'un acide nucléique spécifique de
*Cryptovirus* dans le mélange réactionnel d'amplification, dans lequel la présence des produits d'amplification dans le mélange réactionnel indique la présence de l'ARN de *Cryptovirus* dans l'échantillon.

27. Procédé selon l'une quelconque des revendications 25 ou 26, dans lequel le matériel biologique est un matériel cellulaire, ou
est du sang ou du sérum, ou
est du liquide céphalo-rachidien, ou
est un tissu lymphoïde, ou
est un tissu nerveux.

28. Procédé selon la revendication 27, dans lequel le tissu nerveux est du tissu cérébral.

29. Procédé de détection de la présence ou l'absence d'un anticorps spécifique de *Cryptovirus* dans un échantillon d'un matériel biologique, comprenant :
la mise en contact de l'échantillon avec la protéine selon la revendication 11 : permettre la formation d'un complexe protéine-anticorps spécifique ; la détection de la présence du complexe protéine-anticorps spécifique, dans lequel la présence d'un complexe protéine-anticorps spécifique indique la présence de l'anticorps spécifique de *Cryptovirus* dans l'échantillon.

30. Procédé de détection de la présence ou l'absence d'un anticorps spécifique de *Cryptovirus* dans un échantillon d'un matériel biologique, comprenant :
la mise en contact de l'échantillon avec la protéine selon la revendication 14 ; permettre la formation d'un complexe protéine-anticorps spécifique ; la détection de la présence du complexe protéine-anticorps spécifique, dans lequel la présence d'un complexe protéine-anticorps spécifique indique la présence de l'anticorps spécifique de *Cryptovirus* dans l'échantillon.

31. Procédé de dosage pour détecter la présence ou l'absence d'un anticorps qui se lie sélectivement à un *Cryptovirus* dans un échantillon d'un matériel biologique contenant un anticorps provenant d'un humain, comprenant :
la mise en contact de l'échantillon, l'échantillon provenant d'un sujet soupçonné d'avoir une infection à *Cryptovirus*, avec la protéine d'enveloppe selon la revendication 12, de telle sorte que, si un anticorps se liant sélectivement à un *Cryptovirus* est présent, un complexe de protéine d'enveloppe liée à l'anticorps se forme ;
la mise en contact de tous les complexes de protéine d'enveloppe liée à l'anticorps ainsi formés avec un anticorps anti-liaison à un anticorps humain, et laisser la formation de complexes de l'anticorps, avec les complexes de protéine d'enveloppe liée à l'anticorps ; et
la détection de la présence ou l'absence de tous les complexes de protéine d'enveloppe liée à l'anticorps ainsi formés, la présence de tels complexes indiquant la présence dans l'échantillon d'un anticorps se liant sélectivement à un *Cryptovirus*.

32. Procédé de dosage pour détecter la présence ou l'absence d'un anticorps qui se lie sélectivement à un antigène de *Cryptovirus* dans un échantillon d'un matériel biologique contenant un anticorps provenant d'un humain, le procédé comprenant :
la mise en contact de l'échantillon, l'échantillon provenant d'un sujet soupçonné d'avoir une infection à *Cryptovirus*, avec la particule virale selon la revendication 20, de telle sorte que, si l'anticorps se liant sélectivement à un antigène de *Cryptovirus* est présent, un complexe de virus lié à l'anticorps se forme ;
la mise en contact de tous les complexes de virus lié à l'anticorps ainsi formés avec un anticorps anti-liaison à un anticorps humain, et permettre la formation de complexes de l'anticorps anti-liaison à un anticorps humain avec les complexes de virus lié à un anticorps ; et la détection de la présence ou l'absence de tous les complexes formés, la présence de tels complexes, indiquant la présence dans l'échantillon d'un anticorps se liant sélectivement à un antigène de *Cryptovirus*.

33. Procédé de détection d'une infection à *Cryptovirus* chez un mammifère, comprenant :
la mise en oeuvre du procédé selon l'une quelconque des revendications 25, 26, 30, 31, ou 32, en utilisant l'échantillon, ainsi la détection de la présence de la protéine de *Cryptovirus*, l'ARN spécifique de *Cryptovirus*, et/ou l'anticorps spécifique de *Cryptovirus* dans l'échantillon indique une infection à *Cryptovirus* chez le mammifère.

34. Procédé selon la revendication 33, dans lequel le mammifère est un humain, ou dans lequel l'humain a une maladie neurologique, neurodégénérative, et/ou neuropsychiatrique, ou
dans lequel l'humain a une maladie trachéo-bronchique primaire et/ou une maladie associée à une lymphadénopathie.

35. Procédé d'isolement d'un virion de *Cryptovirus*, comprenant la séquence d'acide nucléique selon la revendication 1 le procédé comprenant :
la culture d'une pluralité de cellules mononucléées du sang périphérique qui ont été obtenues à partir d'un humain ayant une infection à *Cryptovirus*, l'humain ayant été infecté par la particule virale selon la revendication 21, dans un milieu aqueux artificiel comprenant un agent qui augmente l'activité guanylyl cyclase cellulaire ;
la co-culture de la pluralité de cellules mononucléées du sang périphérique avec une pluralité de cellules amniotiques de mammifère dans du milieu aqueux artificiel frais comprenant un agent qui augmente l'activité guanylyl cyclase cellulaire ;
le repiquage des cellules mononucléées du sang périphérique avec les cellules amniotiques de mammifère en co-culture ;
la co-culture d'une pluralité de cellules épithéliales de mammifère avec les cellules mononucléées du sang périphérique et les cellules amniotiques de mammifère dans du milieu aqueux artificiel frais comprenant un agent qui augmente l'activité guanylyl cyclase cellulaire ; et
la séparation d'un surnageant du milieu aqueux des cellules, pour obtenir un virion de *Cryptovirus* dans le surnageant.

36. Procédé de propagation d'un *Cryptovirus*, comprenant :
(a) l'exposition d'une pluralité de cellules épithéliales de mammifère à une pluralité de virions de *Cryptovirus* acellulaires, lesdits virions de *Cryptovirus* ayant été isolés par le procédé de la revendication 35 ; et
(b) en outre la culture des cellules épithéliales de mammifère, ainsi exposées aux virions, dans un milieu aqueux artificiel comprenant un agent qui augmente l'activité de la guanylyl cyclase cellulaire.

37. Procédé de production d'une lignée cellulaire de mammifère infectée de manière non productive par un *Cryptovirus*, codé par un acide nucléique selon la revendication 1 comprenant :
(a) la co-culture de cellules mononucléées du sang périphérique qui ont été obtenues à partir d'un humain ayant une infection à *Cryptovirus*, avec des cellules amniotiques de mammifère, dans un milieu aqueux artificiel comprenant un agent qui augmente l'activité guanylyl cyclase cellulaire, de telle sorte que les cellules amniotiques de mammifère deviennent infectées de manière non productive par le *Cryptovirus* ; et
(b) le repiquage des cellules amniotiques de mammifère infectées de manière non productive avec les cellules mononucléées du sang périphérique, ainsi la co-culture devient une monoculture des cellules amniotiques de mammifère infectées de manière non productive.

38. Procédé selon la revendication 35 ou la revendication 37, dans lequel les cellules amniotiques de mammifère sont des cellules amniotiques humaines.

39. Procédé selon la revendication 38, dans lequel les cellules amniotiques humaines sont des cellules AV₃.

40. Procédé selon la revendication 35 ou la revendication 36, dans lequel les cellules épithéliales de mammifère sont des cellules épithéliales simiennes choisies dans le groupe constitué des cellules Vero ou CV-1.

41. Procédé selon la revendication 40, dans lequel les cellules CV-1 sont une sous-lignée de cellules CV-1.

42. Procédé selon les revendications 35, 36 ou 37, dans lequel l'agent qui augmente l'activité guanylyl cyclase cellulaire est le GMP cyclique, l'insuline, un dication de zinc, ou une combinaison de ceux-ci.

43. Procédé selon la revendication 42, dans lequel le GMP cyclique est à une concentration d'environ 0,05 à environ 5 mM dans le milieu aqueux artificiel.

44. Procédé selon la revendication 35, 36 ou 37, dans lequel l'agent qui augmente l'activité guanylyl cyclase cellulaire est l'oxyde nitrique ou un donneur d'oxyde nitrique choisi dans le groupe constitué par les composés de nitrate organiques, les composés nitrosyle de fer, les composés S-nitrosothiol, les composés de sydnonimine, et les composés NONOate.

45. Procédé selon les revendications 35, 36 ou 37, dans lequel le milieu aqueux comprend en outre de la glutamine.

46. Procédé de production d'une lignée de cellules épithéliales de mammifère présentant une infection aiguë à *Cryptovirus*, comprenant le procédé selon la revendication 36.

47. Cellule épithéliale de mammifère infectée de façon aiguë par un *Cryptovirus*, ladite cellule étant produite par le procédé selon la revendication 36.

48. Cellule infectée de manière non productive par un *Cryptovirus*, dans laquelle ladite cellule est produite selon le procédé de la revendication 37.

49. Procédé de criblage *in vitro* d'un agent thérapeutique antiviral potentiel, comprenant :
(a) la culture de la cellule selon la revendication 47 ;
(b) l'exposition des cellules à l'agent thérapeutique antiviral potentiel ; et
(c) la mesure de l'effet de l'agent sur la réplication du *Cryptovirus* et/ou l'assemblage du virion de *Cryptovirus*, dans lequel l'inhibition de la réplication du *Cryptovirus* et/ou l'assemblage du virion de *Cryptovirus* par rapport à un témoin indique une activité antivirale de l'agent thérapeutique potentiel.

50. Procédé de criblage *in vitro* d'un agent thérapeutique antiviral potentiel, comprenant :
(a) la culture de la cellule selon la revendication 47 ;
(b) l'exposition des cellules à l'agent thérapeutique antiviral potentiel ; et
(c) la mesure de l'effet de l'agent sur la réplication du *Cryptovirus*, la réplication du génome du *Cryptovirus*, et/ou la transcription spécifique du *Cryptovirus,* dans lequel l'inhibition de la réplication du *Cryptovirus,* la réplication du génome du *Cryptovirus*, et/ou la transcription spécifique du *Cryptovirus*, par rapport à un témoin, indique l'activité antivirale de l'agent thérapeutique potentiel.

51. Modèle animal pour l'étude des maladies humaines, comprenant un mammifère non-humain, ledit mammifère non-humain ayant été inoculé artificiellement avec un *Cryptovirus* acellulaire infectieux ayant un génome comprenant un ARN simple brin complémentaire de (SEQ ID NO: 1), ou ayant été inoculé avec une cellule infectée de manière non productive par le *Cryptovirus*, par quoi le mammifère non-humain présente au moins un symptôme caractéristique d'une maladie humaine après avoir été ainsi inoculé, ledit symptôme n'étant pas déjà présenté par le mammifère non-humain.

52. Modèle animal selon la revendication 51, dans lequel le mammifère non-humain est un rongeur ou un lagomorphe, ou dans lequel le mammifère non-humain est un primate non-humain, ou dans lequel la maladie humaine est une maladie neurologique, neurodégénérative, et/ou neuropsychiatrique.

53. Kit de détection d'anticorps *anti-Cryptovirus*, comprenant :
la particule de *Cryptovirus* selon la revendication 18 ; et
un anticorps anti-liaison à un anticorps humain marqué.

54. Kit de détection d'anticorps *anti-Cryptovirus* selon la revendication 53, comprenant en outre une matrice solide pour supporter ladite particule de *Cryptovirus*.

55. Kit de détection d'anticorps *anti-Cryptovirus*, comprenant : la protéine selon la revendication 11 ou la revendication 14 : et
un anticorps anti-liaison à un anticorps humain marqué.

56. Kit de détection d'anticorps *anti-Cryptovirus* selon la revendication 54, comprenant en outre une matrice solide pour supporter ladite protéine.


### SEQUENCE LISTING

<110> Cryptic Afflictions, LLC (Assignee) Steven J. Robbins (Inventor)
<120> A Novel Virus (Cryptovirus) Within The Rubulavirus Genus and Uses Therefor
<130> Cryptic 81010
<140> PCT UNKNOWN
   <141> 2002-02-07
<150> US 60/267,253
   <151> 2001-02-07
<160> 49
<170> FastSEQ for Windows Version 4.0
<210> 1
   <211> 15246
   <212> DNA
   <213> Cryptovirus
<220>
   <223> cDNA in mRNA sense of Crypotovirus minus strand RNA genome
<400> 1
   accaagggga aaatgaagtg gtgactcaaa tcatcgaaga ccctcgagat tacataggtc 60
   cggaacctat ggccttcgtg accgacctcg agtcagagta gttcaataag gacctatcaa 120
   gtttgggcaa tttttcgtcc ctgacacaaa aatgtcatcc gtgcttaaag catatgagag 180
   attcacactc actcaagaac tgcaagatca gagtgaagaa gggacaatcc cacctacaac 240
   actaaaaccg gttatcaggg tatttgtact aacctctaat aacccagagc taagatcccg 300
   gcttcttcta ttctgcctac ggattgttct cagtaatggt gcaagggatt cccatcgctt 360
   tggagcatta cttacaatgt tttcgctacc atcagctaca atgctcaatc atgtcaaatt 420
   agctgaccag tcaccagaag ctgatatcga aagggtagag atcgatggct ttgaggaggg 480
   atcattccgc ttaattccca atgctcgctc aggtatgagc cgtggagaga tcaatgccta 540
   tgctgcactt gcagaagatc tacctgacac actaaaccat gcaacacctt tcgttgattc 600
   cgaagtcgag ggaactgcat gggacgagat tgagactttc ttagatatgt gttacagtgt 660
   cctaatgcag gcatggatag tgacttgcaa gtgcatgact gcgccagacc aacctgctgc 720
   ttctattgag aaacgcctgc aaaaatatcg tcagcaaggc aggatcaacc caagatatct 780
   cctgcaaccg gaggctcgac gaataatcca gaatgtaatc cggaagggaa tggtggtcag 840
   acatttcctc acctttgaac tgcagcttgc ccgagcacaa agccttgtat caaataggta 900
   ttatgctatg gtaggggatg ttggaaagta tatagagaat tgtggaatgg gaggcttctt 960
   tttgacacta aaatatgcat taggaaccag atggcccaca cttgctttag ctgcattttc 1020
   aggagagcta acaaagctaa agtccctcat ggcattgtac cagacccttg gtgagcaggc 1080
   ccgatatttg gccctattgg agtcaccaca cttgatggat tttgctgcag caaactatcc 1140
   attgctatat agctatgcta tgggaatagg ctatgtgtta gatgtcaaca tgaggaacta 1200
   cgctttctcc agatcataca tgaataagac atatttccaa ttgggaatgg aaactgcaag 1260
   aaaacaacag ggtgcagttg acatgaggat ggcagaagat ctcggtctaa ctcaagccga 1320
   acgcaccgag atggcaaata cacttgccaa attgaccaca gcaaatcgag gggcagacac 1380
   caggggagga gtcaacccgt tctcatctgt cactgggaca actcagatgc ccgctgcagc 1440
   aacaggtgac acattcgaga gttacatggc agcggatcga ctgaggcaga gatatgctga 1500
   tgcaggcacc catgatgatg agatgccacc attagaagag gaggaagagg acgacacatc 1560
   tgcaggtcca cgcactgaac caactcctga acaagtggcc ttggacatcc agagcgcagc 1620
   agttggagct cccatccata cagacgacct gaatgccgca ctaggtgatc ttgacatcta 1680
   gacaattcag atcccaatcc taaattgata tacctaattg attagttaga tggaactaca 1740
   gtggattcca taaggttcct gcctaccatc ggcttttaag aaaaaaatag gcccggacgg 1800
   gttagcaaca agcgactgcc gatgccaaca gcgcaatcca caatctacaa tggatcccac 1860
   tgatctgagc ttctccccag atgagattaa taagctcata gagacaggcc tgaatactgt 1920
   ggagtatttt acttcccaac aagtcacagg aacatcctct cttggaaaga atacaatacc 1980
   accaggggtc acaggactac taaccaatgc tgcagaggca aagatccaag agtcaatcaa 2040
   ccatcagaag ggttcagttg gtgggggtac aaacccaaag aaaccgcgat caaaaattgc 2100
   cattgtgcca gcagatgaca aaacagtgcc cgaaaagccg atcccaaacc ctctactagg 2160
   tctggactcc accccgagca cccaaaccgt gcttgatcta agtgggaaaa cattaccatc 2220
   aggatcctat aagggggtta agcttgcgaa atttgggaaa gaaaatctga tgacacggtt 2280
   catcgaggaa cccagagaga atcctatcgc aaccagttcc cccatcgatt ttaagagggg 2340
   cagagatacc ggtgggttcc atagaaggga gtactcaatc ggatgggtgg gagatgaagt 2400
   caaggtcact gagtggtgca atccatcctg ttctccaatc accgctgcag caaggcgatt 2460
   taaatgcact tgtcaccaat gtccagtcac ttgctctgaa tgtgaacgag atacttaata 2520
   cagtgagaaa tttggactct cggatgaatc aactggagac aaaagtagat cgcattctct 2580
   catctcagtc tctaatccag accatcaaga atgacataat tggacttaaa gcagggatgg 2640
   ctactttaga aggaatgatt acaactgtga aaatcatgga cccgggagtt cccagtaatg 2700
   ttactgtgga agatgtacgc aagaaactaa gtaaccatgc tgttgttgtg ccagaatcat 2760
   tcaatgatag tttcttgact caatctgaag atgtaatttc acttgatgag ttggctcgac 2820
   caactgcaac aagtgttaag aagattgtca ggaaggttcc tcctcagaag gatctgactg 2880
   gattgaagat cacactagag caattggcaa aggattgcat cagcaaaccg aagatgaggg 2940
   aagattatct cctcaagatc aaccaggctt ctagtgaggc tcagctaatt gacctcaaga 3000
   aagcaatcat ccgcagtgca atttgatcaa gaaacaccca attacactac actggtatga 3060
   cactgtacta accctgaggg ttttagaaaa aacgattaac gataaataag cccgaacact 3120
   acatactacc tgaggcagcc atgccatcca tcagcattcc cgcagacccc accaatccac 3180
   gtcaatcaat aaaagcgttc ccaattgtga ttaacagtga tgggggtgag aaaggccgct 3240
   tggttaaaca actacgtaca acctacttga atgacctaga tactcatgag ccactggtga 3300
   cattcgtaaa tacctatgga ttcatctacg aacagaatcg ggggaatgcc attgtcggag 3360
   aggatcaact tgggaagaaa agagaggctg tgactgctgc aatggttacc cttggatgtg 3420
   ggcctaatct accatcatta gggaatgtcc tgagacaact gagtgaattc caagtcattg 3480
   ttaggaagac atccagcaaa gcggaagaga tggtctttga aattgttaag tatccgagaa 3540
   tatttcgggg tcatacatta atccagaaag gactagtctg tgtctccgca gaaaaatttg 3600
   ttaagtcacc agggaaagta caatctggaa tggactatct cttcattccg acatttctgt 3660
   cagtgactta ttgtccagct gcaatcaaat ttcaggtacc tggccccatg ttgaaaatga 3720
   ggtcaagata cactcagagc ttacaacttg aactaatgat aagaatcctg tgtaagcccg 3780
   attcgccact tatgaaggtc catatccctg acaaggaggg aagaggatgt cttgtatcag 3840
   tatggctgca tgtatgcaac atcttcaaat caggaaacaa gaatggcagt gagtggcagg 3900
   aatactggat gagaaagtgt gctaacatgc aacttgaagt gtcgattgca gatatgtggg 3960
   gaccaactat cataattcat gccagaggtc acattcccaa aagtgctaag ttgttttttg 4020
   gaaagggtgg atggagctgc catccacttc acgaaattgt tccaagtgtc actaaaacac 4080
   tatggtccgt aggttgtgaa attacaaagg cgaaggcaat aatacaagag agtagcatct 4140
   ctcttctcgt ggagactact gacatcataa gtccaaaagt taaaatttca tctaagcatc 4200
   gccgctttgg gaaatcaaat tggggtctgt tcaagaaaac taaatcacta cctaacctaa 4260
   cggagctgga atgactgacc tctaatcgag attacaccaa ctcaaactat aggtgggtag 4320
   taccccagtg attaatcttg caagtactga tcgttggcta caacacacca atattatcca 4380
   gattagagag cttaattaac tctgtattaa taataacact actattccaa taactggaat 4440
   taccagcttg atttatctcc aaaatgattc aaagaaaaca gatcatatta agactaccct 4500
   aagcacgaac ccatatcgtc cttcaaatca tgagcactat aattcaatct ctggtggtct 4560
   cctgtctatt ggcaggagca ggcagccttg atccagcagc cctcatgcaa atcggtgtca 4620
   ttccaacaaa tgtccggcaa cttatgtatt atactgaggc ctcatcagca ttcattgttg 4680
   tgaagttaat gcctacaatt gactcgccga ttagtggatg taatataaca tcaatttcaa 4740
   gctataatgc aacagtgaca aaactcctac agccgatcgg tgagaatttg gagacgatta 4800
   ggaaccagtt gattccaact cggaggagac gccggtttgc aggggtggtg attggattag 4860
   ctgcattagg agtagctact gccgcacaag tcactgccgc agtagcacta gttaaggcaa 4920
   atgaaaatac tgcggctata ctcaatctca aaaatgcaat ccaaaaaaca aatgcagcag 4980
   ttgcagatgt ggtccaggcc acacaatcac taggaacggc agttcaagca gttcaagatc 5040
   acataaacag tgtgataagt ccagcaatta cagcagccaa ttgtaaggcc caagatgcta 5100
   tcattggctc aatcctcaat ctctatttga ccgagttgac aactatcttc cacaatcaaa 5160
   ttacaaaccc tgcattgagt cctattacaa ttcaagcttt aaggatccta ctggggagta 5220
   ccttgccgac tgtggtcgaa aaatctttca atacccagat aagtgcagct gagcttctct 5280
   catcagggtt gttgacaggc cagattgtgg gattagattt gacctatatg cagatggtca 5340
   taaaaattga gctgccaact ttaactgtac aacctgcaac ccagatcata gatctggcca 5400
   ccatttctgc attcattaac aatcaagaag tcatggccca attaccaaca cgtgttattg 5460
   tgactggcag cttgatccaa gcctatcccg catcgcaatg cactattaca cccaacactg 5520
   tgtactgtag gtataatgat gcccaagtac tctcagatga tacgatggct tgcctccaag 5580
   gtaacttgac aagatgcacc ttctctccag tggttgggag ctttctcact cgattcgtgc 5640
   tgttcgatgg aatagtttat gcaaattgca ggtcgatgct gtgcaagtgc atgcaacctg 5700
   ctgccgtgat cctacagccg agttcatccc ctgtaactgt cattgacatg cacaaatgtg 5760
   tgagtctgca gcttgacgat ctcagattca ccatcactca attggccaat gtaacctaca 5820
   atagcaccat caagcttgaa acatcccaga tcttgcctat tgatccgttg gatatatccc 5880
   agaatttagc tgcggtgaat aagagtctaa gtgatgcact acaacactta gcacaaagtg 5940
   acacatacct ttctgcaatc acatcagcta cgactacaag tgtattatcc ataatagcaa 6000
   tctgtcttgg atcgttaggt ttaatattaa taatcttgct cagtgtagtt gtgtggaagt 6060
   tattgaccat tgtcgctgct aatcgaaata gaatggagaa ttttgtttat cataattcag 6120
   cattccacca cccacgatct gatctcagtg agaaaaatca acctgcaact cttggaacaa 6180
   gataagacag tcatccatta gtaatctcaa agaaaaaaac gataggaccg aaactagtat 6240
   tgaaagaact gtctcggtca atttaggtaa tcgagctgat accgtctcgg aaagctcaaa 6300
   tcatgctgcc tgatccggaa gatccggaaa gcaaaaaagc tacaaggaga acaggaaacc 6360
   taattatctg cttcctattc atcttctttc tgtttgtaac cctcattgtt ccaactctaa 6420
   gacacttgct atcttaacac ctgccatagg ctatccactg catcatctct tctgccatac 6480
   ttcctactca catcatatct attttaaaga aaaaataggc ccgaacacta atcgtgccgg 6540
   cagtgccact gcacacacaa cactacacat acaatacact ataatgattg cagaagatgc 6600
   ccctgttaag ggcacttgcc gagtattatt tcggacaaca actttaattt ttctatgcac 6660
   actactagca ttaagcatct ctatccttta tgagagttta ataacccaaa agcaaatcat 6720
   gagtcaagca ggctcaactg gatctaattc tggattagga ggtattactg atcttcttaa 6780
   taatattctt tctgtcgcaa atcagattat atataactct gcagtcgctc tacctctaca 6840
   attggacact cttgaatcaa cactccttac agccattaag tctcttcaaa ccagtgacaa 6900
   gctagaacag aactgctcgt ggggtgctgc actgattaat gataatagat acattaatgg 6960
   catcaatcag ttctatttct caattgctga gggtcgcaat ctgacacttg gcccacttct 7020
   taatatacct agtttcattc caactgccac gacaccagag ggctgcacca ggatcccatc 7080
   attctcgctc actaagacac actggtgtta tacgcacaat gttatcctga atggatgcca 7140
   ggatcatgta tcctcaaatc aatttgtttc catgggaatc attgaaccca cttctgccgg 7200
   gtttccatcc tttcgaacct taaagactct atatctcagc gatggggtca atcgtaagag 7260
   ctgctctatc agtacagttc cggggggttg tatgatgtac tgttttgtct ctactcaacc 7320
   agagagggat gactactttt ctaccgctcc tccagaacaa cgaattatta taatgtacta 7380
   taatgataca atcgtggagc gcataattaa tccacccggg gtactagacg tatgggcaac 7440
   attgaaccca ggaacaggaa gcggggtata ttatttaggt tgggtgctct ttccaatata 7500
   tggcggcgtg attaaaaata cgagtttatg gaataatcaa gcaaataaat acttcattcc 7560
   ccagatggtt gctgctctct gctcacaaaa ccaggcaact caagtccaaa atgctaagtc 7620
   atcatactat agcagctggt ttggcaatcg aatgattcag tctgggatcc tggcatgccc 7680
   tcttcaacag gatctaacca atgagtgttt agttctgccc ttttctaatg atcaggtgct 7740
   tatgggtgct gaagggagat tatatatgta tggtgactcg gtgtattact accaaagaag 7800
   caatagttgg tggcctatga ccatgctgta taaggtaacc ataacattca ctaatggtca 7860
   gccatccgct atatcagctc agaatgtgcc cacacagcag gtccctagac ctgggacagg 7920
   agactgcttt gcaaccaata gatgtcccgg tttttgcttg acaggagtgt atgctgatgc 7980
   ttggttactg accaaccctt cgtctaccag tacatttgga tcggaagcaa ccttcactgg 8040
   ttcttatctc aacgcagcaa ctcagcgtat caatccgacg atgtatatcg cgaacaacac 8100
   acagatcata agctcacagc aatttggatc aagcggtcaa gaagcagcat atggccacac 8160
   aacttgtttt agggacacag gctctgttat ggtatactgt atctatatta ttgaattgtc 8220
   ctcatctctc ttaggacaat ttcagattgt cccatttatc cgtcaggtga cactatccta 8280
   aaggcagaag cctccagatc tgacctagcc aatcaaagca ttataccaga ccatggaatg 8340
   cataccaaac attattgaca ctaatgacac acaaaattgg ttttaagaaa aaccaagaga 8400
   acaataagcc agaatggctg ggtctcggga gatattactc cctgaagtcc atctcaattc 8460
   accaattgta aagcataagc tatactatta cattctactt ggaaacctcc caaatgagat 8520
   cgacattgac gatttaggtc cattacataa tcaaaattgg aatcaaatag cacatgaaga 8580
   gtctaactta gctcaacgct tggtaaatgt aagaaatttt ctaattaccc acatccctga 8640
   tcttagaaag ggccattggc aagagtatgt aaatgtaata ctgtggccgc gaattcttcc 8700
   cttgatcccg gattttaaaa tcaatgacca attgcctctg ctcaaaaatt gggacaagtt 8760
   agttaaagaa tcatgttcag taatcaatgc gggtacttcc cagtgcattc agaatctcag 8820
   ctatggactg acaggtcgtg ggaacctctt tacacgatca cgtgaactct ctggtgaccg 8880
   cagggatatt gatcttaaga cggttgtggc agcatggcat gactcagact ggaaaagaat 8940
   aagtgatttt tggattatga tcaaattcca gatgagacaa ttaattgtta ggcaaacaga 9000
   tcataatgat cctgatttaa tcacgtatat cgaaaataga gaaggcataa tcatcataac 9060
   ccctgaactg gtagcattat ttaatactga gaatcataca ctaacataca tgacctttga 9120
   aattgtactg atggtttcag atatgtacga aggtcgtcac aacattttat cactatgcac 9180
   agttagcact tacctgaatc ctctgaagaa aagaataaca tatttattga gccttgtaga 9240
   taacttagct tttcagatag gtgatgctgt atataacata attgctttgc tagaatcctt 9300
   tgtatatgca cagttgcaaa tgtcagatcc catcccagaa ctcagaggac aattccatgc 9360
   attcgtatgt tctgagattc ttgatgcact aaggggaact aatagtttca cccaggatga 9420
   attaagaact gtgacaacta atttgatatc cccattccaa gatctgaccc cagatcttac 9480
   ggctgaattg ctctgtataa tgaggctttg gggacacccc atgctcactg ccagtcaagc 9540
   tgcgggaaag gtacgcgagt ctatgtgtgc tgggaaagta ctggactttc ccactattat 9600
   gaaaacacta gcctttttcc atactattct gatcaatgga tacaggagga agcatcatgg 9660
   agtatggcca cccttaaact taccaggtaa tgcttcaaag ggtctcacag aacttatgaa 9720
   tgacaacact gagataagct atgaattcac acttaagcat tggaaggaaa tctctcttat 9780
   aaaattcaag aaatgttttg atgcagacgc aggtgaggaa ctcagtatat ttatgaaaga 9840
   taaagcaatt agtgccccaa aacaagactg gatgagtgtg tttagaagaa gcctaatcaa 9900
   acagcgccat cagcatcatc aggtccccct accaaatcca ttcaatcgac ggctattgct 9960
   aaactttctc ggagatgaca aattcgaccc gaatgtggag ctacagtatg taacatcagg 10020
   tgagtatcta catgatgaca cgttttgtgc atcatattca ctaaaagaga aggaaattaa 10080
   acctgatggt cgaatttttg caaagttgac taagagaatg agatcatgtc aagttatagc 10140
   agaatctctt ttagcgaacc atgctgggaa gttaatgaaa gagaatggtg ttgtgatgaa 10200
   tcagctatca ttaacaaaat cactattaac aatgagtcag attggaataa tatccgagag 10260
   agctagaaag tcgactcgag ataacataaa tcgacctggt ttccagaata tccagagaaa 10320
   taaatcacat cactccaagc aagtcaatca gcgagatcca agtgatgact ttgaattggc 10380
   agcatctttt ttaactactg atctcaaaaa atattgttta caatggaggt atcagacaat 10440
   tatcccattt gctcaatcac taaacagaat gtatggttat cctcatctct tcgagtggat 10500
   tcacttgcgg ctaatgcgta gtacacttta cgtgggggac cccttcaacc caccagcaga 10560
   taccagtcaa tttgatctag ataaagtaat taatggagat atcttcattg tatcacccag 10620
   aggtggaatt gaagggctgt gtcaaaaagc ttggacaatg atatctatct ctgtgataat 10680
   tctatctgcc acagagtctg gcacacgagt aatgagcatg gtgcagggag ataatcaagc 10740
   aattgctgtc accacacgag taccaaggag cctgccgact cttgagaaaa agactattgc 10800
   ttttagatct tgtaatttat tctttgagag gttaaaatgt aataattttg gattaggtca 10860
   ccatttgaaa gaacaagaga ctatcattag ttctcacttc tttgtttata gcaagagaat 10920
   attctatcag gggaggattc taacgcaagc cttaaaaaat gctagtaagc tctgcttgac 10980
   agctgatgtc ctaggagaat gcacccaatc atcatgttct aatcttgcaa ctactgtcat 11040
   gaggttaact gagaatggtg ttgaaaaaga tatctgtttc tacttgaata tctatatgac 11100
   catcaaacag ctctcctatg atatcatctt ccctcaagtg tcgattcctg gagatcagat 11160
   cacattagaa tacataaata atccacacct ggtatcacga ttggctcttt taccatccca 11220
   gttaggaggt ctaaactact tgtcatgcag taggctgttc aatcgaaaca taggtgaccc 11280
   ggtggtttcc gcagttgcag atcttaagag attaattaaa tcaggatgta tggattactg 11340
   gatcctttat aacttattag ggagaaaacc gggaaacggc tcatgggcta ctttagcagc 11400
   tgacccgtac tcaatcaata tagagtatca atacccccca actacagctc ttaagaggca 11460
   cacccaacaa gttctgatgg aactcagtac gaatccaatg ttacgtggca tattctctga 11520
   caatgcacag gcagaagaaa ataatcttgc taggtttctc ctggataggg aggtgatctt 11580
   tccgcgtgta gctcacatca tcattgagca aaccagtgtc gggaggagaa agcagattca 11640
   aggatatttg gattcaacta gatcgataat gaggaaatca ctggaaatta agcccttgtc 11700
   caataggaag cttaatgaaa tactggatta caacatcaat tacttagctt acaatttggc 11760
   attactcaag aatgctattg aacctccgac ttatttgaag gcaatgaccc ttgaaacatg 11820
   tagcatcgac attgcaagga gcctccggaa gctctcctgg gccccactct tgggtgggag 11880
   aaatcttgaa ggactagaga cgccagatcc cattgaaatc actgcaggag cattaattgt 11940
   tggatcgggc tactgtgaac agtgtgctgc aggagacaat cgattcacat ggtttttctt 12000
   gccatctggt atcgagatag gaggggatcc ccgtgataat cctcctatcc gtgtaccgta 12060
   cattggctcc aggactgatg agaggagggt agcctcaatg gcatacatca ggggtgcctc 12120
   aagtagccta aaagcagttc ttagactggc gggagtgtac atctgggcat tcggagatac 12180
   tctggagaat tggatagatg cactggattt gtctcacact agagttaaca tcacacttga 12240
   acagttacaa tccctcaccc cacttccaac ctctgccaat ctaacccatc ggttggatga 12300
   tggcacaact accctaaagt ttactcctgc aagctcttac accttttcaa gtttcactca 12360
   tatatcaaat gatgagcaat atctgacaat taatgacaaa actgcagatt caaatataat 12420
   ctaccaacag ttaatgatca ctggactcgg gatcttagaa acatggaata atcccccaat 12480
   caatagaaca ttcgaagaat ctaccctaca tttgcacact ggtgcatcat gttgtgtccg 12540
   acctgtggac tcctgcatta tctcagaagc attaacagtc aagccacata ttacagtacc 12600
   gtacagcaat aaatttgtat ttgatgaaga cccgctatct gaatatgaga ctgcaaaact 12660
   ggaatcgtta tcattccaag cccaattagg caacattgat gctgtagata tgacaggtaa 12720
   attaacatta ttgtcccaat tcactgcaag gcagattatc aatgcaatca ctggactcga 12780
   tgagtctgtt tctcttacta atgatgccat tgttgcatca gactatgtct ccaattggat 12840
   tagtgaatgc atgtatacca aattagatga attatttatg tattgtgggt gggaactact 12900
   attggaacta tcctatcaaa tgtattatct gagggtagtt gggtggagta atatagtgga 12960
   ttattcttac atgatcttga gaagaatccc gggtgcagca ttaaacaatc tggcatctac 13020
   attaagtcat ccaaaacttt tccgacgagc tatcaaccta gatatagttg cccccttaaa 13080
   tgctcctcat tttgcatctc tggactacat caagatgagt atggatgcaa tactctgggg 13140
   ctgtaaaaga gtcatcaatg tgctctccaa tggaggggac ttagaattag ttgtgacatc 13200
   tgaagatagc cttattctca gtgaccgatc catgaatctc attgcaagga aattaacttt 13260
   attatcactg attcaccata atggtttgga actaccaaag attaaggggt tctctcctga 13320
   tgagaagtgt ttcgctttga cagaattttt gaggaaagtg gtgaactcag ggttgagttc 13380
   aatagagaac ctatcaaatt ttatgtacaa tgtggagaac ccacggcttg cagcattcgc 13440
   cagcaacaat tactacctga ccagaaaatt attgaattca atacgagata ctgagtcagg 13500
   tcaagtagca gtcacctcat attatgaatc attagaatat attgatagtc ttaagctaac 13560
   cccacatgtg cctggcacct catgcattga ggatgatagt ctatgtacaa atgattacat 13620
   aatctggatc atagagtcta atgcaaactt ggagaagtat ccaattccaa atagccctga 13680
   ggatgattcc aatttccata actttaagtt gaatgctcca tcgcaccata ccttacgccc 13740
   attagggttg tcatcgactg cttggtataa gggtataagc tgctgcaggt accttgagcg 13800
   attaaagcta ccacaaggtg atcatttata tattgcagaa ggtagtggtg ccagtatgac 13860
   aatcatagaa tacctattcc caggaagaaa gatatattac aattctttat ttagtagtgg 13920
   tgacaatccc ccacaaagaa attatgcacc aatgcctact cagttcattg agagtgtccc 13980
   atacaagctc tggcaagcac acacagatca atatcccgag atttttgagg acttcatccc 14040
   tctatggaac ggaaacgccg ccatgactga cataggaatg acagcttgtg tagaattcat 14100
   catcaatcga gttggcccaa ggacttgcag tttagtacat gtagatttgg aatcaagtgc 14160
   aagcttaaat caacaatgcc tgtcaaagcc gataattaat gctatcatca ctgctacaac 14220
   tgttttgtgc cctcatgggg tgcttattct gaaatatagt tggttgccat ttactagatt 14280
   tagtactttg atcactttct tatggtgcta ctttgagaga atcactgttc ttaggagcac 14340
   atattctgat ccagctaatc atgaggttta tttaatttgt atccttgcca acaactttgc 14400
   attccagact gtctcgcagg caacaggaat ggcgatgact ttaaccgatc aagggtttac 14460
   cttgatatca cctgaaagaa taaatcagta ttgggatggt cacttaaagc aagaacgtat 14520
   cgtagcagaa gcaattgata aggtggttct aggagaaaat gctctattta attcgagtga 14580
   taatgaatta attctcaaat gtggagggac accaaatgca cggaatctta tcgatatcga 14640
   gccagtcgca actttcatag aatttgaaca actaatctgc acaatgttga caacccactt 14700
   gaaggaaata attgatataa caaggtctgg aacccaggat tatgaaagtt tattactcac 14760
   tccttacaat ttaggtcttc ttggtaaaat cagtacgata gtgagattat taacagaaag 14820
   gattctaaat catactatca ggaattggtt gatcctccca ccttcgctcc agatgatcgt 14880
   gaagcaggac ttggaattcg gcatattcag gattacttcc atcctcaatt ctgatcggtt 14940
   cctgaaactt tctccaaata ggaaatactt gattacacaa ttaactgcag gctacattag 15000
   gaaattgatt gagggggatt gtaatatcga tctaaccaga cctatccaaa agcaaatctg 15060
   gaaagcatta ggttgtgtag tctactgtca cgatccaatg gatcaaaggg agtcaacaga 15120
   gtttattgat ataaatatta atgaagaaat agaccgcggg atcgatggcg aggaaatcta 15180
   aatatatcaa gaatcagaat taatttaaga aaaaagaagc ggattaatct tggttttccc 15240
   cttggt 15246
<210> 2
   <211> 15246
   <212> DNA
   <213> Cryptovirus
<220>
   <223> cDNA in mRNA sense of Simian Virus 5 minus strand RNA genome
<400> 2
   accaagggga aaatgaagtg gtgactcaaa tcatcgaaga ccctcgagat tacataggtc 60
   cggaacctat ggccttcgtg accgacctcg agtcagagta gttcaataag gacctatcaa 120
   gtttgggcaa tttttcgtcc ccgacacaaa aatgtcatcc gtgcttaaag catatgagcg 180
   attcacgctc actcaagaac tgcaagatca gagtgaggaa ggtacaatcc cacctacaac 240
   actaaaaccg gtaatcaggg tatttatact aacctctaat aacccagagc taagatcccg 300
   gcttcttcta ttctgcctac ggattgttct cagtaatggt gcaagggatt cccatcgctt 360
   tggagcatta ctcacaatgt tttcgctacc atcagccaca atgctcaatc atgtcaaatt 420
   agctgaccag tcaccagaag ctgatatcga aagggtagag atcgatggct ttgaggaggg 480
   atcattccgc ttaatcccca atgcacgttc aggtatgagc cgtggagaga tcaatgccta 540
   tgctgcactt gcagaagatc tacctgacac actaaaccat gcaacacctt tcgttgattc 600
   cgaagtcgag ggaactgcat gggatgagat tgagactttc ttagatatgt gttacagtgt 660
   cctaatgcag gcatggatag tgacttgcaa gtgcatgact gcgccagacc aacctgctgc 720
   ttctattgag aaacgcctgc aaaaatatcg tcagcaaggc aggatcaacc cgagatatct 780
   cctgcaaccg gaggctcgac gaataatcca gaatgtaatc cggaagggaa tggtggtcag 840
   acatttcctc acctttgaac tgcagcttgc ccgagcacaa agccttgtat caaataggta 900
   ttatgctatg gtaggggatg ttggaaagta tatagagaat tgtggaatgg gaggcttctt 960
   tttgacacta aaatatgcat taggaactag atggcccaca cttgctttag ctgcattttc 1020
   aggagagcta acaaagctaa agtccctcat ggcattatac cagacccttg gtgagcaggc 1080
   ccgatatttg gccctattgg agtcaccaca tttgatggat tttgctgcag caaactaccc 1140
   actgctatat agctatgcta tgggaatagg ctatgtgtta gatgtcaaca tgaggaacta 1200
   cgctttctcc agatcataca tgaacaagac atatttccaa ttgggaatgg aaactgcaag 1260
   aaaacaacag ggtgcagttg acatgaggat ggcagaagat ctcggtctaa ctcaagccga 1320
   acgcaccgag atggcaaata cacttgccaa attgaccaca gcaaatcgag gggcagacac 1380
   caggggagga gtcaacccgt tctcatctgt cactgggaca actcaggtgc ccgctgcagc 1440
   aacaggtgac acactcgaga gttacatggc agcggatcga ctgaggcaga gatatgctga 1500
   tgcaggcacc catgatgatg agatgccacc attggaagag gaggaagagg acgacacatc 1560
   tgcaggtcca cgcactggac caactcttga acaagtggcc ttggacatcc agaacgcagc 1620
   agttggagct cccatccata cagatgacct gaatgccgca ctgggtgatc ttgacatcta 1680
   gacaattcag atcccaatct aaaattgaca tacctaattg attagttaga tggaactaca 1740
   gtggattcca taaggttcct gcctaccatc ggctttaaag aaaaaaatag gcccggacgg 1800
   gttagcaaca agcgactgcc ggtgccaaca gcgcaatcca caatctacaa tggatcccac 1860
   tgatctgagc ttctccccag atgagatcaa taagctcata gagacaggcc tgaatactgt 1920
   agagtatttt acttcccaac aagtcacagg aacatcctct cttggaaaga atacaatacc 1980
   accaggggtc acaggactac taaccaatgc tgcagaggca aagatccaag agtcaactaa 2040
   ccatcagaag ggctcagttg gtgggggtgc aaaaccaaag aaaccgcgac caaaaattgc 2100
   cattgtgcca gcagatgaca aaacagtgcc cggaaagccg atcccaaacc ctctattagg 2160
   tctggactcc accccgagca cccaaactgt gcttgatcta agtgggaaaa cattaccatc 2220
   aggatcctat aagggggtta agcttgcgaa atttggaaaa gaaaatctga tgacacggtt 2280
   catcgaggaa cccagagaga atcctatcgc aaccagttcc cccatcgatt ttaagagggg 2340
   cagggatacc ggcgggttcc atagaaggga gtactcaatc ggatgggtgg gagatgaagt 2400
   caaggtcact gagtggtgca atccatcctg ttctccaatc accgctgcag caaggcgatt 2460
   tgaatgcact tgtcaccagt gtccagtcac ttgctctgaa tgtgaacgag atacttaata 2520
   cagtgagaaa tttggactct cggatgaatc aactggagac aaaagtagat cgcattctct 2580
   catctcagtc tctaatccag accatcaaga atgacatagt tggacttaaa gcagggatgg 2640
   ctactttaga aggaatgatt acaactgtga aaatcatgga cccgggagtt cccagtaatg 2700
   ttactgtgga agatgtacgc aagacactaa gtaaccatgc tgttgttgtg ccagaatcat 2760
   tcaatgatag tttcttgact caatctgaag atgtaatttc acttgatgag ttggctcgac 2820
   caactgcaac aagtgttaag aagattgtca ggaaggttcc tcctcagaag gatctgactg 2880
   gattgaagat tacactagag caattggcaa aggattgcat cagcaaaccg aagatgaggg 2940
   aagagtatct cctcaaaatc aaccaggctt ccagtgaggc tcagctaatt gacctcaaga 3000
   aagcaatcat ccgcagtgca atttgatcaa gaaacaccca attacactac actggtatga 3060
   cactgtacta accctgaggg ttttagaaaa aacgattaac gataaataag cccgaacact 3120
   acacactacc tgaggcagcc atgccatcca tcagcattcc cgcagacccc accaatccac 3180
   gtcaatcaat aaaagcgttc ccaattgtga tcaacagtga tgggggtgag aaaggccgct 3240
   tggttaaaca actacgcaca acctacttga atgacctaga tactcatgag ccactggtga 3300
   cattcataaa tacctatgga ttcatctacg aacaggatcg ggggaatacc attgtcggag 3360
   aggatcaact tgggaagaaa agagaggctg tgaccgctgc aatggttacc cttggatgtg 3420
   ggcctaatct accatcatta gggaatgtcc tgggacaact gagggaattc caggtcactg 3480
   ttaggaagac atccagcaaa gcggaagaga tggtctttga aattgttaag tatccgagaa 3540
   tatttcgggg tcatacatta atccagaaag gactagtctg tgtctccgca gaaaaatttg 3600
   ttaagtcacc agggaaaata caatctggaa tggactatct cttcattccg acatttctgt 3660
   cagtgactta ctgtccagct gcaatcaaat ttcaggtacc tggccccatg ttgaaaatga 3720
   gatcaagata cactcagagc ttacaacttg aactaatgat aagaatcctg tgtaagcccg 3780
   attcgccact tatgaaggtc catacccctg acaaggaggg aagaggatgt cttgtatcag 3840
   tatggctgca tgtatgcaac atcttcaaat caggaaacaa gaatggcagt gagtggcagg 3900
   aatactggat gagaaagtgt gctaacatgc aacttgaagt gtcgattgca gatatgtggg 3960
   gaccaactat cataattcat gccagaggtc acattcccaa aagtgctaag ttgttttttg 4020
   gaaagggtgg atggagctgc catccacttc acgaagttgt tccaagtgtc actaaaacac 4080
   tatggtccgt gggctgtgag attacaaagg cgaaggcaat aatacaagag agtagcatct 4140
   ctcttctcgt ggagactact gacatcataa gtccaaaagt caaaatttca tctaagcatc 4200
   gccgctttgt gaaatcaaat tggggtctgt tcaagaaaac taaatcactg cctaacctga 4260
   cggagctgga atgactgacc tctaatcgag actacaccgc cgcaaactat aggtgggtgg 4320
   tacctcagtg attaatcttg taagcactga tcgtaggcta caacacacta atattatcca 4380
   gattagagag cttaattagc tctgtattaa taataacact actattccaa taactggaat 4440
   caccagcttg atttatctcc aaaatgattc aaagaaaaca aatcatatta agactatcct 4500
   aagcacgaac ccatatcgtc cttcaaatca tgggtactat aattcaattt ctggtggtct 4560
   cctgtctatt ggcaggagca ggcagccttg atccagcagc cctcatgcaa atcggtgtca 4620
   ttccaacaaa tgtccggcaa cttatgtatt atactgaggc ctcatcagca ttcattgttg 4680
   tgaagttaat gcctacaatt gactcgccga ttagtggatg taatataaca tcaatttcaa 4740
   gctataatgc aacagtgaca aaactcctac agccgatcgg tgagaatttg gagacgatta 4800
   ggaaccagtt gattccaact cggaggagac gccggtttgc aggggtggtg attggattag 4860
   ctgcattagg agtagctact gccgcacagg tcactgccgc agtggcacta gtaaaggcaa 4920
   atgaaaatgc tgcggctata ctcaatctca aaaatgcaat ccaaaaaaca aatgcagcag 4980
   ttgcagatgt ggtccaggcc acacaatcac taggaacggc agttcaagca gttcaagatc 5040
   acataaacag tgtggtaagt ccagcaatta cagcagccaa ttgtaaggcc caagatgcta 5100
   tcattggctc aatcctcaat ctctatttga ccgagttgac aaccatcttc cacaatcaaa 5160
   ttacaaaccc tgcattgagt cccattacaa ttcaagcttt aaggatccta ctggggagta 5220
   ccttgccgac tgtggtcgaa aaatctttca atacccagat aagtgcagct gagcttctct 5280
   catcagggtt attgacaggc cagattgtgg gattagattt gacctatatg cagatggtca 5340
   taaaaattga gctgccaact ttaactgtac aacctgcaac ccagatcata gatctggcca 5400
   ccatttctgc attcattaac aatcaagaag tcatggccca attaccaaca cgtgttatgg 5460
   tgactggcag cttgatccaa gcctatcccg catcgcaatg caccattaca cccaacactg 5520
   tgtactgtag gtataatgat gcccaagtac tctcagatga tactatggct tgcctccaag 5580
   gtaacttgac aagatgcacc ttctctccag tggttgggag ctttctcact cgattcgtgc 5640
   tgttcgatgg aatagtttat gcaaattgca ggtcgatgtt gtgcaagtgc atgcaacctg 5700
   ctgctgtgat cctacagccg agttcatccc ctgtaactgt cattgacatg tacaaatgtg 5760
   tgagtctgca gcttgacaat ctcagattca ccatcactca attggccaat gtaacctaca 5820
   atagcaccat caagcttgaa tcatcccaga tcttgtctat tgatccgttg gatatatccc 5880
   aaaatctagc tgcggtgaat aagagtctaa gtgatgcact acaacactta gcacaaagtg 5940
   acacatatct ttctgcaatc acatcagcta cgactacaag tgtattatcc ataatagcaa 6000
   tctgtcttgg atcgttaggt ttaatattaa taatcttgct cagtgtagtt gtgtggaagt 6060
   tattgaccat tgtcgttgct aatcgaaata gaatggagaa ttttgtttat cataaataag 6120
   cattccacca ctcacgatct gatctcagtg agaaaaatca acctgcaact cttggaacaa 6180
   gataagacag tcatccatta gtaattttta agaaaaaaac gataggaccg aacctagtat 6240
   tgaaagaacc gtctcggtca atctaggtaa tcgagctgat accgtctcgg aaagctcaaa 6300
   tcatgctgcc tgatccggaa gatccggaaa gcaagaaagc tacaaggaga gcaggaaacc 6360
   taattatctg cttcctattc atcttctttc tgtttgtaac cttcattgtt ccaactctaa 6420
   gacacttgct gtcctaacac ctgctatagg ctatccactg catcatctct cctgccatac 6480
   ttcctactca catcatatct attttaaaga aaaaataggc ccgaacacta atcgtgccgg 6540
   cagtgccact gcacacacaa cactacacat acaatacact acaatggttg cagaagatgc 6600
   ccctgttagg gccacttgcc gagtattatt tcgaacaaca actttaatct ttctatgcac 6660
   actactagca ttaagcatct ctatccttta tgagagttta ataacccaaa agcaaatcat 6720
   gagccaagca ggctcaactg gatctaattc tggattagga agtatcactg atcttcttaa 6780
   taatattctc tctgtcgcaa atcagattat atataactct gcagtcgctc tacctctaca 6840
   attggacact cttgaatcaa cactccttac agccattaag tctcttcaaa ccagtgacaa 6900
   gctagaacag aactgctcgt ggagtgctgc actgattaat gataatagat acattaatgg 6960
   catcaatcag ttctattttt caattgctga gggtcgcaat ctgacacttg gcccacttct 7020
   taatatgcct agtttcattc caactgccac gacaccagag ggctgcacca ggatcccatc 7080
   attctcgctc actaagacac actggtgtta tacacacaat gttatcctga atggatgcca 7140
   ggatcatgta tcctcaaatc aatttgtttc catgggaatc attgaaccca cttctgccgg 7200
   gtttccattc tttcgaaccc taaagactct atatctcagc gatggggtca atcgtaagag 7260
   ctgctctatc agtacagttc cggggggttg tatgatgtac tgttttgttt ctactcaacc 7320
   agagagggat gactactttt ctgccgctcc tccagaacaa cgaattatta taatgtacta 7380
   taatgataca atcgtggagc gcataattaa tccacccggg gtactagatg tatgggcaac 7440
   attgaaccca ggaacaggaa gcggggtata ttatttaggt tgggtgctct ttccaatata 7500
   tggcggcgtg attaaaggta cgagtttatg gaataatcaa gcaaataaat actttatccc 7560
   ccagatggtt gctgctctct gctcacaaaa ccaggcaact caagtccaaa atgctaagtc 7620
   atcatactat agcagctggt ttggcaatcg aatgattcag tctgggatcc tggcatgtcc 7680
   tcttcgacag gatctaacca atgagtgttt agttctgccc ttttctaatg atcaggtgct 7740
   tatgggtgct gaagggagat tatacatgta tggtgactcg gtgtattact atcaaagaag 7800
   caatagttgg tggcctatga ccatgctgta taaggtaacc ataacattca ctaatggtca 7860
   gccatctgct atatcagctc agaatgtgcc cacacagcag gtccctagac ctgggacagg 7920
   agactgctct gcaaccaata gatgtcccgg tttttgcttg acaggagtgt atgccgatgc 7980
   ctggttactg accaaccctt cgtctaccag tacatttgga tcagaagcaa ccttcactgg 8040
   ttcttatctc aacacagcaa ctcagcgtat caatccgacg atgtatatcg cgaacaacac 8100
   acagatcata agctcacagc aatttggatc aagcggtcaa gaagcagcat atggccacac 8160
   aacctgtttt agggacacag gctctgttat ggtatactgt atctatatta ttgaattgtc 8220
   ctcatctctc ttaggacaat ttcagattgt cccatttatc cgtcaggtga cactatccta 8280
   aaggcagaag ccttcaggtc tgacccagcc aatcaaagca ttataccaga ccatggaatg 8340
   cataccaaac attattgaca ctaatgacac acaaaattgg ttttaagaaa aaccaagaga 8400
   acaataggcc agaatggctg ggtctcggga gatattactc cctgaagtcc atctcaattc 8460
   accaattgta aagcataagc tatactatta cattctactt ggaaacctcc caaatgagat 8520
   cgaccttgac gatttaggtc cattacataa tcaaaattgg aatcagatag cacatgaaga 8580
   gtctaactta gctcaacgct tggtaaatgt aagaaatttt ctaattaccc acatccctga 8640
   tcttagaaag ggccattggc aagagtatgt caatgtaata ctgtggccgc gaattcttcc 8700
   cttgatcccg gattttaaaa tcaatgacca attgcctctg ctcaaaaatt gggacaagtt 8760
   agttaaagaa tcatgttcag taatcaatgc aggtacttcc cagtgcattc agaatctcag 8820
   ctatggactg acaggtcgtg ggaacctctt tacacgatca cgtgaactct ctggtgaccg 8880
   cagggatatt gatcttaaga cagttgtggc agcatggcat gactcagact ggaaaagaat 8940
   aagtgatttt tggattatga tcaaattcca gatgagacaa ttaattgtta ggcaaacaga 9000
   tcataatgat tctgatttaa tcacgtatat cgaaaataga gaaggcataa tcatcataac 9060
   ccctgaactg gtagcattat ttaacactga gaatcataca ctaacataca tgacctttga 9120
   aattgtactg atggtttcag atatgtacga aggtcgtcac aacattttat cactatgcac 9180
   agttagcact tacctgaatc ctctgaagaa aagaataaca tatttattga gccttgtaga 9240
   taacttagct tttcagatag gtgatgctgt atataacata attgctttgc tagaatcctt 9300
   tgtatatgca cagttgcaaa tgtcagatcc catcccagaa ctcagaggac aattccatgc 9360
   attcgtatgt tctgagattc ttgatgcact aagaggaact aatagtttca cccaggatga 9420
   attaagaact gtgacaacta atttgatatc cccattccaa gatctgaccc cagatcttac 9480
   ggctgaattg ctctgtataa tgaggctttg gggacacccc atgctcactg ccagtcaagc 9540
   tgcaggaaag gtacgcgagt ctatgtgtgc tggaaaagta ttagactttc ccaccattat 9600
   gaaaacacta gcctttttcc atactattct gatcaatgga tacaggagga agcatcatgg 9660
   agtatggcca cccttaaact taccgggtaa tgcttcaaag ggtctcacgg aacttatgaa 9720
   tgacaatact gaaataagct atgaattcac acttaagcat tggaaggaag tctctcttat 9780
   aaaattcaag aaatgttttg atgcagacgc aggtgaggaa ctcagtatat ttatgaaaga 9840
   taaggcaatt agtgccccaa aacaagactg gatgagtgtg tttagaagaa gcctaatcaa 9900
   acagcgccat cagcatcatc aggtccccct accaaatcca ttcaatcgac ggctgttgct 9960
   aaactttctc ggagatgaca aattcgaccc gaatgtggag ctacagtatg taacatcagg 10020
   tgagtatcta catgatgaca cgttttgtgc atcatattca ctaaaagaga aggaaattaa 10080
   acctgatggt cgaatttttg caaagttgac taagagaatg agatcatgtc aagttatagc 10140
   agaatctctt ttagcgaacc atgctgggaa gttaatgaaa gagaatggtg ttgtgatgaa 10200
   tcagctatca ttaacaaaat cactattaac aatgagtcag attggaataa tatccgagaa 10260
   agctagaaag tcaactcgag ataacataaa tcaacctggt ttccagaata tccagagaaa 10320
   taaatcacat cactccaagc aagtcaatca gcgagatcca agtgatgact ttgaattggc 10380
   agcatctttt ttaactactg atctcaaaaa atattgttta caatggaggt accagacaat 10440
   tatcccattt gctcaatcat taaacagaat gtatggttat cctcatctct ttgagtggat 10500
   tcacttacgg ctaatgcgta gtacacttta cgtgggggat cccttcaacc caccagcaga 10560
   taccagtcaa tttgatctag ataaagtaat taatggagat atcttcattg tatcacccag 10620
   aggtggaatt gaagggctgt gtcaaaaggc ttggacaatg atatctatcg ctgtgataat 10680
   tctatctgcc acagagtctg gcacacgagt aatgagtatg gtgcagggag ataatcaagc 10740
   aattgctgtc accacacgag taccaaggag cctgccgact cttgagaaaa agactattgc 10800
   ttttagatct tgtaatctat tctttgagag gttaaaatgt aataattttg gattaggtca 10860
   ccatttgaaa gaacaagaga ctatcattag ttctcacttc tttgtttata gcaagagaat 10920
   attctatcag gggaggattc taacgcaagc cttaaaaaat gctagtaagc tctgcttgac 10980
   agctgatgtc ctaggagaat gcacccaatc atcatgttct aatcttgcaa ctactgtcat 11040
   gaggttaact gagaatggtg ttgaaaaaga tatctgtttc tacttgaata tctatatgac 11100
   catcaaacag ctctcctatg atatcatctt ccctcaagtg tcaattcctg gagatcagat 11160
   cacattagaa tacataaata atccacacct ggtatcacga ttggctcttt tgccatccca 11220
   gttaggaggt ctaaactacc tgtcatgcag taggctgttc aatcgaaaca taggcgaccc 11280
   ggtggtttcc gcagttgcag atcttaagag attaattaaa tcaggatgta tggattactg 11340
   gatcctttat aacttattag ggagaaaacc gggaaacggc tcatgggcta ctttagcagc 11400
   tgacccgtac tcaatcaata tagagtatca ataccctcca actacagctc ttaagaggca 11460
   cacccaacaa gctctgatgg aactcagtac gaatccaatg ttacgtggca tattctctga 11520
   caatgcacag gcagaagaaa ataaccttgc taggtttctc ctggataggg aggtgatctt 11580
   tccgcgtgta gctcacatca tcattgagca aaccagtgtc gggaggagaa aacagattca 11640
   aggatatttg gattcaacta gatcgataat gaggaaatca ctagaaatta agcccttatc 11700
   caataggaag cttaatgaaa tactggatta caacatcaat tacctagctt acaatttggc 11760
   attactcaag aatgctattg aacctccgac ttatttgaag gcaatgacac ttgaaacatg 11820
   tagcatcgac attgcaagga acctccggaa gctctcctgg gccccactct tgggtgggag 11880
   aaatcttgaa ggattagaga cgccagatcc cattgaaatt actgcaggag cattaattgt 11940
   tggatcgggc tactgtgaac agtgtgctgc aggagacaat cgattcacat ggtttttctt 12000
   gccatctggt atcgagatag gaggggatcc ccgtgataat cctcctatcc gtgtaccgta 12060
   cattggctcc aggactgatg agaggagggt agcctcaatg gcatacatca ggggtgcctc 12120
   gagtagctta aaagcagttc ttagactggc gggagtgtac atctgggcat tcggagatac 12180
   tctggagaat tggatagatg cactggattt gtctcacact agagttaaca tcacacttga 12240
   acagctgcaa tccctcaccc cacttccaac ctctgccaat ctaacccatc ggttggatga 12300
   tggcacaact accctaaagt ttactcctgc gagctcttac accttttcaa gtttcactca 12360
   tatatcaaat gatgagcaat acctgacaat taatgacaaa actgcagatt caaatataat 12420
   ctaccaacag ttaatgatca ctggactcgg aatcttagaa acatggaata atcccccaat 12480
   caatagaaca ttcgaagaat ctaccctaca tttgcacact ggtgcatcat gttgtgtccg 12540
   acctgtggac tcctgcattc tctcagaagc attaacagtc aagccacata ttacagtacc 12600
   gtacagcaat aaatttgtat ttgatgagga cccgctatct gaatatgaaa ctgcaaaact 12660
   ggaatcgtta tcattccaag cccaattagg caacattgat gctgtagata tgacaggtaa 12720
   attaacatta ttgtcccaat tcactgcaag gcagattatc aatgcaatca ctggactcga 12780
   tgagtctgtc tctcttacta atgatgccat tgttgcatca gactatgtct ccaattggat 12840
   tagtgaatgc atgtatacca aattagatga attatttatg tattgtgggt gggaactact 12900
   attggaacta tcctatcaaa tgtattatct gagggtagtt gggtggagta atatagtgga 12960
   ttattcttac atgatcttga gaagaatccc gggtgcagca ttaaacaatc tggcatctac 13020
   attaagtcat ccaaaacttt tccgacgagc tatcaaccta gatatagttg cccccttaaa 13080
   tgctcctcat tttgcatctc tggactacat caagatgagt gtggatgcaa tactctgggg 13140
   ctgtaaaaga gtcatcaatg tgctctccaa tggaggggac ttagaattag ttgtgacatc 13200
   tgaagatagc cttattctca gtgaccgatc catgaatctc attgcaagga aattaacttt 13260
   attatcactg attcaccata atggtttgga actaccaaag attaaggggt tctctcctga 13320
   tgagaagtgt ttcgctttga cagaattttt gaggaaagtg gtgaactcag ggttgagttc 13380
   aatagagaac ctatcaaatt ttatgtacaa tgtggagaac ccacggcttg cagcattcgc 13440
   cagcaacaat tactacctga ccagaaaatt attgaattca atacgagata ctgagtcggg 13500
   tcaagtagca gtcacctcat attatgaatc attagaatat attgatagtc ttaagctaac 13560
   cccacatgtg cctggcacct catgcattga ggatgatagt ctatgtacaa atgattacat 13620
   aatctggatc atagagtcta atgcaaactt ggagaagtat ccaattccaa atagccctga 13680
   ggatgattcc aatttccata actttaagtt gaatgctcca tcgcaccata ccttacgccc 13740
   attagggttg tcatcaactg cttggtataa gggtataagc tgctgcaggt accttgagcg 13800
   attaaagcta ccacaaggtg atcatttata tattgcagaa ggtagtggtg ccagtatgac 13860
   aatcatagaa tacctattcc caggaagaaa gatatattac aattctttat ttagtagtgg 13920
   tgacaatccc ccacaaagaa attatgcacc aatgcctact cagttcattg agagtgtccc 13980
   atacaagctc tggcaagcac acacagatca atatcccgag atttttgagg acttcatccc 14040
   tctatggaac ggaaacgccg ccatgactga cataggaatg acagcttgtg tagaattcat 14100
   catcaatcga gtcggcccaa ggacttgcag tttagtacat gtagatttgg aatcaagtgc 14160
   aagcttaaat caacaatgcc tgtcaaagcc gataattaat gctatcatca ctgctacaac 14220
   tgttttgtgc cctcatgggg tgcttattct gaaatatagt tggttgccat ttactagatt 14280
   tagtactttg atcactttct tatggtgcta ctttgagaga atcactgttc ttaggagcac 14340
   atattctgat ccagctaatc atgaggttta tttaatttgt atccttgcca acaactttgc 14400
   attccagact gtctcgcagg caacaggaat ggcgatgact ttaactgatc aagggtttac 14460
   tttgatatca cctgaaagaa taaatcagta ttgggatggt cacttgaagc aagaacgtat 14520
   cgtagcagaa gcaattgata aggtggttct aggagaaaat gctctattta attcgagtga 14580
   taatgaatta attctcaaat gtggagggac accaaatgca cggaatctca tcgatatcga 14640
   gccagtcgca actttcatag aatttgaaca attgatctgc acaatgttga caacccactt 14700
   gaaggaaata attgatataa caaggtctgg aacccaggat tatgaaagtt tattactcac 14760
   tccttacaat ttaggtcttc ttggtaaaat cagtacgata gtgagattat taacagaaag 14820
   gattctaaat catactatca ggaattggtt gatcctccca ccttcgctcc ggatgatcgt 14880
   gaagcaggac ttggaattcg gcatattcag gattacttcc atcctcaatt ctgatcggtt 14940
   cctgaagctt tctccaaata ggaaatactt gattgcacaa ttaactgcag gctacattag 15000
   gaaattgatt gagggggatt gcaatatcga tctaaccaga cctatccaaa agcaaatctg 15060
   gaaagcatta ggttgtgtag tctattgtca cgatccaatg gatcaaaggg agtcaacaga 15120
   gtttattgat ataaatatta atgaagaaat agaccgcggg atcgatggcg aggaaatcta 15180
   aacatatcaa gaatcagaat tagtttaaga aaaaagaaga ggattaatct tggttttccc 15240
   cttggt                                                            15246
<210> 3
   <211> 1527
   <212> DNA
   <213> Cryptovirus
<220>
   <223> Cryptovirus NP Protein encoding sequence; cDNA in mRNA sense
<221> CDS
   <222> (1)...(1527)
<400> 3
   atg tca tcc gtg ctt aaa gca tat gag aga ttc aca ctc act caa gaa    48
   Met Ser Ser Val Leu Lys Ala Tyr Glu Arg Phe Thr Leu Thr Gln Glu
    1               5                  10                  15
   ctg caa gat cag agt gaa gaa ggg aca atc cca cct aca aca cta aaa    96
   Leu Gln Asp Gln Ser Glu Glu Gly Thr Ile Pro Pro Thr Thr Leu Lys
               20                  25                  30
   ccg gtt atc agg gta ttt gta cta acc tct aat aac cca gag cta aga    144
   Pro Val Ile Arg Val Phe Val Leu Thr Ser Asn Asn Pro Glu Leu Arg
           35                  40                  45
   tcc cgg ctt ctt cta ttc tgc cta cgg att gtt ctc agt aat ggt gca    192
   Ser Arg Leu Leu Leu Phe Cys Leu Arg Ile Val Leu Ser Asn Gly Ala
       50                  55                  60
   agg gat tcc cat cgc ttt gga gca tta ctt aca atg ttt tcg cta cca    240
   Arg Asp Ser His Arg Phe Gly Ala Leu Leu Thr Met Phe Ser Leu Pro
   65                  70                  75                  80
   tca gct aca atg ctc aat cat gtc aaa tta gct gac cag tca cca gaa    288
   Ser Ala Thr Met Leu Asn His Val Lys Leu Ala Asp Gln Ser Pro Glu
                   85                  90                  95
   gct gat atc gaa agg gta gag atc gat ggc ttt gag gag gga tca ttc    336
   Ala Asp Ile Glu Arg Val Glu Ile Asp Gly Phe Glu Glu Gly Ser Phe
               100                 105                 110
   cgc tta att ccc aat gct cgc tca ggt atg agc cgt gga gag atc aat    384
   Arg Leu Ile Pro Asn Ala Arg Ser Gly Met Ser Arg Gly Glu Ile Asn
           115                 120                 125
   gcc tat gct gca ctt gca gaa gat cta cct gac aca cta aac cat gca    432
   Ala Tyr Ala Ala Leu Ala Glu Asp Leu Pro Asp Thr Leu Asn His Ala
       130                 135                 140
   aca cct ttc gtt gat tcc gaa gtc gag gga act gca tgg gac gag att    480
   Thr Pro Phe Val Asp Ser Glu Val Glu Gly Thr Ala Trp Asp Glu Ile
   145                 150                 155                 160
   gag act ttc tta gat atg tgt tac agt gtc cta atg cag gca tgg ata    528
   Glu Thr Phe Leu Asp Met Cys Tyr Ser Val Leu Met Gln Ala Trp Ile
                   165                 170                 175
   gtg act tgc aag tgc atg act gcg cca gac caa cct gct gct tct att    576
   Val Thr Cys Lys Cys Met Thr Ala Pro Asp Gln Pro Ala Ala Ser Ile
               180                 185                 190
   gag aaa cgc ctg caa aaa tat cgt cag caa ggc agg atc aac cca aga    624
   Glu Lys Arg Leu Gln Lys Tyr Arg Gln Gln Gly Arg Ile Asn Pro Arg
           195                 200                 205
   tat ctc ctg caa ccg gag gct cga cga ata atc cag aat gta atc cgg    672
   Tyr Leu Leu Gln Pro Glu Ala Arg Arg Ile Ile Gln Asn Val Ile Arg
       210                 215                 220
   aag gga atg gtg gtc aga cat ttc ctc acc ttt gaa ctg cag ctt gcc    720
   Lys Gly Met Val Val Arg His Phe Leu Thr Phe Glu Leu Gln Leu Ala
   225                 230                 235                 240
   cga gca caa agc ctt gta tca aat agg tat tat gct atg gta ggg gat    768
   Arg Ala Gln Ser Leu Val Ser Asn Arg Tyr Tyr Ala Met Val Gly Asp
                   245                 250                 255
   gtt gga aag tat ata gag aat tgt gga atg gga ggc ttc ttt ttg aca    816
   Val Gly Lys Tyr Ile Glu Asn Cys Gly Met Gly Gly Phe Phe Leu Thr
               260                 265                 270
   cta aaa tat gca tta gga acc aga tgg ccc aca ctt gct tta gct gca    864
   Leu Lys Tyr Ala Leu Gly Thr Arg Trp Pro Thr Leu Ala Leu Ala Ala
           275                 280                 285
   ttt tca gga gag cta aca aag cta aag tcc ctc atg gca ttg tac cag    912
   Phe Ser Gly Glu Leu Thr Lys Leu Lys Ser Leu Met Ala Leu Tyr Gln
       290                 295                 300
   acc ctt ggt gag cag gcc cga tat ttg gcc cta ttg gag tca cca cac    960
   Thr Leu Gly Glu Gln Ala Arg Tyr Leu Ala Leu Leu Glu Ser Pro His
   305                 310                 315                 320
   ttg atg gat ttt gct gca gca aac tat cca ttg cta tat agc tat gct    1008
   Leu Met Asp Phe Ala Ala Ala Asn Tyr Pro Leu Leu Tyr Ser Tyr Ala
                   325                 330                 335
   atg gga ata ggc tat gtg tta gat gtc aac atg agg aac tac gct ttc    1056
   Met Gly Ile Gly Tyr Val Leu Asp Val Asn Met Arg Asn Tyr Ala Phe
               340                 345                 350
   tcc aga tca tac atg aat aag aca tat ttc caa ttg gga atg gaa act    1104
   Ser Arg Ser Tyr Met Asn Lys Thr Tyr Phe Gln Leu Gly Met Glu Thr
           355                 360                 365
   gca aga aaa caa cag ggt gca gtt gac atg agg atg gca gaa gat ctc      1152
   Ala Arg Lys Gln Gln Gly Ala Val Asp Met Arg Met Ala Glu Asp Leu
       370                 375                 380
   ggt cta act caa gcc gaa cgc acc gag atg gca aat aca ctt gcc aaa      1200
   Gly Leu Thr Gln Ala Glu Arg Thr Glu Met Ala Asn Thr Leu Ala Lys
   385                 390                 395                 400
   ttg acc aca gca aat cga ggg gca gac acc agg gga gga gtc aac ccg      1248
   Leu Thr Thr Ala Asn Arg Gly Ala Asp Thr Arg Gly Gly Val Asn Pro
                   405                 410                 415
   ttc tca tct gtc act ggg aca act cag atg ccc gct gca gca aca ggt      1296
   Phe Ser Ser Val Thr Gly Thr Thr Gln Met Pro Ala Ala Ala Thr Gly
               420                 425                 430
   gac aca ttc gag agt tac atg gca gcg gat cga ctg agg cag aga tat      1344
   Asp Thr Phe Glu Ser Tyr Met Ala Ala Asp Arg Leu Arg Gln Arg Tyr
           435                 440                 445
   gct gat gca ggc acc cat gat gat gag atg cca cca tta gaa gag gag      1392
   Ala Asp Ala Gly Thr His Asp Asp Glu Met Pro Pro Leu Glu Glu Glu
       450                 455                 460
   gaa gag gac gac aca tct gca ggt cca cgc act gaa cca act cct gaa      1440
   Glu Glu Asp Asp Thr Ser Ala Gly Pro Arg Thr Glu Pro Thr Pro Glu
   465                 470                 475                 480
   caa gtg gcc ttg gac atc cag agc gca gca gtt gga gct ccc atc cat      1488
   Gln Val Ala Leu Asp Ile Gln Ser Ala Ala Val Gly Ala Pro Ile His
                   485                 490                 495
   aca gac gac ctg aat gcc gca cta ggt gat ctt gac atc                  1527
   Thr Asp Asp Leu Asn Ala Ala Leu Gly Asp Leu Asp Ile
               500                 505
<210> 4
   <211> 509
   <212> PRT
   <213> Cryptovirus NP Protein
<400> 4
   Met Ser Ser Val Leu Lys Ala Tyr Glu Arg Phe Thr Leu Thr Gln Glu
    1               5                  10                  15
   Leu Gln Asp Gln Ser Glu Glu Gly Thr Ile Pro Pro Thr Thr Leu Lys
               20                  25                  30
   Pro Val Ile Arg Val Phe Val Leu Thr Ser Asn Asn Pro Glu Leu Arg
           35                  40                  45
   Ser Arg Leu Leu Leu Phe Cys Leu Arg Ile Val Leu Ser Asn Gly Ala
       50                  55                  60
   Arg Asp Ser His Arg Phe Gly Ala Leu Leu Thr Met Phe Ser Leu Pro
   65                  70                  75                  80
   Ser Ala Thr Met Leu Asn His Val Lys Leu Ala Asp Gln Ser Pro Glu
                   85                  90                  95
   Ala Asp Ile Glu Arg Val Glu Ile Asp Gly Phe Glu Glu Gly Ser Phe
               100                 105                 110
   Arg Leu Ile Pro Asn Ala Arg Ser Gly Met Ser Arg Gly Glu Ile Asn
           115                 120                 125
   Ala Tyr Ala Ala Leu Ala Glu Asp Leu Pro Asp Thr Leu Asn His Ala
       130                 135                 140
   Thr Pro Phe Val Asp Ser Glu Val Glu Gly Thr Ala Trp Asp Glu Ile
   145                 150                 155                 160
   Glu Thr Phe Leu Asp Met Cys Tyr Ser Val Leu Met Gln Ala Trp Ile
                   165                 170                 175
   Val Thr Cys Lys Cys Met Thr Ala Pro Asp Gln Pro Ala Ala Ser Ile
               180                 185                 190
   Glu Lys Arg Leu Gln Lys Tyr Arg Gln Gln Gly Arg Ile Asn Pro Arg
           195                 200                 205
   Tyr Leu Leu Gln Pro Glu Ala Arg Arg Ile Ile Gln Asn Val Ile Arg
       210                 215                 220
   Lys Gly Met Val Val Arg His Phe Leu Thr Phe Glu Leu Gln Leu Ala
   225                 230                 235                 240
   Arg Ala Gln Ser Leu Val Ser Asn Arg Tyr Tyr Ala Met Val Gly Asp
                   245                 250                 255
   Val Gly Lys Tyr Ile Glu Asn Cys Gly Met Gly Gly Phe Phe Leu Thr
               260                 265                 270
   Leu Lys Tyr Ala Leu Gly Thr Arg Trp Pro Thr Leu Ala Leu Ala Ala
           275                 280                 285
   Phe Ser Gly Glu Leu Thr Lys Leu Lys Ser Leu Met Ala Leu Tyr Gln
       290                 295                 300
   Thr Leu Gly Glu Gln Ala Arg Tyr Leu Ala Leu Leu Glu Ser Pro His
   305                 310                 315                 320
   Leu Met Asp Phe Ala Ala Ala Asn Tyr Pro Leu Leu Tyr Ser Tyr Ala
                   325                 330                 335
   Met Gly Ile Gly Tyr Val Leu Asp Val Asn Met Arg Asn Tyr Ala Phe
               340                 345                 350
   Ser Arg Ser Tyr Met Asn Lys Thr Tyr Phe Gln Leu Gly Met Glu Thr
           355                 360                 365
   Ala Arg Lys Gln Gln Gly Ala Val Asp Met Arg Met Ala Glu Asp Leu
       370                 375                 380
   Gly Leu Thr Gln Ala Glu Arg Thr Glu Met Ala Asn Thr Leu Ala Lys
   385                 390                 395                 400
   Leu Thr Thr Ala Asn Arg Gly Ala Asp Thr Arg Gly Gly Val Asn Pro
                   405                 410                 415
   Phe Ser Ser Val Thr Gly Thr Thr Gln Met Pro Ala Ala Ala Thr Gly
               420                 425                 430
   Asp Thr Phe Glu Ser Tyr Met Ala Ala Asp Arg Leu Arg Gln Arg Tyr
           435                 440                 445
   Ala Asp Ala Gly Thr His Asp Asp Glu Met Pro Pro Leu Glu Glu Glu
       450                 455                 460
   Glu Glu Asp Asp Thr Ser Ala Gly Pro Arg Thr Glu Pro Thr Pro Glu
   465                 470                 475                 480
   Gln Val Ala Leu Asp Ile Gln Ser Ala Ala Val Gly Ala Pro Ile His
                   485                 490                 495
   Thr Asp Asp Leu Asn Ala Ala Leu Gly Asp Leu Asp Ile
               500                 505
<210> 5
   <211> 666
   <212> DNA
   <213> Cryptovirus
<220>
   <221> CDS
   <222> (1)...(666)
   <223> Cryptovirus V protein encoding sequence; cDNA in mRNA sense
<400> 5
   atg gat ccc act gat ctg agc ttc tcc cca gat gag att aat aag ctc    48
   Met Asp Pro Thr Asp Leu Ser Phe Ser Pro Asp Glu Ile Asn Lys Leu
    1               5                  10                  15
   ata gag aca ggc ctg aat act gtg gag tat ttt act tcc caa caa gtc    96
   Ile Glu Thr Gly Leu Asn Thr Val Glu Tyr Phe Thr Ser Gln Gln Val
               20                  25                  30
   aca gga aca tcc tct ctt gga aag aat aca ata cca cca ggg gtc aca    144
   Thr Gly Thr Ser Ser Leu Gly Lys Asn Thr Ile Pro Pro Gly Val Thr
           35                  40                  45
   gga cta cta acc aat gct gca gag gca aag atc caa gag tca atc aac    192
   Gly Leu Leu Thr Asn Ala Ala Glu Ala Lys Ile Gln Glu Ser Ile Asn
       50                  55                  60
   cat cag aag ggt tca gtt ggt ggg ggt aca aac cca aag aaa ccg cga    240
   His Gln Lys Gly Ser Val Gly Gly Gly Thr Asn Pro Lys Lys Pro Arg
   65                  70                  75                  80
   tca aaa att gcc att gtg cca gca gat gac aaa aca gtg ccc gaa aag    288
   Ser Lys Ile Ala Ile Val Pro Ala Asp Asp Lys Thr Val Pro Glu Lys
                   85                  90                  95
   ccg atc cca aac cct cta cta ggt ctg gac tcc acc ccg agc acc caa    336
   Pro Ile Pro Asn Pro Leu Leu Gly Leu Asp Ser Thr Pro Ser Thr Gln
               100                 105                 110
   acc gtg ctt gat cta agt ggg aaa aca tta cca tca gga tcc tat aag    384
   Thr Val Leu Asp Leu Ser Gly Lys Thr Leu Pro Ser Gly Ser Tyr Lys
           115                 120                 125
   ggg gtt aag ctt gcg aaa ttt ggg aaa gaa aat ctg atg aca cgg ttc    432
   Gly Val Lys Leu Ala Lys Phe Gly Lys Glu Asn Leu Met Thr Arg Phe
       130                 135                 140
   atc gag gaa ccc aga gag aat cct atc gca acc agt tcc ccc atc gat    480
   Ile Glu Glu Pro Arg Glu Asn Pro Ile Ala Thr Ser Ser Pro Ile Asp
   145                 150                 155                 160
   ttt aag agg ggc aga gat acc ggt ggg ttc cat aga agg gag tac tca    528
   Phe Lys Arg Gly Arg Asp Thr Gly Gly Phe His Arg Arg Glu Tyr Ser
                   165                 170                 175
   atc gga tgg gtg gga gat gaa gtc aag gtc act gag tgg tgc aat cca    576
   Ile Gly Trp Val Gly Asp Glu Val Lys Val Thr Glu Trp Cys Asn Pro
               180                 185                 190
   tcc tgt tct cca atc acc gct gca gca agg cga ttt aaa tgc act tgt    624
   Ser Cys Ser Pro Ile Thr Ala Ala Ala Arg Arg Phe Lys Cys Thr Cys
           195                 200                 205
   cac caa tgt cca gtc act tgc tct gaa tgt gaa cga gat act            666
   His Gln Cys Pro Val Thr Cys Ser Glu Cys Glu Arg Asp Thr
       210                 215                 220
<210> 6
   <211> 222
   <212> PRT
   <213> Cryptovirus V Protein
<400> 6
   Met Asp Pro Thr Asp Leu Ser Phe Ser Pro Asp Glu Ile Asn Lys Leu
   1               5                   10                  15
   Ile Glu Thr Gly Leu Asn Thr Val Glu Tyr Phe Thr Ser Gln Gln Val
               20                  25                  30
   Thr Gly Thr Ser Ser Leu Gly Lys Asn Thr Ile Pro Pro Gly Val Thr
           35                  40                  45
   Gly Leu Leu Thr Asn Ala Ala Glu Ala Lys Ile Gln Glu Ser Ile Asn
       50                  55                  60
   His Gln Lys Gly Ser Val Gly Gly Gly Thr Asn Pro Lys Lys Pro Arg
   65                  70                  75                  80
   Ser Lys Ile Ala Ile Val Pro Ala Asp Asp Lys Thr Val Pro Glu Lys
                   85                  90                  95
   Pro Ile Pro Asn Pro Leu Leu Gly Leu Asp Ser Thr Pro Ser Thr Gln
               100                 105                 110
   Thr Val Leu Asp Leu Ser Gly Lys Thr Leu Pro Ser Gly Ser Tyr Lys
           115                 120                 125
   Gly Val Lys Leu Ala Lys Phe Gly Lys Glu Asn Leu Met Thr Arg Phe
       130                 135                 140
   Ile Glu Glu Pro Arg Glu Asn Pro Ile Ala Thr Ser Ser Pro Ile Asp
   145                 150                 155                 160
   Phe Lys Arg Gly Arg Asp Thr Gly Gly Phe His Arg Arg Glu Tyr Ser
                   165                 170                 175
   Ile Gly Trp Val Gly Asp Glu Val Lys Val Thr Glu Trp Cys Asn Pro
               180                 185                 190
   Ser Cys Ser Pro Ile Thr Ala Ala Ala Arg Arg Phe Lys Cys Thr Cys
           195                 200                 205
   His Gln Cys Pro Val Thr Cys Ser Glu Cys Glu Arg Asp Thr
       210                 215                 220
<210> 7
   <211> 1176
   <212> DNA
   <213> Cryptovirus
<220>
   <221> CDS
   <222> (1)...(1176)
   <223> Cryptovirus P protein encoding sequence; cDNA in mRNA sense
<400> 7
   atg gat ccc act gat ctg agc ttc tcc cca gat gag att aat aag ctc      48
   Met Asp Pro Thr Asp Leu Ser Phe Ser Pro Asp Glu Ile Asn Lys Leu
    1               5                  10                  15
   ata gag aca ggc ctg aat act gtg gag tat ttt act tcc caa caa gtc      96
   Ile Glu Thr Gly Leu Asn Thr Val Glu Tyr Phe Thr Ser Gln Gln Val
               20                  25                  30
   aca gga aca tcc tct ctt gga aag aat aca ata cca cca ggg gtc aca     144
   Thr Gly Thr Ser Ser Leu Gly Lys Asn Thr Ile Pro Pro Gly Val Thr
           35                  40                  45
   gga cta cta acc aat gct gca gag gca aag atc caa gag tca atc aac     192
   Gly Leu Leu Thr Asn Ala Ala Glu Ala Lys Ile Gln Glu Ser Ile Asn
       50                  55                  60
   cat cag aag ggt tca gtt ggt ggg ggt aca aac cca aag aaa ccg cga     240
   His Gln Lys Gly Ser Val Gly Gly Gly Thr Asn Pro Lys Lys Pro Arg
   65                  70                  75                  80
   tca aaa att gcc att gtg cca gca gat gac aaa aca gtg ccc gaa aag     288
   Ser Lys Ile Ala Ile Val Pro Ala Asp Asp Lys Thr Val Pro Glu Lys
                   85                  90                  95
   ccg atc cca aac cct cta cta ggt ctg gac tcc acc ccg agc acc caa     336
   Pro Ile Pro Asn Pro Leu Leu Gly Leu Asp Ser Thr Pro Ser Thr Gln
               100                 105                 110
   acc gtg ctt gat cta agt ggg aaa aca tta cca tca gga tcc tat aag     384
   Thr Val Leu Asp Leu Ser Gly Lys Thr Leu Pro Ser Gly Ser Tyr Lys
           115                 120                 125
   ggg gtt aag ctt gcg aaa ttt ggg aaa gaa aat ctg atg aca cgg ttc     432
   Gly Val Lys Leu Ala Lys Phe Gly Lys Glu Asn Leu Met Thr Arg Phe
       130                 135                 140
   atc gag gaa ccc aga gag aat cct atc gca acc agt tcc ccc atc gat     480
   Ile Glu Glu Pro Arg Glu Asn Pro Ile Ala Thr Ser Ser Pro Ile Asp
   145                 150                 155                 160
   ttt aag agg ggg gca gag ata ccg gtg ggt tcc ata gaa ggg agt act     528
   Phe Lys Arg Gly Ala Glu Ile Pro Val Gly Ser Ile Glu Gly Ser Thr
                   165                 170                 175
   caa tcg gat ggg tgg gag atg aag tca agg tca ctg agt ggt gca atc     576
   Gln Ser Asp Gly Trp Glu Met Lys Ser Arg Ser Leu Ser Gly Ala Ile
               180                 185                 190
   cat cct gtt ctc caa tca ccg ctg cag caa ggc gat tta aat gca ctt      624
   His Pro Val Leu Gln Ser Pro Leu Gln Gln Gly Asp Leu Asn Ala Leu
           195                 200                 205
   gtc acc aat gtc cag tca ctt gct ctg aat gtg aac gag ata ctt aat      672
   Val Thr Asn Val Gln Ser Leu Ala Leu Asn Val Asn Glu Ile Leu Asn
       210                 215                 220
   aca gtg aga aat ttg gac tct cgg atg aat caa ctg gag aca aaa gta      720
   Thr Val Arg Asn Leu Asp Ser Arg Met Asn Gln Leu Glu Thr Lys Val
   225                 230                 235                 240
   gat cgc att ctc tca tct cag tct cta atc cag acc atc aag aat gac      768
   Asp Arg Ile Leu Ser Ser Gln Ser Leu Ile Gln Thr Ile Lys Asn Asp
                   245                 250                 255
   ata att gga ctt aaa gca ggg atg gct act tta gaa gga atg att aca      816
   Ile Ile Gly Leu Lys Ala Gly Met Ala Thr Leu Glu Gly Met Ile Thr
               260                 265                 270
   act gtg aaa atc atg gac ccg gga gtt ccc agt aat gtt act gtg gaa      864
   Thr Val Lys Ile Met Asp Pro Gly Val Pro Ser Asn Val Thr Val Glu
           275                 280                 285
   gat gta cgc aag aaa cta agt aac cat gct gtt gtt gtg cca gaa tca      912
   Asp Val Arg Lys Lys Leu Ser Asn His Ala Val Val Val Pro Glu Ser
       290                 295                 300
   ttc aat gat agt ttc ttg act caa tct gaa gat gta att tca ctt gat      960
   Phe Asn Asp Ser Phe Leu Thr Gln Ser Glu Asp Val Ile Ser Leu Asp
   305                 310                 315                 320
   gag ttg gct cga cca act gca aca agt gtt aag aag att gtc agg aag     1008
   Glu Leu Ala Arg Pro Thr Ala Thr Ser Val Lys Lys Ile Val Arg Lys
                   325                 330                 335
   gtt cct cct cag aag gat ctg act gga ttg aag atc aca cta gag caa     1056
   Val Pro Pro Gln Lys Asp Leu Thr Gly Leu Lys Ile Thr Leu Glu Gln
               340                 345                 350
   ttg gca aag gat tgc atc agc aaa ccg aag atg agg gaa gat tat ctc     1104
   Leu Ala Lys Asp Cys Ile Ser Lys Pro Lys Met Arg Glu Asp Tyr Leu
           355                 360                 365
   ctc aag atc aac cag gct tct agt gag gct cag cta att gac ctc aag     1152
   Leu Lys Ile Asn Gln Ala Ser Ser Glu Ala Gln Leu Ile Asp Leu Lys
       370                 375                 380
   aaa gca atc atc cgc agt gca att                                     1176
   Lys Ala Ile Ile Arg Ser Ala Ile
   385                 390
<210> 8
   <211> 392
   <212> PRT
   <213> Cryptovirus P Protein
<400> 8
   Met Asp Pro Thr Asp Leu Ser Phe Ser Pro Asp Glu Ile Asn Lys Leu
    1               5                  10                  15
   Ile Glu Thr Gly Leu Asn Thr Val Glu Tyr Phe Thr Ser Gln Gln Val
               20                  25                  30
   Thr Gly Thr Ser Ser Leu Gly Lys Asn Thr Ile Pro Pro Gly Val Thr
           35                  40                  45
   Gly Leu Leu Thr Asn Ala Ala Glu Ala Lys Ile Gln Glu Ser Ile Asn
       50                  55                  60
   His Gln Lys Gly Ser Val Gly Gly Gly Thr Asn Pro Lys Lys Pro Arg
   65                  70                  75                  80
   Ser Lys Ile Ala Ile Val Pro Ala Asp Asp Lys Thr Val Pro Glu Lys
                   85                  90                  95
   Pro Ile Pro Asn Pro Leu Leu Gly Leu Asp Ser Thr Pro Ser Thr Gln
               100                 105                 110
   Thr Val Leu Asp Leu Ser Gly Lys Thr Leu Pro Ser Gly Ser Tyr Lys
           115                 120                 125
   Gly Val Lys Leu Ala Lys Phe Gly Lys Glu Asn Leu Met Thr Arg Phe
       130                 135                 140
   Ile Glu Glu Pro Arg Glu Asn Pro Ile Ala Thr Ser Ser Pro Ile Asp
   145                 150                 155                 160
   Phe Lys Arg Gly Ala Glu Ile Pro Val Gly Ser Ile Glu Gly Ser Thr
                   165                 170                 175
   Gln Ser Asp Gly Trp Glu Met Lys Ser Arg Ser Leu Ser Gly Ala Ile
               180                 185                 190
   His Pro Val Leu Gln Ser Pro Leu Gln Gln Gly Asp Leu Asn Ala Leu
           195                 200                 205
   Val Thr Asn Val Gln Ser Leu Ala Leu Asn Val Asn Glu Ile Leu Asn
       210                 215                 220
   Thr Val Arg Asn Leu Asp Ser Arg Met Asn Gln Leu Glu Thr Lys Val
   225                 230                 235                 240
   Asp Arg Ile Leu Ser Ser Gln Ser Leu Ile Gln Thr Ile Lys Asn Asp
                   245                 250                 255
   Ile Ile Gly Leu Lys Ala Gly Met Ala Thr Leu Glu Gly Met Ile Thr
               260                 265                 270
   Thr Val Lys Ile Met Asp Pro Gly Val Pro Ser Asn Val Thr Val Glu
           275                 280                 285
   Asp Val Arg Lys Lys Leu Ser Asn His Ala Val Val Val Pro Glu Ser
       290                 295                 300
   Phe Asn Asp Ser Phe Leu Thr Gln Ser Glu Asp Val Ile Ser Leu Asp
   305                 310                 315                 320
   Glu Leu Ala Arg Pro Thr Ala Thr Ser Val Lys Lys Ile Val Arg Lys
                   325                 330                 335
   Val Pro Pro Gln Lys Asp Leu Thr Gly Leu Lys Ile Thr Leu Glu Gln
               340                 345                 350
   Leu Ala Lys Asp Cys Ile Ser Lys Pro Lys Met Arg Glu Asp Tyr Leu
           355                 360                 365
   Leu Lys Ile Asn Gln Ala Ser Ser Glu Ala Gln Leu Ile Asp Leu Lys
       370                 375                 380
   Lys Ala Ile Ile Arg Ser Ala Ile
   385                 390
<210> 9
   <211> 1131
   <212> DNA
   <213> Cryptovirus
<220>
   <221> CDS
   <222> (1) ... (1131)
   <223> Crypotovirus M protein encoding sequence; cDNA in mRNA sense
<400> 9
   atg cca tcc atc agc att ccc gca gac ccc acc aat cca cgt caa tca     48
   Met Pro Ser Ile Ser Ile Pro Ala Asp Pro Thr Asn Pro Arg Gln Ser
    1               5                  10                  15
   ata aaa gcg ttc cca att gtg att aac agt gat ggg ggt gag aaa ggc     96
   Ile Lys Ala Phe Pro Ile Val Ile Asn Ser Asp Gly Gly Glu Lys Gly
               20                  25                  30
   cgc ttg gtt aaa caa cta cgt aca acc tac ttg aat gac cta gat act    144
   Arg Leu Val Lys Gln Leu Arg Thr Thr Tyr Leu Asn Asp Leu Asp Thr
           35                  40                  45
   cat gag cca ctg gtg aca ttc gta aat acc tat gga ttc atc tac gaa    192
   His Glu Pro Leu Val Thr Phe Val Asn Thr Tyr Gly Phe Ile Tyr Glu
       50                  55                  60
   cag aat cgg ggg aat gcc att gtc gga gag gat caa ctt ggg aag aaa    240
   Gln Asn Arg Gly Asn Ala Ile Val Gly Glu Asp Gln Leu Gly Lys Lys
   65                  70                  75                  80
   aga gag gct gtg act gct gca atg gtt acc ctt gga tgt ggg cct aat    288
   Arg Glu Ala Val Thr Ala Ala Met Val Thr Leu Gly Cys Gly Pro Asn
                   85                  90                  95
   cta cca tca tta ggg aat gtc ctg aga caa ctg agt gaa ttc caa gtc    336
   Leu Pro Ser Leu Gly Asn Val Leu Arg Gln Leu Ser Glu Phe Gln Val
               100                 105                 110
   att gtt agg aag aca tcc agc aaa gcg gaa gag atg gtc ttt gaa att    384
   Ile Val Arg Lys Thr Ser Ser Lys Ala Glu Glu Met Val Phe Glu Ile
           115                 120                 125
   gtt aag tat ccg aga ata ttt cgg ggt cat aca tta atc cag aaa gga    432
   Val Lys Tyr Pro Arg Ile Phe Arg Gly His Thr Leu Ile Gln Lys Gly
       130                 135                 140
   cta gtc tgt gtc tcc gca gaa aaa ttt gtt aag tca cca ggg aaa gta    480
   Leu Val Cys Val Ser Ala Glu Lys Phe Val Lys Ser Pro Gly Lys Val
   145                 150                 155                 160
   caa tct gga atg gac tat ctc ttc att ccg aca ttt ctg tca gtg act    528
   Gln Ser Gly Met Asp Tyr Leu Phe Ile Pro Thr Phe Leu Ser Val Thr
                   165                 170                 175
   tat tgt cca gct gca atc aaa ttt cag gta cct ggc ccc atg ttg aaa    576
   Tyr Cys Pro Ala Ala Ile Lys Phe Gln Val Pro Gly Pro Met Leu Lys
               180                 185                 190
   atg agg tca aga tac act cag agc tta caa ctt gaa cta atg ata aga    624
   Met Arg Ser Arg Tyr Thr Gln Ser Leu Gln Leu Glu Leu Met Ile Arg
           195                 200                 205
   atc ctg tgt aag ccc gat tcg cca ctt atg aag gtc cat atc cct gac    672
   Ile Leu Cys Lys Pro Asp Ser Pro Leu Met Lys Val His Ile Pro Asp
       210                 215                 220
   aag gag gga aga gga tgt ctt gta tca gta tgg ctg cat gta tgc aac    720
   Lys Glu Gly Arg Gly Cys Leu Val Ser Val Trp Leu His Val Cys Asn
   225                 230                 235                 240
   atc ttc aaa tca gga aac aag aat ggc agt gag tgg cag gaa tac tgg    768
   Ile Phe Lys Ser Gly Asn Lys Asn Gly Ser Glu Trp Gln Glu Tyr Trp
                   245                 250                 255
   atg aga aag tgt gct aac atg caa ctt gaa gtg tcg att gca gat atg    816
   Met Arg Lys Cys Ala Asn Met Gln Leu Glu Val Ser Ile Ala Asp Met
               260                 265                 270
   tgg gga cca act atc ata att cat gcc aga ggt cac att ccc aaa agt    864
   Trp Gly Pro Thr Ile Ile Ile His Ala Arg Gly His Ile Pro Lys Ser
           275                 280                 285
   gct aag ttg ttt ttt gga aag ggt gga tgg agc tgc cat cca ctt cac    912
   Ala Lys Leu Phe Phe Gly Lys Gly Gly Trp Ser Cys His Pro Leu His
       290                 295                 300
   gaa att gtt cca agt gtc act aaa aca cta tgg tcc gta ggt tgt gaa    960
   Glu Ile Val Pro Ser Val Thr Lys Thr Leu Trp Ser Val Gly Cys Glu
   305                 310                 315                 320
   att aca aag gcg aag gca ata ata caa gag agt agc atc tct ctt ctc    1008
   Ile Thr Lys Ala Lys Ala Ile Ile Gln Glu Ser Ser Ile Ser Leu Leu
                   325                 330                 335
   gtg gag act act gac atc ata agt cca aaa gtt aaa att tca tct aag    1056
   Val Glu Thr Thr Asp Ile Ile Ser Pro Lys Val Lys Ile Ser Ser Lys
               340                 345                 350
   cat cgc cgc ttt ggg aaa tca aat tgg ggt ctg ttc aag aaa act aaa    1104
   His Arg Arg Phe Gly Lys Ser Asn Trp Gly Leu Phe Lys Lys Thr Lys
           355                 360                 365
   tca cta cct aac cta acg gag ctg gaa                                1131
   Ser Leu Pro Asn Leu Thr Glu Leu Glu
   370 375
<210> 10
   <211> 377
   <212> PRT
   <213> Cryptovirus M Protein
<400> 10
   Met Pro Ser Ile Ser Ile Pro Ala Asp Pro Thr Asn Pro Arg Gln Ser
    1               5                  10                  15
   Ile Lys Ala Phe Pro Ile Val Ile Asn Ser Asp Gly Gly Glu Lys Gly
               20                  25                  30
   Arg Leu Val Lys Gln Leu Arg Thr Thr Tyr Leu Asn Asp Leu Asp Thr
           35                  40                  45
   His Glu Pro Leu Val Thr Phe Val Asn Thr Tyr Gly Phe Ile Tyr Glu
       50                  55                  60
   Gln Asn Arg Gly Asn Ala Ile Val Gly Glu Asp Gln Leu Gly Lys Lys
   65                  70                  75                  80
   Arg Glu Ala Val Thr Ala Ala Met Val Thr Leu Gly Cys Gly Pro Asn
                   85                  90                  95
   Leu Pro Ser Leu Gly Asn Val Leu Arg Gln Leu Ser Glu Phe Gln Val
               100                 105                 110
   Ile Val Arg Lys Thr Ser Ser Lys Ala Glu Glu Met Val Phe Glu Ile
           115                 120                 125
   Val Lys Tyr Pro Arg Ile Phe Arg Gly His Thr Leu Ile Gln Lys Gly
       130                 135                 140
   Leu Val Cys Val Ser Ala Glu Lys Phe Val Lys Ser Pro Gly Lys Val
   145                 150                 155                 160
   Gln Ser Gly Met Asp Tyr Leu Phe Ile Pro Thr Phe Leu Ser Val Thr
                   165                 170                 175
   Tyr Cys Pro Ala Ala Ile Lys Phe Gln Val Pro Gly Pro Met Leu Lys
               180                 185                 190
   Met Arg Ser Arg Tyr Thr Gln Ser Leu Gln Leu Glu Leu Met Ile Arg
           195                 200                 205
   Ile Leu Cys Lys Pro Asp Ser Pro Leu Met Lys Val His Ile Pro Asp
       210                 215                 220
   Lys Glu Gly Arg Gly Cys Leu Val Ser Val Trp Leu His Val Cys Asn
   225                 230                 235                 240
   Ile Phe Lys Ser Gly Asn Lys Asn Gly Ser Glu Trp Gln Glu Tyr Trp
                   245                 250                 255
   Met Arg Lys Cys Ala Asn Met Gln Leu Glu Val Ser Ile Ala Asp Met
               260                 265                 270
   Trp Gly Pro Thr Ile Ile Ile His Ala Arg Gly His Ile Pro Lys Ser
           275                 280                 285
   Ala Lys Leu Phe Phe Gly Lys Gly Gly Trp Ser Cys His Pro Leu His
       290                 295                 300
   Glu Ile Val Pro Ser Val Thr Lys Thr Leu Trp Ser Val Gly Cys Glu
   305                 310                 315                 320
   Ile Thr Lys Ala Lys Ala Ile Ile Gln Glu Ser Ser Ile Ser Leu Leu
                   325                 330                 335
   Val Glu Thr Thr Asp Ile Ile Ser Pro Lys Val Lys Ile Ser Ser Lys
               340                 345                 350
   His Arg Arg Phe Gly Lys Ser Asn Trp Gly Leu Phe Lys Lys Thr Lys
           355                 360                 365
   Ser Leu Pro Asn Leu Thr Glu Leu Glu
       370                 375
<210> 11
   <211> 1653
   <212> DNA
   <213> Cryptovirus
<220>
   <221> CDS
   <222> (1)...(1653)
   <223> Cryptovirus F protein encoding sequence; cDNA in mRNA sense
<400> 11
   atg agc act ata att caa tct ctg gtg gtc tcc tgt cta ttg gca gga      48
   Met Ser Thr Ile Ile Gln Ser Leu Val Val Ser Cys Leu Leu Ala Gly
    1               5                  10                  15
   gca ggc agc ctt gat cca gca gcc ctc atg caa atc ggt gtc att cca      96
   Ala Gly Ser Leu Asp Pro Ala Ala Leu Met Gln Ile Gly Val Ile Pro
               20                  25                  30
   aca aat gtc cgg caa ctt atg tat tat act gag gcc tca tca gca ttc     144
   Thr Asn Val Arg Gln Leu Met Tyr Tyr Thr Glu Ala Ser Ser Ala Phe
           35                  40                  45
   att gtt gtg aag tta atg cct aca att gac tcg ccg att agt gga tgt     192
   Ile Val Val Lys Leu Met Pro Thr Ile Asp Ser Pro Ile Ser Gly Cys
       50                  55                  60
   aat ata aca tca att tca agc tat aat gca aca gtg aca aaa ctc cta     240
   Asn Ile Thr Ser Ile Ser Ser Tyr Asn Ala Thr Val Thr Lys Leu Leu
   65                  70                  75                  80
   cag ccg atc ggt gag aat ttg gag acg att agg aac cag ttg att cca     288
   Gln Pro Ile Gly Glu Asn Leu Glu Thr Ile Arg Asn Gln Leu Ile Pro
                   85                  90                  95
   act cgg agg aga cgc cgg ttt gca ggg gtg gtg att gga tta gct gca     336
   Thr Arg Arg Arg Arg Arg Phe Ala Gly Val Val Ile Gly Leu Ala Ala
               100                 105                 110
   tta gga gta gct act gcc gca caa gtc act gcc gca gta gca cta gtt     384
   Leu Gly Val Ala Thr Ala Ala Gln Val Thr Ala Ala Val Ala Leu Val
           115                 120                 125
   aag gca aat gaa aat act gcg gct ata ctc aat ctc aaa aat gca atc     432
   Lys Ala Asn Glu Asn Thr Ala Ala Ile Leu Asn Leu Lys Asn Ala Ile
       130                 135                 140
   caa aaa aca aat gca gca gtt gca gat gtg gtc cag gcc aca caa tca     480
   Gln Lys Thr Asn Ala Ala Val Ala Asp Val Val Gln Ala Thr Gln Ser
   145                 150                 155                 160
   cta gga acg gca gtt caa gca gtt caa gat cac ata aac agt gtg ata     528
   Leu Gly Thr Ala Val Gln Ala Val Gln Asp His Ile Asn Ser Val Ile
                   165                 170                 175
   agt cca gca att aca gca gcc aat tgt aag gcc caa gat gct atc att     576
   Ser Pro Ala Ile Thr Ala Ala Asn Cys Lys Ala Gln Asp Ala Ile Ile
               180                 185                 190
   ggc tca atc ctc aat ctc tat ttg acc gag ttg aca act atc ttc cac     624
   Gly Ser Ile Leu Asn Leu Tyr Leu Thr Glu Leu Thr Thr Ile Phe His
           195                 200                 205
   aat caa att aca aac cct gca ttg agt cct att aca att caa gct tta     672
   Asn Gln Ile Thr Asn Pro Ala Leu Ser Pro Ile Thr Ile Gln Ala Leu
       210                 215                 220
   agg atc cta ctg ggg agt acc ttg ccg act gtg gtc gaa aaa tct ttc     720
   Arg Ile Leu Leu Gly Ser Thr Leu Pro Thr Val Val Glu Lys Ser Phe
   225                 230                 235                 240
   aat acc cag ata agt gca gct gag ctt ctc tca tca ggg ttg ttg aca     768
   Asn Thr Gln Ile Ser Ala Ala Glu Leu Leu Ser Ser Gly Leu Leu Thr
                   245                 250                 255
   ggc cag att gtg gga tta gat ttg acc tat atg cag atg gtc ata aaa     816
   Gly Gln Ile Val Gly Leu Asp Leu Thr Tyr Met Gln Met Val Ile Lys
               260                 265                 270
   att gag ctg cca act tta act gta caa cct gca acc cag atc ata gat     864
   Ile Glu Leu Pro Thr Leu Thr Val Gln Pro Ala Thr Gln Ile Ile Asp
           275                 280                 285
   ctg gcc acc att tct gca ttc att aac aat caa gaa gtc atg gcc caa     912
   Leu Ala Thr Ile Ser Ala Phe Ile Asn Asn Gln Glu Val Met Ala Gln
       290                 295                 300
   tta cca aca cgt gtt att gtg act ggc agc ttg atc caa gcc tat ccc     960
   Leu Pro Thr Arg Val Ile Val Thr Gly Ser Leu Ile Gln Ala Tyr Pro
   305                 310                 315                 320
   gca tcg caa tgc act att aca ccc aac act gtg tac tgt agg tat aat    1008
   Ala Ser Gln Cys Thr Ile Thr Pro Asn Thr Val Tyr Cys Arg Tyr Asn
                   325                 330                 335
   gat gcc caa gta ctc tca gat gat acg atg gct tgc ctc caa ggt aac    1056
   Asp Ala Gln Val Leu Ser Asp Asp Thr Met Ala Cys Leu Gln Gly Asn
               340                 345                 350
   ttg aca aga tgc acc ttc tct cca gtg gtt ggg agc ttt ctc act cga    1104
   Leu Thr Arg Cys Thr Phe Ser Pro Val Val Gly Ser Phe Leu Thr Arg
           355                 360                 365
   ttc gtg ctg ttc gat gga ata gtt tat gca aat tgc agg tcg atg ctg    1152
   Phe Val Leu Phe Asp Gly Ile Val Tyr Ala Asn Cys Arg Ser Met Leu
               370                 375                 380
   tgc aag tgc atg caa cct gct gcc gtg atc cta cag ccg agt tca tcc      1200
   Cys Lys Cys Met Gln Pro Ala Ala Val Ile Leu Gln Pro Ser Ser Ser
   385                 390                 395                 400
   cct gta act gtc att gac atg cac aaa tgt gtg agt ctg cag ctt gac      1248
   Pro Val Thr Val Ile Asp Met His Lys Cys Val Ser Leu Gln Leu Asp
                   405                 410                 415
   gat ctc aga ttc acc atc act caa ttg gcc aat gta acc tac aat agc      1296
   Asp Leu Arg Phe Thr Ile Thr Gln Leu Ala Asn Val Thr Tyr Asn Ser
               420                 425                 430
   acc atc aag ctt gaa aca tcc cag atc ttg cct att gat ccg ttg gat      1344
   Thr Ile Lys Leu Glu Thr Ser Gln Ile Leu Pro Ile Asp Pro Leu Asp
           435                 440                 445
   ata tcc cag aat tta gct gcg gtg aat aag agt cta agt gat gca cta      1392
   Ile Ser Gln Asn Leu Ala Ala Val Asn Lys Ser Leu Ser Asp Ala Leu
       450                 455                 460
   caa cac tta gca caa agt gac aca tac ctt tct gca atc aca tca gct      1440
   Gln His Leu Ala Gln Ser Asp Thr Tyr Leu Ser Ala Ile Thr Ser Ala
   465                 470                 475                 480
   acg act aca agt gta tta tcc ata ata gca atc tgt ctt gga tcg tta      1488
   Thr Thr Thr Ser Val Leu Ser Ile Ile Ala Ile Cys Leu Gly Ser Leu
                   485                 490                 495
   ggt tta ata tta ata atc ttg ctc agt gta gtt gtg tgg aag tta ttg      1536
   Gly Leu Ile Leu Ile Ile Leu Leu Ser Val Val Val Trp Lys Leu Leu
               500                 505                 510
   acc att gtc gct gct aat cga aat aga atg gag aat ttt gtt tat cat      1584
   Thr Ile Val Ala Ala Asn Arg Asn Arg Met Glu Asn Phe Val Tyr His
           515                 520                 525
   aat tca gca ttc cac cac cca cga tct gat ctc agt gag aaa aat caa      1632
   Asn Ser Ala Phe His His Pro Arg Ser Asp Leu Ser Glu Lys Asn Gln
       530                 535                 540
   cct gca act ctt gga aca aga                                          1653
   Pro Ala Thr Leu Gly Thr Arg
   545                 550
<210> 12
   <211> 551
   <212> PRT
   <213> Cryptovirus F protein
<400> 12
   Met Ser Thr Ile Ile Gln Ser Leu Val Val Ser Cys Leu Leu Ala Gly
    1               5                  10                  15
   Ala Gly Ser Leu Asp Pro Ala Ala Leu Met Gln Ile Gly Val Ile Pro
   20 25 30
   Thr Asn Val Arg Gln Leu Met Tyr Tyr Thr Glu Ala Ser Ser Ala Phe
   35 40 45
   Ile Val Val Lys Leu Met Pro Thr Ile Asp Ser Pro Ile Ser Gly Cys
   50 55 60
   Asn Ile Thr Ser Ile Ser Ser Tyr Asn Ala Thr Val Thr Lys Leu Leu
   65 70 75 80
   Gln Pro Ile Gly Glu Asn Leu Glu Thr Ile Arg Asn Gln Leu Ile Pro
   85 90 95
   Thr Arg Arg Arg Arg Arg Phe Ala Gly Val Val Ile Gly Leu Ala Ala
   100 105 110
   Leu Gly Val Ala Thr Ala Ala Gln Val Thr Ala Ala Val Ala Leu Val
   115 120 125
   Lys Ala Asn Glu Asn Thr Ala Ala Ile Leu Asn Leu Lys Asn Ala Ile
   130 135 140
   Gln Lys Thr Asn Ala Ala Val Ala Asp Val Val Gln Ala Thr Gln Ser
   145 150 155 160
   Leu Gly Thr Ala Val Gln Ala Val Gln Asp His Ile Asn Ser Val Ile
   165 170 175
   Ser Pro Ala Ile Thr Ala Ala Asn Cys Lys Ala Gln Asp Ala Ile Ile
   180 185 190
   Gly Ser Ile Leu Asn Leu Tyr Leu Thr Glu Leu Thr Thr Ile Phe His
   195 200 205
   Asn Gln Ile Thr Asn Pro Ala Leu Ser Pro Ile Thr Ile Gln Ala Leu
   210 215 220
   Arg Ile Leu Leu Gly Ser Thr Leu Pro Thr Val Val Glu Lys Ser Phe
   225 230 235 240
   Asn Thr Gln Ile Ser Ala Ala Glu Leu Leu Ser Ser Gly Leu Leu Thr
   245 250 255
   Gly Gln Ile Val Gly Leu Asp Leu Thr Tyr Met Gln Met Val Ile Lys
   260 265 270
   Ile Glu Leu Pro Thr Leu Thr Val Gln Pro Ala Thr Gln Ile Ile Asp
   275 280 285
   Leu Ala Thr Ile Ser Ala Phe Ile Asn Asn Gln Glu Val Met Ala Gln
   290 295 300
   Leu Pro Thr Arg Val Ile Val Thr Gly Ser Leu Ile Gln Ala Tyr Pro
   305 310 315 320
   Ala Ser Gln Cys Thr Ile Thr Pro Asn Thr Val Tyr Cys Arg Tyr Asn
   325 330 335
   Asp Ala Gln Val Leu Ser Asp Asp Thr Met Ala Cys Leu Gln Gly Asn
   340 345 350
   Leu Thr Arg Cys Thr Phe Ser Pro Val Val Gly Ser Phe Leu Thr Arg
   355 360 365
   Phe Val Leu Phe Asp Gly Ile Val Tyr Ala Asn Cys Arg Ser Met Leu
   370 375 380
   Cys Lys Cys Met Gln Pro Ala Ala Val Ile Leu Gln Pro Ser Ser Ser
   385 390 395 400
   Pro Val Thr Val Ile Asp Met His Lys Cys Val Ser Leu Gln Leu Asp
   405 410 415
   Asp Leu Arg Phe Thr Ile Thr Gln Leu Ala Asn Val Thr Tyr Asn Ser
   420 425 430
   Thr Ile Lys Leu Glu Thr Ser Gln Ile Leu Pro Ile Asp Pro Leu Asp
   435 440 445
   Ile Ser Gln Asn Leu Ala Ala Val Asn Lys Ser Leu Ser Asp Ala Leu
       450                 455                 460
   Gln His Leu Ala Gln Ser Asp Thr Tyr Leu Ser Ala Ile Thr Ser Ala
   465                 470                 475                 480
   Thr Thr Thr Ser Val Leu Ser Ile Ile Ala Ile Cys Leu Gly Ser Leu
                   485                 490                 495
   Gly Leu Ile Leu Ile Ile Leu Leu Ser Val Val Val Trp Lys Leu Leu
               500                 505                 510
   Thr Ile Val Ala Ala Asn Arg Asn Arg Met Glu Asn Phe Val Tyr His
           515                 520                 525
   Asn Ser Ala Phe His His Pro Arg Ser Asp Leu Ser Glu Lys Asn Gln
       530                 535                 540
   Pro Ala Thr Leu Gly Thr Arg
   545                 550
<210> 13
   <211> 1596
   <212> DNA
   <213> Cryptovirus
<220>
   <221> CDS
   <222> (1)...(1596)
   <223> Cryptovirus F0 protein encoding sequence; cDNA in mRNA sense
<400> 13
   ctt gat cca gca gcc ctc atg caa atc ggt gtc att cca aca aat gtc    48
   Leu Asp Pro Ala Ala Leu Met Gln Ile Gly Val Ile Pro Thr Asn Val
    1               5                  10                  15
   cgg caa ctt atg tat tat act gag gcc tca tca gca ttc att gtt gtg    96
   Arg Gln Leu Met Tyr Tyr Thr Glu Ala Ser Ser Ala Phe Ile Val Val
                   20                  25                  30
   aag tta atg cct aca att gac tcg ccg att agt gga tgt aat ata aca    144
   Lys Leu Met Pro Thr Ile Asp Ser Pro Ile Ser Gly Cys Asn Ile Thr
               35                  40                  45
   tca att tca agc tat aat gca aca gtg aca aaa ctc cta cag ccg atc    192
   Ser Ile Ser Ser Tyr Asn Ala Thr Val Thr Lys Leu Leu Gln Pro Ile
       50                  55                  60
   ggt gag aat ttg gag acg att agg aac cag ttg att cca act cgg agg    240
   Gly Glu Asn Leu Glu Thr Ile Arg Asn Gln Leu Ile Pro Thr Arg Arg
   65                  70                  75                  80
   aga cgc cgg ttt gca ggg gtg gtg att gga tta gct gca tta gga gta    288
   Arg Arg Arg Phe Ala Gly Val Val Ile Gly Leu Ala Ala Leu Gly Val
                   85                  90                  95
   gct act gcc gca caa gtc act gcc gca gta gca cta gtt aag gca aat    336
   Ala Thr Ala Ala Gln Val Thr Ala Ala Val Ala Leu Val Lys Ala Asn
               100                 105                 110
   gaa aat act gcg gct ata ctc aat ctc aaa aat gca atc caa aaa aca     384
   Glu Asn Thr Ala Ala Ile Leu Asn Leu Lys Asn Ala Ile Gln Lys Thr
           115                 120                 125
   aat gca gca gtt gca gat gtg gtc cag gcc aca caa tca cta gga acg     432
   Asn Ala Ala Val Ala Asp Val Val Gln Ala Thr Gln Ser Leu Gly Thr
       130                 135                 140
   gca gtt caa gca gtt caa gat cac ata aac agt gtg ata agt cca gca     480
   Ala Val Gln Ala Val Gln Asp His Ile Asn Ser Val Ile Ser Pro Ala
   145                 150                 155                 160
   att aca gca gcc aat tgt aag gcc caa gat gct atc att ggc tca atc     528
   Ile Thr Ala Ala Asn Cys Lys Ala Gln Asp Ala Ile Ile Gly Ser Ile
                   165                 170                 175
   ctc aat ctc tat ttg acc gag ttg aca act atc ttc cac aat caa att     576
   Leu Asn Leu Tyr Leu Thr Glu Leu Thr Thr Ile Phe His Asn Gln Ile
               180                 185                 190
   aca aac cct gca ttg agt cct att aca att caa gct tta agg atc cta     624
   Thr Asn Pro Ala Leu Ser Pro Ile Thr Ile Gln Ala Leu Arg Ile Leu
           195                 200                 205
   ctg ggg agt acc ttg ccg act gtg gtc gaa aaa tct ttc aat acc cag     672
   Leu Gly Ser Thr Leu Pro Thr Val Val Glu Lys Ser Phe Asn Thr Gln
       210                 215                 220
   ata agt gca gct gag ctt ctc tca tca ggg ttg ttg aca ggc cag att     720
   Ile Ser Ala Ala Glu Leu Leu Ser Ser Gly Leu Leu Thr Gly Gln Ile
   225                 230                 235                 240
   gtg gga tta gat ttg acc tat atg cag atg gtc ata aaa att gag ctg     768
   Val Gly Leu Asp Leu Thr Tyr Met Gln Met Val Ile Lys Ile Glu Leu
                   245                 250                 255
   cca act tta act gta caa cct gca acc cag atc ata gat ctg gcc acc     816
   Pro Thr Leu Thr Val Gln Pro Ala Thr Gln Ile Ile Asp Leu Ala Thr
               260                 265                 270
   att tct gca ttc att aac aat caa gaa gtc atg gcc caa tta cca aca     864
   Ile Ser Ala Phe Ile Asn Asn Gln Glu Val Met Ala Gln Leu Pro Thr
           275                 280                 285
   cgt gtt att gtg act ggc agc ttg atc caa gcc tat ccc gca tcg caa     912
   Arg Val Ile Val Thr Gly Ser Leu Ile Gln Ala Tyr Pro Ala Ser Gln
       290                 295                 300
   tgc act att aca ccc aac act gtg tac tgt agg tat aat gat gcc caa     960
   Cys Thr Ile Thr Pro Asn Thr Val Tyr Cys Arg Tyr Asn Asp Ala Gln
   305                 310                 315                 320
   gta ctc tca gat gat acg atg gct tgc ctc caa ggt aac ttg aca aga     1008
   Val Leu Ser Asp Asp Thr Met Ala Cys Leu Gln Gly Asn Leu Thr Arg
                   325                 330                 335
   tgc acc ttc tct cca gtg gtt ggg agc ttt ctc act cga ttc gtg ctg      1056
   Cys Thr Phe Ser Pro Val Val Gly Ser Phe Leu Thr Arg Phe Val Leu
               340                 345                 350
   ttc gat gga ata gtt tat gca aat tgc agg tcg atg ctg tgc aag tgc      1104
   Phe Asp Gly Ile Val Tyr Ala Asn Cys Arg Ser Met Leu Cys Lys Cys
           355                 360                 365
   atg caa cct gct gcc gtg atc cta cag ccg agt tca tcc cct gta act      1152
   Met Gln Pro Ala Ala Val Ile Leu Gln Pro Ser Ser Ser Pro Val Thr
       370                 375                 380
   gtc att gac atg cac aaa tgt gtg agt ctg cag ctt gac gat ctc aga      1200
   Val Ile Asp Met His Lys Cys Val Ser Leu Gln Leu Asp Asp Leu Arg
   385                 390                 395                 400
   ttc acc atc act caa ttg gcc aat gta acc tac aat agc acc atc aag      1248
   Phe Thr Ile Thr Gln Leu Ala Asn Val Thr Tyr Asn Ser Thr Ile Lys
                   405                 410                 415
   ctt gaa aca tcc cag atc ttg cct att gat ccg ttg gat ata tcc cag      1296
   Leu Glu Thr Ser Gln Ile Leu Pro Ile Asp Pro Leu Asp Ile Ser Gln
               420                 425                 430
   aat tta gct gcg gtg aat aag agt cta agt gat gca cta caa cac tta      1344
   Asn Leu Ala Ala Val Asn Lys Ser Leu Ser Asp Ala Leu Gln His Leu
           435                 440                 445
   gca caa agt gac aca tac ctt tct gca atc aca tca gct acg act aca      1392
   Ala Gln Ser Asp Thr Tyr Leu Ser Ala Ile Thr Ser Ala Thr Thr Thr
       450                 455                 460
   agt gta tta tcc ata ata gca atc tgt ctt gga tcg tta ggt tta ata      1440
   Ser Val Leu Ser Ile Ile Ala Ile Cys Leu Gly Ser Leu Gly Leu Ile
   465                 470                 475                 480
   tta ata atc ttg ctc agt gta gtt gtg tgg aag tta ttg acc att gtc      1488
   Leu Ile Ile Leu Leu Ser Val Val Val Trp Lys Leu Leu Thr Ile Val
                   485                 490                 495
   gct gct aat cga aat aga atg gag aat ttt gtt tat cat aat tca gca      1536
   Ala Ala Asn Arg Asn Arg Met Glu Asn Phe Val Tyr His Asn Ser Ala
               500                 505                 510
   ttc cac cac cca cga tct gat ctc agt gag aaa aat caa cct gca act      1584
   Phe His His Pro Arg Ser Asp Leu Ser Glu Lys Asn Gln Pro Ala Thr
           515                 520                 525
   ctt gga aca aga                                                      1596
   Leu Gly Thr Arg
       530
<210> 14
   <211> 532
   <212> PRT
   <213> Cryptovirus F0 protein
<400> 14
   Leu Asp Pro Ala Ala Leu Met Gln Ile Gly Val Ile Pro Thr Asn Val
   1               5                   10                  15
   Arg Gln Leu Met Tyr Tyr Thr Glu Ala Ser Ser Ala Phe Ile Val Val
               20                  25                  30
   Lys Leu Met Pro Thr Ile Asp Ser Pro Ile Ser Gly Cys Asn Ile Thr
           35                  40                  45
   Ser Ile Ser Ser Tyr Asn Ala Thr Val Thr Lys Leu Leu Gln Pro Ile
       50                  55                  60
   Gly Glu Asn Leu Glu Thr Ile Arg Asn Gln Leu Ile Pro Thr Arg Arg
   65                  70                  75                  80
   Arg Arg Arg Phe Ala Gly Val Val Ile Gly Leu Ala Ala Leu Gly Val
                   85                  90                  95
   Ala Thr Ala Ala Gln Val Thr Ala Ala Val Ala Leu Val Lys Ala Asn
               100                 105                 110
   Glu Asn Thr Ala Ala Ile Leu Asn Leu Lys Asn Ala Ile Gln Lys Thr
           115                 120                 125
   Asn Ala Ala Val Ala Asp Val Val Gln Ala Thr Gln Ser Leu Gly Thr
       130                 135                 140
   Ala Val Gln Ala Val Gln Asp His Ile Asn Ser Val Ile Ser Pro Ala
   145                 150                 155                 160
   Ile Thr Ala Ala Asn Cys Lys Ala Gln Asp Ala Ile Ile Gly Ser Ile
                   165                 170                 175
   Leu Asn Leu Tyr Leu Thr Glu Leu Thr Thr Ile Phe His Asn Gln Ile
               180                 185                 190
   Thr Asn Pro Ala Leu Ser Pro Ile Thr Ile Gln Ala Leu Arg Ile Leu
           195                 200                 205
   Leu Gly Ser Thr Leu Pro Thr Val Val Glu Lys Ser Phe Asn Thr Gln
       210                 215                 220
   Ile Ser Ala Ala Glu Leu Leu Ser Ser Gly Leu Leu Thr Gly Gln Ile
   225                 230                 235                 240
   Val Gly Leu Asp Leu Thr Tyr Met Gln Met Val Ile Lys Ile Glu Leu
                   245                 250                 255
   Pro Thr Leu Thr Val Gln Pro Ala Thr Gln Ile Ile Asp Leu Ala Thr
               260                 265                 270
   Ile Ser Ala Phe Ile Asn Asn Gln Glu Val Met Ala Gln Leu Pro Thr
           275                 280                 285
   Arg Val Ile Val Thr Gly Ser Leu Ile Gln Ala Tyr Pro Ala Ser Gln
       290                 295                 300
   Cys Thr Ile Thr Pro Asn Thr Val Tyr Cys Arg Tyr Asn Asp Ala Gln
   305                 310                 315                 320
   Val Leu Ser Asp Asp Thr Met Ala Cys Leu Gln Gly Asn Leu Thr Arg
                   325                 330                 335
   Cys Thr Phe Ser Pro Val Val Gly Ser Phe Leu Thr Arg Phe Val Leu
               340                 345                 350
   Phe Asp Gly Ile Val Tyr Ala Asn Cys Arg Ser Met Leu Cys Lys Cys
           355                 360                 365
   Met Gln Pro Ala Ala Val Ile Leu Gln Pro Ser Ser Ser Pro Val Thr
       370                 375                 380
   Val Ile Asp Met His Lys Cys Val Ser Leu Gln Leu Asp Asp Leu Arg
   385                 390                 395                 400
   Phe Thr Ile Thr Gln Leu Ala Asn Val Thr Tyr Asn Ser Thr Ile Lys
                   405                 410                 415
   Leu Glu Thr Ser Gln Ile Leu Pro Ile Asp Pro Leu Asp Ile Ser Gln
               420                 425                 430
   Asn Leu Ala Ala Val Asn Lys Ser Leu Ser Asp Ala Leu Gln His Leu
           435                 440                 445
   Ala Gln Ser Asp Thr Tyr Leu Ser Ala Ile Thr Ser Ala Thr Thr Thr
       450                 455                 460
   Ser Val Leu Ser Ile Ile Ala Ile Cys Leu Gly Ser Leu Gly Leu Ile
   465                 470                 475                 480
   Leu Ile Ile Leu Leu Ser Val Val Val Trp Lys Leu Leu Thr Ile Val
                   485                 490                 495
   Ala Ala Asn Arg Asn Arg Met Glu Asn Phe Val Tyr His Asn Ser Ala
               500                 505                 510
   Phe His His Pro Arg Ser Asp Leu Ser Glu Lys Asn Gln Pro Ala Thr
           515                 520                 525
   Leu Gly Thr Arg
       530
<210> 15
   <211> 249
   <212> DNA
   <213> Cryptovirus
<220>
   <221> CDS
   <222> (1) ... (249)
   <223> Cryptovirus F2 coding sequence; cDNA in mRNA sense
<400> 15
   ctt gat cca gca gcc ctc atg caa atc ggt gtc att cca aca aat gtc   48
   Leu Asp Pro Ala Ala Leu Met Gln Ile Gly Val Ile Pro Thr Asn Val
    1               5                  10                  15
   cgg caa ctt atg tat tat act gag gcc tca tca gca ttc att gtt gtg   96
   Arg Gln Leu Met Tyr Tyr Thr Glu Ala Ser Ser Ala Phe Ile Val Val
               20                  25                  30
   aag tta atg cct aca att gac tcg ccg att agt gga tgt aat ata aca   144
   Lys Leu Met Pro Thr Ile Asp Ser Pro Ile Ser Gly Cys Asn Ile Thr
           35                  40                  45
   tca att tca agc tat aat gca aca gtg aca aaa ctc cta cag ccg atc   192
   Ser Ile Ser Ser Tyr Asn Ala Thr Val Thr Lys Leu Leu Gln Pro Ile
       50                  55                  60
   ggt gag aat ttg gag acg att agg aac cag ttg att cca act cgg agg   240
   Gly Glu Asn Leu Glu Thr Ile Arg Asn Gln Leu Ile Pro Thr Arg Arg
   65                  70                  75                  80
   aga cgc cgg                                                       249
   Arg Arg Arg
<210> 16
   <211> 83
   <212> PRT
   <213> Cryptovirus F2 protein
<400> 16
   Leu Asp Pro Ala Ala Leu Met Gln Ile Gly Val Ile Pro Thr Asn Val
    1               5                  10                  15
   Arg Gln Leu Met Tyr Tyr Thr Glu Ala Ser Ser Ala Phe Ile Val Val
               20                  25                  30
   Lys Leu Met Pro Thr Ile Asp Ser Pro Ile Ser Gly Cys Asn Ile Thr
           35                  40                  45
   Ser Ile Ser Ser Tyr Asn Ala Thr Val Thr Lys Leu Leu Gln Pro Ile
       50                  55                  60
   Gly Glu Asn Leu Glu Thr Ile Arg Asn Gln Leu Ile Pro Thr Arg Arg
   65                  70                  75                  80
   Arg Arg Arg
<210> 17
   <211> 1347
   <212> DNA
   <213> Cryptovirus
<220>
   <221> CDS
   <222> (1)...(1347)
   <223> Cryptovirus F1 protein encoding sequence; cDNA in mRNA sense
<400> 17
   ttt gca ggg gtg gtg att gga tta gct gca tta gga gta gct act gcc      48
   Phe Ala Gly Val Val Ile Gly Leu Ala Ala Leu Gly Val Ala Thr Ala
    1               5                  10                  15
   gca caa gtc act gcc gca gta gca cta gtt aag gca aat gaa aat act      96
   Ala Gln Val Thr Ala Ala Val Ala Leu Val Lys Ala Asn Glu Asn Thr
               20                  25                  30
   gcg gct ata ctc aat ctc aaa aat gca atc caa aaa aca aat gca gca     144
   Ala Ala Ile Leu Asn Leu Lys Asn Ala Ile Gln Lys Thr Asn Ala Ala
           35                  40                  45
   gtt gca gat gtg gtc cag gcc aca caa tca cta gga acg gca gtt caa     192
   Val Ala Asp Val Val Gln Ala Thr Gln Ser Leu Gly Thr Ala Val Gln
       50                  55                  60
   gca gtt caa gat cac ata aac agt gtg ata agt cca gca att aca gca     240
   Ala Val Gln Asp His Ile Asn Ser Val Ile Ser Pro Ala Ile Thr Ala
   65                  70                  75                  80
   gcc aat tgt aag gcc caa gat gct atc att ggc tca atc ctc aat ctc     288
   Ala Asn Cys Lys Ala Gln Asp Ala Ile Ile Gly Ser Ile Leu Asn Leu
                   85                  90                  95
   tat ttg acc gag ttg aca act atc ttc cac aat caa att aca aac cct      336
   Tyr Leu Thr Glu Leu Thr Thr Ile Phe His Asn Gln Ile Thr Asn Pro
               100                 105                 110
   gca ttg agt cct att aca att caa gct tta agg atc cta ctg ggg agt      384
   Ala Leu Ser Pro Ile Thr Ile Gln Ala Leu Arg Ile Leu Leu Gly Ser
           115                 120                 125
   acc ttg ccg act gtg gtc gaa aaa tct ttc aat acc cag ata agt gca      432
   Thr Leu Pro Thr Val Val Glu Lys Ser Phe Asn Thr Gln Ile Ser Ala
       130                 135                 140
   gct gag ctt ctc tca tca ggg ttg ttg aca ggc cag att gtg gga tta      480
   Ala Glu Leu Leu Ser Ser Gly Leu Leu Thr Gly Gln Ile Val Gly Leu
   145                 150                 155                 160
   gat ttg acc tat atg cag atg gtc ata aaa att gag ctg cca act tta      528
   Asp Leu Thr Tyr Met Gln Met Val Ile Lys Ile Glu Leu Pro Thr Leu
                   165                 170                 175
   act gta caa cct gca acc cag atc ata gat ctg gcc acc att tct gca      576
   Thr Val Gln Pro Ala Thr Gln Ile Ile Asp Leu Ala Thr Ile Ser Ala
               180                 185                 190
   ttc att aac aat caa gaa gtc atg gcc caa tta cca aca cgt gtt att      624
   Phe Ile Asn Asn Gln Glu Val Met Ala Gln Leu Pro Thr Arg Val Ile
           195                 200                 205
   gtg act ggc agc ttg atc caa gcc tat ccc gca tcg caa tgc act att      672
   Val Thr Gly Ser Leu Ile Gln Ala Tyr Pro Ala Ser Gln Cys Thr Ile
       210                 215                 220
   aca ccc aac act gtg tac tgt agg tat aat gat gcc caa gta ctc tca      720
   Thr Pro Asn Thr Val Tyr Cys Arg Tyr Asn Asp Ala Gln Val Leu Ser
   225                 230                 235                 240
   gat gat acg atg gct tgc ctc caa ggt aac ttg aca aga tgc acc ttc      768
   Asp Asp Thr Met Ala Cys Leu Gln Gly Asn Leu Thr Arg Cys Thr Phe
                   245                 250                 255
   tct cca gtg gtt ggg agc ttt ctc act cga ttc gtg ctg ttc gat gga      816
   Ser Pro Val Val Gly Ser Phe Leu Thr Arg Phe Val Leu Phe Asp Gly
               260                 265                 270
   ata gtt tat gca aat tgc agg tcg atg ctg tgc aag tgc atg caa cct      864
   Ile Val Tyr Ala Asn Cys Arg Ser Met Leu Cys Lys Cys Met Gln Pro
           275                 280                 285
   gct gcc gtg atc cta cag ccg agt tca tcc cct gta act gtc att gac      912
   Ala Ala Val Ile Leu Gln Pro Ser Ser Ser Pro Val Thr Val Ile Asp
       290                 295                 300
   atg cac aaa tgt gtg agt ctg cag ctt gac gat ctc aga ttc acc atc      960
   Met His Lys Cys Val Ser Leu Gln Leu Asp Asp Leu Arg Phe Thr Ile
   305                 310                 315                 320
   act caa ttg gcc aat gta acc tac aat agc acc atc aag ctt gaa aca     1008
   Thr Gln Leu Ala Asn Val Thr Tyr Asn Ser Thr Ile Lys Leu Glu Thr
                   325                 330                 335
   tcc cag atc ttg cct att gat ccg ttg gat ata tcc cag aat tta gct     1056
   Ser Gln Ile Leu Pro Ile Asp Pro Leu Asp Ile Ser Gln Asn Leu Ala
               340                 345                 350
   gcg gtg aat aag agt cta agt gat gca cta caa cac tta gca caa agt     1104
   Ala Val Asn Lys Ser Leu Ser Asp Ala Leu Gln His Leu Ala Gln Ser
           355                 360                 365
   gac aca tac ctt tct gca atc aca tca gct acg act aca agt gta tta     1152
   Asp Thr Tyr Leu Ser Ala Ile Thr Ser Ala Thr Thr Thr Ser Val Leu
       370                 375                 380
   tcc ata ata gca atc tgt ctt gga tcg tta ggt tta ata tta ata atc     1200
   Ser Ile Ile Ala Ile Cys Leu Gly Ser Leu Gly Leu Ile Leu Ile Ile
   385                 390                 395                 400
   ttg ctc agt gta gtt gtg tgg aag tta ttg acc att gtc gct gct aat     1248
   Leu Leu Ser Val Val Val Trp Lys Leu Leu Thr Ile Val Ala Ala Asn
                   405                 410                 415
   cga aat aga atg gag aat ttt gtt tat cat aat tca gca ttc cac cac     1296
   Arg Asn Arg Met Glu Asn Phe Val Tyr His Asn Ser Ala Phe His His
               420                 425                 430
   cca cga tct gat ctc agt gag aaa aat caa cct gca act ctt gga aca     1344
   Pro Arg Ser Asp Leu Ser Glu Lys Asn Gln Pro Ala Thr Leu Gly Thr
           435                 440                 445
   aga                                                                 1347
   Arg
<210> 18
   <211> 449
   <212> PRT
   <213> Cryptovirus
<400> 18
   Phe Ala Gly Val Val Ile Gly Leu Ala Ala Leu Gly Val Ala Thr Ala
   1               5                   10                  15
   Ala Gln Val Thr Ala Ala Val Ala Leu Val Lys Ala Asn Glu Asn Thr
               20                  25                  30
   Ala Ala Ile Leu Asn Leu Lys Asn Ala Ile Gln Lys Thr Asn Ala Ala
           35                  40                  45
   Val Ala Asp Val Val Gln Ala Thr Gln Ser Leu Gly Thr Ala Val Gln
       50                  55                  60
   Ala Val Gln Asp His Ile Asn Ser Val Ile Ser Pro Ala Ile Thr Ala
   65                  70                  75                  80
   Ala Asn Cys Lys Ala Gln Asp Ala Ile Ile Gly Ser Ile Leu Asn Leu
                   85                  90                  95
   Tyr Leu Thr Glu Leu Thr Thr Ile Phe His Asn Gln Ile Thr Asn Pro
               100                 105                 110
   Ala Leu Ser Pro Ile Thr Ile Gln Ala Leu Arg Ile Leu Leu Gly Ser
           115                 120                 125
   Thr Leu Pro Thr Val Val Glu Lys Ser Phe Asn Thr Gln Ile Ser Ala
       130                 135                 140
   Ala Glu Leu Leu Ser Ser Gly Leu Leu Thr Gly Gln Ile Val Gly Leu
   145                 150                 155                 160
   Asp Leu Thr Tyr Met Gln Met Val Ile Lys Ile Glu Leu Pro Thr Leu
                   165                 170                 175
   Thr Val Gln Pro Ala Thr Gln Ile Ile Asp Leu Ala Thr Ile Ser Ala
               180                 185                 190
   Phe Ile Asn Asn Gln Glu Val Met Ala Gln Leu Pro Thr Arg Val Ile
           195                 200                 205
   Val Thr Gly Ser Leu Ile Gln Ala Tyr Pro Ala Ser Gln Cys Thr Ile
       210                 215                 220
   Thr Pro Asn Thr Val Tyr Cys Arg Tyr Asn Asp Ala Gln Val Leu Ser
   225                 230                 235                 240
   Asp Asp Thr Met Ala Cys Leu Gln Gly Asn Leu Thr Arg Cys Thr Phe
                   245                 250                 255
   Ser Pro Val Val Gly Ser Phe Leu Thr Arg Phe Val Leu Phe Asp Gly
               260                 265                 270
   Ile Val Tyr Ala Asn Cys Arg Ser Met Leu Cys Lys Cys Met Gln Pro
           275                 280                 285
   Ala Ala Val Ile Leu Gln Pro Ser Ser Ser Pro Val Thr Val Ile Asp
       290                 295                 300
   Met His Lys Cys Val Ser Leu Gln Leu Asp Asp Leu Arg Phe Thr Ile
   305                 310                 315                 320
   Thr Gln Leu Ala Asn Val Thr Tyr Asn Ser Thr Ile Lys Leu Glu Thr
                   325                 330                 335
   Ser Gln Ile Leu Pro Ile Asp Pro Leu Asp Ile Ser Gln Asn Leu Ala
               340                 345                 350
   Ala Val Asn Lys Ser Leu Ser Asp Ala Leu Gln His Leu Ala Gln Ser
           355                 360                 365
   Asp Thr Tyr Leu Ser Ala Ile Thr Ser Ala Thr Thr Thr Ser Val Leu
       370                 375                 380
   Ser Ile Ile Ala Ile Cys Leu Gly Ser Leu Gly Leu Ile Leu Ile Ile
   385                 390                 395                 400
   Leu Leu Ser Val Val Val Trp Lys Leu Leu Thr Ile Val Ala Ala Asn
                   405                 410                 415
   Arg Asn Arg Met Glu Asn Phe Val Tyr His Asn Ser Ala Phe His His
               420                 425                 430
   Pro Arg Ser Asp Leu Ser Glu Lys Asn Gln Pro Ala Thr Leu Gly Thr
           435                 440                 445
   Arg
<210> 19
   <211> 132
   <212> DNA
   <213> Cryptovirus
<220>
   <221> CDS
   <222> (1)...(132)
   <223> Cryptovirus SH protein encoding sequence; cDNA in mRNA sense
<400> 19
   atg ctg cct gat ccg gaa gat ccg gaa agc aaa aaa gct aca agg aga    48
   Met Leu Pro Asp Pro Glu Asp Pro Glu Ser Lys Lys Ala Thr Arg Arg
    1               5                  10                  15
   aca gga aac cta att atc tgc ttc cta ttc atc ttc ttt ctg ttt gta    96
   Thr Gly Asn Leu Ile Ile Cys Phe Leu Phe Ile Phe Phe Leu Phe Val
               20                  25                  30
   acc ctc att gtt cca act cta aga cac ttg cta tct                   132
   Thr Leu Ile Val Pro Thr Leu Arg His Leu Leu Ser
           35                  40
<210> 20
   <211> 44
   <212> PRT
   <213> Cryptovirus
<400> 20
   Met Leu Pro Asp Pro Glu Asp Pro Glu Ser Lys Lys Ala Thr Arg Arg
    1               5                  10                  15
   Thr Gly Asn Leu Ile Ile Cys Phe Leu Phe Ile Phe Phe Leu Phe Val
               20                  25                  30
   Thr Leu Ile Val Pro Thr Leu Arg His Leu Leu Ser
           35                  40
<210> 21
   <211> 1695
   <212> DNA
   <213> Cryptovirus
<220>
   <221> CDS
   <222> (1)...(1695)
   <223> Cryptovirus HN protein encoding sequence; cDNA in mRNA sense
<400> 21
   atg att gca gaa gat gcc cct gtt aag ggc act tgc cga gta tta ttt    48
   Met Ile Ala Glu Asp Ala Pro Val Lys Gly Thr Cys Arg Val Leu Phe
    1               5                  10                  15
   cgg aca aca act tta att ttt cta tgc aca cta cta gca tta agc atc    96
   Arg Thr Thr Thr Leu Ile Phe Leu Cys Thr Leu Leu Ala Leu Ser Ile
               20                  25                  30
   tct atc ctt tat gag agt tta ata acc caa aag caa atc atg agt caa      144
   Ser Ile Leu Tyr Glu Ser Leu Ile Thr Gln Lys Gln Ile Met Ser Gln
           35                  40                  45
   gca ggc tca act gga tct aat tct gga tta gga ggt att act gat ctt      192
   Ala Gly Ser Thr Gly Ser Asn Ser Gly Leu Gly Gly Ile Thr Asp Leu
       50                  55                  60
   ctt aat aat att ctt tct gtc gca aat cag att ata tat aac tct gca      240
   Leu Asn Asn Ile Leu Ser Val Ala Asn Gln Ile Ile Tyr Asn Ser Ala
   65                  70                  75                  80
   gtc gct cta cct cta caa ttg gac act ctt gaa tca aca ctc ctt aca      288
   Val Ala Leu Pro Leu Gln Leu Asp Thr Leu Glu Ser Thr Leu Leu Thr
                   85                  90                  95
   gcc att aag tct ctt caa acc agt gac aag cta gaa cag aac tgc tcg      336
   Ala Ile Lys Ser Leu Gln Thr Ser Asp Lys Leu Glu Gln Asn Cys Ser
               100                 105                 110
   tgg ggt gct gca ctg att aat gat aat aga tac att aat ggc atc aat      384
   Trp Gly Ala Ala Leu Ile Asn Asp Asn Arg Tyr Ile Asn Gly Ile Asn
           115                 120                 125
   cag ttc tat ttc tca att gct gag ggt cgc aat ctg aca ctt ggc cca      432
   Gln Phe Tyr Phe Ser Ile Ala Glu Gly Arg Asn Leu Thr Leu Gly Pro
       130                 135                 140
   ctt ctt aat ata cct agt ttc att cca act gcc acg aca cca gag ggc      480
   Leu Leu Asn Ile Pro Ser Phe Ile Pro Thr Ala Thr Thr Pro Glu Gly
   145                 150                 155                 160
   tgc acc agg atc cca tca ttc tcg ctc act aag aca cac tgg tgt tat      528
   Cys Thr Arg Ile Pro Ser Phe Ser Leu Thr Lys Thr His Trp Cys Tyr
                   165                 170                 175
   acg cac aat gtt atc ctg aat gga tgc cag gat cat gta tcc tca aat      576
   Thr His Asn Val Ile Leu Asn Gly Cys Gln Asp His Val Ser Ser Asn
               180                 185                 190
   caa ttt gtt tcc atg gga atc att gaa ccc act tct gcc ggg ttt cca      624
   Gln Phe Val Ser Met Gly Ile Ile Glu Pro Thr Ser Ala Gly Phe Pro
           195                 200                 205
   tcc ttt cga acc tta aag act cta tat ctc agc gat ggg gtc aat cgt      672
   Ser Phe Arg Thr Leu Lys Thr Leu Tyr Leu Ser Asp Gly Val Asn Arg
       210                 215                 220
   aag agc tgc tct atc agt aca gtt ccg ggg ggt tgt atg atg tac tgt      720
   Lys Ser Cys Ser Ile Ser Thr Val Pro Gly Gly Cys Met Met Tyr Cys
   225                 230                 235                 240
   ttt gtc tct act caa cca gag agg gat gac tac ttt tct acc gct cct      768
   Phe Val Ser Thr Gln Pro Glu Arg Asp Asp Tyr Phe Ser Thr Ala Pro
                   245                 250                 255
   cca gaa caa cga att att ata atg tac tat aat gat aca atc gtg gag     816
   Pro Glu Gln Arg Ile Ile Ile Met Tyr Tyr Asn Asp Thr Ile Val Glu
               260                 265                 270
   cgc ata att aat cca ccc ggg gta cta gac gta tgg gca aca ttg aac     864
   Arg Ile Ile Asn Pro Pro Gly Val Leu Asp Val Trp Ala Thr Leu Asn
           275                 280                 285
   cca gga aca gga agc ggg gta tat tat tta ggt tgg gtg ctc ttt cca     912
   Pro Gly Thr Gly Ser Gly Val Tyr Tyr Leu Gly Trp Val Leu Phe Pro
       290                 295                 300
   ata tat ggc ggc gtg att aaa aat acg agt tta tgg aat aat caa gca     960
   Ile Tyr Gly Gly Val Ile Lys Asn Thr Ser Leu Trp Asn Asn Gln Ala
   305                 310                 315                 320
   aat aaa tac ttc att ccc cag atg gtt gct gct ctc tgc tca caa aac     1008
   Asn Lys Tyr Phe Ile Pro Gln Met Val Ala Ala Leu Cys Ser Gln Asn
                   325                 330                 335
   cag gca act caa gtc caa aat gct aag tca tca tac tat agc agc tgg     1056
   Gln Ala Thr Gln Val Gln Asn Ala Lys Ser Ser Tyr Tyr Ser Ser Trp
               340                 345                 350
   ttt ggc aat cga atg att cag tct ggg atc ctg gca tgc cct ctt caa     1104
   Phe Gly Asn Arg Met Ile Gln Ser Gly Ile Leu Ala Cys Pro Leu Gln
           355                 360                 365
   cag gat cta acc aat gag tgt tta gtt ctg ccc ttt tct aat gat cag     1152
   Gln Asp Leu Thr Asn Glu Cys Leu Val Leu Pro Phe Ser Asn Asp Gln
       370                 375                 380
   gtg ctt atg ggt gct gaa ggg aga tta tat atg tat ggt gac tcg gtg     1200
   Val Leu Met Gly Ala Glu Gly Arg Leu Tyr Met Tyr Gly Asp Ser Val
   385                 390                 395                 400
   tat tac tac caa aga agc aat agt tgg tgg cct atg acc atg ctg tat     1248
   Tyr Tyr Tyr Gln Arg Ser Asn Ser Trp Trp Pro Met Thr Met Leu Tyr
                   405                 410                 415
   aag gta acc ata aca ttc act aat ggt cag cca tcc gct ata tca gct     1296
   Lys Val Thr Ile Thr Phe Thr Asn Gly Gln Pro Ser Ala Ile Ser Ala
               420                 425                 430
   cag aat gtg ccc aca cag cag gtc cct aga cct ggg aca gga gac tgc     1344
   Gln Asn Val Pro Thr Gln Gln Val Pro Arg Pro Gly Thr Gly Asp Cys
           435                 440                 445
   ttt gca acc aat aga tgt ccc ggt ttt tgc ttg aca gga gtg tat gct     1392
   Phe Ala Thr Asn Arg Cys Pro Gly Phe Cys Leu Thr Gly Val Tyr Ala
       450                 455                 460
   gat gct tgg tta ctg acc aac cct tcg tct acc agt aca ttt gga tcg     1440
   Asp Ala Trp Leu Leu Thr Asn Pro Ser Ser Thr Ser Thr Phe Gly Ser
   465                 470                 475                 480
   gaa gca acc ttc act ggt tct tat ctc aac gca gca act cag cgt atc      1488
   Glu Ala Thr Phe Thr Gly Ser Tyr Leu Asn Ala Ala Thr Gln Arg Ile
                   485                 490                 495
   aat ccg acg atg tat atc gcg aac aac aca cag atc ata agc tca cag      1536
   Asn Pro Thr Met Tyr Ile Ala Asn Asn Thr Gln Ile Ile Ser Ser Gln
               500                 505                 510
   caa ttt gga tca agc ggt caa gaa gca gca tat ggc cac aca act tgt      1584
   Gln Phe Gly Ser Ser Gly Gln Glu Ala Ala Tyr Gly His Thr Thr Cys
           515                 520                 525
   ttt agg gac aca ggc tct gtt atg gta tac tgt atc tat att att gaa      1632
   Phe Arg Asp Thr Gly Ser Val Met Val Tyr Cys Ile Tyr Ile Ile Glu
       530                 535                 540
   ttg tcc tca tct ctc tta gga caa ttt cag att gtc cca ttt atc cgt      1680
   Leu Ser Ser Ser Leu Leu Gly Gln Phe Gln Ile Val Pro Phe Ile Arg
   545                 550                 555                 560
   cag gtg aca cta tcc                                                  1695
   Gln Val Thr Leu Ser
                   565
<210> 22
   <211> 565
   <212> PRT
   <213> Cryptovirus
<400> 22
   Met Ile Ala Glu Asp Ala Pro Val Lys Gly Thr Cys Arg Val Leu Phe
   1               5                   10                  15
   Arg Thr Thr Thr Leu Ile Phe Leu Cys Thr Leu Leu Ala Leu Ser Ile
               20                  25                  30
   Ser Ile Leu Tyr Glu Ser Leu Ile Thr Gln Lys Gln Ile Met Ser Gln
           35                  40                  45
   Ala Gly Ser Thr Gly Ser Asn Ser Gly Leu Gly Gly Ile Thr Asp Leu
       50                  55                  60
   Leu Asn Asn Ile Leu Ser Val Ala Asn Gln Ile Ile Tyr Asn Ser Ala
   65                  70                  75                  80
   Val Ala Leu Pro Leu Gln Leu Asp Thr Leu Glu Ser Thr Leu Leu Thr
                   85                  90                  95
   Ala Ile Lys Ser Leu Gln Thr Ser Asp Lys Leu Glu Gln Asn Cys Ser
               100                 105                 110
   Trp Gly Ala Ala Leu Ile Asn Asp Asn Arg Tyr Ile Asn Gly Ile Asn
           115                 120                 125
   Gln Phe Tyr Phe Ser Ile Ala Glu Gly Arg Asn Leu Thr Leu Gly Pro
       130                 135                 140
   Leu Leu Asn Ile Pro Ser Phe Ile Pro Thr Ala Thr Thr Pro Glu Gly
   145                 150                 155                 160
   Cys Thr Arg Ile Pro Ser Phe Ser Leu Thr Lys Thr His Trp Cys Tyr
                   165                 170                 175
   Thr His Asn Val Ile Leu Asn Gly Cys Gln Asp His Val Ser Ser Asn
               180                 185                 190
   Gln Phe Val Ser Met Gly Ile Ile Glu Pro Thr Ser Ala Gly Phe Pro
           195                 200                 205
   Ser Phe Arg Thr Leu Lys Thr Leu Tyr Leu Ser Asp Gly Val Asn Arg
       210                 215                 220
   Lys Ser Cys Ser Ile Ser Thr Val Pro Gly Gly Cys Met Met Tyr Cys
   225                 230                 235                 240
   Phe Val Ser Thr Gln Pro Glu Arg Asp Asp Tyr Phe Ser Thr Ala Pro
                   245                 250                 255
   Pro Glu Gln Arg Ile Ile Ile Met Tyr Tyr Asn Asp Thr Ile Val Glu
               260                 265                 270
   Arg Ile Ile Asn Pro Pro Gly Val Leu Asp Val Trp Ala Thr Leu Asn
           275                 280                 285
   Pro Gly Thr Gly Ser Gly Val Tyr Tyr Leu Gly Trp Val Leu Phe Pro
       290                 295                 300
   Ile Tyr Gly Gly Val Ile Lys Asn Thr Ser Leu Trp Asn Asn Gln Ala
   305                 310                 315                 320
   Asn Lys Tyr Phe Ile Pro Gln Met Val Ala Ala Leu Cys Ser Gln Asn
                   325                 330                 335
   Gln Ala Thr Gln Val Gln Asn Ala Lys Ser Ser Tyr Tyr Ser Ser Trp
               340                 345                 350
   Phe Gly Asn Arg Met Ile Gln Ser Gly Ile Leu Ala Cys Pro Leu Gln
           355                 360                 365
   Gln Asp Leu Thr Asn Glu Cys Leu Val Leu Pro Phe Ser Asn Asp Gln
       370                 375                 380
   Val Leu Met Gly Ala Glu Gly Arg Leu Tyr Met Tyr Gly Asp Ser Val
   385                 390                 395                 400
   Tyr Tyr Tyr Gln Arg Ser Asn Ser Trp Trp Pro Met Thr Met Leu Tyr
                   405                 410                 415
   Lys Val Thr Ile Thr Phe Thr Asn Gly Gln Pro Ser Ala Ile Ser Ala
               420                 425                 430
   Gln Asn Val Pro Thr Gln Gln Val Pro Arg Pro Gly Thr Gly Asp Cys
           435                 440                 445
   Phe Ala Thr Asn Arg Cys Pro Gly Phe Cys Leu Thr Gly Val Tyr Ala
       450                 455                 460
   Asp Ala Trp Leu Leu Thr Asn Pro Ser Ser Thr Ser Thr Phe Gly Ser
   465                 470                 475                 480
   Glu Ala Thr Phe Thr Gly Ser Tyr Leu Asn Ala Ala Thr Gln Arg Ile
                   485                 490                 495
   Asn Pro Thr Met Tyr Ile Ala Asn Asn Thr Gln Ile Ile Ser Ser Gln
               500                 505                 510
   Gln Phe Gly Ser Ser Gly Gln Glu Ala Ala Tyr Gly His Thr Thr Cys
           515                 520                 525
   Phe Arg Asp Thr Gly Ser Val Met Val Tyr Cys Ile Tyr Ile Ile Glu
       530                 535                 540
   Leu Ser Ser Ser Leu Leu Gly Gln Phe Gln Ile Val Pro Phe Ile Arg
   545                 550                 555                 560
   Gln Val Thr Leu Ser
                   565
<210> 23
   <211> 6765
   <212> DNA
   <213> Cryptovirus
<220>
   <221> CDS
   <222> (1)...(6765)
   <223> Cryptovirus L protein encoding sequence; cDNA in mRNA sense
<400> 23
   atg gct ggg tct cgg gag ata tta ctc cct gaa gtc cat ctc aat tca    48
   Met Ala Gly Ser Arg Glu Ile Leu Leu Pro Glu Val His Leu Asn Ser
    1               5                  10                  15
   cca att gta aag cat aag cta tac tat tac att cta ctt gga aac ctc    96
   Pro Ile Val Lys His Lys Leu Tyr Tyr Tyr Ile Leu Leu Gly Asn Leu
               20                  25                  30
   cca aat gag atc gac att gac gat tta ggt cca tta cat aat caa aat   144
   Pro Asn Glu Ile Asp Ile Asp Asp Leu Gly Pro Leu His Asn Gln Asn
           35                  40                  45
   tgg aat caa ata gca cat gaa gag tct aac tta gct caa cgc ttg gta   192
   Trp Asn Gln Ile Ala His Glu Glu Ser Asn Leu Ala Gln Arg Leu Val
       50                  55                  60
   aat gta aga aat ttt cta att acc cac atc cct gat ctt aga aag ggc   240
   Asn Val Arg Asn Phe Leu Ile Thr His Ile Pro Asp Leu Arg Lys Gly
   65                  70                  75                  80
   cat tgg caa gag tat gta aat gta ata ctg tgg ccg cga att ctt ccc   288
   His Trp Gln Glu Tyr Val Asn Val Ile Leu Trp Pro Arg Ile Leu Pro
                   85                  90                  95
   ttg atc ccg gat ttt aaa atc aat gac caa ttg cct ctg ctc aaa aat   336
   Leu Ile Pro Asp Phe Lys Ile Asn Asp Gln Leu Pro Leu Leu Lys Asn
               100                 105                 110
   tgg gac aag tta gtt aaa gaa tca tgt tca gta atc aat gcg ggt act   384
   Trp Asp Lys Leu Val Lys Glu Ser Cys Ser Val Ile Asn Ala Gly Thr
           115                 120                 125
   tcc cag tgc att cag aat ctc agc tat gga ctg aca ggt cgt ggg aac   432
   Ser Gln Cys Ile Gln Asn Leu Ser Tyr Gly Leu Thr Gly Arg Gly Asn
       130                 135                 140
   ctc ttt aca cga tca cgt gaa ctc tct ggt gac cgc agg gat att gat   480
   Leu Phe Thr Arg Ser Arg Glu Leu Ser Gly Asp Arg Arg Asp Ile Asp
   145                 150                 155                 160
   ctt aag acg gtt gtg gca gca tgg cat gac tca gac tgg aaa aga ata   528
   Leu Lys Thr Val Val Ala Ala Trp His Asp Ser Asp Trp Lys Arg Ile
                   165                 170                 175
   agt gat ttt tgg att atg atc aaa ttc cag atg aga caa tta att gtt   576
   Ser Asp Phe Trp Ile Met Ile Lys Phe Gln Met Arg Gln Leu Ile Val
               180                 185                 190
   agg caa aca gat cat aat gat cct gat tta atc acg tat atc gaa aat     624
   Arg Gln Thr Asp His Asn Asp Pro Asp Leu Ile Thr Tyr Ile Glu Asn
           195                 200                 205
   aga gaa ggc ata atc atc ata acc cct gaa ctg gta gca tta ttt aat     672
   Arg Glu Gly Ile Ile Ile Ile Thr Pro Glu Leu Val Ala Leu Phe Asn
       210                 215                 220
   act gag aat cat aca cta aca tac atg acc ttt gaa att gta ctg atg     720
   Thr Glu Asn His Thr Leu Thr Tyr Met Thr Phe Glu Ile Val Leu Met
   225                 230                 235                 240
   gtt tca gat atg tac gaa ggt cgt cac aac att tta tca cta tgc aca     768
   Val Ser Asp Met Tyr Glu Gly Arg His Asn Ile Leu Ser Leu Cys Thr
                   245                 250                 255
   gtt agc act tac ctg aat cct ctg aag aaa aga ata aca tat tta ttg     816
   Val Ser Thr Tyr Leu Asn Pro Leu Lys Lys Arg Ile Thr Tyr Leu Leu
               260                 265                 270
   agc ctt gta gat aac tta gct ttt cag ata ggt gat gct gta tat aac     864
   Ser Leu Val Asp Asn Leu Ala Phe Gln Ile Gly Asp Ala Val Tyr Asn
           275                 280                 285
   ata att gct ttg cta gaa tcc ttt gta tat gca cag ttg caa atg tca     912
   Ile Ile Ala Leu Leu Glu Ser Phe Val Tyr Ala Gln Leu Gln Met Ser
       290                 295                 300
   gat ccc atc cca gaa ctc aga gga caa ttc cat gca ttc gta tgt tct     960
   Asp Pro Ile Pro Glu Leu Arg Gly Gln Phe His Ala Phe Val Cys Ser
   305                 310                 315                 320
   gag att ctt gat gca cta agg gga act aat agt ttc acc cag gat gaa    1008
   Glu Ile Leu Asp Ala Leu Arg Gly Thr Asn Ser Phe Thr Gln Asp Glu
                   325                 330                 335
   tta aga act gtg aca act aat ttg ata tcc cca ttc caa gat ctg acc    1056
   Leu Arg Thr Val Thr Thr Asn Leu Ile Ser Pro Phe Gln Asp Leu Thr
               340                 345                 350
   cca gat ctt acg gct gaa ttg ctc tgt ata atg agg ctt tgg gga cac    1104
   Pro Asp Leu Thr Ala Glu Leu Leu Cys Ile Met Arg Leu Trp Gly His
           355                 360                 365
   ccc atg ctc act gcc agt caa gct gcg gga aag gta cgc gag tct atg    1152
   Pro Met Leu Thr Ala Ser Gln Ala Ala Gly Lys Val Arg Glu Ser Met
       370                 375                 380
   tgt gct ggg aaa gta ctg gac ttt ccc act att atg aaa aca cta gcc    1200
   Cys Ala Gly Lys Val Leu Asp Phe Pro Thr Ile Met Lys Thr Leu Ala
   385                 390                 395                 400
   ttt ttc cat act att ctg atc aat gga tac agg agg aag cat cat gga    1248
   Phe Phe His Thr Ile Leu Ile Asn Gly Tyr Arg Arg Lys His His Gly
                   405                 410                 415
   gta tgg cca ccc tta aac tta cca ggt aat gct tca aag ggt ctc aca    1296
   Val Trp Pro Pro Leu Asn Leu Pro Gly Asn Ala Ser Lys Gly Leu Thr
               420                 425                 430
   gaa ctt atg aat gac aac act gag ata agc tat gaa ttc aca ctt aag    1344
   Glu Leu Met Asn Asp Asn Thr Glu Ile Ser Tyr Glu Phe Thr Leu Lys
           435                 440                 445
   cat tgg aag gaa atc tct ctt ata aaa ttc aag aaa tgt ttt gat gca    1392
   His Trp Lys Glu Ile Ser Leu Ile Lys Phe Lys Lys Cys Phe Asp Ala
       450                 455                 460
   gac gca ggt gag gaa ctc agt ata ttt atg aaa gat aaa gca att agt    1440
   Asp Ala Gly Glu Glu Leu Ser Ile Phe Met Lys Asp Lys Ala Ile Ser
   465                 470                 475                 480
   gcc cca aaa caa gac tgg atg agt gtg ttt aga aga agc cta atc aaa    1488
   Ala Pro Lys Gln Asp Trp Met Ser Val Phe Arg Arg Ser Leu Ile Lys
                   485                 490                 495
   cag cgc cat cag cat cat cag gtc ccc cta cca aat cca ttc aat cga    1536
   Gln Arg His Gln His His Gln Val Pro Leu Pro Asn Pro Phe Asn Arg
               500                 505                 510
   cgg cta ttg cta aac ttt ctc gga gat gac aaa ttc gac ccg aat gtg    1584
   Arg Leu Leu Leu Asn Phe Leu Gly Asp Asp Lys Phe Asp Pro Asn Val
           515                 520                 525
   gag cta cag tat gta aca tca ggt gag tat cta cat gat gac acg ttt    1632
   Glu Leu Gln Tyr Val Thr Ser Gly Glu Tyr Leu His Asp Asp Thr Phe
       530                 535                 540
   tgt gca tca tat tca cta aaa gag aag gaa att aaa cct gat ggt cga    1680
   Cys Ala Ser Tyr Ser Leu Lys Glu Lys Glu Ile Lys Pro Asp Gly Arg
   545                 550                 555                 560
   att ttt gca aag ttg act aag aga atg aga tca tgt caa gtt ata gca    1728
   Ile Phe Ala Lys Leu Thr Lys Arg Met Arg Ser Cys Gln Val Ile Ala
                   565                 570                 575
   gaa tct ctt tta gcg aac cat gct ggg aag tta atg aaa gag aat ggt    1776
   Glu Ser Leu Leu Ala Asn His Ala Gly Lys Leu Met Lys Glu Asn Gly
               580                 585                 590
   gtt gtg atg aat cag cta tca tta aca aaa tca cta tta aca atg agt    1824
   Val Val Met Asn Gln Leu Ser Leu Thr Lys Ser Leu Leu Thr Met Ser
           595                 600                 605
   cag att gga ata ata tcc gag aga gct aga aag tcg act cga gat aac    1872
   Gln Ile Gly Ile Ile Ser Glu Arg Ala Arg Lys Ser Thr Arg Asp Asn
       610                 615                 620
   ata aat cga cct ggt ttc cag aat atc cag aga aat aaa tca cat cac     1920
   Ile Asn Arg Pro Gly Phe Gln Asn Ile Gln Arg Asn Lys Ser His His
   625                 630                 635                 640
   tcc aag caa gtc aat cag cga gat cca agt gat gac ttt gaa ttg gca     1968
   Ser Lys Gln Val Asn Gln Arg Asp Pro Ser Asp Asp Phe Glu Leu Ala
                   645                 650                 655
   gca tct ttt tta act act gat ctc aaa aaa tat tgt tta caa tgg agg     2016
   Ala Ser Phe Leu Thr Thr Asp Leu Lys Lys Tyr Cys Leu Gln Trp Arg
               660                 665                 670
   tat cag aca att atc cca ttt gct caa tca cta aac aga atg tat ggt     2064
   Tyr Gln Thr Ile Ile Pro Phe Ala Gln Ser Leu Asn Arg Met Tyr Gly
           675                 680                 685
   tat cct cat ctc ttc gag tgg att cac ttg cgg cta atg cgt agt aca     2112
   Tyr Pro His Leu Phe Glu Trp Ile His Leu Arg Leu Met Arg Ser Thr
       690                 695                 700
   ctt tac gtg ggg gac ccc ttc aac cca cca gca gat acc agt caa ttt     2160
   Leu Tyr Val Gly Asp Pro Phe Asn Pro Pro Ala Asp Thr Ser Gln Phe
   705                 710                 715                 720
   gat cta gat aaa gta att aat gga gat atc ttc att gta tca ccc aga     2208
   Asp Leu Asp Lys Val Ile Asn Gly Asp Ile Phe Ile Val Ser Pro Arg
                   725                 730                 735
   ggt gga att gaa ggg ctg tgt caa aaa gct tgg aca atg ata tct atc     2256
   Gly Gly Ile Glu Gly Leu Cys Gln Lys Ala Trp Thr Met Ile Ser Ile
               740                 745                 750
   tct gtg ata att cta tct gcc aca gag tct ggc aca cga gta atg agc     2304
   Ser Val Ile Ile Leu Ser Ala Thr Glu Ser Gly Thr Arg Val Met Ser
           755                 760                 765
   atg gtg cag gga gat aat caa gca att gct gtc acc aca cga gta cca     2352
   Met Val Gln Gly Asp Asn Gln Ala Ile Ala Val Thr Thr Arg Val Pro
       770                 775                 780
   agg agc ctg ccg act ctt gag aaa aag act att gct ttt aga tct tgt     2400
   Arg Ser Leu Pro Thr Leu Glu Lys Lys Thr Ile Ala Phe Arg Ser Cys
   785                 790                 795                 800
   aat tta ttc ttt gag agg tta aaa tgt aat aat ttt gga tta ggt cac     2448
   Asn Leu Phe Phe Glu Arg Leu Lys Cys Asn Asn Phe Gly Leu Gly His
                   805                 810                 815
   cat ttg aaa gaa caa gag act atc att agt tct cac ttc ttt gtt tat     2496
   His Leu Lys Glu Gln Glu Thr Ile Ile Ser Ser His Phe Phe Val Tyr
               820                 825                 830
   agc aag aga ata ttc tat cag ggg agg att cta acg caa gcc tta aaa     2544
   Ser Lys Arg Ile Phe Tyr Gln Gly Arg Ile Leu Thr Gln Ala Leu Lys
           835                 840                 845
   aat gct agt aag ctc tgc ttg aca gct gat gtc cta gga gaa tgc acc      2592
   Asn Ala Ser Lys Leu Cys Leu Thr Ala Asp Val Leu Gly Glu Cys Thr
       850                 855                 860
   caa tca tca tgt tct aat ctt gca act act gtc atg agg tta act gag      2640
   Gln Ser Ser Cys Ser Asn Leu Ala Thr Thr Val Met Arg Leu Thr Glu
   865                 870                 875                 880
   aat ggt gtt gaa aaa gat atc tgt ttc tac ttg aat atc tat atg acc      2688
   Asn Gly Val Glu Lys Asp Ile Cys Phe Tyr Leu Asn Ile Tyr Met Thr
                   885                 890                 895
   atc aaa cag ctc tcc tat gat atc atc ttc cct caa gtg tcg att cct      2736
   Ile Lys Gln Leu Ser Tyr Asp Ile Ile Phe Pro Gln Val Ser Ile Pro
               900                 905                 910
   gga gat cag atc aca tta gaa tac ata aat aat cca cac ctg gta tca      2784
   Gly Asp Gln Ile Thr Leu Glu Tyr Ile Asn Asn Pro His Leu Val Ser
           915                 920                 925
   cga ttg gct ctt tta cca tcc cag tta gga ggt cta aac tac ttg tca      2832
   Arg Leu Ala Leu Leu Pro Ser Gln Leu Gly Gly Leu Asn Tyr Leu Ser
       930                 935                 940
   tgc agt agg ctg ttc aat cga aac ata ggt gac ccg gtg gtt tcc gca      2880
   Cys Ser Arg Leu Phe Asn Arg Asn Ile Gly Asp Pro Val Val Ser Ala
   945                 950                 955                 960
   gtt gca gat ctt aag aga tta att aaa tca gga tgt atg gat tac tgg      2928
   Val Ala Asp Leu Lys Arg Leu Ile Lys Ser Gly Cys Met Asp Tyr Trp
                   965                 970                 975
   atc ctt tat aac tta tta ggg aga aaa ccg gga aac ggc tca tgg gct      2976
   Ile Leu Tyr Asn Leu Leu Gly Arg Lys Pro Gly Asn Gly Ser Trp Ala
               980                 985                 990
   act tta gca gct gac ccg tac tca atc aat ata gag tat caa tac ccc      3024
   Thr Leu Ala Ala Asp Pro Tyr Ser Ile Asn Ile Glu Tyr Gln Tyr Pro
           995                 1000                1005
   cca act aca gct ctt aag agg cac acc caa caa gtt ctg atg gaa ctc      3072
   Pro Thr Thr Ala Leu Lys Arg His Thr Gln Gln Val Leu Met Glu Leu
       1010                1015                1020
   agt acg aat cca atg tta cgt ggc ata ttc tct gac aat gca cag gca      3120
   Ser Thr Asn Pro Met Leu Arg Gly Ile Phe Ser Asp Asn Ala Gln Ala
   1025                1030                1035                1040
   gaa gaa aat aat ctt gct agg ttt ctc ctg gat agg gag gtg atc ttt      3168
   Glu Glu Asn Asn Leu Ala Arg Phe Leu Leu Asp Arg Glu Val Ile Phe
                   1045                1050                1055
   ccg cgt gta gct cac atc atc att gag caa acc agt gtc ggg agg aga      3216
   Pro Arg Val Ala His Ile Ile Ile Glu Gln Thr Ser Val Gly Arg Arg
               1060                1065                1070
   aag cag att caa gga tat ttg gat tca act aga tcg ata atg agg aaa     3264
   Lys Gln Ile Gln Gly Tyr Leu Asp Ser Thr Arg Ser Ile Met Arg Lys
           1075                1080                1085
   tca ctg gaa att aag ccc ttg tcc aat agg aag ctt aat gaa ata ctg     3312
   Ser Leu Glu Ile Lys Pro Leu Ser Asn Arg Lys Leu Asn Glu Ile Leu
       1090                1095                1100
   gat tac aac atc aat tac tta gct tac aat ttg gca tta ctc aag aat     3360
   Asp Tyr Asn Ile Asn Tyr Leu Ala Tyr Asn Leu Ala Leu Leu Lys Asn
   1105                1110                1115                1120
   gct att gaa cct ccg act tat ttg aag gca atg acc ctt gaa aca tgt     3408
   Ala Ile Glu Pro Pro Thr Tyr Leu Lys Ala Met Thr Leu Glu Thr Cys
                   1125                1130                1135
   agc atc gac att gca agg agc ctc cgg aag ctc tcc tgg gcc cca ctc     3456
   Ser Ile Asp Ile Ala Arg Ser Leu Arg Lys Leu Ser Trp Ala Pro Leu
               1140                1145                1150
   ttg ggt ggg aga aat ctt gaa gga cta gag acg cca gat ccc att gaa     3504
   Leu Gly Gly Arg Asn Leu Glu Gly Leu Glu Thr Pro Asp Pro Ile Glu
           1155                1160                1165
   atc act gca gga gca tta att gtt gga tcg ggc tac tgt gaa cag tgt     3552
   Ile Thr Ala Gly Ala Leu Ile Val Gly Ser Gly Tyr Cys Glu Gln Cys
       1170                1175                1180
   gct gca gga gac aat cga ttc aca tgg ttt ttc ttg cca tct ggt atc     3600
   Ala Ala Gly Asp Asn Arg Phe Thr Trp Phe Phe Leu Pro Ser Gly Ile
   1185                1190                1195                1200
   gag ata gga ggg gat ccc cgt gat aat cct cct atc cgt gta ccg tac     3648
   Glu Ile Gly Gly Asp Pro Arg Asp Asn Pro Pro Ile Arg Val Pro Tyr
                   1205                1210                1215
   att ggc tcc agg act gat gag agg agg gta gcc tca atg gca tac atc     3696
   Ile Gly Ser Arg Thr Asp Glu Arg Arg Val Ala Ser Met Ala Tyr Ile
               1220                1225                1230
   agg ggt gcc tca agt agc cta aaa gca gtt ctt aga ctg gcg gga gtg     3744
   Arg Gly Ala Ser Ser Ser Leu Lys Ala Val Leu Arg Leu Ala Gly Val
           1235                1240                1245
   tac atc tgg gca ttc gga gat act ctg gag aat tgg ata gat gca ctg     3792
   Tyr Ile Trp Ala Phe Gly Asp Thr Leu Glu Asn Trp Ile Asp Ala Leu
       1250                1255                1260
   gat ttg tct cac act aga gtt aac atc aca ctt gaa cag tta caa tcc     3840
   Asp Leu Ser His Thr Arg Val Asn Ile Thr Leu Glu Gln Leu Gln Ser
   1265                1270                1275                1280
   ctc acc cca ctt cca acc tct gcc aat cta acc cat cgg ttg gat gat     3888
   Leu Thr Pro Leu Pro Thr Ser Ala Asn Leu Thr His Arg Leu Asp Asp
                   1285                1290                1295
   ggc aca act acc cta aag ttt act cct gca agc tct tac acc ttt tca    3936
   Gly Thr Thr Thr Leu Lys Phe Thr Pro Ala Ser Ser Tyr Thr Phe Ser
               1300                1305                1310
   agt ttc act cat ata tca aat gat gag caa tat ctg aca att aat gac    3984
   Ser Phe Thr His Ile Ser Asn Asp Glu Gln Tyr Leu Thr Ile Asn Asp
           1315                1320                1325
   aaa act gca gat tca aat ata atc tac caa cag tta atg atc act gga    4032
   Lys Thr Ala Asp Ser Asn Ile Ile Tyr Gln Gln Leu Met Ile Thr Gly
       1330                1335                1340
   ctc ggg atc tta gaa aca tgg aat aat ccc cca atc aat aga aca ttc    4080
   Leu Gly Ile Leu Glu Thr Trp Asn Asn Pro Pro Ile Asn Arg Thr Phe
   1345                1350                1355                1360
   gaa gaa tct acc cta cat ttg cac act ggt gca tca tgt tgt gtc cga    4128
   Glu Glu Ser Thr Leu His Leu His Thr Gly Ala Ser Cys Cys Val Arg
                   1365                1370                1375
   cct gtg gac tcc tgc att atc tca gaa gca tta aca gtc aag cca cat    4176
   Pro Val Asp Ser Cys Ile Ile Ser Glu Ala Leu Thr Val Lys Pro His
               1380                1385                1390
   att aca gta ccg tac agc aat aaa ttt gta ttt gat gaa gac ccg cta    4224
   Ile Thr Val Pro Tyr Ser Asn Lys Phe Val Phe Asp Glu Asp Pro Leu
           1395                1400                1405
   tct gaa tat gag act gca aaa ctg gaa tcg tta tca ttc caa gcc caa    4272
   Ser Glu Tyr Glu Thr Ala Lys Leu Glu Ser Leu Ser Phe Gln Ala Gln
       1410                1415                1420
   tta ggc aac att gat gct gta gat atg aca ggt aaa tta aca tta ttg    4320
   Leu Gly Asn Ile Asp Ala Val Asp Met Thr Gly Lys Leu Thr Leu Leu
   1425                1430                1435                1440
   tcc caa ttc act gca agg cag att atc aat gca atc act gga ctc gat    4368
   Ser Gln Phe Thr Ala Arg Gln Ile Ile Asn Ala Ile Thr Gly Leu Asp
                   1445                1450                1455
   gag tct gtt tct ctt act aat gat gcc att gtt gca tca gac tat gtc    4416
   Glu Ser Val Ser Leu Thr Asn Asp Ala Ile Val Ala Ser Asp Tyr Val
               1460                1465                1470
   tcc aat tgg att agt gaa tgc atg tat acc aaa tta gat gaa tta ttt    4464
   Ser Asn Trp Ile Ser Glu Cys Met Tyr Thr Lys Leu Asp Glu Leu Phe
           1475                1480                1485
   atg tat tgt ggg tgg gaa cta cta ttg gaa cta tcc tat caa atg tat    4512
   Met Tyr Cys Gly Trp Glu Leu Leu Leu Glu Leu Ser Tyr Gln Met Tyr
       1490                1495                1500
   tat ctg agg gta gtt ggg tgg agt aat ata gtg gat tat tct tac atg     4560
   Tyr Leu Arg Val Val Gly Trp Ser Asn Ile Val Asp Tyr Ser Tyr Met
   1505                1510                1515                1520
   atc ttg aga aga atc ccg ggt gca gca tta aac aat ctg gca tct aca     4608
   Ile Leu Arg Arg Ile Pro Gly Ala Ala Leu Asn Asn Leu Ala Ser Thr
                   1525                1530                1535
   tta agt cat cca aaa ctt ttc cga cga gct atc aac cta gat ata gtt     4656
   Leu Ser His Pro Lys Leu Phe Arg Arg Ala Ile Asn Leu Asp Ile Val
               1540                1545                1550
   gcc ccc tta aat gct cct cat ttt gca tct ctg gac tac atc aag atg     4704
   Ala Pro Leu Asn Ala Pro His Phe Ala Ser Leu Asp Tyr Ile Lys Met
           1555                1560                1565
   agt atg gat gca ata ctc tgg ggc tgt aaa aga gtc atc aat gtg ctc     4752
   Ser Met Asp Ala Ile Leu Trp Gly Cys Lys Arg Val Ile Asn Val Leu
       1570                1575                1580
   tcc aat gga ggg gac tta gaa tta gtt gtg aca tct gaa gat agc ctt     4800
   Ser Asn Gly Gly Asp Leu Glu Leu Val Val Thr Ser Glu Asp Ser Leu
   1585                1590                1595                1600
   att ctc agt gac cga tcc atg aat ctc att gca agg aaa tta act tta     4848
   Ile Leu Ser Asp Arg Ser Met Asn Leu Ile Ala Arg Lys Leu Thr Leu
                   1605                1610                1615
   tta tca ctg att cac cat aat ggt ttg gaa cta cca aag att aag ggg     4896
   Leu Ser Leu Ile His His Asn Gly Leu Glu Leu Pro Lys Ile Lys Gly
               1620                1625                1630
   ttc tct cct gat gag aag tgt ttc gct ttg aca gaa ttt ttg agg aaa     4944
   Phe Ser Pro Asp Glu Lys Cys Phe Ala Leu Thr Glu Phe Leu Arg Lys
           1635                1640                1645
   gtg gtg aac tca ggg ttg agt tca ata gag aac cta tca aat ttt atg     4992
   Val Val Asn Ser Gly Leu Ser Ser Ile Glu Asn Leu Ser Asn Phe Met
       1650                1655                1660
   tac aat gtg gag aac cca cgg ctt gca gca ttc gcc agc aac aat tac     5040
   Tyr Asn Val Glu Asn Pro Arg Leu Ala Ala Phe Ala Ser Asn Asn Tyr
   1665                1670                1675                1680
   tac ctg acc aga aaa tta ttg aat tca ata cga gat act gag tca ggt     5088
   Tyr Leu Thr Arg Lys Leu Leu Asn Ser Ile Arg Asp Thr Glu Ser Gly
                   1685                1690                1695
   caa gta gca gtc acc tca tat tat gaa tca tta gaa tat att gat agt     5136
   Gln Val Ala Val Thr Ser Tyr Tyr Glu Ser Leu Glu Tyr Ile Asp Ser
               1700                1705                1710
   ctt aag cta acc cca cat gtg cct ggc acc tca tgc att gag gat gat     5184
   Leu Lys Leu Thr Pro His Val Pro Gly Thr Ser Cys Ile Glu Asp Asp
           1715                1720                1725
   agt cta tgt aca aat gat tac ata atc tgg atc ata gag tct aat gca    5232
   Ser Leu Cys Thr Asn Asp Tyr Ile Ile Trp Ile Ile Glu Ser Asn Ala
       1730                1735                1740
   aac ttg gag aag tat cca att cca aat agc cct gag gat gat tcc aat    5280
   Asn Leu Glu Lys Tyr Pro Ile Pro Asn Ser Pro Glu Asp Asp Ser Asn
   1745                1750                1755                1760
   ttc cat aac ttt aag ttg aat gct cca tcg cac cat acc tta cgc cca    5328
   Phe His Asn Phe Lys Leu Asn Ala Pro Ser His His Thr Leu Arg Pro
                   1765                1770                1775
   tta ggg ttg tca tcg act gct tgg tat aag ggt ata agc tgc tgc agg    5376
   Leu Gly Leu Ser Ser Thr Ala Trp Tyr Lys Gly Ile Ser Cys Cys Arg
               1780                1785                1790
   tac ctt gag cga tta aag cta cca caa ggt gat cat tta tat att gca    5424
   Tyr Leu Glu Arg Leu Lys Leu Pro Gln Gly Asp His Leu Tyr Ile Ala
           1795                1800                1805
   gaa ggt agt ggt gcc agt atg aca atc ata gaa tac cta ttc cca gga    5472
   Glu Gly Ser Gly Ala Ser Met Thr Ile Ile Glu Tyr Leu Phe Pro Gly
       1810                1815                1820
   aga aag ata tat tac aat tct tta ttt agt agt ggt gac aat ccc cca    5520
   Arg Lys Ile Tyr Tyr Asn Ser Leu Phe Ser Ser Gly Asp Asn Pro Pro
   1825                1830                1835                1840
   caa aga aat tat gca cca atg cct act cag ttc att gag agt gtc cca    5568
   Gln Arg Asn Tyr Ala Pro Met Pro Thr Gln Phe Ile Glu Ser Val Pro
                   1845                1850                1855
   tac aag ctc tgg caa gca cac aca gat caa tat ccc gag att ttt gag    5616
   Tyr Lys Leu Trp Gln Ala His Thr Asp Gln Tyr Pro Glu Ile Phe Glu
               1860                1865                1870
   gac ttc atc cct cta tgg aac gga aac gcc gcc atg act gac ata gga    5664
   Asp Phe Ile Pro Leu Trp Asn Gly Asn Ala Ala Met Thr Asp Ile Gly
           1875                1880                1885
   atg aca gct tgt gta gaa ttc atc atc aat cga gtt ggc cca agg act    5712
   Met Thr Ala Cys Val Glu Phe Ile Ile Asn Arg Val Gly Pro Arg Thr
       1890                1895                1900
   tgc agt tta gta cat gta gat ttg gaa tca agt gca agc tta aat caa    5760
   Cys Ser Leu Val His Val Asp Leu Glu Ser Ser Ala Ser Leu Asn Gln
   1905                1910                1915                1920
   caa tgc ctg tca aag ccg ata att aat gct atc atc act gct aca act    5808
   Gln Cys Leu Ser Lys Pro Ile Ile Asn Ala Ile Ile Thr Ala Thr Thr
                   1925                1930                1935
   gtt ttg tgc cct cat ggg gtg ctt att ctg aaa tat agt tgg ttg cca    5856
   Val Leu Cys Pro His Gly Val Leu Ile Leu Lys Tyr Ser Trp Leu Pro
   1940 1945 1950
   ttt act aga ttt agt act ttg atc act ttc tta tgg tgc tac ttt gag 5904
   Phe Thr Arg Phe Ser Thr Leu Ile Thr Phe Leu Trp Cys Tyr Phe Glu
   1955 1960 1965
   aga atc act gtt ctt agg agc aca tat tct gat cca gct aat cat gag 5952
   Arg Ile Thr Val Leu Arg Ser Thr Tyr Ser Asp Pro Ala Asn His Glu
   1970 1975 1980
   gtt tat tta att tgt atc ctt gcc aac aac ttt gca ttc cag act gtc 6000
   Val Tyr Leu Ile Cys Ile Leu Ala Asn Asn Phe Ala Phe Gln Thr Val
   1985 1990 1995 2000
   tcg cag gca aca gga atg gcg atg act tta acc gat caa ggg ttt acc 6048
   Ser Gln Ala Thr Gly Met Ala Met Thr Leu Thr Asp Gln Gly Phe Thr
   2005 2010 2015
   ttg ata tca cct gaa aga ata aat cag tat tgg gat ggt cac tta aag 6096
   Leu Ile Ser Pro Glu Arg Ile Asn Gln Tyr Trp Asp Gly His Leu Lys
   2020 2025 2030
   caa gaa cgt atc gta gca gaa gca att gat aag gtg gtt cta gga gaa 6144
   Gln Glu Arg Ile Val Ala Glu Ala Ile Asp Lys Val Val Leu Gly Glu
   2035 2040 2045
   aat gct cta ttt aat tcg agt gat aat gaa tta att ctc aaa tgt gga 6192
   Asn Ala Leu Phe Asn Ser Ser Asp Asn Glu Leu Ile Leu Lys Cys Gly
   2050 2055 2060
   ggg aca cca aat gca cgg aat ctt atc gat atc gag cca gtc gca act 6240
   Gly Thr Pro Asn Ala Arg Asn Leu Ile Asp Ile Glu Pro Val Ala Thr
   2065 2070 2075 2080
   ttc ata gaa ttt gaa caa cta atc tgc aca atg ttg aca acc cac ttg 6288
   Phe Ile Glu Phe Glu Gln Leu Ile Cys Thr Met Leu Thr Thr His Leu
   2085 2090 2095
   aag gaa ata att gat ata aca agg tct gga acc cag gat tat gaa agt 6336
   Lys Glu Ile Ile Asp Ile Thr Arg Ser Gly Thr Gln Asp Tyr Glu Ser
   2100 2105 2110
   tta tta ctc act cct tac aat tta ggt ctt ctt ggt aaa atc agt acg 6384
   Leu Leu Leu Thr Pro Tyr Asn Leu Gly Leu Leu Gly Lys Ile Ser Thr
   2115 2120 2125
   ata gtg aga tta tta aca gaa agg att cta aat cat act atc agg aat 6432
   Ile Val Arg Leu Leu Thr Glu Arg Ile Leu Asn His Thr Ile Arg Asn
   2130 2135 2140
   tgg ttg atc ctc cca cct tcg ctc cag atg atc gtg aag cag gac ttg 6480
   Trp Leu Ile Leu Pro Pro Ser Leu Gln Met Ile Val Lys Gln Asp Leu
   2145 2150 2155 2160
   gaa ttc ggc ata ttc agg att act tcc atc ctc aat tct gat cgg ttc 6528
   Glu Phe Gly Ile Phe Arg Ile Thr Ser Ile Leu Asn Ser Asp Arg Phe
                   2165                2170                2175
   ctg aaa ctt tct cca aat agg aaa tac ttg att aca caa tta act gca    6576
   Leu Lys Leu Ser Pro Asn Arg Lys Tyr Leu Ile Thr Gln Leu Thr Ala
               2180                2185                2190
   ggc tac att agg aaa ttg att gag ggg gat tgt aat atc gat cta acc    6624
   Gly Tyr Ile Arg Lys Leu Ile Glu Gly Asp Cys Asn Ile Asp Leu Thr
           2195                2200                2205
   aga cct atc caa aag caa atc tgg aaa gca tta ggt tgt gta gtc tac    6672
   Arg Pro Ile Gln Lys Gln Ile Trp Lys Ala Leu Gly Cys Val Val Tyr
       2210                2215                2220
   tgt cac gat cca atg gat caa agg gag tca aca gag ttt att gat ata    6720
   Cys His Asp Pro Met Asp Gln Arg Glu Ser Thr Glu Phe Ile Asp Ile
   2225                2230                2235                2240
   aat att aat gaa gaa ata gac cgc ggg atc gat ggc gag gaa atc        6765
   Asn Ile Asn Glu Glu Ile Asp Arg Gly Ile Asp Gly Glu Glu Ile
                   2245                2250                2255
<210> 24
   <211> 2255
   <212> PRT
   <213> Cryptovirus
<400> 24
   Met Ala Gly Ser Arg Glu Ile Leu Leu Pro Glu Val His Leu Asn Ser
    1               5                  10                  15
   Pro Ile Val Lys His Lys Leu Tyr Tyr Tyr Ile Leu Leu Gly Asn Leu
               20                  25                  30
   Pro Asn Glu Ile Asp Ile Asp Asp Leu Gly Pro Leu His Asn Gln Asn
           35                  40                  45
   Trp Asn Gln Ile Ala His Glu Glu Ser Asn Leu Ala Gln Arg Leu Val
       50                  55                  60
   Asn Val Arg Asn Phe Leu Ile Thr His Ile Pro Asp Leu Arg Lys Gly
   65                  70                  75                  80
   His Trp Gln Glu Tyr Val Asn Val Ile Leu Trp Pro Arg Ile Leu Pro
                   85                  90                  95
   Leu Ile Pro Asp Phe Lys Ile Asn Asp Gln Leu Pro Leu Leu Lys Asn
               100                 105                 110
   Trp Asp Lys Leu Val Lys Glu Ser Cys Ser Val Ile Asn Ala Gly Thr
           115                 120                 125
   Ser Gln Cys Ile Gln Asn Leu Ser Tyr Gly Leu Thr Gly Arg Gly Asn
       130                 135                 140
   Leu Phe Thr Arg Ser Arg Glu Leu Ser Gly Asp Arg Arg Asp Ile Asp
   145                 150                 155                 160
   Leu Lys Thr Val Val Ala Ala Trp His Asp Ser Asp Trp Lys Arg Ile
                   165                 170                 175
   Ser Asp Phe Trp Ile Met Ile Lys Phe Gln Met Arg Gln Leu Ile Val
               180                 185                 190
   Arg Gln Thr Asp His Asn Asp Pro Asp Leu Ile Thr Tyr Ile Glu Asn
           195                 200                 205
   Arg Glu Gly Ile Ile Ile Ile Thr Pro Glu Leu Val Ala Leu Phe Asn
       210                 215                 220
   Thr Glu Asn His Thr Leu Thr Tyr Met Thr Phe Glu Ile Val Leu Met
   225                 230                 235                 240
   Val Ser Asp Met Tyr Glu Gly Arg His Asn Ile Leu Ser Leu Cys Thr
                   245                 250                 255
   Val Ser Thr Tyr Leu Asn Pro Leu Lys Lys Arg Ile Thr Tyr Leu Leu
               260                 265                 270
   Ser Leu Val Asp Asn Leu Ala Phe Gln Ile Gly Asp Ala Val Tyr Asn
           275                 280                 285
   Ile Ile Ala Leu Leu Glu Ser Phe Val Tyr Ala Gln Leu Gln Met Ser
       290                 295                 300
   Asp Pro Ile Pro Glu Leu Arg Gly Gln Phe His Ala Phe Val Cys Ser
   305                 310                 315                 320
   Glu Ile Leu Asp Ala Leu Arg Gly Thr Asn Ser Phe Thr Gln Asp Glu
                   325                 330                 335
   Leu Arg Thr Val Thr Thr Asn Leu Ile Ser Pro Phe Gln Asp Leu Thr
               340                 345                 350
   Pro Asp Leu Thr Ala Glu Leu Leu Cys Ile Met Arg Leu Trp Gly His
           355                 360                 365
   Pro Met Leu Thr Ala Ser Gln Ala Ala Gly Lys Val Arg Glu Ser Met
       370                 375                 380
   Cys Ala Gly Lys Val Leu Asp Phe Pro Thr Ile Met Lys Thr Leu Ala
   385                 390                 395                 400
   Phe Phe His Thr Ile Leu Ile Asn Gly Tyr Arg Arg Lys His His Gly
                   405                 410                 415
   Val Trp Pro Pro Leu Asn Leu Pro Gly Asn Ala Ser Lys Gly Leu Thr
               420                 425                 430
   Glu Leu Met Asn Asp Asn Thr Glu Ile Ser Tyr Glu Phe Thr Leu Lys
           435                 440                 445
   His Trp Lys Glu Ile Ser Leu Ile Lys Phe Lys Lys Cys Phe Asp Ala
       450                 455                 460
   Asp Ala Gly Glu Glu Leu Ser Ile Phe Met Lys Asp Lys Ala Ile Ser
   465                 470                 475                 480
   Ala Pro Lys Gln Asp Trp Met Ser Val Phe Arg Arg Ser Leu Ile Lys
                   485                 490                 495
   Gln Arg His Gln His His Gln Val Pro Leu Pro Asn Pro Phe Asn Arg
               500                 505                 510
   Arg Leu Leu Leu Asn Phe Leu Gly Asp Asp Lys Phe Asp Pro Asn Val
           515                 520                 525
   Glu Leu Gln Tyr Val Thr Ser Gly Glu Tyr Leu His Asp Asp Thr Phe
       530                 535                 540
   Cys Ala Ser Tyr Ser Leu Lys Glu Lys Glu Ile Lys Pro Asp Gly Arg
   545                 550                 555                 560
   Ile Phe Ala Lys Leu Thr Lys Arg Met Arg Ser Cys Gln Val Ile Ala
                   565                 570                 575
   Glu Ser Leu Leu Ala Asn His Ala Gly Lys Leu Met Lys Glu Asn Gly
               580                 585                 590
   Val Val Met Asn Gln Leu Ser Leu Thr Lys Ser Leu Leu Thr Met Ser
           595                 600                 605
   Gln Ile Gly Ile Ile Ser Glu Arg Ala Arg Lys Ser Thr Arg Asp Asn
       610                 615                 620
   Ile Asn Arg Pro Gly Phe Gln Asn Ile Gln Arg Asn Lys Ser His His
   625 630 635 640
   Ser Lys Gln Val Asn Gln Arg Asp Pro Ser Asp Asp Phe Glu Leu Ala
   645 650 655
   Ala Ser Phe Leu Thr Thr Asp Leu Lys Lys Tyr Cys Leu Gln Trp Arg
   660 665 670
   Tyr Gln Thr Ile Ile Pro Phe Ala Gln Ser Leu Asn Arg Met Tyr Gly
   675 680 685
   Tyr Pro His Leu Phe Glu Trp Ile His Leu Arg Leu Met Arg Ser Thr
   690 695 700
   Leu Tyr Val Gly Asp Pro Phe Asn Pro Pro Ala Asp Thr Ser Gln Phe
   705 710 715 720
   Asp Leu Asp Lys Val Ile Asn Gly Asp Ile Phe Ile Val Ser Pro Arg
   725 730 735
   Gly Gly Ile Glu Gly Leu Cys Gln Lys Ala Trp Thr Met Ile Ser Ile
   740 745 750
   Ser Val Ile Ile Leu Ser Ala Thr Glu Ser Gly Thr Arg Val Met Ser
   755 760 765
   Met Val Gln Gly Asp Asn Gln Ala Ile Ala Val Thr Thr Arg Val Pro
   770 775 780
   Arg Ser Leu Pro Thr Leu Glu Lys Lys Thr Ile Ala Phe Arg Ser Cys
   785 790 795 800
   Asn Leu Phe Phe Glu Arg Leu Lys Cys Asn Asn Phe Gly Leu Gly His
   805 810 815
   His Leu Lys Glu Gln Glu Thr Ile Ile Ser Ser His Phe Phe Val Tyr
   820 825 830
   Ser Lys Arg Ile Phe Tyr Gln Gly Arg Ile Leu Thr Gln Ala Leu Lys
   835 840 845
   Asn Ala Ser Lys Leu Cys Leu Thr Ala Asp Val Leu Gly Glu Cys Thr
   850 855 860
   Gln Ser Ser Cys Ser Asn Leu Ala Thr Thr Val Met Arg Leu Thr Glu
   865 870 875 880
   Asn Gly Val Glu Lys Asp Ile Cys Phe Tyr Leu Asn Ile Tyr Met Thr
   885 890 895
   Ile Lys Gln Leu Ser Tyr Asp Ile Ile Phe Pro Gln Val Ser Ile Pro
   900 905 910
   Gly Asp Gln Ile Thr Leu Glu Tyr Ile Asn Asn Pro His Leu Val Ser
   915 920 925
   Arg Leu Ala Leu Leu Pro Ser Gln Leu Gly Gly Leu Asn Tyr Leu Ser
   930 935 940
   Cys Ser Arg Leu Phe Asn Arg Asn Ile Gly Asp Pro Val Val Ser Ala
   945 950 955 960
   Val Ala Asp Leu Lys Arg Leu Ile Lys Ser Gly Cys Met Asp Tyr Trp
   965 970 975
   Ile Leu Tyr Asn Leu Leu Gly Arg Lys Pro Gly Asn Gly Ser Trp Ala
   980 985 990
   Thr Leu Ala Ala Asp Pro Tyr Ser Ile Asn Ile Glu Tyr Gln Tyr Pro
   995 1000 1005
   Pro Thr Thr Ala Leu Lys Arg His Thr Gln Gln Val Leu Met Glu Leu
   1010 1015 1020
   Ser Thr Asn Pro Met Leu Arg Gly Ile Phe Ser Asp Asn Ala Gln Ala
   1025 1030 1035 1040
   Glu Glu Asn Asn Leu Ala Arg Phe Leu Leu Asp Arg Glu Val Ile Phe
   1045 1050 1055
   Pro Arg Val Ala His Ile Ile Ile Glu Gln Thr Ser Val Gly Arg Arg
   1060 1065 1070
   Lys Gln Ile Gln Gly Tyr Leu Asp Ser Thr Arg Ser Ile Met Arg Lys
   1075 1080 1085
   Ser Leu Glu Ile Lys Pro Leu Ser Asn Arg Lys Leu Asn Glu Ile Leu
   1090 1095 1100
   Asp Tyr Asn Ile Asn Tyr Leu Ala Tyr Asn Leu Ala Leu Leu Lys Asn
   1105 1110 1115 1120
   Ala Ile Glu Pro Pro Thr Tyr Leu Lys Ala Met Thr Leu Glu Thr Cys
   1125 1130 1135
   Ser Ile Asp Ile Ala Arg Ser Leu Arg Lys Leu Ser Trp Ala Pro Leu
   1140 1145 1150
   Leu Gly Gly Arg Asn Leu Glu Gly Leu Glu Thr Pro Asp Pro Ile Glu
   1155 1160 1165
   Ile Thr Ala Gly Ala Leu Ile Val Gly Ser Gly Tyr Cys Glu Gln Cys
   1170 1175 1180
   Ala Ala Gly Asp Asn Arg Phe Thr Trp Phe Phe Leu Pro Ser Gly Ile
   1185 1190 1195 1200
   Glu Ile Gly Gly Asp Pro Arg Asp Asn Pro Pro Ile Arg Val Pro Tyr
   1205 1210 1215
   Ile Gly Ser Arg Thr Asp Glu Arg Arg Val Ala Ser Met Ala Tyr Ile
   1220 1225 1230
   Arg Gly Ala Ser Ser Ser Leu Lys Ala Val Leu Arg Leu Ala Gly Val
   1235 1240 1245
   Tyr Ile Trp Ala Phe Gly Asp Thr Leu Glu Asn Trp Ile Asp Ala Leu
   1250 1255 1260
   Asp Leu Ser His Thr Arg Val Asn Ile Thr Leu Glu Gln Leu Gln Ser
   1265 1270 1275 1280
   Leu Thr Pro Leu Pro Thr Ser Ala Asn Leu Thr His Arg Leu Asp Asp
   1285 1290 1295
   Gly Thr Thr Thr Leu Lys Phe Thr Pro Ala Ser Ser Tyr Thr Phe Ser
   1300 1305 1310
   Ser Phe Thr His Ile Ser Asn Asp Glu Gln Tyr Leu Thr Ile Asn Asp
   1315 1320 1325
   Lys Thr Ala Asp Ser Asn Ile Ile Tyr Gln Gln Leu Met Ile Thr Gly
   1330 1335 1340
   Leu Gly Ile Leu Glu Thr Trp Asn Asn Pro Pro Ile Asn Arg Thr Phe
   1345 1350 1355 1360
   Glu Glu Ser Thr Leu His Leu His Thr Gly Ala Ser Cys Cys Val Arg
   1365 1370 1375
   Pro Val Asp Ser Cys Ile Ile Ser Glu Ala Leu Thr Val Lys Pro His
   1380 1385 1390
   Ile Thr Val Pro Tyr Ser Asn Lys Phe Val Phe Asp Glu Asp Pro Leu
   1395 1400 1405
   Ser Glu Tyr Glu Thr Ala Lys Leu Glu Ser Leu Ser Phe Gln Ala Gln
   1410 1415 1420
   Leu Gly Asn Ile Asp Ala Val Asp Met Thr Gly Lys Leu Thr Leu Leu
   1425 1430 1435 1440
   Ser Gln Phe Thr Ala Arg Gln Ile Ile Asn Ala Ile Thr Gly Leu Asp
   1445 1450 1455
   Glu Ser Val Ser Leu Thr Asn Asp Ala Ile Val Ala Ser Asp Tyr Val
   1460 1465 1470
   Ser Asn Trp Ile Ser Glu Cys Met Tyr Thr Lys Leu Asp Glu Leu Phe
   1475 1480 1485
   Met Tyr Cys Gly Trp Glu Leu Leu Leu Glu Leu Ser Tyr Gln Met Tyr
   1490 1495 1500
   Tyr Leu Arg Val Val Gly Trp Ser Asn Ile Val Asp Tyr Ser Tyr Met
   1505 1510 1515 1520
   Ile Leu Arg Arg Ile Pro Gly Ala Ala Leu Asn Asn Leu Ala Ser Thr
   1525 1530 1535
   Leu Ser His Pro Lys Leu Phe Arg Arg Ala Ile Asn Leu Asp Ile Val
   1540 1545 1550
   Ala Pro Leu Asn Ala Pro His Phe Ala Ser Leu Asp Tyr Ile Lys Met
   1555 1560 1565
   Ser Met Asp Ala Ile Leu Trp Gly Cys Lys Arg Val Ile Asn Val Leu
   1570 1575 1580
   Ser Asn Gly Gly Asp Leu Glu Leu Val Val Thr Ser Glu Asp Ser Leu
   1585 1590 1595 1600
   Ile Leu Ser Asp Arg Ser Met Asn Leu Ile Ala Arg Lys Leu Thr Leu
   1605 1610 1615
   Leu Ser Leu Ile His His Asn Gly Leu Glu Leu Pro Lys Ile Lys Gly
   1620 1625 1630
   Phe Ser Pro Asp Glu Lys Cys Phe Ala Leu Thr Glu Phe Leu Arg Lys
   1635 1640 1645
   Val Val Asn Ser Gly Leu Ser Ser Ile Glu Asn Leu Ser Asn Phe Met
   1650 1655 1660
   Tyr Asn Val Glu Asn Pro Arg Leu Ala Ala Phe Ala Ser Asn Asn Tyr
   1665 1670 1675 1680
   Tyr Leu Thr Arg Lys Leu Leu Asn Ser Ile Arg Asp Thr Glu Ser Gly
   1685 1690 1695
   Gln Val Ala Val Thr Ser Tyr Tyr Glu Ser Leu Glu Tyr Ile Asp Ser
   1700 1705 1710
   Leu Lys Leu Thr Pro His Val Pro Gly Thr Ser Cys Ile Glu Asp Asp
   1715 1720 1725
   Ser Leu Cys Thr Asn Asp Tyr Ile Ile Trp Ile Ile Glu Ser Asn Ala
   1730 1735 1740
   Asn Leu Glu Lys Tyr Pro Ile Pro Asn Ser Pro Glu Asp Asp Ser Asn
   1745 1750 1755 1760
   Phe His Asn Phe Lys Leu Asn Ala Pro Ser His His Thr Leu Arg Pro
   1765 1770 1775
   Leu Gly Leu Ser Ser Thr Ala Trp Tyr Lys Gly Ile Ser Cys Cys Arg
   1780 1785 1790
   Tyr Leu Glu Arg Leu Lys Leu Pro Gln Gly Asp His Leu Tyr Ile Ala
   1795 1800 1805
   Glu Gly Ser Gly Ala Ser Met Thr Ile Ile Glu Tyr Leu Phe Pro Gly
   1810 1815 1820
   Arg Lys Ile Tyr Tyr Asn Ser Leu Phe Ser Ser Gly Asp Asn Pro Pro
   1825 1830 1835 1840
   Gln Arg Asn Tyr Ala Pro Met Pro Thr Gln Phe Ile Glu Ser Val Pro
   1845 1850 1855
   Tyr Lys Leu Trp Gln Ala His Thr Asp Gln Tyr Pro Glu Ile Phe Glu
   1860 1865 1870
   Asp Phe Ile Pro Leu Trp Asn Gly Asn Ala Ala Met Thr Asp Ile Gly
   1875 1880 1885
   Met Thr Ala Cys Val Glu Phe Ile Ile Asn Arg Val Gly Pro Arg Thr
   1890 1895 1900
   Cys Ser Leu Val His Val Asp Leu Glu Ser Ser Ala Ser Leu Asn Gln
   1905 1910 1915 1920
   Gln Cys Leu Ser Lys Pro Ile Ile Asn Ala Ile Ile Thr Ala Thr Thr
   1925 1930 1935
   Val Leu Cys Pro His Gly Val Leu Ile Leu Lys Tyr Ser Trp Leu Pro
   1940 1945 1950
   Phe Thr Arg Phe Ser Thr Leu Ile Thr Phe Leu Trp Cys Tyr Phe Glu
           1955                1960                1965
   Arg Ile Thr Val Leu Arg Ser Thr Tyr Ser Asp Pro Ala Asn His Glu
       1970                1975                1980
   Val Tyr Leu Ile Cys Ile Leu Ala Asn Asn Phe Ala Phe Gln Thr Val
   1985                1990                1995                2000
   Ser Gln Ala Thr Gly Met Ala Met Thr Leu Thr Asp Gln Gly Phe Thr
                   2005                2010                2015
   Leu Ile Ser Pro Glu Arg Ile Asn Gln Tyr Trp Asp Gly His Leu Lys
               2020                2025                2030
   Gln Glu Arg Ile Val Ala Glu Ala Ile Asp Lys Val Val Leu Gly Glu
           2035                2040                2045
   Asn Ala Leu Phe Asn Ser Ser Asp Asn Glu Leu Ile Leu Lys Cys Gly
       2050                2055                2060
   Gly Thr Pro Asn Ala Arg Asn Leu Ile Asp Ile Glu Pro Val Ala Thr
   2065                2070                2075                2080
   Phe Ile Glu Phe Glu Gln Leu Ile Cys Thr Met Leu Thr Thr His Leu
                   2085                2090                2095
   Lys Glu Ile Ile Asp Ile Thr Arg Ser Gly Thr Gln Asp Tyr Glu Ser
               2100                2105                2110
   Leu Leu Leu Thr Pro Tyr Asn Leu Gly Leu Leu Gly Lys Ile Ser Thr
           2115                2120                2125
   Ile Val Arg Leu Leu Thr Glu Arg Ile Leu Asn His Thr Ile Arg Asn
       2130                2135                2140
   Trp Leu Ile Leu Pro Pro Ser Leu Gln Met Ile Val Lys Gln Asp Leu
   2145                2150                2155                2160
   Glu Phe Gly Ile Phe Arg Ile Thr Ser Ile Leu Asn Ser Asp Arg Phe
                   2165                2170                2175
   Leu Lys Leu Ser Pro Asn Arg Lys Tyr Leu Ile Thr Gln Leu Thr Ala
               2180                2185                2190
   Gly Tyr Ile Arg Lys Leu Ile Glu Gly Asp Cys Asn Ile Asp Leu Thr
           2195                2200                2205
   Arg Pro Ile Gln Lys Gln Ile Trp Lys Ala Leu Gly Cys Val Val Tyr
       2210                2215                2220
   Cys His Asp Pro Met Asp Gln Arg Glu Ser Thr Glu Phe Ile Asp Ile
   2225                2230                2235                2240
   Asn Ile Asn Glu Glu Ile Asp Arg Gly Ile Asp Gly Glu Glu Ile
                   2245                2250                2255
<210> 25
   <211> 1656
   <212> DNA
   <213> Canine parainfluenza virus
<220>
   <221> CDS
   <222> (1)...(1656)
   <223> Canine parainfluenza virus F protein encoding sequence with "TAA" termination codon; cDNA in mRNA sense
<400> 25
   atg ggt act aga att caa ttt ctg gtg gtc tcc tgt cta ttg gca gga    48
   Met Gly Thr Arg Ile Gln Phe Leu Val Val Ser Cys Leu Leu Ala Gly
   1               5                   10                  15
   aca ggc agc ctt gat cca gca gcc ctc atg caa atc ggt gtc att cca      96
   Thr Gly Ser Leu Asp Pro Ala Ala Leu Met Gln Ile Gly Val Ile Pro
               20                  25                  30
   aca aat gtc cgg caa ctt atg tat tat act gag gct tca tca gca ttc     144
   Thr Asn Val Arg Gln Leu Met Tyr Tyr Thr Glu Ala Ser Ser Ala Phe
           35                  40                  45
   att gtt gtg aag tta atg cct aca att gac tcg ccg att agt ggg tgt     192
   Ile Val Val Lys Leu Met Pro Thr Ile Asp Ser Pro Ile Ser Gly Cys
       50                  55                  60
   aat ata aca tcc att tca agc tat aat gca aca atg aca aaa ctt cta     240
   Asn Ile Thr Ser Ile Ser Ser Tyr Asn Ala Thr Met Thr Lys Leu Leu
   65                  70                  75                  80
   cag ccg atc ggt gag aat tta gag acg att agg tac cag ttg att cca     288
   Gln Pro Ile Gly Glu Asn Leu Glu Thr Ile Arg Tyr Gln Leu Ile Pro
                   85                  90                  95
   act cgg agg aga cgc cgg ttt gca ggg gtg gtg att gga tta gcc gca     336
   Thr Arg Arg Arg Arg Arg Phe Ala Gly Val Val Ile Gly Leu Ala Ala
               100                 105                 110
   tta gga gta gct act gca gca cag gtc act gcc gca gta gca cta gta     384
   Leu Gly Val Ala Thr Ala Ala Gln Val Thr Ala Ala Val Ala Leu Val
           115                 120                 125
   aag gcg aat aaa aat gct gtg gct ata ctc aat ctc aaa aac gca atc     432
   Lys Ala Asn Lys Asn Ala Val Ala Ile Leu Asn Leu Lys Asn Ala Ile
       130                 135                 140
   caa aaa aca aat gca gca gtt gca gac gtg gtt cag gcc aca caa tca     480
   Gln Lys Thr Asn Ala Ala Val Ala Asp Val Val Gln Ala Thr Gln Ser
   145                 150                 155                 160
   cta gga acg gca gtt caa gca gtt caa gat cac ata aat agt gtg gta     528
   Leu Gly Thr Ala Val Gln Ala Val Gln Asp His Ile Asn Ser Val Val
                   165                 170                 175
   agt cca gca att aca gca gcc aat tgc aaa gcc caa gat gct atc att     576
   Ser Pro Ala Ile Thr Ala Ala Asn Cys Lys Ala Gln Asp Ala Ile Ile
               180                 185                 190
   ggc tca att ctc aat ctc tat ttg acc gag ttg aca act atc ttc cac     624
   Gly Ser Ile Leu Asn Leu Tyr Leu Thr Glu Leu Thr Thr Ile Phe His
           195                 200                 205
   aat caa att aca aac ccc gca ttg agt cct att aca att caa gct ttg     672
   Asn Gln Ile Thr Asn Pro Ala Leu Ser Pro Ile Thr Ile Gln Ala Leu
       210                 215                 220
   agg atc cta cta ggg agt acc ttg ccg acc gtg gtc gaa aaa tct ttc     720
   Arg Ile Leu Leu Gly Ser Thr Leu Pro Thr Val Val Glu Lys Ser Phe
   225                 230                 235                 240
   aat acc cag ata agt gca gct gag ctt ctc tca tca ggg tta ttg aca     768
   Asn Thr Gln Ile Ser Ala Ala Glu Leu Leu Ser Ser Gly Leu Leu Thr
                   245                 250                 255
   ggc cag att gtg gga tta gat ttg acc tac atg cag atg gtc ata aaa     816
   Gly Gln Ile Val Gly Leu Asp Leu Thr Tyr Met Gln Met Val Ile Lys
               260                 265                 270
   att gag ctg cca act tta act gta caa cct gca acc cag atc ata gat     864
   Ile Glu Leu Pro Thr Leu Thr Val Gln Pro Ala Thr Gln Ile Ile Asp
           275                 280                 285
   ctg gtc acc att tct gca ttc att aac aat caa gaa gtt atg gcc caa     912
   Leu Val Thr Ile Ser Ala Phe Ile Asn Asn Gln Glu Val Met Ala Gln
       290                 295                 300
   tta cca aca cgt gtt att gtg act ggc agc ttg atc caa gcc tat ccc     960
   Leu Pro Thr Arg Val Ile Val Thr Gly Ser Leu Ile Gln Ala Tyr Pro
   305                 310                 315                 320
   gca tcg caa tgc act atc acc ccc aac act gtg tac tgt agg tat aat    1008
   Ala Ser Gln Cys Thr Ile Thr Pro Asn Thr Val Tyr Cys Arg Tyr Asn
                   325                 330                 335
   gat gcc caa gta ctc tca gat gat acg atg gct tgc ctc caa ggt aac    1056
   Asp Ala Gln Val Leu Ser Asp Asp Thr Met Ala Cys Leu Gln Gly Asn
               340                 345                 350
   ttg aca aga tgc acc ttc tct cca gtg gtt ggg agc ttt ctc act cga    1104
   Leu Thr Arg Cys Thr Phe Ser Pro Val Val Gly Ser Phe Leu Thr Arg
           355                 360                 365
   ttc gtg ctg ttt gat gga ata gtt tat gca aat tgc agg tcg atg ttg    1152
   Phe Val Leu Phe Asp Gly Ile Val Tyr Ala Asn Cys Arg Ser Met Leu
       370                 375                 380
   tgc aag tgc atg cag cct gct gct gtt atc cta cag ccg agc tca tcc    1200
   Cys Lys Cys Met Gln Pro Ala Ala Val Ile Leu Gln Pro Ser Ser Ser
   385                 390                 395                 400
   cct gta act gtc att gac atg tac aaa tgt gtg agt ctg cag ctt gac    1248
   Pro Val Thr Val Ile Asp Met Tyr Lys Cys Val Ser Leu Gln Leu Asp
                   405                 410                 415
   aat ctc aga ttc acc atc act caa ttg gcc aat ata acc tac aat agc    1296
   Asn Leu Arg Phe Thr Ile Thr Gln Leu Ala Asn Ile Thr Tyr Asn Ser
               420                 425                 430
   acc atc aag ctt gaa aca tcc cag atc ttg cct atc gat ccg ttg gat    1344
   Thr Ile Lys Leu Glu Thr Ser Gln Ile Leu Pro Ile Asp Pro Leu Asp
           435                 440                 445
   ata tcc cag aat cta gct gcg gtg aat aag agt cta agt gat gca cta      1392
   Ile Ser Gln Asn Leu Ala Ala Val Asn Lys Ser Leu Ser Asp Ala Leu
       450                 455                 460
   caa cac tta gca caa agt gac aca tac ctt tct gca atc aca tca gct      1440
   Gln His Leu Ala Gln Ser Asp Thr Tyr Leu Ser Ala Ile Thr Ser Ala
   465                 470                 475                 480
   acg act aca agt gta tta tcc ata ata gca atc tgt ctt gga tcg tta      1488
   Thr Thr Thr Ser Val Leu Ser Ile Ile Ala Ile Cys Leu Gly Ser Leu
                   485                 490                 495
   ggt tta ata tta ata atc ttg ctc agt gta gtt gtg tgg aag tta ttg      1536
   Gly Leu Ile Leu Ile Ile Leu Leu Ser Val Val Val Trp Lys Leu Leu
               500                 505                 510
   acc att gtt gct gct aat cga aat aga atg gag aat ttt gtt tat cat      1584
   Thr Ile Val Ala Ala Asn Arg Asn Arg Met Glu Asn Phe Val Tyr His
           515                 520                 525
   aat tca gca ttc cac cac tca cgg tct gat ctc agt gag aaa aat caa      1632
   Asn Ser Ala Phe His His Ser Arg Ser Asp Leu Ser Glu Lys Asn Gln
       530                 535                 540
   cct gca act ctt gga aca aga taa                                      1656
   Pro Ala Thr Leu Gly Thr Arg  *
   545                 550
<210> 26
   <211> 551
   <212> PRT
   <213> Canine parainfluenza virus
<400> 26
   Met Gly Thr Arg Ile Gln Phe Leu Val Val Ser Cys Leu Leu Ala Gly
    1               5                  10                  15
   Thr Gly Ser Leu Asp Pro Ala Ala Leu Met Gln Ile Gly Val Ile Pro
               20                  25                  30
   Thr Asn Val Arg Gln Leu Met Tyr Tyr Thr Glu Ala Ser Ser Ala Phe
           35                  40                  45
   Ile Val Val Lys Leu Met Pro Thr Ile Asp Ser Pro Ile Ser Gly Cys
       50                  55                  60
   Asn Ile Thr Ser Ile Ser Ser Tyr Asn Ala Thr Met Thr Lys Leu Leu
   65                  70                  75                  80
   Gln Pro Ile Gly Glu Asn Leu Glu Thr Ile Arg Tyr Gln Leu Ile Pro
                   85                  90                  95
   Thr Arg Arg Arg Arg Arg Phe Ala Gly Val Val Ile Gly Leu Ala Ala
               100                 105                 110
   Leu Gly Val Ala Thr Ala Ala Gln Val Thr Ala Ala Val Ala Leu Val
           115                 120                 125
   Lys Ala Asn Lys Asn Ala Val Ala Ile Leu Asn Leu Lys Asn Ala Ile
       130                 135                 140
   Gln Lys Thr Asn Ala Ala Val Ala Asp Val Val Gln Ala Thr Gln Ser
   145                 150                 155                 160
   Leu Gly Thr Ala Val Gln Ala Val Gln Asp His Ile Asn Ser Val Val
                   165                 170                 175
   Ser Pro Ala Ile Thr Ala Ala Asn Cys Lys Ala Gln Asp Ala Ile Ile
               180                 185                 190
   Gly Ser Ile Leu Asn Leu Tyr Leu Thr Glu Leu Thr Thr Ile Phe His
           195                 200                 205
   Asn Gln Ile Thr Asn Pro Ala Leu Ser Pro Ile Thr Ile Gln Ala Leu
       210                 215                 220
   Arg Ile Leu Leu Gly Ser Thr Leu Pro Thr Val Val Glu Lys Ser Phe
   225                 230                 235                 240
   Asn Thr Gln Ile Ser Ala Ala Glu Leu Leu Ser Ser Gly Leu Leu Thr
                   245                 250                 255
   Gly Gln Ile Val Gly Leu Asp Leu Thr Tyr Met Gln Met Val Ile Lys
               260                 265                 270
   Ile Glu Leu Pro Thr Leu Thr Val Gln Pro Ala Thr Gln Ile Ile Asp
           275                 280                 285
   Leu Val Thr Ile Ser Ala Phe Ile Asn Asn Gln Glu Val Met Ala Gln
       290                 295                 300
   Leu Pro Thr Arg Val Ile Val Thr Gly Ser Leu Ile Gln Ala Tyr Pro
   305                 310                 315                 320
   Ala Ser Gln Cys Thr Ile Thr Pro Asn Thr Val Tyr Cys Arg Tyr Asn
                   325                 330                 335
   Asp Ala Gln Val Leu Ser Asp Asp Thr Met Ala Cys Leu Gln Gly Asn
               340                 345                 350
   Leu Thr Arg Cys Thr Phe Ser Pro Val Val Gly Ser Phe Leu Thr Arg
           355                 360                 365
   Phe Val Leu Phe Asp Gly Ile Val Tyr Ala Asn Cys Arg Ser Met Leu
       370                 375                 380
   Cys Lys Cys Met Gln Pro Ala Ala Val Ile Leu Gln Pro Ser Ser Ser
   385                 390                 395                 400
   Pro Val Thr Val Ile Asp Met Tyr Lys Cys Val Ser Leu Gln Leu Asp
                   405                 410                 415
   Asn Leu Arg Phe Thr Ile Thr Gln Leu Ala Asn Ile Thr Tyr Asn Ser
               420                 425                 430
   Thr Ile Lys Leu Glu Thr Ser Gln Ile Leu Pro Ile Asp Pro Leu Asp
           435                 440                 445
   Ile Ser Gln Asn Leu Ala Ala Val Asn Lys Ser Leu Ser Asp Ala Leu
       450                 455                 460
   Gln His Leu Ala Gln Ser Asp Thr Tyr Leu Ser Ala Ile Thr Ser Ala
   465                 470                 475                 480
   Thr Thr Thr Ser Val Leu Ser Ile Ile Ala Ile Cys Leu Gly Ser Leu
                   485                 490                 495
   Gly Leu Ile Leu Ile Ile Leu Leu Ser Val Val Val Trp Lys Leu Leu
               500                 505                 510
   Thr Ile Val Ala Ala Asn Arg Asn Arg Met Glu Asn Phe Val Tyr His
           515                 520                 525
   Asn Ser Ala Phe His His Ser Arg Ser Asp Leu Ser Glu Lys Asn Gln
       530                 535                 540
   Pro Ala Thr Leu Gly Thr Arg
   545                 550
<210> 27
   <211> 1656
   <212> DNA
   <213> Porcine Rubulavirus
<220>
   <221> CDS
   <222> (1)...(1656)
   <223> Porcine Rubulavirus F protein encoding sequence with "TAA" termination codon; cDNA in mRNA sense
<400> 27
   atg ggt act ata att caa ttt ctg gtg gtc tcc tgt cta ttg gca gga    48
   Met Gly Thr Ile Ile Gln Phe Leu Val Val Ser Cys Leu Leu Ala Gly
    1               5                  10                  15
   gca ggc agc ctt gat cca gca gcc ctc atg caa atc ggt gtc att cca    96
   Ala Gly Ser Leu Asp Pro Ala Ala Leu Met Gln Ile Gly Val Ile Pro
               20                  25                  30
   aca aat gtc cgg caa ctt atg tat tat act gag gcc tca tca gca ttc    144
   Thr Asn Val Arg Gln Leu Met Tyr Tyr Thr Glu Ala Ser Ser Ala Phe
           35                  40                  45
   att gtt gtg aag tta atg cct aca att gac tcg ccg att agt gga tgt    192
   Ile Val Val Lys Leu Met Pro Thr Ile Asp Ser Pro Ile Ser Gly Cys
       50                  55                  60
   aat ata aca tca att tca agc tat aat gca aca gtg aca aaa ctc cta    240
   Asn Ile Thr Ser Ile Ser Ser Tyr Asn Ala Thr Val Thr Lys Leu Leu
   65                  70                  75                  80
   cag ccg atc ggt gag aat ttg gaa acg att agg aac cag ttg att cca    288
   Gln Pro Ile Gly Glu Asn Leu Glu Thr Ile Arg Asn Gln Leu Ile Pro
                   85                  90                  95
   act cgg agg aga cgc cgg ttt gca ggg gtg gtg att gga tta gct gca    336
   Thr Arg Arg Arg Arg Arg Phe Ala Gly Val Val Ile Gly Leu Ala Ala
               100                 105                 110
   tta gga gta gct act gcc gca cag gtc act gcc gca gta gca cta gta    384
   Leu Gly Val Ala Thr Ala Ala Gln Val Thr Ala Ala Val Ala Leu Val
           115                 120                 125
   aag gca aat aaa aat gct gcg gct ata ctc aat ctc aaa aat gca atc    432
   Lys Ala Asn Lys Asn Ala Ala Ala Ile Leu Asn Leu Lys Asn Ala Ile
       130                 135                 140
   caa aaa aca aat aca gca gtt gca gat gtg gtc cag gcc aca caa tca    480
   Gln Lys Thr Asn Thr Ala Val Ala Asp Val Val Gln Ala Thr Gln Ser
   145                 150                 155                 160
   cta gga acg gca gtt caa gca gtt caa gat cac ata aac agt gtg gta    528
   Leu Gly Thr Ala Val Gln Ala Val Gln Asp His Ile Asn Ser Val Val
                   165                 170                 175
   agt cca gca att aca gca gcc aat tgt aag gcc caa gat gct atc att    576
   Ser Pro Ala Ile Thr Ala Ala Asn Cys Lys Ala Gln Asp Ala Ile Ile
               180                 185                 190
   ggc tca atc ctc aat ctc tat ttg acc gag ttg aca act atc ttc cac      624
   Gly Ser Ile Leu Asn Leu Tyr Leu Thr Glu Leu Thr Thr Ile Phe His
           195                 200                 205
   aat caa att aca aac cct gca ttg agt cct att aca att caa gct tta      672
   Asn Gln Ile Thr Asn Pro Ala Leu Ser Pro Ile Thr Ile Gln Ala Leu
       210                 215                 220
   agg atc cta ctg ggg agt acc ttg ccg act gtg gtc gaa aaa tct ttc      720
   Arg Ile Leu Leu Gly Ser Thr Leu Pro Thr Val Val Glu Lys Ser Phe
   225                 230                 235                 240
   aat acc cag ata agt gca gct gag ctt ctc tca tca ggg tta ttg aca      768
   Asn Thr Gln Ile Ser Ala Ala Glu Leu Leu Ser Ser Gly Leu Leu Thr
                   245                 250                 255
   ggc cag att gtg gga tta gat ttg acc tat atg cag atg gtc ata aaa      816
   Gly Gln Ile Val Gly Leu Asp Leu Thr Tyr Met Gln Met Val Ile Lys
               260                 265                 270
   att gag ctg cca act tta act gta caa cct gca acc cag atc ata gat      864
   Ile Glu Leu Pro Thr Leu Thr Val Gln Pro Ala Thr Gln Ile Ile Asp
           275                 280                 285
   ctg gcc acc att tct gca ttc att aac aat caa gaa gtc atg gcc caa      912
   Leu Ala Thr Ile Ser Ala Phe Ile Asn Asn Gln Glu Val Met Ala Gln
       290                 295                 300
   tta cca aca cgt gtt att gtg act ggc agc ttg atc caa gcc tat ccc      960
   Leu Pro Thr Arg Val Ile Val Thr Gly Ser Leu Ile Gln Ala Tyr Pro
   305                 310                 315                 320
   gca tcg caa tgc act att aca ccc aac act gtg tac tgt agg tat aat     1008
   Ala Ser Gln Cys Thr Ile Thr Pro Asn Thr Val Tyr Cys Arg Tyr Asn
                   325                 330                 335
   gat gcc caa gta ctc tca gat gat acg atg gct tgc ctc caa ggt aac     1056
   Asp Ala Gln Val Leu Ser Asp Asp Thr Met Ala Cys Leu Gln Gly Asn
               340                 345                 350
   ttg aca aga tgc acc ttc tct ccg gtg gtt ggg agc ttt ctc act cga     1104
   Leu Thr Arg Cys Thr Phe Ser Pro Val Val Gly Ser Phe Leu Thr Arg
           355                 360                 365
   ttc atg ctg ttc gat gga ata gtt tat gca aat tgc agg tcg atg tta     1152
   Phe Met Leu Phe Asp Gly Ile Val Tyr Ala Asn Cys Arg Ser Met Leu
       370                 375                 380
   tgc aag tgc atg cag cct gct gct gtg atc cta cag ccg agt tca tcc     1200
   Cys Lys Cys Met Gln Pro Ala Ala Val Ile Leu Gln Pro Ser Ser Ser
   385                 390                 395                 400
   cct gta act gtc att gac atg tac aaa tgt gtg agt ctg cag ctt gac     1248
   Pro Val Thr Val Ile Asp Met Tyr Lys Cys Val Ser Leu Gln Leu Asp
                   405                 410                 415
   aat ctc aga ttc acc atc act caa ttg gcc aat gta acc tac aat agc     1296
   Asn Leu Arg Phe Thr Ile Thr Gln Leu Ala Asn Val Thr Tyr Asn Ser
               420                 425                 430
   acc atc aag ctt gaa aca tcc cag atc ttg cct att gat ccg ttg gat     1344
   Thr Ile Lys Leu Glu Thr Ser Gln Ile Leu Pro Ile Asp Pro Leu Asp
           435                 440                 445
   ata tcc cag aat cta gct gcg gtg aat aag agt cta agt gat gca cta     1392
   Ile Ser Gln Asn Leu Ala Ala Val Asn Lys Ser Leu Ser Asp Ala Leu
       450                 455                 460
   caa cac tta gca caa agt gac aca tac ctt tct gca atc aca tca gct     1440
   Gln His Leu Ala Gln Ser Asp Thr Tyr Leu Ser Ala Ile Thr Ser Ala
   465                 470                 475                 480
   acg act aca agt gta tta tcc ata atg gca atc tgt ctt gga tcg tta     1488
   Thr Thr Thr Ser Val Leu Ser Ile Met Ala Ile Cys Leu Gly Ser Leu
                   485                 490                 495
   ggt tta ata tta ata atc ttg ctc agt gta gtt gtg tgg aag tta ttg     1536
   Gly Leu Ile Leu Ile Ile Leu Leu Ser Val Val Val Trp Lys Leu Leu
               500                 505                 510
   acc att gtc act gct aat cga aat aga atg gag aat ttt gtt tat cat     1584
   Thr Ile Val Thr Ala Asn Arg Asn Arg Met Glu Asn Phe Val Tyr His
           515                 520                 525
   aat tca gca ttc cac cac tca cga tct gat ctc agt gag aaa aat caa     1632
   Asn Ser Ala Phe His His Ser Arg Ser Asp Leu Ser Glu Lys Asn Gln
       530                 535                 540
   cct gca act ctt gga aca aga taa                                     1656
   Pro Ala Thr Leu Gly Thr Arg  *
   545                 550
<210> 28
   <211> 551
   <212> PRT
   <213> Porcine Rubulavirus
<400> 28
   Met Gly Thr Ile Ile Gln Phe Leu Val Val Ser Cys Leu Leu Ala Gly
    1               5                  10                  15
   Ala Gly Ser Leu Asp Pro Ala Ala Leu Met Gln Ile Gly Val Ile Pro
               20                  25                  30
   Thr Asn Val Arg Gln Leu Met Tyr Tyr Thr Glu Ala Ser Ser Ala Phe
           35                  40                  45
   Ile Val Val Lys Leu Met Pro Thr Ile Asp Ser Pro Ile Ser Gly Cys
       50                  55                  60
   Asn Ile Thr Ser Ile Ser Ser Tyr Asn Ala Thr Val Thr Lys Leu Leu
   65                  70                  75                  80
   Gln Pro Ile Gly Glu Asn Leu Glu Thr Ile Arg Asn Gln Leu Ile Pro
                   85                  90                  95
   Thr Arg Arg Arg Arg Arg Phe Ala Gly Val Val Ile Gly Leu Ala Ala
               100                 105                 110
   Leu Gly Val Ala Thr Ala Ala Gln Val Thr Ala Ala Val Ala Leu Val
           115                 120                 125
   Lys Ala Asn Lys Asn Ala Ala Ala Ile Leu Asn Leu Lys Asn Ala Ile
       130                 135                 140
   Gln Lys Thr Asn Thr Ala Val Ala Asp Val Val Gln Ala Thr Gln Ser
   145                 150                 155                 160
   Leu Gly Thr Ala Val Gln Ala Val Gln Asp His Ile Asn Ser Val Val
                   165                 170                 175
   Ser Pro Ala Ile Thr Ala Ala Asn Cys Lys Ala Gln Asp Ala Ile Ile
               180                 185                 190
   Gly Ser Ile Leu Asn Leu Tyr Leu Thr Glu Leu Thr Thr Ile Phe His
           195                 200                 205
   Asn Gln Ile Thr Asn Pro Ala Leu Ser Pro Ile Thr Ile Gln Ala Leu
       210                 215                 220
   Arg Ile Leu Leu Gly Ser Thr Leu Pro Thr Val Val Glu Lys Ser Phe
   225                 230                 235                 240
   Asn Thr Gln Ile Ser Ala Ala Glu Leu Leu Ser Ser Gly Leu Leu Thr
                   245                 250                 255
   Gly Gln Ile Val Gly Leu Asp Leu Thr Tyr Met Gln Met Val Ile Lys
               260                 265                 270
   Ile Glu Leu Pro Thr Leu Thr Val Gln Pro Ala Thr Gln Ile Ile Asp
           275                 280                 285
   Leu Ala Thr Ile Ser Ala Phe Ile Asn Asn Gln Glu Val Met Ala Gln
       290                 295                 300
   Leu Pro Thr Arg Val Ile Val Thr Gly Ser Leu Ile Gln Ala Tyr Pro
   305                 310                 315                 320
   Ala Ser Gln Cys Thr Ile Thr Pro Asn Thr Val Tyr Cys Arg Tyr Asn
                   325                 330                 335
   Asp Ala Gln Val Leu Ser Asp Asp Thr Met Ala Cys Leu Gln Gly Asn
               340                 345                 350
   Leu Thr Arg Cys Thr Phe Ser Pro Val Val Gly Ser Phe Leu Thr Arg
           355                 360                 365
   Phe Met Leu Phe Asp Gly Ile Val Tyr Ala Asn Cys Arg Ser Met Leu
       370                 375                 380
   Cys Lys Cys Met Gln Pro Ala Ala Val Ile Leu Gln Pro Ser Ser Ser
   385                 390                 395                 400
   Pro Val Thr Val Ile Asp Met Tyr Lys Cys Val Ser Leu Gln Leu Asp
                   405                 410                 415
   Asn Leu Arg Phe Thr Ile Thr Gln Leu Ala Asn Val Thr Tyr Asn Ser
               420                 425                 430
   Thr Ile Lys Leu Glu Thr Ser Gln Ile Leu Pro Ile Asp Pro Leu Asp
           435                 440                 445
   Ile Ser Gln Asn Leu Ala Ala Val Asn Lys Ser Leu Ser Asp Ala Leu
       450                 455                 460
   Gln His Leu Ala Gln Ser Asp Thr Tyr Leu Ser Ala Ile Thr Ser Ala
   465                 470                 475                 480
   Thr Thr Thr Ser Val Leu Ser Ile Met Ala Ile Cys Leu Gly Ser Leu
                   485                 490                 495
   Gly Leu Ile Leu Ile Ile Leu Leu Ser Val Val Val Trp Lys Leu Leu
               500                 505                 510
   Thr Ile Val Thr Ala Asn Arg Asn Arg Met Glu Asn Phe Val Tyr His
           515                 520                 525
   Asn Ser Ala Phe His His Ser Arg Ser Asp Leu Ser Glu Lys Asn Gln
       530                 535                 540
   Pro Ala Thr Leu Gly Thr Arg
   545                 550
<210> 29
   <211> 1656
   <212> DNA
   <213> Simian virus 5
<220>
   <221> CDS
   <222> (1)...(1590)
   <223> Simian virus 5 W3A strain F protein encoding sequence; cDNA in mRNA sense
<400> 29
   atg ggt act ata att caa ttt ctg gtg gtc tcc tgt cta ttg gca gga      48
   Met Gly Thr Ile Ile Gln Phe Leu Val Val Ser Cys Leu Leu Ala Gly
    1               5                  10                  15
   gca ggc agc ctt gat cca gca gcc ctc atg caa atc ggt gtc att cca      96
   Ala Gly Ser Leu Asp Pro Ala Ala Leu Met Gln Ile Gly Val Ile Pro
               20                  25                  30
   aca aat gtc cgg caa ctt atg tat tat act gag gcc tca tca gca ttc     144
   Thr Asn Val Arg Gln Leu Met Tyr Tyr Thr Glu Ala Ser Ser Ala Phe
           35                  40                  45
   att gtt gtg aag tta atg cct aca att gac tcg ccg att agt gga tgt     192
   Ile Val Val Lys Leu Met Pro Thr Ile Asp Ser Pro Ile Ser Gly Cys
       50                  55                  60
   aat ata aca tca att tca agc tat aat gca aca gtg aca aaa ctc cta     240
   Asn Ile Thr Ser Ile Ser Ser Tyr Asn Ala Thr Val Thr Lys Leu Leu
   65                  70                  75                  80
   cag ccg atc ggt gag aat ttg gag acg att agg aac cag ttg att cca     288
   Gln Pro Ile Gly Glu Asn Leu Glu Thr Ile Arg Asn Gln Leu Ile Pro
                   85                  90                  95
   act cgg agg aga cgc cgg ttt gca ggg gtg gtg att gga tta gct gca     336
   Thr Arg Arg Arg Arg Arg Phe Ala Gly Val Val Ile Gly Leu Ala Ala
               100                 105                 110
   tta gga gta gct act gcc gca cag gtc act gcc gca gtg gca cta gta     384
   Leu Gly Val Ala Thr Ala Ala Gln Val Thr Ala Ala Val Ala Leu Val
           115                 120                 125
   aag gca aat gaa aat gct gcg gct ata ctc aat ctc aaa aat gca atc     432
   Lys Ala Asn Glu Asn Ala Ala Ala Ile Leu Asn Leu Lys Asn Ala Ile
       130                 135                 140
   caa aaa aca aat gca gca gtt gca gat gtg gtc cag gcc aca caa tca    480
   Gln Lys Thr Asn Ala Ala Val Ala Asp Val Val Gln Ala Thr Gln Ser
   145                 150                 155                 160
   cta gga acg gca gtt caa gca gtt caa gat cac ata aac agt gtg gta    528
   Leu Gly Thr Ala Val Gln Ala Val Gln Asp His Ile Asn Ser Val Val
                   165                 170                 175
   agt cca gca att aca gca gcc aat tgt aag gcc caa gat gct atc att    576
   Ser Pro Ala Ile Thr Ala Ala Asn Cys Lys Ala Gln Asp Ala Ile Ile
               180                 185                 190
   ggc tca atc ctc aat ctc tat ttg acc gag ttg aca acc atc ttc cac    624
   Gly Ser Ile Leu Asn Leu Tyr Leu Thr Glu Leu Thr Thr Ile Phe His
           195                 200                 205
   aat caa att aca aac cct gca ttg agt ccc att aca att caa gct tta    672
   Asn Gln Ile Thr Asn Pro Ala Leu Ser Pro Ile Thr Ile Gln Ala Leu
       210                 215                 220
   agg atc cta ctg ggg agt acc ttg ccg act gtg gtc gaa aaa tct ttc    720
   Arg Ile Leu Leu Gly Ser Thr Leu Pro Thr Val Val Glu Lys Ser Phe
   225                 230                 235                 240
   aat acc cag ata agt gca gct gag ctt ctc tca tca ggg tta ttg aca    768
   Asn Thr Gln Ile Ser Ala Ala Glu Leu Leu Ser Ser Gly Leu Leu Thr
                   245                 250                 255
   ggc cag att gtg gga tta gat ttg acc tat atg cag atg gtc ata aaa    816
   Gly Gln Ile Val Gly Leu Asp Leu Thr Tyr Met Gln Met Val Ile Lys
               260                 265                 270
   att gag ctg cca act tta act gta caa cct gca acc cag atc ata gat    864
   Ile Glu Leu Pro Thr Leu Thr Val Gln Pro Ala Thr Gln Ile Ile Asp
           275                 280                 285
   ctg gcc acc att tct gca ttc att aac aat caa gaa gtc atg gcc caa    912
   Leu Ala Thr Ile Ser Ala Phe Ile Asn Asn Gln Glu Val Met Ala Gln
       290                 295                 300
   tta cca aca cgt gtt atg gtg act ggc agc ttg atc caa gcc tat ccc    960
   Leu Pro Thr Arg Val Met Val Thr Gly Ser Leu Ile Gln Ala Tyr Pro
   305                 310                 315                 320
   gca tcg caa tgc acc att aca ccc aac act gtg tac tgt agg tat aat    1008
   Ala Ser Gln Cys Thr Ile Thr Pro Asn Thr Val Tyr Cys Arg Tyr Asn
                   325                 330                 335
   gat gcc caa gta ctc tca gat gat act atg gct tgc ctc caa ggt aac    1056
   Asp Ala Gln Val Leu Ser Asp Asp Thr Met Ala Cys Leu Gln Gly Asn
               340                 345                 350
   ttg aca aga tgc acc ttc tct cca gtg gtt ggg agc ttt ctc act cga    1104
   Leu Thr Arg Cys Thr Phe Ser Pro Val Val Gly Ser Phe Leu Thr Arg
           355                 360                 365
   ttc gtg ctg ttc gat gga ata gtt tat gca aat tgc agg tcg atg ttg      1152
   Phe Val Leu Phe Asp Gly Ile Val Tyr Ala Asn Cys Arg Ser Met Leu
       370                 375                 380
   tgc aag tgc atg caa cct gct gct gtg atc cta cag ccg agt tca tcc      1200
   Cys Lys Cys Met Gln Pro Ala Ala Val Ile Leu Gln Pro Ser Ser Ser
   385                 390                 395                 400
   cct gta act gtc att gac atg tac aaa tgt gtg agt ctg cag ctt gac      1248
   Pro Val Thr Val Ile Asp Met Tyr Lys Cys Val Ser Leu Gln Leu Asp
                   405                 410                 415
   aat ctc aga ttc acc atc act caa ttg gcc aat gta acc tac aat agc      1296
   Asn Leu Arg Phe Thr Ile Thr Gln Leu Ala Asn Val Thr Tyr Asn Ser
               420                 425                 430
   acc atc aag ctt gaa tca tcc cag atc ttg tct att gat ccg ttg gat      1344
   Thr Ile Lys Leu Glu Ser Ser Gln Ile Leu Ser Ile Asp Pro Leu Asp
           435                 440                 445
   ata tcc caa aat cta gct gcg gtg aat aag agt cta agt gat gca cta      1392
   Ile Ser Gln Asn Leu Ala Ala Val Asn Lys Ser Leu Ser Asp Ala Leu
       450                 455                 460
   caa cac tta gca caa agt gac aca tat ctt tct gca atc aca tca gct      1440
   Gln His Leu Ala Gln Ser Asp Thr Tyr Leu Ser Ala Ile Thr Ser Ala
   465                 470                 475                 480
   acg act aca agt gta tta tcc ata ata gca atc tgt ctt gga tcg tta      1488
   Thr Thr Thr Ser Val Leu Ser Ile Ile Ala Ile Cys Leu Gly Ser Leu
                   485                 490                 495
   ggt tta ata tta ata atc ttg ctc agt gta gtt gtg tgg aag tta ttg      1536
   Gly Leu Ile Leu Ile Ile Leu Leu Ser Val Val Val Trp Lys Leu Leu
               500                 505                 510
   acc att gtc gtt gct aat cga aat aga atg gag aat ttt gtt tat cat      1584
   Thr Ile Val Val Ala Asn Arg Asn Arg Met Glu Asn Phe Val Tyr His
           515                 520                 525
   aaa taa gcattccacc actcacgatc tgatctcagt gagaaaaatc aacctgcaac       1640
   Lys  *
   tcttggaaca agataa                                                    1656
<210> 30
   <211> 529
   <212> PRT
   <213> Simian virus 5
<400> 30
   Met Gly Thr Ile Ile Gln Phe Leu Val Val Ser Cys Leu Leu Ala Gly
   1               5                   10                  15
   Ala Gly Ser Leu Asp Pro Ala Ala Leu Met Gln Ile Gly Val Ile Pro
               20                  25                  30
   Thr Asn Val Arg Gln Leu Met Tyr Tyr Thr Glu Ala Ser Ser Ala Phe
           35                  40                  45
   Ile Val Val Lys Leu Met Pro Thr Ile Asp Ser Pro Ile Ser Gly Cys
       50                  55                  60
   Asn Ile Thr Ser Ile Ser Ser Tyr Asn Ala Thr Val Thr Lys Leu Leu
   65                  70                  75                  80
   Gln Pro Ile Gly Glu Asn Leu Glu Thr Ile Arg Asn Gln Leu Ile Pro
                   85                  90                  95
   Thr Arg Arg Arg Arg Arg Phe Ala Gly Val Val Ile Gly Leu Ala Ala
               100                 105                 110
   Leu Gly Val Ala Thr Ala Ala Gln Val Thr Ala Ala Val Ala Leu Val
           115                 120                 125
   Lys Ala Asn Glu Asn Ala Ala Ala Ile Leu Asn Leu Lys Asn Ala Ile
       130                 135                 140
   Gln Lys Thr Asn Ala Ala Val Ala Asp Val Val Gln Ala Thr Gln Ser
   145                 150                 155                 160
   Leu Gly Thr Ala Val Gln Ala Val Gln Asp His Ile Asn Ser Val Val
                   165                 170                 175
   Ser Pro Ala Ile Thr Ala Ala Asn Cys Lys Ala Gln Asp Ala Ile Ile
               180                 185                 190
   Gly Ser Ile Leu Asn Leu Tyr Leu Thr Glu Leu Thr Thr Ile Phe His
           195                 200                 205
   Asn Gln Ile Thr Asn Pro Ala Leu Ser Pro Ile Thr Ile Gln Ala Leu
       210                 215                 220
   Arg Ile Leu Leu Gly Ser Thr Leu Pro Thr Val Val Glu Lys Ser Phe
   225                 230                 235                 240
   Asn Thr Gln Ile Ser Ala Ala Glu Leu Leu Ser Ser Gly Leu Leu Thr
                   245                 250                 255
   Gly Gln Ile Val Gly Leu Asp Leu Thr Tyr Met Gln Met Val Ile Lys
               260                 265                 270
   Ile Glu Leu Pro Thr Leu Thr Val Gln Pro Ala Thr Gln Ile Ile Asp
           275                 280                 285
   Leu Ala Thr Ile Ser Ala Phe Ile Asn Asn Gln Glu Val Met Ala Gln
       290                 295                 300
   Leu Pro Thr Arg Val Met Val Thr Gly Ser Leu Ile Gln Ala Tyr Pro
   305                 310                 315                 320
   Ala Ser Gln Cys Thr Ile Thr Pro Asn Thr Val Tyr Cys Arg Tyr Asn
                   325                 330                 335
   Asp Ala Gln Val Leu Ser Asp Asp Thr Met Ala Cys Leu Gln Gly Asn
               340                 345                 350
   Leu Thr Arg Cys Thr Phe Ser Pro Val Val Gly Ser Phe Leu Thr Arg
           355                 360                 365
   Phe Val Leu Phe Asp Gly Ile Val Tyr Ala Asn Cys Arg Ser Met Leu
       370                 375                 380
   Cys Lys Cys Met Gln Pro Ala Ala Val Ile Leu Gln Pro Ser Ser Ser
   385                 390                 395                 400
   Pro Val Thr Val Ile Asp Met Tyr Lys Cys Val Ser Leu Gln Leu Asp
                   405                 410                 415
   Asn Leu Arg Phe Thr Ile Thr Gln Leu Ala Asn Val Thr Tyr Asn Ser
               420                 425                 430
   Thr Ile Lys Leu Glu Ser Ser Gln Ile Leu Ser Ile Asp Pro Leu Asp
           435                 440                 445
   Ile Ser Gln Asn Leu Ala Ala Val Asn Lys Ser Leu Ser Asp Ala Leu
       450                 455                 460
   Gln His Leu Ala Gln Ser Asp Thr Tyr Leu Ser Ala Ile Thr Ser Ala
   465                 470                 475                 480
   Thr Thr Thr Ser Val Leu Ser Ile Ile Ala Ile Cys Leu Gly Ser Leu
                   485                 490                 495
   Gly Leu Ile Leu Ile Ile Leu Leu Ser Val Val Val Trp Lys Leu Leu
               500                 505                 510
   Thr Ile Val Val Ala Asn Arg Asn Arg Met Glu Asn Phe Val Tyr His
           515                 520                 525
   Lys
<210> 31
   <211> 1656
   <212> DNA
   <213> Simian virus 5
<220>
   <221> CDS
   <222> (1)...(1590)
   <223> Simian virus 5 WR strain F protein encoding sequence; cDNA in mRNA sense
<400> 31
   atg ggt act att att caa ttt ctg gtg gtc tcc tgt cta ttg gca gga      48
   Met Gly Thr Ile Ile Gln Phe Leu Val Val Ser Cys Leu Leu Ala Gly
    1               5                  10                  15
   gca ggc agc ctt gat cta gca gcc ctc atg caa atc ggt gtc att cca      96
   Ala Gly Ser Leu Asp Leu Ala Ala Leu Met Gln Ile Gly Val Ile Pro
               20                  25                  30
   aca aat gtc cgg caa ctt atg tat tat act gag gcc tca tcg gca ttc     144
   Thr Asn Val Arg Gln Leu Met Tyr Tyr Thr Glu Ala Ser Ser Ala Phe
           35                  40                  45
   att gtt gtg aag tta atg cct aca att gac tcg ccg att agt gga tgt     192
   Ile Val Val Lys Leu Met Pro Thr Ile Asp Ser Pro Ile Ser Gly Cys
       50                  55                  60
   aat ata aca tca att tca agc tat aat gca aca gtg aca aaa ctc cta     240
   Asn Ile Thr Ser Ile Ser Ser Tyr Asn Ala Thr Val Thr Lys Leu Leu
   65                  70                  75                  80
   cag ccg atc ggt gag aat ttg gag acg att agg aac cag ttg att cca     288
   Gln Pro Ile Gly Glu Asn Leu Glu Thr Ile Arg Asn Gln Leu Ile Pro
                   85                  90                  95
   act cgg aga aga cgc cgg ttt gca ggg gtg gtg att gga tta gct gca     336
   Thr Arg Arg Arg Arg Arg Phe Ala Gly Val Val Ile Gly Leu Ala Ala
               100                 105                 110
   tta gga gta gct act gcc gca cag gtc act gcc gca gta gca cta gta     384
   Leu Gly Val Ala Thr Ala Ala Gln Val Thr Ala Ala Val Ala Leu Val
           115                 120                 125
   aag gca aat gaa aat gct gcg gct ata ctc aat ctc aaa aat gca atc      432
   Lys Ala Asn Glu Asn Ala Ala Ala Ile Leu Asn Leu Lys Asn Ala Ile
       130                 135                 140
   caa aaa aca aat gca gca gtt gca gat gtg gtc cag gcc aca caa tca      480
   Gln Lys Thr Asn Ala Ala Val Ala Asp Val Val Gln Ala Thr Gln Ser
   145                 150                 155                 160
   cta gga acg gca gtt caa gca gtt caa gat cac ata aac agt gtg gta      528
   Leu Gly Thr Ala Val Gln Ala Val Gln Asp His Ile Asn Ser Val Val
                   165                 170                 175
   agt cca gca att aca gca gcc aat tgt aag gcc caa gat gct atc att      576
   Ser Pro Ala Ile Thr Ala Ala Asn Cys Lys Ala Gln Asp Ala Ile Ile
               180                 185                 190
   ggc tca atc ctc aat ctc tat ttg acc gag ttg aca acc atc ttc cac      624
   Gly Ser Ile Leu Asn Leu Tyr Leu Thr Glu Leu Thr Thr Ile Phe His
           195                 200                 205
   aat caa att aca aac cct gca ttg agt ccc att aca att caa gct tta      672
   Asn Gln Ile Thr Asn Pro Ala Leu Ser Pro Ile Thr Ile Gln Ala Leu
       210                 215                 220
   agg atc cta ctg ggg agt acc ttg ccg act gtg gtc gaa aaa tct ttc      720
   Arg Ile Leu Leu Gly Ser Thr Leu Pro Thr Val Val Glu Lys Ser Phe
   225                 230                 235                 240
   aat acc cag ata agt gca gct gag ctt ctc tca tca ggg tta ttg aca      768
   Asn Thr Gln Ile Ser Ala Ala Glu Leu Leu Ser Ser Gly Leu Leu Thr
                   245                 250                 255
   ggc cag att gtg gga tta gat ttg acc tat atg cag atg gtc ata aaa      816
   Gly Gln Ile Val Gly Leu Asp Leu Thr Tyr Met Gln Met Val Ile Lys
               260                 265                 270
   att gag ctg cca act tta act gta caa cct gca acc cag atc ata gat      864
   Ile Glu Leu Pro Thr Leu Thr Val Gln Pro Ala Thr Gln Ile Ile Asp
           275                 280                 285
   ctg gcc acc att tct gca ttc att aac aat caa gaa gtc atg gcc caa      912
   Leu Ala Thr Ile Ser Ala Phe Ile Asn Asn Gln Glu Val Met Ala Gln
       290                 295                 300
   tta cca aca cgt gtt atg gtg act ggc agc ttg atc caa gcc tat ccc      960
   Leu Pro Thr Arg Val Met Val Thr Gly Ser Leu Ile Gln Ala Tyr Pro
   305                 310                 315                 320
   gca tcg caa tgc acc att aca ccc aac act gtg tac tgt agg tat aat     1008
   Ala Ser Gln Cys Thr Ile Thr Pro Asn Thr Val Tyr Cys Arg Tyr Asn
                   325                 330                 335
   gat gcc caa gta ctc tca gat gat act atg gct tgc ctc caa ggt aac     1056
   Asp Ala Gln Val Leu Ser Asp Asp Thr Met Ala Cys Leu Gln Gly Asn
               340                 345                 350
   ttg aca aga tgc acc ttc tct cca gtg gtt ggg agc ttt ctc act cga     1104
   Leu Thr Arg Cys Thr Phe Ser Pro Val Val Gly Ser Phe Leu Thr Arg
           355                 360                 365
   ttc gtg ctg ttc gat gga ata gtt tat gca aat tgc agg tcg atg ttg     1152
   Phe Val Leu Phe Asp Gly Ile Val Tyr Ala Asn Cys Arg Ser Met Leu
       370                 375                 380
   tgc aag tgc atg caa cct gct gct gtg atc cta cag ccg agt tca tcc     1200
   Cys Lys Cys Met Gln Pro Ala Ala Val Ile Leu Gln Pro Ser Ser Ser
   385                 390                 395                 400
   cct gta act gtc att gac atg tac aaa tgt gtg agt ctg cag ctt gac     1248
   Pro Val Thr Val Ile Asp Met Tyr Lys Cys Val Ser Leu Gln Leu Asp
                   405                 410                 415
   aat ctc aga ttc acc atc act caa ttg gcc aat gta acc tac aat agc     1296
   Asn Leu Arg Phe Thr Ile Thr Gln Leu Ala Asn Val Thr Tyr Asn Ser
               420                 425                 430
   acc atc aag ctt gaa tca tcc cag atc ttg cct att gat ccg ttg gat     1344
   Thr Ile Lys Leu Glu Ser Ser Gln Ile Leu Pro Ile Asp Pro Leu Asp
           435                 440                 445
   ata tcc cag aat cta gct gcg gtg aat aag agt cta agt gat gca cta     1392
   Ile Ser Gln Asn Leu Ala Ala Val Asn Lys Ser Leu Ser Asp Ala Leu
       450                 455                 460
   caa cac tta gca caa agt gac aca tat ctt tct gca atc aca tca gct     1440
   Gln His Leu Ala Gln Ser Asp Thr Tyr Leu Ser Ala Ile Thr Ser Ala
   465                 470                 475                 480
   acg act aca agt gta tta tcc ata ata gca atc tgt ctt gga tcg tta     1488
   Thr Thr Thr Ser Val Leu Ser Ile Ile Ala Ile Cys Leu Gly Ser Leu
                   485                 490                 495
   ggt tta ata tta ata atc ttg ctc agt gta gtt gtg tgg aag tta ttg     1536
   Gly Leu Ile Leu Ile Ile Leu Leu Ser Val Val Val Trp Lys Leu Leu
               500                 505                 510
   acc att gtc gct gct aat cga aat aga atg gag aat ttt gtt tat cat     1584
   Thr Ile Val Ala Ala Asn Arg Asn Arg Met Glu Asn Phe Val Tyr His
           515                 520                 525
   aaa taa gcattccacc actcacgatc tgatctcagt gagaaaaatc aacctgcaac     1640
   Lys  *
   tcttggaaca agataa                                                    1656
<210> 32
   <211> 529
   <212> PRT
   <213> Simian virus 5
<400> 32
   Met Gly Thr Ile Ile Gln Phe Leu Val Val Ser Cys Leu Leu Ala Gly
   1               5                   10                  15
   Ala Gly Ser Leu Asp Leu Ala Ala Leu Met Gln Ile Gly Val Ile Pro
               20                  25                  30
   Thr Asn Val Arg Gln Leu Met Tyr Tyr Thr Glu Ala Ser Ser Ala Phe
           35                  40                  45
   Ile Val Val Lys Leu Met Pro Thr Ile Asp Ser Pro Ile Ser Gly Cys
       50                  55                  60
   Asn Ile Thr Ser Ile Ser Ser Tyr Asn Ala Thr Val Thr Lys Leu Leu
   65                  70                  75                  80
   Gln Pro Ile Gly Glu Asn Leu Glu Thr Ile Arg Asn Gln Leu Ile Pro
                   85                  90                  95
   Thr Arg Arg Arg Arg Arg Phe Ala Gly Val Val Ile Gly Leu Ala Ala
               100                 105                 110
   Leu Gly Val Ala Thr Ala Ala Gln Val Thr Ala Ala Val Ala Leu Val
           115                 120                 125
   Lys Ala Asn Glu Asn Ala Ala Ala Ile Leu Asn Leu Lys Asn Ala Ile
       130                 135                 140
   Gln Lys Thr Asn Ala Ala Val Ala Asp Val Val Gln Ala Thr Gln Ser
   145                 150                 155                 160
   Leu Gly Thr Ala Val Gln Ala Val Gln Asp His Ile Asn Ser Val Val
                   165                 170                 175
   Ser Pro Ala Ile Thr Ala Ala Asn Cys Lys Ala Gln Asp Ala Ile Ile
               180                 185                 190
   Gly Ser Ile Leu Asn Leu Tyr Leu Thr Glu Leu Thr Thr Ile Phe His
           195                 200                 205
   Asn Gln Ile Thr Asn Pro Ala Leu Ser Pro Ile Thr Ile Gln Ala Leu
       210                 215                 220
   Arg Ile Leu Leu Gly Ser Thr Leu Pro Thr Val Val Glu Lys Ser Phe
   225                 230                 235                 240
   Asn Thr Gln Ile Ser Ala Ala Glu Leu Leu Ser Ser Gly Leu Leu Thr
                   245                 250                 255
   Gly Gln Ile Val Gly Leu Asp Leu Thr Tyr Met Gln Met Val Ile Lys
               260                 265                 270
   Ile Glu Leu Pro Thr Leu Thr Val Gln Pro Ala Thr Gln Ile Ile Asp
           275                 280                 285
   Leu Ala Thr Ile Ser Ala Phe Ile Asn Asn Gln Glu Val Met Ala Gln
       290                 295                 300
   Leu Pro Thr Arg Val Met Val Thr Gly Ser Leu Ile Gln Ala Tyr Pro
   305                 310                 315                 320
   Ala Ser Gln Cys Thr Ile Thr Pro Asn Thr Val Tyr Cys Arg Tyr Asn
                   325                 330                 335
   Asp Ala Gln Val Leu Ser Asp Asp Thr Met Ala Cys Leu Gln Gly Asn
               340                 345                 350
   Leu Thr Arg Cys Thr Phe Ser Pro Val Val Gly Ser Phe Leu Thr Arg
           355                 360                 365
   Phe Val Leu Phe Asp Gly Ile Val Tyr Ala Asn Cys Arg Ser Met Leu
       370                 375                 380
   Cys Lys Cys Met Gln Pro Ala Ala Val Ile Leu Gln Pro Ser Ser Ser
   385                 390                 395                 400
   Pro Val Thr Val Ile Asp Met Tyr Lys Cys Val Ser Leu Gln Leu Asp
                   405                 410                 415
   Asn Leu Arg Phe Thr Ile Thr Gln Leu Ala Asn Val Thr Tyr Asn Ser
               420                 425                 430
   Thr Ile Lys Leu Glu Ser Ser Gln Ile Leu Pro Ile Asp Pro Leu Asp
           435                 440                 445
   Ile Ser Gln Asn Leu Ala Ala Val Asn Lys Ser Leu Ser Asp Ala Leu
       450                 455                 460
   Gln His Leu Ala Gln Ser Asp Thr Tyr Leu Ser Ala Ile Thr Ser Ala
   465                 470                 475                 480
   Thr Thr Thr Ser Val Leu Ser Ile Ile Ala Ile Cys Leu Gly Ser Leu
                   485                 490                 495
   Gly Leu Ile Leu Ile Ile Leu Leu Ser Val Val Val Trp Lys Leu Leu
               500                 505                 510
   Thr Ile Val Ala Ala Asn Arg Asn Arg Met Glu Asn Phe Val Tyr His
           515                 520                 525
   Lys
<210> 33
   <211> 1174
   <212> DNA
   <213> Cryptovirus
<220>
   <223> cDNA in mRNA sense
<400> 33
   atggatccca ctgatctgag cttctcccca gatgagatta ataagctcat agagacaggc 60
   ctgaatactg tggagtattt tacttcccaa caagtcacag gaacatcctc tcttggaaag 120
   aatacaatac caccaggggt cacaggacta ctaaccaatg ctgcagaggc aaagatccaa 180
   gagtcaatca accatcagaa gggttcagtt ggtgggggta caaacccaaa gaaaccgcga 240
   tcaaaaattg ccattgtgcc agcagatgac aaaacagtgc ccgaaaagcc gatcccaaac 300
   cctctactag gtctggactc caccccgagc acccaaaccg tgcttgatct aagtgggaaa 360
   acattaccat caggatccta taagggggtt aagcttgcga aatttgggaa agaaaatctg 420
   atgacacggt tcatcgagga acccagagag aatcctatcg caaccagttc ccccatcgat 480
   tttaagaggg gcagagatac cggtgggttc catagaaggg agtactcaat cggatgggtg 540
   ggagatgaag tcaaggtcac tgagtggtgc aatccatcct gttctccaat caccgctgca 600
   gcaaggcgat ttaaatgcac ttgtcaccaa tgtccagtca cttgctctga atgtgaacga 660
   gatacttaat acagtgagaa atttggactc tcggatgaat caactggaga caaaagtaga 720
   tcgcattctc tcatctcagt ctctaatcca gaccatcaag aatgacataa ttggacttaa 780
   agcagggatg gctactttag aaggaatgat tacaactgtg aaaatcatgg acccgggagt 840
   tcccagtaat gttactgtgg aagatgtacg caagaaacta agtaaccatg ctgttgttgt 900
   gccagaatca ttcaatgata gtttcttgac tcaatctgaa gatgtaattt cacttgatga 960
   gttggctcga ccaactgcaa caagtgttaa gaagattgtc aggaaggttc ctcctcagaa 1020
   ggatctgact ggattgaaga tcacactaga gcaattggca aaggattgca tcagcaaacc 1080
   gaagatgagg gaagattatc tcctcaagat caaccaggct tctagtgagg ctcagctaat 1140
   tgacctcaag aaagcaatca tccgcagtgc aatt                             1174
<210> 34
   <211> 2244
   <212> DNA
   <213> Cryptovirus
<220>
   <223> cDNA in mRNA sense
<400> 34
   tgactgacct ctaatcgaga ttacaccaac tcaaactata ggtgggtagt accccagtga 60
   ttaatcttgc aagtactgat cgttggctac aacacaccaa tattatccag attagagagc 120
   ttaattaact ctgtattaat aataacacta ctattccaat aactggaatt accagcttga 180
   tttatctcca aaatgattca aagaaaacag atcatattaa gactacccta agcacgaacc 240
   catatcgtcc ttcaaatcat gagcactata attcaatctc tggtggtctc ctgtctattg 300
   gcaggagcag gcagccttga tccagcagcc ctcatgcaaa tcggtgtcat tccaacaaat 360
   gtccggcaac ttatgtatta tactgaggcc tcatcagcat tcattgttgt gaagttaatg 420
   cctacaattg actcgccgat tagtggatgt aatataacat caatttcaag ctataatgca 480
   acagtgacaa aactcctaca gccgatcggt gagaatttgg agacgattag gaaccagttg 540
   attccaactc ggaggagacg ccggtttgca ggggtggtga ttggattagc tgcattagga 600
   gtagctactg ccgcacaagt cactgccgca gtagcactag ttaaggcaaa tgaaaatact 660
   gcggctatac tcaatctcaa aaatgcaatc caaaaaacaa atgcagcagt tgcagatgtg 720
   gtccaggcca cacaatcact aggaacggca gttcaagcag ttcaagatca cataaacagt 780
   gtgataagtc cagcaattac agcagccaat tgtaaggccc aagatgctat cattggctca 840
   atcctcaatc tctatttgac cgagttgaca actatcttcc acaatcaaat tacaaaccct 900
   gcattgagtc ctattacaat tcaagcttta aggatcctac tggggagtac cttgccgact 960
   gtggtcgaaa aatctttcaa tacccagata agtgcagctg agcttctctc atcagggttg 1020
   ttgacaggcc agattgtggg attagatttg acctatatgc agatggtcat aaaaattgag 1080
   ctgccaactt taactgtaca acctgcaacc cagatcatag atctggccac catttctgca 1140
   ttcattaaca atcaagaagt catggcccaa ttaccaacac gtgttattgt gactggcagc 1200
   ttgatccaag cctatcccgc atcgcaatgc actattacac ccaacactgt gtactgtagg 1260
   tataatgatg cccaagtact ctcagatgat acgatggctt gcctccaagg taacttgaca 1320
   agatgcacct tctctccagt ggttgggagc tttctcactc gattcgtgct gttcgatgga 1380
   atagtttatg caaattgcag gtcgatgctg tgcaagtgca tgcaacctgc tgccgtgatc 1440
   ctacagccga gttcatcccc tgtaactgtc attgacatgc acaaatgtgt gagtctgcag 1500
   cttgacgatc tcagattcac catcactcaa ttggccaatg taacctacaa tagcaccatc 1560
   aagcttgaaa catcccagat cttgcctatt gatccgttgg atatatccca gaatttagct 1620
   gcggtgaata agagtctaag tgatgcacta caacacttag cacaaagtga cacatacctt 1680
   tctgcaatca catcagctac gactacaagt gtattatcca taatagcaat ctgtcttgga 1740
   tcgttaggtt taatattaat aatcttgctc agtgtagttg tgtggaagtt attgaccatt 1800
   gtcgctgcta atcgaaatag aatggagaat tttgtttatc ataattcagc attccaccac 1860
   ccacgatctg atctcagtga gaaaaatcaa cctgcaactc ttggaacaag ataagacagt 1920
   catccattag taatctcaaa gaaaaaaacg ataggaccga aactagtatt gaaagaactg 1980
   tctcggtcaa tttaggtaat cgagctgata ccgtctcgga aagctcaaat catgctgcct 2040
   gatccggaag atccggaaag caaaaaagct acaaggagaa caggaaacct aattatctgc 2100
   ttcctattca tcttctttct gtttgtaacc ctcattgttc caactctaag acacttgcta 2160
   tcttaacacc tgccataggc tatccactgc atcatctctt ctgccatact tcctactcac 2220
   atcatatcta ttttaaagaa aaaa                                        2244
<210> 35
   <211> 18
   <212 DNA
   <213> Cryptovirus
<220>
   <223> cDNA in mRNA sense
<400> 35
   aattcagatc ccaatcct 18
<210> 36
   <211> 18
   <212> DNA
   <213> Cryptovirus
<220>
   <223> cDNA in mRNA sense
<400> 36
   ctaaattgat atacctaa 18
<210> 37
   <211> 18
   <212> DNA
   <213> Cryptovirus
<220>
   <223> cDNA in mRNA sense
<400> 37
   taatcgagat tacaccaa 18
<210> 38
   <211> 18
   <212> DNA
   <213> Cryptovirus
<220>
   <223> cDNA in mRNA sense
<400> 38
   aactcaaact ataggtgg 18
<210> 39
   <211> 18
   <212> DNA
   <213> Cryptovirus
<220>
   <223> cDNA in mRNA sense
<400> 39
   atcgagatta caccaact 18
<210> 40
   <211> 18
   <212> DNA
   <213> Cryptovirus
<220>
   <223> cDNA in mRNA sense
<400> 40
   actcaaacta taggtggg 18
<210> 41
   <211> 18
   <212> DNA
   <213> Cryptovirus
<220>
   <223> cDNA in mRNA sense
<400> 41
   gtccttcaaa tcatgagc 18
<210> 42
   <211> 18
   <212> DNA
   <213> Cryptovirus
<220>
   <223> cDNA in mRNA sense
<400> 42
   agcactataa ttcaatct 18
<210> 43
   <211> 18
   <212> DNA
   <213> Cryptovirus
<220>
   <223> cDNA in mRNA sense
<400> 43
   tcatccatta gtaatctc 18
<210> 44
   <211> 18
   <212> DNA
   <213> Cryptovirus
<220>
   <223> cDNA in mRNA sense
<400> 44
   ttcagcattc caccaccc 18
<210> 45
   <211> 18
   <212> DNA
   <213> Cryptovirus
<220>
   <223> cDNA in mRNA sense
<400> 45
   catccattag taatctca 18
<210> 46
   <211> 18
   <212> DNA
   <213> Cryptovirus
<220>
   <223> cDNA in mRNA sense
<400> 46
   tggcggcgtg attaaaaa 18
<210> 47
   <211> 18
   <212> DNA
   <213> Cryptovirus
<220>
   <223> cDNA in mRNA sense
<400> 47
   aatacgagtt tatggaat 18
<210> 48
   <211> 258
   <212> DNA
   <213> Cryptovirus
<220>
   <223> cDNA in mRNA sense
<400> 48
   ttacaccaac tcaaactata ggtgggtagt accccagtga ttaatcttgc aagtactgat 60
   cgttggctac aacacaccaa tattatccag attagagagc ttaattaact ctgtattaat 120
   aataacacta ctattccaat aactggaatt accagcttga tttatctcca aaatgattca 180
   aagaaaacag atcatattaa gactacccta agcacgaacc catatcgtcc ttcaaatcat 240
   gagcactata attcaatc 258
<210> 49
   <211> 1656
   <212> DNA
   <213> Cryptovirus
<220>
   <223> cDNA in mRNA sense
<400> 49
   atgagcacta taattcaatc tctggtggtc tcctgtctat tggcaggagc aggcagcctt 60
   gatccagcag ccctcatgca aatcggtgtc attccaacaa atgtccggca acttatgtat 120
   tatactgagg cctcatcagc attcattgtt gtgaagttaa tgcctacaat tgactcgccg 180
   attagtggat gtaatataac atcaatttca agctataatg caacagtgac aaaactccta 240
   cagccgatcg gtgagaattt ggagacgatt aggaaccagt tgattccaac tcggaggaga 300
   cgccggtttg caggggtggt gattggatta gctgcattag gagtagctac tgccgcacaa 360
   gtcactgccg cagtagcact agttaaggca aatgaaaata ctgcggctat actcaatctc 420
   aaaaatgcaa tccaaaaaac aaatgcagca gttgcagatg tggtccaggc cacacaatca 480
   ctaggaacgg cagttcaagc agttcaagat cacataaaca gtgtgataag tccagcaatt 540
   acagcagcca attgtaaggc ccaagatgct atcattggct caatcctcaa tctctatttg 600
   accgagttga caactatctt ccacaatcaa attacaaacc ctgcattgag tcctattaca 660
   attcaagctt taaggatcct actggggagt accttgccga ctgtggtcga aaaatctttc 720
   aatacccaga taagtgcagc tgagcttctc tcatcagggt tgttgacagg ccagattgtg 780
   ggattagatt tgacctatat gcagatggtc ataaaaattg agctgccaac tttaactgta 840
   caacctgcaa cccagatcat agatctggcc accatttctg cattcattaa caatcaagaa 900
   gtcatggccc aattaccaac acgtgttatt gtgactggca gcttgatcca agcctatccc 960
   gcatcgcaat gcactattac acccaacact gtgtactgta ggtataatga tgcccaagta 1020
   ctctcagatg atacgatggc ttgcctccaa ggtaacttga caagatgcac cttctctcca 1080
   gtggttggga gctttctcac tcgattcgtg ctgttcgatg gaatagttta tgcaaattgc 1140
   aggtcgatgc tgtgcaagtg catgcaacct gctgccgtga tcctacagcc gagttcatcc 1200
   cctgtaactg tcattgacat gcacaaatgt gtgagtctgc agcttgacga tctcagattc 1260
   accatcactc aattggccaa tgtaacctac aatagcacca tcaagcttga aacatcccag 1320
   atcttgccta ttgatccgtt ggatatatcc cagaatttag ctgcggtgaa taagagtcta 1380
   agtgatgcac tacaacactt agcacaaagt gacacatacc tttctgcaat cacatcagct 1440
   acgactacaa gtgtattatc cataatagca atctgtcttg gatcgttagg tttaatatta 1500
   ataatcttgc tcagtgtagt tgtgtggaag ttattgacca ttgtcgctgc taatcgaaat 1560
   agaatggaga attttgttta tcataattca gcattccacc acccacgatc tgatctcagt 1620
   gagaaaaatc aacctgcaac tcttggaaca agataa                          1656